# EUROPEAN PATENT APPLICATION

(11) **EP 4 831 187 A1**
(43) Date of publication of application: **28.10.2026**
(21) Application number: 25305596.6
(22) Date of filing: 25.04.2025
(51) Int. Cl.: C07F 9/6584

(54) **COMPOUNDS, PHARMACEUTICAL COMPOSITION AND THEIR USE IN TREATING NEURODEGENERATIVE AND OTHER DISEASES**

(71) Applicant: UNIVERSITE DE MONTPELLIER, 34090 Montpellier (FR); Institut National de la Santé et de la Recherche Médicale, 75013 Paris (FR); Centre National de la Recherche Scientifique, 75016 Paris (FR); Ecole Nationale Supérieure de Chimie de Montpellier, 34090 Montpellier (FR); Ecole Pratique des Hautes Etudes, 75014 Paris (FR)
(72) Inventor: VIRIEUX, David, 34000 MONTPELLIER (FR); DE ZORDO, Arnaud, 91940 LES ULIS (FR); MELE, Lucas, 73100 AIX-LES-BAINS (FR); PIRAT, Jean-Luc, 34410 SAINT-AUNÈS (FR); AYAD, Tahar, 34380 MAS DE LONDRES (FR); DELPRAT, Benjamin, 34830 JACOU (FR); MAURICE, Tangui, 34980 SAINT GÉLY DU FESC (FR); MEUNIER, Johann, 34130 MAUGUIO (FR); CROUZIER, Lucie, 34000 MONTPELLIER (FR)
(74) Representative: Plasseraud IP

(57) **Abstract**

The present invention relates to compounds of Formula I 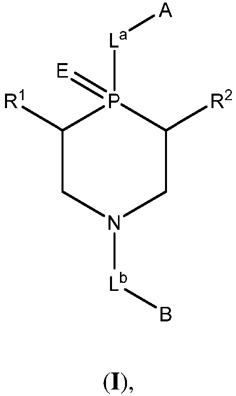 or pharmaceutically acceptable salts thereof, and their use.

## Description

The present invention relates to compounds including their pharmaceutically acceptable salts, which are modulators of functioning of the contact points between the endoplasmic reticulum (ER) and the mitochondria, known as mitochondria-associated ER membrane (MAMs), in particular those which are targeting the sigma-1 receptor (S1R) and are useful as therapeutic compounds, particularly in the treatment or prevention of a disease, disorder or condition that is associated with, caused by or a symptom of a mutation in WFS1 or WFS2 (CISD2) gene (eg, Wolfram syndrome, Wolfram-like syndrome, or disease, disorder or condition associated therewith, caused thereby or a symptom thereof), associated with mitochondrial dysfunction, and/or is treatable, ameliorable or preventable by activation or inhibition of S1R, in particular neurodegenerative diseases.

### BACKGROUND OF THE INVENTION

The sigma-1 receptor (S1R) is a chaperone protein of the endoplasmic reticulum that is attracting a great deal of attention from pharmacologists because its interest as a therapeutic target for small molecules has been demonstrated in a large number of pathologies (T. Maurice, *Expert Opin. Drug Discov.* **2021,** *16*, 373-389). S1R agonists, i.e. molecules capable of activating the chaperone function of the protein, are neuroprotective, anti-amnesic, antidepressant and anticonvulsive agents. S1R is expressed in a large number of cell types and plays a major role in intracellular physiology by chaperoning various client proteins. In particular, it stabilises and facilitates the opening of the IP3 receptor-activated calcium channel present on the endoplasmic reticulum (ER) membrane. It therefore facilitates the entry of calcium into the mitochondria and therefore plays a major role in stabilising communication between these two organelles. This communication is essential to cell physiology and is particularly important within the microdomains that make up the contact points between the ER and the mitochondria, known as mitochondria-associated ER membranes (MAMs). MAM defects are observed in all neurodegenerative diseases, such as Alzheimer's, Parkinson's and Huntington's diseases, amyotrophic lateral sclerosis and Charcot-Marie-Tooth Disease 2A (Rodriguez-Arribas et al., Mol. Neurobiol. 2017, 54, 6287-6303; Cagalinec et al., Biochim. Biophys. Acta Mol. Cell Res. 2025, 1872, 119954) and in many genetic diseases, such as the Wolfram syndrome.

The Wolfram syndrome is a rare autosomal recessive disease affecting many organs with life-threatening consequences. Currently, no treatment is available. The disease is caused by mutations in the WSF1 gene, coding for the protein wolframin, an ER transmembrane protein involved in MAMs. It was demonstrated that WFS1 deficiency leads to an alteration of MAMs, triggering mitochondria dysfunction.

The efficacy of ligands that activate the S1R chaperone protein has been demonstrated in Alzheimer's disease (Maurice et al., Neuroscience 1998, 83, 413-428; Meunier et al., Br. J. Pharmacol. 2006, 149, 998-1012; Villard et al., Neuropsychopharmacology 2009, 34, 1552-1566; Villard et al., J. Psychopharmacol. 2011, 25, 1101-1117) and in Wolfram syndrome (Crouzier et al., Sci. Transl. Med. 2022, 14, eabh3763). Crouzier *et al.* describes a phenotypic alteration in a zebrafish model of Wolfram syndrome, in which the hyperlocomotor response is observed in wfs1ab^{KO} as compared to wfs1^{WT} larvae. This pathological phenotype relates to the alteration of ER-mitochondria communication in MAMs and overexpression or activation of the S1R by a small molecule acting as an agonist allowed a blockade of the hyperlocomotor response in wfs1ab^{KO} zebrafish. These data established the Wolfram syndrome specifically as a MAMopathy and showed that a functional restoration of MAM functioning has therapeutic consequences at the behavioral level.

There is thus a need for new compounds of therapeutic value for treating and/or preventing a disease, disorder or condition that is associated with, caused by or a symptom of a mutation in WFS1 or WFS2 (CISD2) gene (especially Wolfram syndrome, Wolfram-like syndrome, or disease, disorder or condition associated therewith, caused thereby or a symptom thereof), associated with mitochondrial dysfunction, and/or is treatable, ameliorable or preventable by activation or inhibition of S1R, in particular one or more neurodegenerative diseases, disorders or conditions.

### SUMMARY OF THE INVENTION

The inventors have now succeeded in developing compounds having the advantage of modulating, in particular increasing, MAM functioning, as shown by differently affecting a biological response (in a zebrafish model of Wolfram syndrome) between wfs1ab^{KO} larvae and wfs1^{WT} larvae. Compounds of the invention are surprisingly shown to have: (i) direct S1R activity, for example by having the ability to bind to S1R, in particular an activity as direct agonists of S1R, or (ii) to have a different mechanism of modulating, in particular increasing, MAM functioning because they can modulate, in particular activate, another MAM chaperone or another protein acting as an upstream regulator of S1R or another MAM chaperone.

The invention therefore relates to compounds of general Formula I, or their pharmaceutically acceptable salts thereof, and pharmaceutical compositions comprising such compounds, as well as methods of use of such compounds or compositions comprising such compounds for treating and/or preventing a disease, disorder or condition that is associated with, caused by or a symptom of a mutation in WFS1 or WFS2 (CISD2) gene, in particular Wolfram syndrome, Wolfram-like syndrome, or disease, disorder or condition associated therewith, caused thereby or a symptom thereof, associated with mitochondrial dysfunction, and/or is treatable, ameliorable or preventable by activation or inhibition of S1R, in particular neurodegenerative diseases.

In a general aspect, the invention provides compounds of general Formula I: 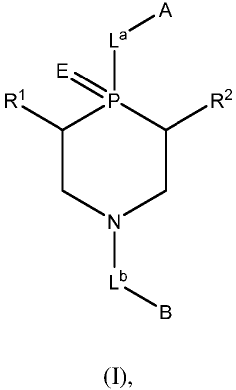 or pharmaceutically acceptable salts thereof,
wherein
A is selected from the group consisting of phenyl, naphthyl, 5 to 10-membered mono-cyclic or bi-cyclic heteroaryl, 3 to 6-membered cycloalkyl and 3 to 6-membered cycloalkenyl, said phenyl, naphthyl, 5 to 10-membered mono-cyclic or bi-cyclic heteroaryl, 3 to 5-membered cycloalkyl and 3 to 6-membered cycloalkenyl being optionally substituted with 1 to 5 substituents **R^{a}**;
   wherein each **R^{a}** is independently selected from the group consisting of -H, -F, -Cl, - Br, -CN, -NH₂, -NO₂, -CH₂F, -CHF₂, -CF₃, -OCH₃, -(CO)OH, -(CO)OCH₃, -SCH₃, -OCH₂F, -OCHF₂, -OCF₃, -O(CH₂)Ph, and C₁₋₄ alkyl;
**L^{a}** is selected from the group consisting of a bond, C₁₋₅ alkanediyl, C₂₋₅ alkenediyl and C₂₋₅ alkynediyl, each optionally substituted with one, two or three substituents independently selected from the group consisting of -OH and =O;
**R¹** and **R²** are independently selected from -H and -C(O)O-C₁₋₄ alkyl;
B is selected from the group consisting of phenyl, naphthyl, 5 to 10-membered mono-cyclic or bi-cyclic heteroaryl, 3 to 6-membered cycloalkyl and 3 to 6-membered cycloalkenyl, said phenyl, naphthyl, 5 to 10-membered mono-cyclic or bi-cyclic heteroaryl, 3 to 6-membered cycloalkyl and 3 to 6-membered cycloalkenyl being optionally substituted with 1 to 5 substituents **R^{b}**;
   wherein each **R^{b}** is independently selected from the group consisting of -H, -F, -Cl, -Br, -CN, -NH₂, -NO₂, -CH₂F, -CHF₂, -CF₃, -OCH₃, -(CO)OH, -(CO)OCH₃, -SCH₃, -OCH₂F, -OCHF₂, -OCF₃, -O(CH₂)Ph, and C₁₋₄ alkyl;
**L^{b}** is a linker selected from the group consisting of a bond, C₁₋₅ alkanediyl, C₂₋₅ alkenediyl and C₂₋₅ alkynediyl, each optionally substituted with one, two or three substituents independently selected from the group consisting of -OH and =O;
**E** is selected from the group consisting of O, S and Se;
   *with the proviso* that when **E** is O and:
   (a) when both of **L^{a}** and **L^{b}** is a bond and **A** is either unsubstituted phenyl or 4-fluorophenyl, then **B** is not 2,6-difluoro-4-nitrophenyl or 2,6-difluoro-4-aminophenyl;
   (b) when **L^{a}** is a bond, **L^{b}** is -(CH₂)- and **B** is unsubstituted phenyl, then **A** is not unsubstituted phenyl, 4-bromophenyl, 4-fluorophenyl, 4-(trifluoromethyl)phenyl, 4-nitrophenyl or unsubstituted cyclopropanyl;
   (c) when **L^{a}** is a bond, **L^{b}** is -(CH₂)- and **A** is 4-fluorophenyl, then **B** is not 4-methoxyphenyl;
   (d) when **L^{a}** is -CH₂-, **L^{b}** is -(CH₂)- and **B** is unsubstituted phenyl, then A is not 4-fluorophenyl or unsubstituted cyclopropanyl;
   (e) when **L^{a}** is a bond, **L^{b}** is -(CH₂)- and **B** is 2,4-di-methoxyphenyl, then A is not 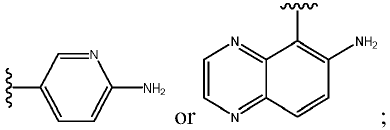
   (f) when **L^{a}** is a bond and **A** is unsubstituted phenyl, then **L^{b}-B** is not -(CH₂)₃-(1-isoquinoline), -(CH₂)₂-(4-carboxyphenyl), -(CH₂)-(4-methylbenzoate) or 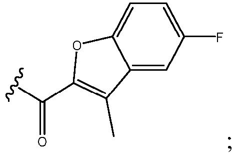 and/or (g) when **L^{a}** is a bond and **A** is 3-methoxy-4-aminophenyl or 3-methoxy-4-nitrophenyl, then **L^{b}-B** is not unsubstituted phenyl, unsubstituted cyclopropanyl or -(CO)-cyclopropanyl.

In another aspect, the present invention provides a pharmaceutical composition comprising at least one compound of Formula I or a pharmaceutically acceptable salt thereof and at least one pharmaceutically acceptable carrier, diluent, excipient and/or adjuvant.

The invention also relates to a compound of Formula I: 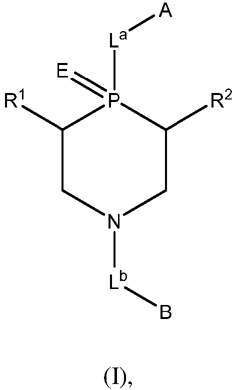 or a pharmaceutically acceptable salt thereof,
wherein
**A** is selected from the group consisting of phenyl, naphthyl, 5 to 10-membered mono-cyclic or bi-cyclic heteroaryl, 3 to 6-membered cycloalkyl and 3 to 6-membered cycloalkenyl, said phenyl, naphthyl, 5 to 10-membered mono-cyclic or bi-cyclic heteroaryl, 3 to 5-membered cycloalkyl and 3 to 6-membered cycloalkenyl being optionally substituted with 1 to 5 substituents **R^{a}**; wherein each **R^{a}** is independently selected from the group consisting of -H, -F, -Cl, - Br, -CN, -NH₂, -NO₂, -CH₂F, -CHF₂, -CF₃, -OCH₃, -(CO)OH, -(CO)OCH₃, -SCH₃, -OCH₂F, -OCHF₂, -OCF₃, -O(CH₂)Ph, and C₁₋₄ alkyl;
**L^{a}** is selected from the group consisting of a bond, C₁₋₅ alkanediyl, C₂₋₅ alkenediyl and C₂₋₅ alkynediyl, each optionally substituted with one, two or three substituents independently selected from the group consisting of -OH and =O;
**R¹** and **R²** are independently selected from -H and -C(O)O-C₁₋₄ alkyl;
**B** is selected from the group consisting of phenyl, naphthyl, 5 to 10-membered mono-cyclic or bi-cyclic heteroaryl, 3 to 6-membered cycloalkyl and 3 to 6-membered cycloalkenyl, said phenyl, naphthyl, 5 to 10-membered mono-cyclic or bi-cyclic heteroaryl, 3 to 6-membered cycloalkyl and 3 to 6-membered cycloalkenyl being optionally substituted with 1 to 5 substituents **R^{b}**;
   wherein each **R^{b}** is independently selected from the group consisting of -H, -F, -Cl, -Br, -CN, -NH₂, -NO₂, -CH₂F, -CHF₂, -CF₃, -OCH₃, -(CO)OH, -(CO)OCH₃, -SCH₃, -OCH₂F, -OCHF₂, -OCF₃, -O(CH₂)Ph, and C₁₋₄ alkyl;
**L^{b}** is a linker selected from the group consisting of a bond, C₁₋₅ alkanediyl, C₂₋₅ alkenediyl and C₂₋₅ alkynediyl, each optionally substituted with one, two or three substituents independently selected from the group consisting of -OH and =O;
**E** is selected from the group consisting of O, S and Se; for use in treating and/or preventing a disease, disorder or condition that is:
   (a) associated with, caused by or a symptom of a mutation in WFS1 or WFS2 (CISD2) gene;
   (b) a neurodegenerative disease, disorder or condition;
   (c) selected from the group consisting of stroke, stroke-induced neuronal damage, depression, anxiety, addiction, epilepsy, multiple sclerosis, pain, neuropathic pain and cancer;
   (d) associated with mitochondrial dysfunction, endoplasmic reticulum (ER) stress, or disfunction of nucleocytoplasmic transport; and/or
   (e) treatable, ameliorable or preventable by activation or inhibition of sigma-1 receptor.

### DETAILED DESCRIPTION OF THE INVENTION

As detailed above, the invention relates to compounds of Formula I, as well as their pharmaceutically acceptable salts.

Particular compounds of Formula I and pharmaceutically acceptable salts thereof are those wherein one or more of A, **L^{a}**, **R¹**, **R²**, **B, L^{b}**, and **E** are defined as follows:
**A** is selected from the group consisting of phenyl, naphthyl, 5 to 10-membered mono-cyclic or bi-cyclic heteroaryl, 3 to 6-membered cycloalkyl and 3 to 6-membered cycloalkenyl, said phenyl, naphthyl, 5 to 10-membered mono-cyclic or bi-cyclic heteroaryl, 3 to 5-membered cycloalkyl and 3 to 6-membered cycloalkenyl being optionally substituted with 1 to 5 substituents **R^{a}**; in particular **A** is selected from the group consisting of phenyl, naphthyl, and 5 to 10-membered mono-cyclic or bi-cyclic heteroaryl, said phenyl, naphthyl, and 5 to 10-membered mono-cyclic or bi-cyclic heteroaryl being optionally substituted with 1 to 5 substituents **R^{a}**;
   wherein each **R^{a}** is independently selected from the group consisting of -H, -F, -Cl, -Br, -CN, -NH₂, -NO₂, -CH₃, -CH₂F, -CHF₂, -CF₃, -OCH₃, -(CO)OH, -(CO)OCH₃, -SCH₃, - OCH₂F, -OCHF₂, -OCF₃, -O(CH₂)Ph, and C₁₋₄ alkyl; in particular each **R^{a}** is independently selected from the group consisting of -H, -F, -Cl, -Br, -CN, -NO₂, -CH₃, - CF₃, -OCH₃, -(CO)OCH₃, -SCH₃, -OCF₃, and C₁₋₄ alkyl; more particularly each **R^{a}** is independently selected from the group consisting of -H, -F, -Cl, -Br, -CN, -NO₂, -CH₃, - CF₃, -OCH₃, -(CO)OCH₃, -SCH₃, -OCF₃, and t-Bu;
**L^{a}** is a linker selected from the group consisting of a bond, C₁₋₅ alkanediyl, C₂₋₅ alkenediyl and C₂₋₅ alkynediyl, each optionally substituted with one, two or three substituents independently selected from the group consisting of -OH and =O; in particular **L^{a}** is a linker selected from the group consisting of a bond, C₁₋₅ alkanediyl, C₂₋₅ alkenediyl, C₂₋₅ alkynediyl, hydroxy-C₁₋₅ alkanediyl and C₁₋₄ alkanediyl-(CO)-; more particularly **L^{a}** is a linker selected from the group consisting of a bond, C₁₋₄ alkanediyl, C₂₋₄ alkenediyl, C₂₋₄ alkynediyl, hydroxy-C₁₋₄ alkanediyl and C₁₋₃ alkanediyl-(CO)-; still more particularly **L^{a}** is a linker selected from the group consisting of a bond, C₁₋₂ alkanediyl, C₂ alkenediyl, C₂ alkynediyl, hydroxy-C₁₋₂ alkanediyl and C₁ alkanediyl-(CO)-; even more particularly **L^{a}** is a linker selected from the group consisting of a bond, -CH₂-, -(CH₂)₂-, -CH=CH-, -C≡C-, -CH(OH)- and -CH₂-C(O)-;
**R¹** and **R²** are independently selected from -H and -C(O)O-C₁₋₄ alkyl; in particular **R¹** and **R²** are independently selected from -H and -C(O)OtBu; more particulary **R¹** and **R²** are H;
**B** is selected from the group consisting of phenyl, naphthyl, 5 to 10-membered mono-cyclic or bi-cyclic heteroaryl, 3 to 6-membered cycloalkyl and 3 to 6-membered cycloalkenyl, said phenyl, naphthyl, 5 to 10-membered mono-cyclic or bi-cyclic heteroaryl, 3 to 6-membered cycloalkyl and 3 to 6-membered cycloalkenyl being optionally substituted with 1 to 5 substituents **R^{b}**;
   wherein each **R^{b}** is independently selected from the group consisting of -H, -F, -Cl, -Br, -CN, -NH₂, -NO₂, -CH₂F, -CHF₂, -CF₃, -OCH₃, -(CO)OH, -(CO)OCH₃, -SCH₃, -OCH₂F, -OCHF₂, -OCF₃, -O(CH₂)Ph, and C₁₋₄ alkyl; in particular each **R^{b}** is independently selected from the group consisting of -H, -F, -Cl, -CH₃, -CF₃, -OCH₃, and -OCF₃;
**L^{b}** is a linker selected from the group consisting of a bond, C₁₋₅ alkanediyl, C₂₋₅ alkenediyl and C₂₋₅ alkynediyl, each optionally substituted with one, two or three substituents independently selected from the group consisting of -OH and =O; in particular **L^{b}** is a linker selected from the group consisting of a bond, C₁₋₅ alkanediyl, C₂₋₅ alkenediyl and C₂₋₅ alkynediyl, hydroxy-C₁₋₅ alkanediyl, C₁₋₄ alkanediyl-(CO)- and -(CO)-; more particularly **L^{b}** is a linker selected from the group consisting of a bond, C₁₋₄ alkanediyl, C₁₋₄ alkanediyl-(CO)- and -(CO)-; still more particularly **L^{b}** is a linker selected from the group consisting of a bond, C₁₋₃ alkanediyl, C₁₋₃ alkanediyl-(CO)- and -(CO)-; even more particularly **L^{b}** is a linker selected from the group consisting of a bond, C₁₋₃ alkanediyl, C₁₋₃ alkanediyl-(CO)- and -(CO)-; even more particularly **L^{b}** is a linker selected from the group consisting of a bond, -CH₂-, -(CH₂)₂-, -CH(CH₃)-, -CH₂-C(O)-, -(CH₂)₂-C(O)-, -(CH₂)₃-C(O)- and - (CO)-; and
**E** is selected from the group consisting of O, S and Se; in particular **E** is O;
   *with the proviso* that when **E** is O and:
   (a) when both of **L^{a}** and **L^{b}** is a bond and A is either unsubstituted phenyl or 4-fluorophenyl, then **B** is not 2,6-difluoro-4-nitrophenyl or 2,6-difluoro-4-aminophenyl;
   (b) when **L^{a}** is a bond, **L^{b}** is -(CH₂)- and **B** is unsubstituted phenyl, then **A** is not unsubstituted phenyl, 4-bromophenyl, 4-fluorophenyl, 4-(trifluoromethyl)phenyl, 4-nitrophenyl or unsubstituted cyclopropanyl;
   (c) when **L^{a}** is a bond, **L^{b}** is -(CH₂)- and **A** is 4-fluorophenyl, then **B** is not 4-methoxyphenyl;
   (d) when **L^{a}** is -CH₂-, **L^{b}** is -(CH₂)- and **B** is unsubstituted phenyl, then **A** is not 4-fluorophenyl or unsubstituted cyclopropanyl;
   (e) when **L^{a}** is a bond, **L^{b}** is -(CH₂)- and **B** is 2,4-di-methoxyphenyl, then **A** is not 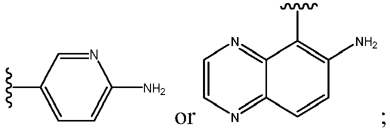
   (f) when **L^{a}** is a bond and A is unsubstituted phenyl, then **L^{b}-B** is not -(CH₂)₃-(1-isoquinoline), -(CH₂)₂-(4-carboxyphenyl), -(CH₂)-(4-methylbenzoate) or 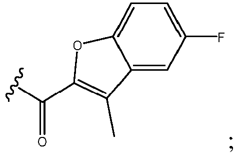 and/or (g) when **L^{a}** is a bond and **A** is 3-methoxy-4-aminophenyl or 3-methoxy-4-nitrophenyl, then **L^{b}-B** is not unsubstituted phenyl, unsubstituted cyclopropanyl or -(CO)-cyclopropanyl.

The sign " 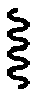 " as used herein indicates the position of a substituent by which said subsituent is linked to the remainder of the compound.

In one embodiment, the compounds of Formula I are those wherein **E** is O.

In one embodiment, the compounds of Formula I are those wherein **E** is S.

In one embodiment, the compounds of Formula I are those wherein **E** is Se.

In one embodiment, the compounds of Formula I are those wherein **R¹** and **R²** are H.

In one embodiment, the compounds of Formula I are those wherein **L^{a}** is a linker selected from the group consisting of a bond, -CH₂-, -(CH₂)₂-, -CH=CH-, -C≡C-, -CH(OH)-, - CH₂-C(O)-, -(CH₂)₂-C(O)- and -(CH₂)₃-C(O)-; in particular **L^{a}** is a linker selected from the group consisting of a bond, -CH₂-, -(CH₂)₂-, -CH=CH-, -C≡C-, -CH(OH)- and - CH₂-C(O)-; in particular, **L^{a}** is -CH₂-.

In one embodiment, the compounds of Formula I are those wherein **L^{a}** is a bond.

In one embodiment, the compounds of Formula I are those wherein **L^{a}** is a linker selected from the group consisting of -CH₂-, -(CH₂)₂-, -CH=CH-, -C≡C-, -CH(OH)- and -CH₂-C(O)-.

In one embodiment, the compounds of Formula I are those wherein **A** is phenyl or naphthyl, each optionally substituted with 1 to 5 substituents **R^{a}**, wherein **R^{a}** is as defined as above.

In one embodiment, the compounds of Formula I are those wherein **A** is a 5 to 10-membered mono-cyclic or bi-cyclic heteroaryl, each optionally substituted with 1 to 5 substituents **R^{a}**, wherein **R^{a}** is as defined as above.

In one embodiment, the compounds of Formula I are those wherein A is a 3 to 5-membered cycloalkyl, each optionally substituted with 1 to 5 substituents **R^{a}**, wherein **R^{a}** is as defined as above.

In one embodiment, the compounds of Formula I are those wherein each **R^{a}** is independently selected from the list consisting of -H, -F, -Cl, -Br, -CN, -NO₂, -CH₃, -CF₃, -OCH₃, - (CO)OCH₃, -SCH₃, -OCF₃, and t-Bu; in particular each **R^{a}** is independently selected from the group consisting of H, -F, -Cl, -CH₃, -CF₃, -OCH₃.

In one embodiment, the compounds of Formula I are those wherein **A** is phenyl, optionally substituted with 1, 2 or 3 substituents **R^{a}**, wherein **R^{a}** is as defined as above.

In one embodiment, the compounds of Formula I are those wherein **A** is phenyl substituted by one **R^{a}** at position 4 or position 3 of the phenyl **A.**

In one embodiment, the compounds of Formula I are those wherein **A** is a 5, 6 or 7 membered mono-cyclic heteroaryl consisting or 1,2 or 3 heteroatoms selected from the group consisting or N, S and O, said 5, 6 or 7 membered mono-cyclic heteroaryl optionally substituted with 1, 2 or 3 substituents **R^{a}**, wherein **R^{a}** is as defined as above.

In one embodiment, the compounds of Formula I are those wherein **A** and **R^{a}**, together are selected from the group consisting of phenyl, 4-(trifluoromethyl)phenyl, 4-fluorophenyl, 3-(trifluoromethyl)phenyl, 2-(trifluoromethyl)phenyl, 4-(trifluoromethoxy)phenyl, 3-fluorophenyl, 3,5-difluorophenyl, 4-chlorophenyl, 3-chlorophenyl, 2-chlorophenyl, 3,4-dichlorophenyl, 2,3-dichlorophenyl, 4-methylphenyl, 3-methylphenyl, 2-methylphenyl, 4-t-butylphenyl, naphthalen-1-yl, naphthalen-2-yl, 4-methoxyphenyl, 3-methoxyphenyl, 2-methoxyphenyl, 4-benzyloxyphenyl, 2-(methylthio)phenyl, 4-methyloxycarbonylphenyl, 3-methyloxycarbonylphenyl, 4-cyanophenyl, 3-cyanophenyl, 2-cyanophenyl, pyridin-2-yl, 3-chloro-5-(trifluoromethyl)pyridin-2-yl, pyrimidin-5-yl, isoquinolin-5-yl, benzo[d]thiazol-2-yl, thiophen-3-yl, 4-nitrophenyl, 4-bromophenyl, 2-bromophenyl, 2-(trifluoromethoxy)phenyl, 3,5-dimethylphenyl, 6-bromopyridin-2-yl, perfluorophenyl, 4-(difluoromethyl)phenyl and 2-fluorophenyl.

In one embodiment, the compounds of Formula I are those wherein **A** and **R^{a}**, together are selected from the group consisting of 4-(trifluoromethyl)phenyl, 3-(trifluoromethyl)phenyl, 4-fluorophenyl, phenyl, 4-methylphenyl, 3-methylphenyl, and naphthalen-1-yl.

In one embodiment, the compounds of Formula I are those wherein **L^{b}** is a linker selected from the group consisting of a bond, -CH₂-, -(CH₂)₂-, -CH=CH-, -C≡C-, -CH(OH)-, - CH(CH₃)-, -CH₂-C(O)-, -(CH₂)₂-C(O)-, -(CH₂)₃-C(O)- and -C(O)-; in particular **L^{b}** is a linker selected from the group consisting of a bond, -CH₂-, -(CH₂)₂-, -CH(CH₃)-, -CH₂-C(O)-, -(CH₂)₂-C(O)-, -(CH₂)₃-C(O)- and -C(O)-; in particular **L^{b}** is a linker selected from the group consisting of a -CH₂- and -CH(CH₃)-; more particularly **L^{b}** is -CH₂-.

In one embodiment, the compounds of Formula I are those wherein **L^{b}** is a bond.

In one embodiment, the compounds of Formula I are those wherein **L^{b}** is a linker selected from the group consisting of a bond, -CH₂-, -(CH₂)₂-, -CH(CH₃)-, -CH₂-C(O)-, -(CH₂)₂-C(O)-, -(CH₂)₃-C(O)- and -C(O)-.

In one embodiment, the compounds of Formula I are those wherein **B** is selected from the group consisting of phenyl, naphthalenyl, 5 to 10-membered mono-cyclic or bi-cyclic heteroaryl and 3 to 6-membered cycloalkyl, said phenyl, naphthalenyl, 5 to 10-membered mono-cyclic or bi-cyclic heteroaryl and 3 to 6-membered cycloalkyl being optionally substituted with 1 to 5 substituents **R^{b}**, wherein **R^{b}** is as defined above; in particular the compounds of Formula I are those wherein **B** is selected from the group consisting of phenyl, naphthalenyl, pyridinyl, thiophenyl and cyclopropyl, said phenyl, naphthalenyl, pyridinyl, thiophenyl and cyclopropyl being optionally substituted with 1 or 2 substituents **R^{b}**, wherein **R^{b}** is as defined above.

In one embodiment, the compounds of Formula I are those wherein **B** and **R^{b}**, together are selected from phenyl, 4-methoxyphenyl, 2-methylphenyl, 3-methylphenyl, 4-methylphenyl, 2-bromophenyl, 2-(trifluoromethyl)phenyl, 2-chloro-6-(trifluoromethyl)phenyl, naphthalen-1-yl, naphthalen-2-yl, 4-flurophenyl, 4-(trifluoromethyl)phenyl, 4-chlorophenyl, 2-(trifluoromethoxy)phenyl, 2,5-diflurophenyl, isoquinolin-5-yl, pyridin-4-yl, 2-methoxyphenyl, 4-methoxyphenyl, thiophen-2-yl and cyclopropyl; in particular **B** and **R^{b}**, together are selected from phenyl, 4-methoxyphenyl, 2-methylphenyl, 4-methylphenyl, 4-(trifluoromethyl)phenyl and 3-methylphenyl.

In one embodiment, the compounds of Formula I are those of Formula II: 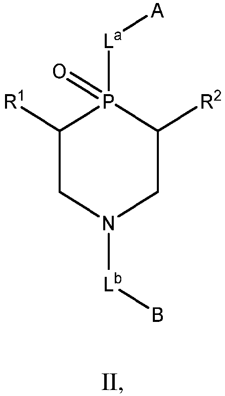 or pharmaceutically acceptable salts thereof,
wherein **A**, **L^{a}**, **R¹**, **R²**, **B** and **L^{b}** are as defined above with respect to Formula I and any of its embodiments.

In one embodiment, the compounds of Formula I are those of Formula III: 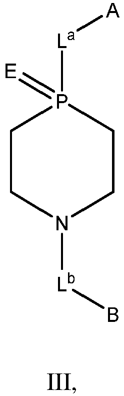 or pharmaceutically acceptable salts thereof,
wherein **A**, **L^{a}**, **B**, **L^{b}** and **E** are as defined above with respect to Formula I and any of its embodiments.

In one embodiment, the compounds of Formula I are those of Formula IV: 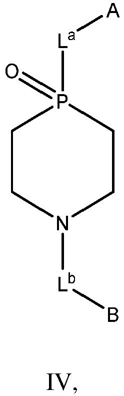 or pharmaceutically acceptable salts thereof,
wherein **A**, **L^{a}**, **B** and **L^{b}** are as defined above with respect to Formula I and any of its embodiments.

Particular compounds of Formula IV, or pharmaceutically acceptable salts thereof, are those wherein one or more of **A**, **L^{a}**, **B** and **L^{b}** are defined as follows:
**A** is selected from the group consisting of phenyl and naphthalenyl, said phenyl and naphthalenyl being optionally substituted with 1 to 5 substituents **R^{a}**, wherein **R^{a}** is as defined as above; in particular **A** is selected from the group consisting of phenyl and naphthalenyl, said phenyl and naphthalenyl being optionally substituted with 1 to 5 substituents **R^{a}**, wherein **R^{a}** is selected from the group consisting of trifluoromethyl, fluro and methyl;
**L^{a}** is a bond or is-CH₂-;
**B** is selected from the group consisting of phenyl, naphthalenyl, pyridinyl, thiophenyl and cyclopropyl, said phenyl, naphthalenyl, pyridinyl, thiophenyl and cyclopropyl being optionally substituted with 1 or 2 substituents **R^{b}**, wherein **R^{b}** is as defined above; in particular **B** is selected from the group consisting of phenyl, naphthalenyl, pyridinyl, thiophenyl and cyclopropyl, said phenyl, naphthalenyl, pyridinyl, thiophenyl and cyclopropyl being optionally substituted with 1 or 2 substituents **R^{b}**, wherein **R^{b}** is selected from the group consisting of methoxy, methyl and trifluromethyl; and
**L^{b}** is a bond or is -CH₂-.

In one embodiment, the compounds of Formula IV are those wherein **A** and **R^{a}**, together are selected from the group consisting of 4-(trifluoromethyl)phenyl, 3-(trifluoromethyl)phenyl, 4-fluorophenyl, phenyl, 4-methylphenyl, 3-methylphenyl, and naphthalen-1-yl.

In one embodiment, the compounds of Formula IV are those wherein **B** and **R^{b}**, together are selected from phenyl, 4-methoxyphenyl, 2-methylphenyl, 4-methylphenyl, 4-(trifluoromethyl)phenyl and 3-methylphenyl.

Particularly preferred compounds of the invention are those listed in Table 1 hereafter:

**Table 1**

| Compound | Structure | Name |
|---|---|---|
| 3 | 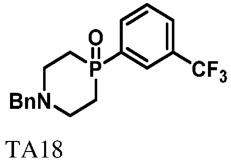 | 1-benzyl-4-(3-(trifluoromethyl)phenyl)-1,4-azaphosphinane 4-oxide |
| 4 | 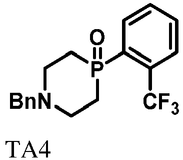 | 1-benzyl-4-(2-(trifluoromethyl)phenyl)-1,4-azaphosphinane 4-oxide |
| 5 | 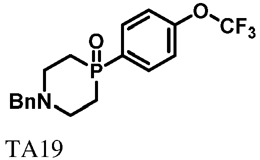 | 1-benzyl-4-(4-(trifluoromethoxy)phenyl)-1,4-azaphosphinane 4-oxide |
| 6 | 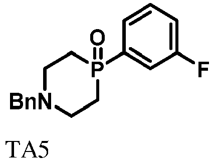 | 1-benzyl-4-(3-fluorophenyl)-1,4-azaphosphinane 4-oxide |
| 7 | 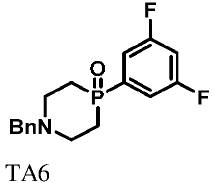 | 1-benzyl-4-(3,5-difluorophenyl)-1,4-azaphosphinane 4-oxide |
| 8 | 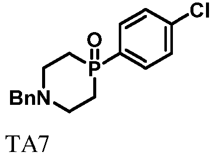 | 1-benzyl-4-(4-chlorophenyl)-1,4-azaphosphinane 4-oxide |
| 9 | 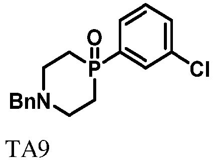 | 1-benzyl-4-(3-chlorophenyl)-1,4-azaphosphinane 4-oxide |
| 10 | 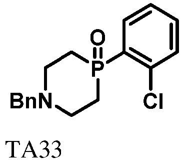 | 1-benzyl-4-(2-chlorophenyl)-1,4-azaphosphinane 4-oxide |
| 11 | 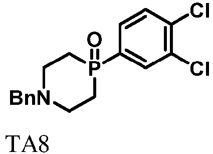 | 1-benzyl-4-(3,4-dichlorophenyl)-1,4-azaphosphinane 4-oxide |
| 12 | 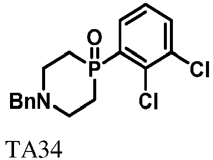 | 1-benzyl-4-(2,3-dichlorophenyl)-1,4-azaphosphinane 4-oxide |
| 14 | 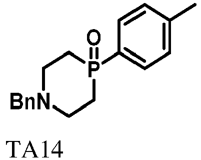 | 1-benzyl-4-(*p*-tolyl)-1,4-azaphosphinane 4-oxide |
| 15 | 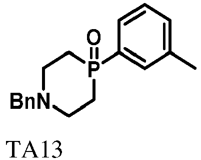 | 1-benzyl-4-(*m*-tolyl)-1,4-azaphosphinane 4-oxide |
| 16 | 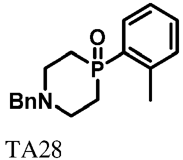 | 1-benzyl-4-(*o*-tolyl)-1,4-azaphosphinane 4-oxide |
| 17 | 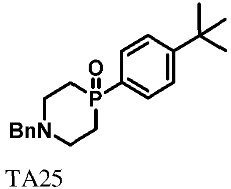 | 1-benzyl-4-(4-(*tert*-butyl)phenyl)-1,4-azaphosphinane 4-oxide |
| 18 | 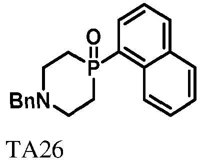 | 1-benzyl-4-(naphthalen-1-yl)-1,4-azaphosphinane 4-oxide |
| 19 | 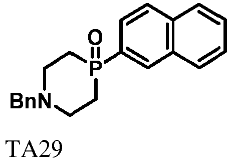 | 1-benzyl-4-(naphthalen-2-yl)-1,4-azaphosphinane 4-oxide |
| 20 | 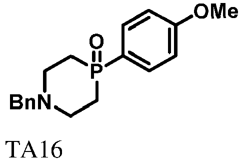 | 1-benzyl-4-(4-methoxyphenyl)-1,4-azaphosphinane 4-oxide |
| 21 | 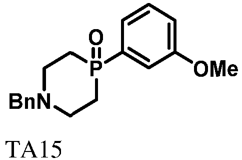 | 1-benzyl-4-(3-methoxyphenyl)-1,4-azaphosphinane 4-oxide |
| 22 | 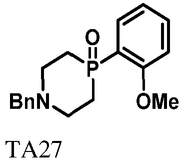 | 1-benzyl-4-(2-methoxyphenyl)-1,4-azaphosphinane 4-oxide |
| 23 | 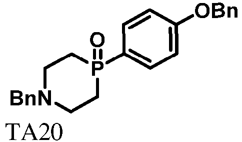 | 1-benzyl-4-(4-(benzyloxy)phenyl)-1,4-azaphosphinane 4-oxide |
| 24 | 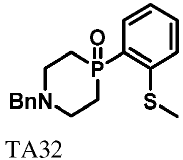 | 1-benzyl-4-(2-(methylthio)phenyl)-1,4-azaphosphinane 4-oxide |
| 25 | 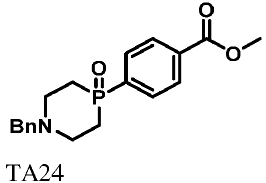 | methyl 4-(1-benzyl-4-oxido-1,4-azaphosphinan-4-yl)benzoate |
| 26 | 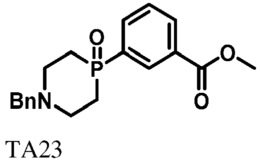 | methyl 3-(1-benzyl-4-oxido-1,4-azaphosphinan-4-yl)benzoate |
| 27 | 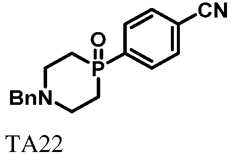 | 4-(1-benzyl-4-oxido-1,4-azaphosphinan-4-yl)benzonitrile |
| 28 | 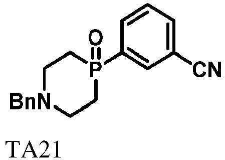 | 3-(1-benzyl-4-oxido-1,4-azaphosphinan-4-yl)benzonitrile |
| 29 | 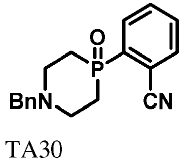 | 2-(1-benzyl-4-oxido-1,4-azaphosphinan-4-yl)benzonitrile |
| 30 | 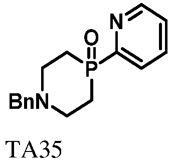 | 1-benzyl-4-(pyridin-2-yl)-1,4-azaphosphinane 4-oxide |
| 31 | 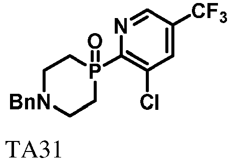 | 1-benzyl-4-(3-chloro-5-(trifluoromethyl)pyridin-2-yl)-1,4-azaphosphinane 4-oxide |
| 32 | 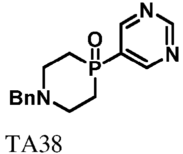 | 1-benzyl-4-(pyrimidin-5-yl)-1,4-azaphosphinane 4-oxide |
| 33 | 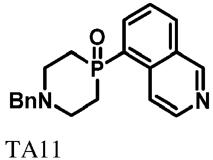 | 1-benzyl-4-(isoquinolin-5-yl)-1,4-azaphosphinane 4-oxide |
| 34 | 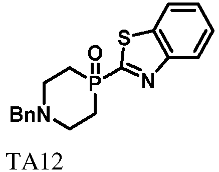 | 4-(benzo[d]thiazol-2-yl)-1-benzyl-1,4-azaphosphinane 4-oxide |
| 35 | 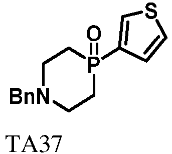 | 1-benzyl-4-(thiophen-3-yl)-1,4-azaphosphinane 4-oxide |
| 36 | 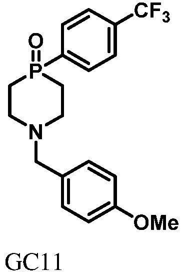 | 1-(4-methoxybenzyl)-4-(4-(trifluoromethyl)phenyl)-1,4-azaphosphinane 4-oxide |
| 37 | 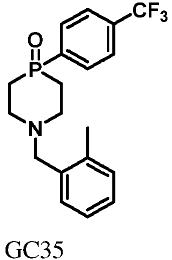 | 1-(2-methylbenzyl)-4-(4-(trifluoromethyl)phenyl)-1,4-azaphosphinane 4-oxide |
| 38 | 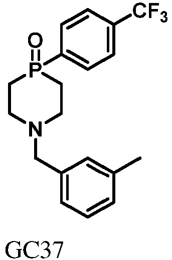 | 1-(3-methylbenzyl)-4-(4-(trifluoromethyl)phenyl)-1,4-azaphosphinane 4-oxide |
| 39 | 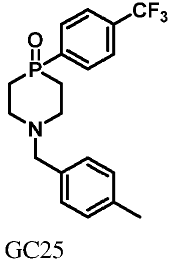 | 1-(4-methylbenzyl)-4-(4-(trifluoromethyl)phenyl)-1,4-azaphosphinane 4-oxide |
| 40 | 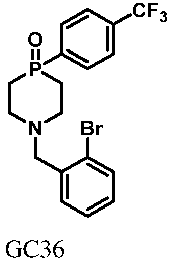 | 1-(2-bromobenzyl)-4-(4-(trifluoromethyl)phenyl)-1,4-azaphosphinane 4-oxide |
| 41 | 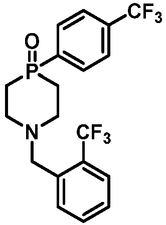 | 1-(2-(trifluoromethyl)benzyl)-4-(4-(trifluoromethyl)phenyl)-1,4-azaphosphinane 4-oxide |
| | 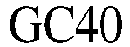 | |
| 42 | 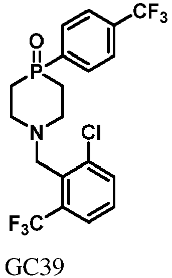 | 1-(2-chloro-6-(trifluoromethyl)benzyl)-4-(4-(trifluoromethyl)phenyl)-1,4-azaphosphinane 4-oxide |
| 43 | 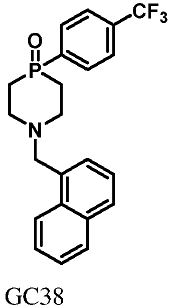 | 1-(naphthalen-1-ylmethyl)-4-(4-(trifluoromethyl)phenyl)-1,4-azaphosphinane 4-oxide |
| 44 | 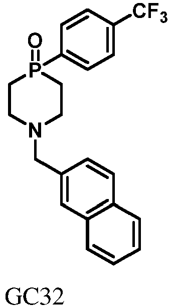 | 1-(naphthalen-2-ylmethyl)-4-(4-(trifluoromethyl)phenyl)-1,4-azaphosphinane 4-oxide |
| 45 | 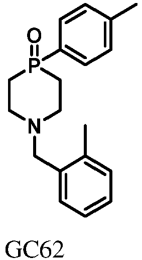 | 1-(2-methylbenzyl)-4-(*p*-tolyl)-1,4-azaphosphinane 4-oxide |
| 46 | 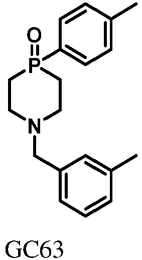 | 1-(3-methylbenzyl)-4-(*p*-tolyl)-1,4-azaphosphinane 4-oxide |
| 47 | 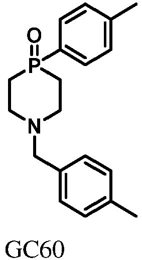 | 1-(4-methylbenzyl)-4-(*p*-tolyl)-1,4-azaphosphinane 4-oxide |
| 48 | 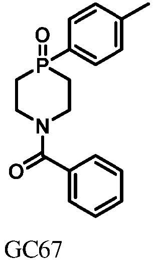 | (4-oxido-4-(*p*-tolyl)-1,4-azaphosphinan-1-yl)(phenyl)methanone |
| 49 | 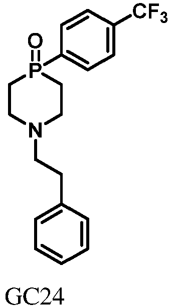 | 1-phenethyl-4-(4-(trifluoromethyl)phenyl)-1,4-azaphosphinane 4-oxide |
| *50* | 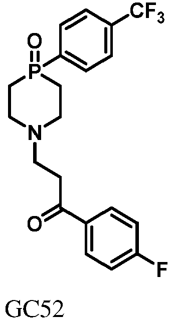 | 1-(4-fluorophenyl)-3-(4-oxido-4-(4-(trifluoromethyl)phenyl)-1,4-azaphosphinan-1-yl)propan-1-one |
| 51 | 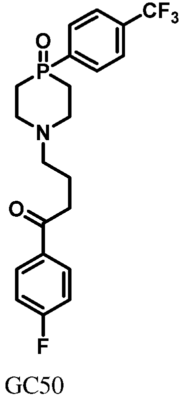 | 1-(4-fluorophenyl)-4-(4-oxido-4-(4-(trifluoromethyl)phenyl)-1,4-azaphosphinan-1-yl)butan-1-one |
| 52 | 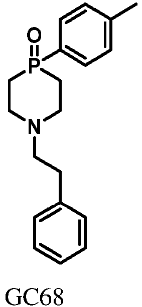 | 1-phenethyl-4-(p-tolyl)-1,4-azaphosphinane 4-oxide |
| 53 | 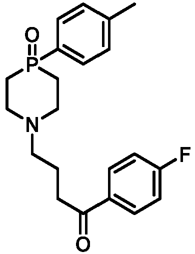 | 1-(4-fluorophenyl)-4-(4-oxido-4-(p-tolyl)-1,4-azaphosphinan-1-yl)butan-1-one |
| | 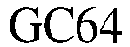 | |
| 54 | 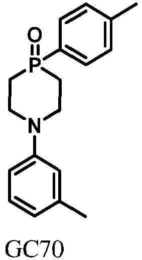 | 1-(m-tolyl)-4-(p-tolyl)-1,4-azaphosphinane 4-oxide |
| 55 | 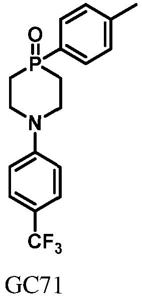 | 4-(p-tolyl)-1-(4-(trifluoromethyl)phenyl)-1,4-azaphosphinane 4-oxide |
| 56 | 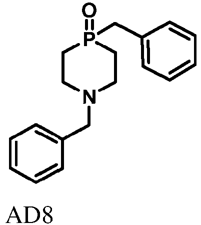 | 1,4-dibenzyl-1,4-azaphosphinane 4-oxide |
| 57 | 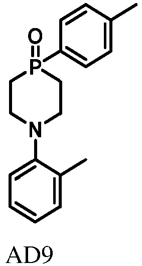 | 1-(o-tolyl)-4-(*p*-tolyl)-1,4-azaphosphinane 4-oxide |
| 58 | 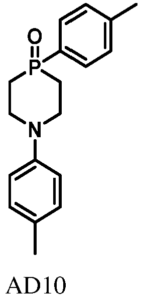 | 1,4-di-p-tolyl-1,4-azaphosphinane 4-oxide |
| 59 | 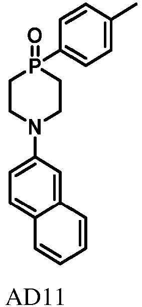 | 1-(naphthalen-2-yl)-4-(*p*-tolyl)-1,4-azaphosphinane 4-oxide |
| 60 | 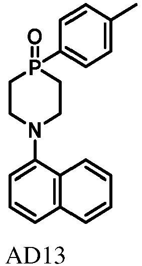 | 1-(naphthalen-1-yl)-4-(*p*-tolyl)-1,4-azaphosphinane 4-oxide |
| 61 | 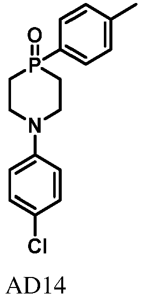 | 1-(4-chlorophenyl)-4-(*p*-tolyl)-1,4-azaphosphinane 4-oxide |
| 62 | 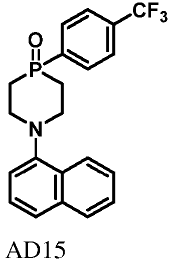 | 1-(naphthalen-1-yl)-4-(4-(trifluoromethyl)phenyl)-1,4-azaphosphinane 4-oxide |
| 63 | 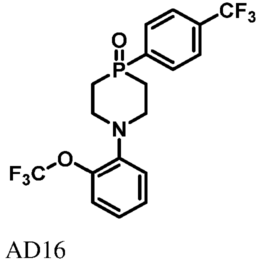 | 1-(2-(trifluoromethoxy)phenyl)-4-(4-(trifluoromethyl)phenyl)-1,4-azaphosphinane 4-oxide |
| 64 | 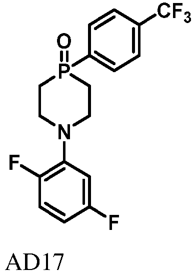 | 1-(2,5-difluorophenyl)-4-(4-(trifluoromethyl)phenyl)-1,4-azaphosphinane 4-oxide |
| 65 | 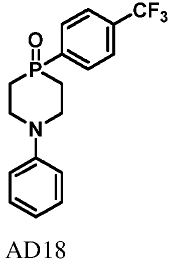 | 1-phenyl-4-(4-(trifluoromethyl)phenyl)-1,4-azaphosphinane 4-oxide |
| 66 | 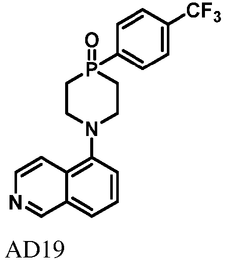 | 1-(isoquinolin-5-yl)-4-(4-(trifluoromethyl)phenyl)-1,4-azaphosphinane 4-oxide |
| 67 | 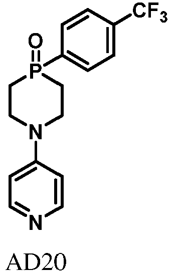 | 1-(pyridin-4-yl)-4-(4-(trifluoromethyl)phenyl)-1,4-azaphosphinane 4-oxide |
| 68 | 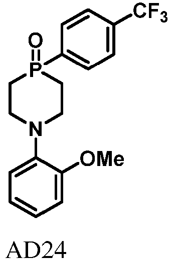 | 1-(2-methoxyphenyl)-4-(4-(trifluoromethyl)phenyl)-1,4-azaphosphinane 4-oxide |
| 69 | 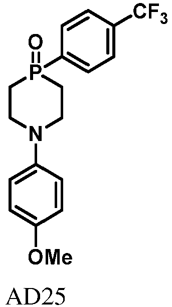 | 1-(4-methoxyphenyl)-4-(4-(trifluoromethyl)phenyl)-1,4-azaphosphinane 4-oxide |
| 70 | 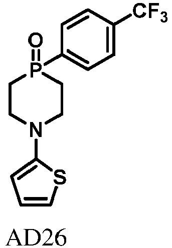 | 1-(thiophen-2-yl)-4-(4-(trifluoromethyl)phenyl)-1,4-azaphosphinane 4-oxide |
| 71 | 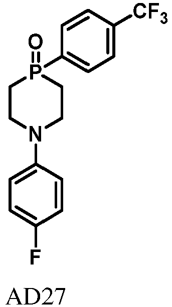 | 1-(4-fluorophenyl)-4-(4-(trifluoromethyl)phenyl)-1,4-azaphosphinane 4-oxide |
| 72 | 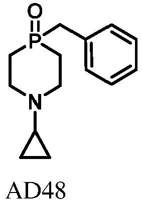 | 4-benzyl-1-cyclopropyl-1,4-azaphosphinane 4-oxide |
| 73 | 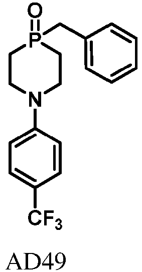 | 4-benzyl-1-(4-(trifluoromethyl)phenyl)-1,4-azaphosphinane 4-oxide |
| 74 | 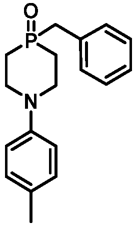 | 4-benzyl-1-(*p*-tolyl)-1,4-azaphosphinane 4-oxide |
| | 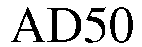 | |
| 75 | 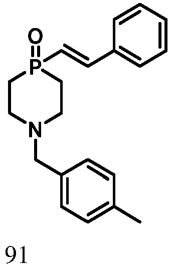 | (E)-1-(4-methylbenzyl)-4-styryl-1,4-azaphosphinane 4-oxide |
| 76 | 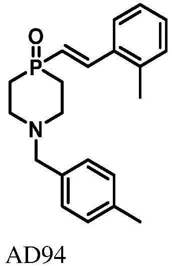 | (E)-1-(4-methylbenzyl)-4-(2-methylstyryl)-1,4-azaphosphinane 4-oxide |
| 77 | 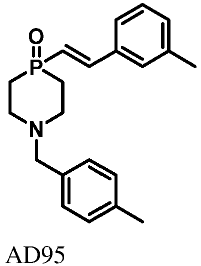 | (E)-1-(4-methylbenzyl)-4-(3-methylstyryl)-1,4-azaphosphinane 4-oxide |
| 78 | 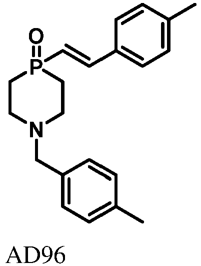 | (E)-1-(4-methylbenzyl)-4-(4-methylstyryl)-1,4-azaphosphinane 4-oxide |
| 79 | 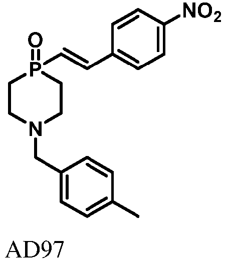 | (E)-1-(4-methylbenzyl)-4-(4-nitrostyryl)-1,4-azaphosphinane 4-oxide |
| 80 | 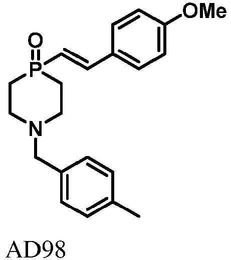 | (E)-4-(4-methoxystyryl)-1-(4-methylbenzyl)-1,4-azaphosphinane 4-oxide |
| 81 | 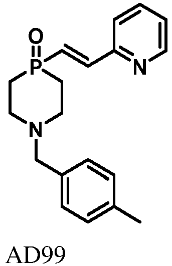 | (E)-1-(4-methylbenzyl)-4-(2-(pyridin-2-yl)vinyl)-1,4-azaphosphinane 4-oxide |
| 82 | 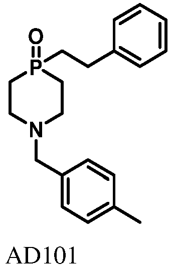 | 1-(4-methylbenzyl)-4-phenethyl-1,4-azaphosphinane 4-oxide |
| 83 | 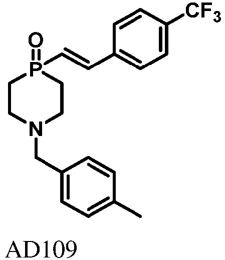 | (E)-1-(4-methylbenzyl)-4-(4-(trifluoromethyl)styryl)-1,4-azaphosphinane 4-oxide |
| 84 | 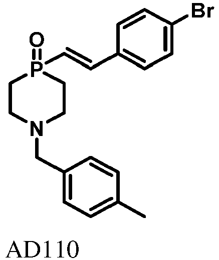 | (E)-4-(4-bromostyryl)-1-(4-methylbenzyl)-1,4-azaphosphinane 4-oxide |
| 85 | 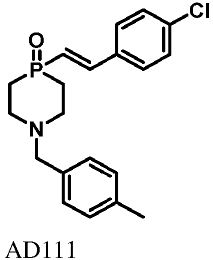 | (E)-4-(4-chlorostyryl)- 1-(4-methylbenzyl)-1,4-azaphosphinane 4-oxide |
| 86 | 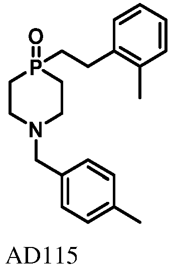 | 1-(4-methylbenzyl)-4-(2-methylphenethyl)-1,4-azaphosphinane 4-oxide |
| 87 | 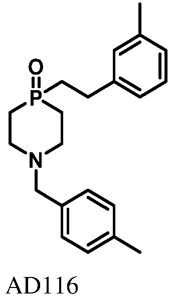 | 1-(4-methylbenzyl)-4-(3-methylphenethyl)-1,4-azaphosphinane 4-oxide |
| 88 | 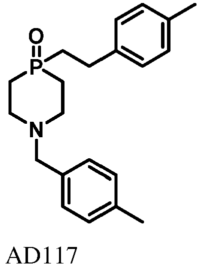 | 1-(4-methylbenzyl)-4-(4-methylphenethyl)-1,4-azaphosphinane 4-oxide |
| 89 | 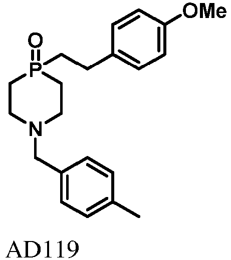 | 4-(4-methoxyphenethyl)-1-(4-methylbenzyl)-1,4-azaphosphinane 4-oxide |
| 90 | 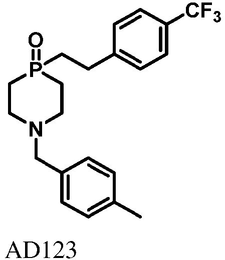 | 1-(4-methylbenzyl)-4-(4-(trifluoromethyl)phenethyl)-1,4-azaphosphinane 4-oxide |
| 91 | 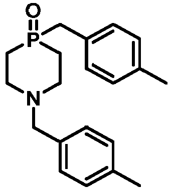 | 1,4-bis(4-methylbenzyl)-1,4-azaphosphinane 4-oxide |
| | 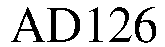 | |
| 92 | 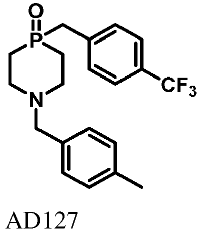 | 1-(4-methylbenzyl)-4-(4-(trifluoromethyl)benzyl)-1,4-azaphosphinane 4-oxide |
| 93 | 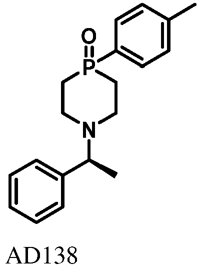 | (S)-1-(1-phenylethyl)-4-(p-tolyl)-1,4-azaphosphinane 4-oxide |
| 94 | 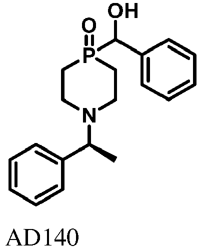 | 4-(hydroxy(phenyl)methyl)-1-((S)-1-phenylethyl)-1,4-azaphosphinane 4-oxide |
| 95 | 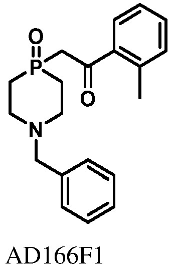 | 2-(1-benzyl-4-oxido-1,4-azaphosphinan-4-yl)-1-(o-tolyl)ethan-1-one |
| 96 | 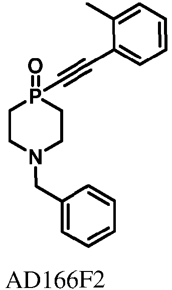 | 1-benzyl-4-(o-tolylethynyl)-1,4-azaphosphinane 4-oxide |
| 97 | 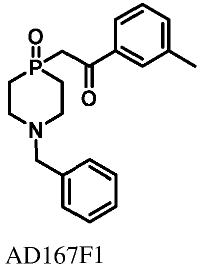 | 2-(1-benzyl-4-oxido-1,4-azaphosphinan-4-yl)-1-(m-tolyl)ethan-1-one |
| 98 | 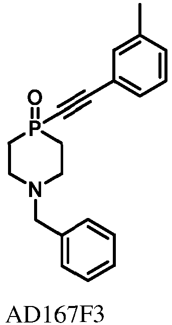 | 1-benzyl-4-(o-tolylethynyl)-1,4-azaphosphinane 4-oxide |
| 99 | 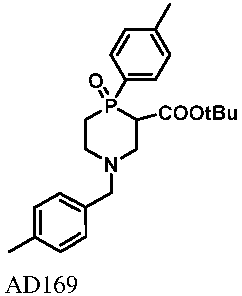 | tert-butyl 1-(4-methylbenzyl)-4-(p-tolyl)-1,4-azaphosphinane-3-carboxylate 4-oxide |
| 100 | 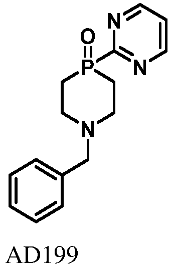 | 1-benzyl-4-(pyrimidin-2-yl)-1,4-azaphosphinane 4-oxide |
| 101 | 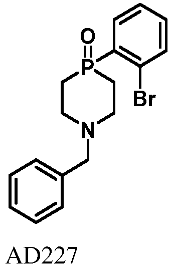 | 1-benzyl-4-(2-bromophenyl)-1,4-azaphosphinane 4-oxide |
| 102 | 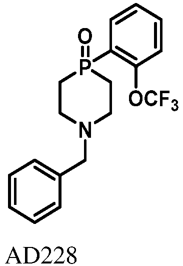 | 1-benzyl-4-(2-(trifluoromethoxy)phenyl)-1,4-azaphosphinane 4-oxide |
| 104 | 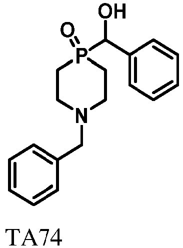 | 1-benzyl-4-(hydroxy(phenyl)methyl)-1,4-azaphosphinane 4-oxide |
| 105 | 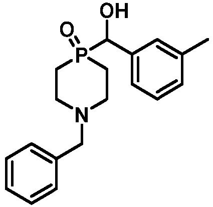 | 1-benzyl-4-(hydroxy(m-tolyl)methyl)-1,4-azaphosphinane 4-oxide |
| | 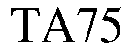 | |
| 106 | 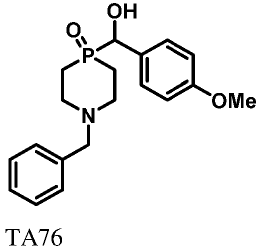 | 1-benzyl-4-(hydroxy(4-methoxyphenyl)methyl)-1,4-azaphosphinane 4-oxide |
| 107 | 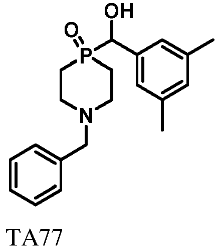 | 1-benzyl-4-((3,5-dimethylphenyl)(hydroxy)methyl)-1,4-azaphosphinane 4-oxide |
| 108 | 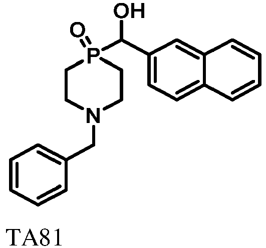 | 1-benzyl-4-(hydroxy(naphthalen-2-yl)methyl)-1,4-azaphosphinane 4-oxide |
| 109 | 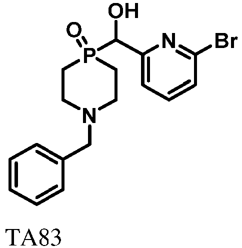 | 1-benzyl-4-((6-bromopyridin-2-yl)(hydroxy)methyl)-1,4-azaphosphinane 4-oxide |
| 110 | 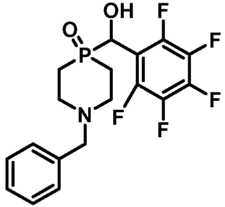 | 1-benzyl-4-(hydroxy(perfluorophenyl)methyl)-1,4-azaphosphinane 4-oxide |
| | 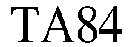 | |
| 111 | 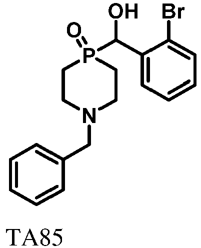 | 1-benzyl-4-((2-bromophenyl)(hydroxy)methyl)-1,4-azaphosphinane 4-oxide |
| 112 | 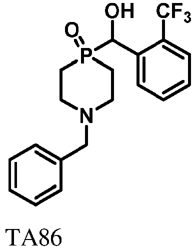 | 1-benzyl-4-(hydroxy(2-(trifluoromethyl)phenyl)methyl)-1,4-azaphosphinane 4-oxide |
| 113 | 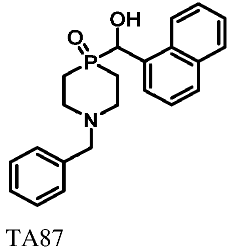 | 1-benzyl-4-(hydroxy(naphthalen-1-yl)methyl)-1,4-azaphosphinane 4-oxide |
| 114 | 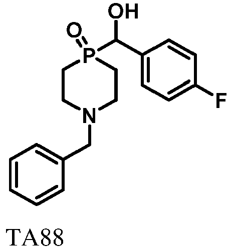 | 1-benzyl-4-((4-fluorophenyl)(hydroxy)methyl)-1,4-azaphosphinane 4-oxide |
| 115 | 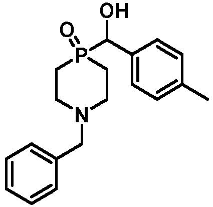 | 1-benzyl-4-(hydroxy(p-tolyl)methyl)-1,4-azaphosphinane 4-oxide |
| | 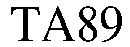 | |
| 116 | 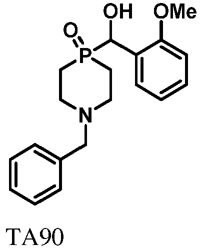 | 1-benzyl-4-(hydroxy(2-methoxyphenyl)methyl)-1,4-azaphosphinane 4-oxide |
| 117 | 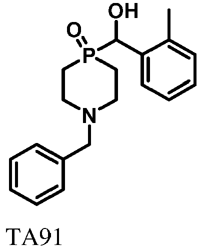 | 1-benzyl-4-(hydroxy(o-tolyl)methyl)-1,4-azaphosphinane 4-oxide |
| 118 | 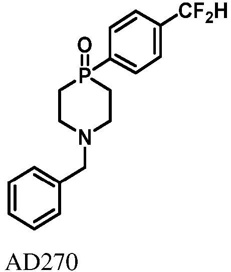 | 1-benzyl-4-(4-(difluoromethyl)phenyl)-1,4-azaphosphinane 4-oxide |
| 119 | 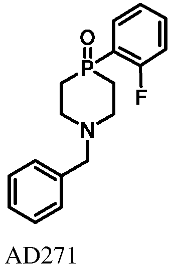 | 1-benzyl-4-(2-fluorophenyl)-1,4-azaphosphinane 4-oxide |
| 120 | 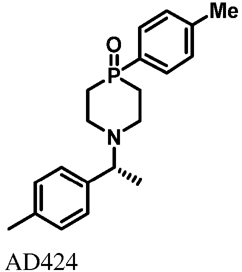 | (R)-4-(p-tolyl)-1-(1-(p-tolyl)ethyl)-1,4-azaphosphinane 4-oxide |
| 121 | 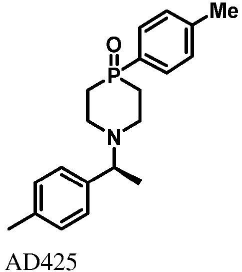 | (S)-4-(p-tolyl)-1-(1-(p-tolyl)ethyl)-1,4-azaphosphinane 4-oxide |

In one embodiment, the compounds of Formula I, or pharmaceutically acceptable salts thereof, are selected from the group consisting of Compound 3 (TA18), Compound 14 (TA14), Compound 15 (TA13), Compound 18 (TA26), Compound 36 (GC11), Compound 37 (GC35), Compound 39 (GC25), Compound 46 (GC63), Compound 47 (GC 60), Compound 49 (GC24), Compound 50 (GC52), Compound 52 (GC68), Compound 54 (GC70), Compound 56 (AD8), Compound 57 (AD9), Compound 61 (AD14), Compound 65 (AD18, Compound 65 (AD18), Compound 67 (AD20), Compound 72 (AD48), Compound 91 (AD126), Compound 93 (AD138), Compound 94 (AD140), Compound 104 (TA74), Compound 105 (TA75), Compound 120 (AD424) and Compound 121 (AD425). In particular, the compounds of Formula I, or pharmaceutically acceptable salts thereof, are selected from the group consisting of Compound 3 (TA18), Compound 14 (TA14), Compound 47 (GC 60), Compound 54 (GC70), Compound 120 (AD424) and Compound 121 (AD425).

The compounds of the invention can be prepared by different ways with reactions known by the person skilled in the art. Reaction schemes as described in the example section illustrate by way of example different possible approaches.

The compounds of the invention are indeed modulators of MAM functioning, as shown by differently affecting a biological response in a zebrafish model of Wolfram syndrome between wfs1ab^{KO} larvae and wfs1^{WT} larvae. Compounds of the invention are surprisingly shown to have: (i) direct sigma-1 receptor activity, for example by having the ability to bind to S1R, in particular an activity as direct agonists of S1R, or (ii) to have a different mechanism of modulating, in particular increasing, MAM functioning because they can modulate, in particular activate, another MAM chaperone or another protein acting as an upstream regulator of S1R or another MAM chaperone.

In particular, the compounds of the invention are modulators, preferably agonists of sigma-1 receptor. The invention thus also provides the use of the compounds of the invention, or pharmaceutically acceptable salts thereof, as modulators of MAM functioning such as by modulating, in particular activating, sigma-1 receptor activity.

Accordingly, in a particularly preferred embodiment, the invention relates to the use of compounds of Formula I and subformulae, or pharmaceutically acceptable salts and thereof, as sigma-1 receptor modulators.

### APPLICATIONS

Unexpectedly, the inventors have discovered that the compounds of Formula I have an activity as modulators of MAM functioning such as by modulating, preferably activating, the sigma-1 receptor activity.

The compounds of the invention are therefore useful in the treatment and/or prevention of a disease, disorder or condition that is associated with, caused by or a symptom of a mutation in WFS1 or WFS2 (CISD2) gene, in particular Wolfram syndrome, Wolfram-like syndrome, or disease, disorder or condition associated therewith, caused thereby or a symptom thereof, associated with mitochondrial dysfunction, and/or is treatable, ameliorable or preventable by activation or inhibition of S1R, in particular one or more neurodegenerative diseases, disorders or conditions.

The invention thus also relates to a compound of Formula I, 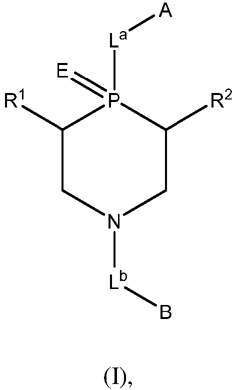 or a pharmaceutically acceptable salt thereof,
wherein
A is selected from the group consisting of phenyl, naphthyl, 5 to 10-membered mono-cyclic or bi-cyclic heteroaryl, 3 to 6-membered cycloalkyl and 3 to 6-membered cycloalkenyl, said phenyl, naphthyl, 5 to 10-membered mono-cyclic or bi-cyclic heteroaryl, 3 to 5-membered cycloalkyl and 3 to 6-membered cycloalkenyl being optionally substituted with 1 to 5 substituents **R^{a}**;
   wherein each **R^{a}** is independently selected from the group consisting of -H, -F, -Cl, - Br, -CN, -NH₂, -NO₂, -CH₂F, -CHF₂, -CF₃, -OCH₃, -(CO)OH, -(CO)OCH₃, -SCH₃, -OCH₂F, -OCHF₂, -OCF₃, -O(CH₂)Ph, and C₁₋₄ alkyl;
**L^{a}** is selected from the group consisting of a bond, C₁₋₅ alkanediyl, C₂₋₅ alkenediyl and C₂₋₅ alkynediyl, each optionally substituted with one, two or three substituents independently selected from the group consisting of -OH and =O;
**R¹** and **R²** are independently selected from -H and -C(O)O-C₁₋₄ alkyl;
**B** is selected from the group consisting of phenyl, naphthyl, 5 to 10-membered mono-cyclic or bi-cyclic heteroaryl, 3 to 6-membered cycloalkyl and 3 to 6-membered cycloalkenyl, said phenyl, naphthyl, 5 to 10-membered mono-cyclic or bi-cyclic heteroaryl, 3 to 6-membered cycloalkyl 3 to 6-membered cycloalkyl and 3 to 6-membered cycloalkenyl being optionally substituted with 1 to 5 substituents **R^{b}**;
   wherein each **R^{b}** is independently selected from the group consisting of -H, -F, -Cl, -Br, -CN, -NH₂, -NO₂, -CH₂F, -CHF₂, -CF₃, -OCH₃, -(CO)OH, -(CO)OCH₃, -SCH₃, -OCH₂F, -OCHF₂, -OCF₃, -O(CH₂)Ph, and C₁₋₄ alkyl;
**L^{b}** is a linker selected from the group consisting of a bond, C₁₋₅ alkanediyl, C₂₋₅ alkenediyl and C₂₋₅ alkynediyl, each optionally substituted with one, two or three substituents independently selected from the group consisting of -OH and =O;
E is selected from the group consisting of O, S and Se;
for use in treating and/or preventing a disease, disorder or condition that is:
   (a) associated, caused by or a symptom of with a mutation in WFS1 or WFS2 (CISD2) gene;
   (b) a neurodegenerative disease, disorder or condition;
   (c) selected from the group consisting of stroke, stroke-induced neuronal damage, depression, anxiety, addiction, epilepsy, multiple sclerosis, pain, including neuropathic pain, and cancer;
   (d) associated with mitochondrial dysfunction, endoplasmic reticulum (ER) stress, or disfunction of nucleocytoplasmic transport; and/or
   (e) treatable, ameliorable or preventable by activation or inhibition of sigma-1 receptor.

Particular compounds for use of Formula I, or pharmaceutically acceptable salts thereof, are those wherein one or more of **A**, **L^{a}**, **R¹**, **R²**, **B**, **L^{b}**, and **E** are defined as follows:
A is selected from the group consisting of phenyl, naphthyl, 5 to 10-membered mono-cyclic or bi-cyclic heteroaryl, 3 to 6-membered cycloalkyl and 3 to 6-membered cycloalkenyl, said phenyl, naphthyl, 5 to 10-membered mono-cyclic or bi-cyclic heteroaryl, 3 to 5-membered cycloalkyl and 3 to 6-membered cycloalkenyl being optionally substituted with 1 to 5 substituents **R^{a}**; in particular A is selected from the group consisting of phenyl, naphthyl, and 5 to 10-membered mono-cyclic or bi-cyclic heteroaryl, said phenyl, naphthyl, and 5 to 10-membered mono-cyclic or bi-cyclic heteroaryl being optionally substituted with 1 to 5 substituents **R^{a}**;
   wherein each **R^{a}** is independently selected from the group consisting of -H, -F, -Cl, -Br, -CN, -NH₂, -NO₂, -CH₂F, -CHF₂, -CF₃, -OCH₃, -(CO)OH, -(CO)OCH₃, -SCH₃, -OCH₂F, -OCHF₂, -OCF₃, -O(CH₂)Ph, and C₁₋₄ alkyl; in particular each **R^{a}** is independently selected from the group consisting of -H, -F, -Cl, -Br, -CN, -NO₂, -CF₃, -OCH₃, - (CO)OCH₃, -SCH₃, -OCF₃, and C₁₋₄ alkyl; more particularly each **R^{a}** is independently selected from the group consisting of -H, -F, -Cl, -Br, -CN, -NO₂, -CH₃, -CF₃, -OCH₃, - (CO)OCH₃, -SCH₃, -OCF₃, and t-Bu;
**L^{a}** is a linker selected from the group consisting of a bond, C₁₋₅ alkanediyl, C₂₋₅ alkenediyl and C₂₋₅ alkynediyl, each optionally substituted with one, two or three substituents independently selected from the group consisting of -OH and =O; in particular **L^{a}** is a linker selected from the group consisting of a bond, C₁₋₅ alkanediyl, C₂₋₅ alkenediyl, C₂₋₅ alkynediyl, hydroxy-C₁₋₅ alkanediyl and C₁₋₄ alkanediyl-(CO)-; more particularly **L^{a}** is a linker selected from the group consisting of a bond, C₁₋₄ alkanediyl, C₂₋₄ alkenediyl, C₂₋₄ alkynediyl, hydroxy-C₁₋₄ alkanediyl and C₁₋₃ alkanediyl-(CO)-; still more particularly **L^{a}** is a linker selected from the group consisting of a bond, C₁₋₂ alkanediyl, C₂ alkenediyl, C₂ alkynediyl, hydroxy-C₁₋₂ alkanediyl and C₁ alkanediyl-(CO)-; even more particularly **L^{a}** is a linker selected from the group consisting of a bond, -CH₂-, -(CH₂)₂-, -CH=CH-, -C≡C-, -CH(OH)- and -CH₂-C(O)-;
**R¹** and **R²** are independently selected from -H and -C(O)O-C₁₋₄ alkyl; in particular **R¹** and **R²** are independently selected from -H and -C(O)OMe;
**B** is selected from the group consisting of phenyl, naphthyl, 5 to 10-membered mono-cyclic or bi-cyclic heteroaryl, 3 to 6-membered cycloalkyl and 3 to 6-membered cycloalkenyl, said phenyl, naphthyl, 5 to 10-membered mono-cyclic or bi-cyclic heteroaryl, 3 to 6-membered cycloalkyl 3 to 6-membered cycloalkyl and 3 to 6-membered cycloalkenyl being optionally substituted with 1 to 5 substituents **R^{b}**;
   wherein each **R^{b}** is independently selected from the group consisting of -H, -F, -Cl, -Br, -CN, -NH₂, -NO₂, -CH₂F, -CHF₂, -CF₃, -OCH₃, -(CO)OH, -(CO)OCH₃, -SCH₃, -OCH₂F, -OCHF₂, -OCF₃, -O(CH₂)Ph, and C₁₋₄ alkyl; in particular each **R^{b}** is independently selected from the group consisting of -H, -F, -Cl, -CH₃, -CF₃, -OCH₃, and -OCF₃;
**L^{b}** is a linker selected from the group consisting of a bond, C₁₋₅ alkanediyl, C₂₋₅ alkenediyl and C₂₋₅ alkynediyl, each optionally substituted with one, two or three substituents independently selected from the group consisting of -OH and =O; in particular **L^{b}** is a linker selected from the group consisting of a bond, C₁₋₅ alkanediyl, C₂₋₅ alkenediyl and C₂₋₅ alkynediyl, hydroxy-C₁₋₅ alkanediyl, C₁₋₄ alkanediyl-(CO)- and -(CO)-; more particularly **L^{b}** is a linker selected from the group consisting of a bond, C₁₋₄ alkanediyl, C₁₋₄ alkanediyl-(CO)- and -(CO)-; still more particularly **L^{b}** is a linker selected from the group consisting of a bond, C₁₋₃ alkanediyl, C₁₋₃ alkanediyl-(CO)- and -(CO)-; even more particularly **L^{b}** is a linker selected from the group consisting of a bond, C₁₋₃ alkanediyl, C₁₋₃ alkanediyl-(CO)- and -(CO)-; even more particularly particularly **L^{b}** is a linker selected from the group consisting of a bond, -CH₂-, -(CH₂)₂-, -CH(CH₃)-, -CH₂-C(O)-, -(CH₂)₂-C(O)-, - (CH₂)₃-C(O)- and -(CO)-; and
E is selected from the group consisting of O, S and Se; in particular **E** is O.

In one embodiment, the compounds for use of Formula I are those wherein **E** is O.

In one embodiment, the compounds for use of Formula I are those wherein **E** is S.

In one embodiment, the compounds for use of Formula I are those wherein **E** is Se.

In one embodiment, the compounds for use of Formula I are those wherein **R¹** and **R²** are H.

In one embodiment, the compounds for use of Formula I are those wherein **L^{a}** is a linker selected from the group consisting of a bond, -CH₂-, -(CH₂)₂-, -CH=CH-, -C≡C-, - CH(OH)-, -CH₂-C(O)-, -(CH₂)₂-C(O)- and -(CH₂)₃-C(O)-; in particular **L^{a}** is a linker selected from the group consisting of a bond, -CH₂-, -(CH₂)₂-, -CH=CH-, -C≡C-, - CH(OH)- and -CH₂-C(O)-.

In one embodiment, the compounds for use of Formula I are those wherein **L^{a}** is a bond.

In one embodiment, the compounds for use of Formula I are those wherein **L^{a}** is a linker selected from the group consisting of -CH₂-, -(CH₂)₂-, -CH=CH-, -C≡C-, -CH(OH)- and -CH₂-C(O)-; in particular, **L^{a}** is -CH₂-.

In one embodiment, the compounds for use of Formula I are those wherein **A** is phenyl or naphthyl, each optionally substituted with 1 to 5 substituents **R^{a}**, wherein **R^{a}** is as defined as above.

In one embodiment, the compounds for use of Formula I are those wherein **A** is a 5 to 10-membered mono-cyclic or bi-cyclic heteroaryl, each optionally substituted with 1 to 5 substituents **R^{a}**, wherein **R^{a}** is as defined as above.

In one embodiment, the compounds for use of Formula I are those wherein **A** is a 3 to 5-membered cycloalkyl, each optionally substituted with 1 to 5 substituents **R^{a}**, wherein **R^{a}** is as defined as above.

In one embodiment, the compounds for use of Formula I are those wherein each **R^{a}** is independently selected from the list consisting of -H, -F, -Cl, -Br, -CN, -NO₂, -CH₃, -CF₃, - OCH₃, -(CO)OCH₃, -SCH₃, -OCF₃, and t-Bu; in particular each **R^{a}** is independently selected from the group consisting of H, -F, -Cl, -CH₃, -CF₃, -OCH₃.

In one embodiment, the compounds for use of Formula I are those wherein **A** is phenyl, optionally substituted with 1, 2 or 3 substituents **R^{a}**, wherein **R^{a}** is as defined as above.

In one embodiment, the compounds for use of Formula I are those wherein **A** is phenyl substituted by one **R^{a}** at position 4 or position 3 of the phenyl **A.**

In one embodiment, the compounds for use of Formula I are those wherein **A** is a 5, 6 or 7 membered mono-cyclic heteroaryl consisting or 1,2 or 3 heteroatoms selected from the group consisting or N, S and O, said 5, 6 or 7 membered mono-cyclic heteroaryl optionally substituted with 1, 2 or 3 substituents **R^{a}**, wherein **R^{a}** is as defined as above.

In one embodiment, the compounds for use of Formula I are those wherein **A** and **R^{a}**, together are selected from the group consisting of phenyl, 4-(trifluoromethyl)phenyl, 4-fluorophenyl, 3-(trifluoromethyl)phenyl, 2-(trifluoromethyl)phenyl, 4-(trifluoromethoxy)phenyl, 3-fluorophenyl, 3,5-difluorophenyl, 4-chlorophenyl, 3-chlorophenyl, 2-chlorophenyl, 3,4-dichlorophenyl, 2,3-dichlorophenyl, 4-methylphenyl, 3-methylphenyl, 2-methylphenyl, 4-t-butylphenyl, naphthalen-1-yl, naphthalen-2-yl, 4-methoxyphenyl, 3-methoxyphenyl, 2-methoxyphenyl, 4-benzyloxyphenyl, 2-(methylthio)phenyl, 4-methyloxycarbonylphenyl, 3-methyloxycarbonylphenyl, 4-cyanophenyl, 3-cyanophenyl, 2-cyanophenyl, pyridin-2-yl, 3-chloro-5-(trifluoromethyl)pyridin-2-yl, pyrimidin-5-yl, isoquinolin-5-yl, benzo[d]thiazol-2-yl, thiophen-3-yl, 4-nitrophenyl, 4-bromophenyl, 2-bromophenyl, 2-(trifluoromethoxy)phenyl, 3,5-dimethylphenyl, 6-bromopyridin-2-yl, perfluorophenyl, 4-(difluoromethyl)phenyl and 2-fluorophenyl.

In one embodiment, the compounds for use of Formula I are those wherein **A** and **R^{a}**, together are selected from the group consisting of 4-(trifluoromethyl)phenyl, 3-(trifluoromethyl)phenyl, 4-fluorophenyl, phenyl, 4-methylphenyl, 3-methylphenyl, and naphthalen-1-yl.

In one embodiment, the compounds for use of Formula I are those wherein **L^{b}** is a linker selected from the group consisting of a bond, -CH₂-, -(CH₂)₂-, -CH=CH-, -C≡C-, - CH(OH)-, -CH(CH₃)-, -CH₂-C(O)-, -(CH₂)₂-C(O)-, -(CH₂)₃-C(O)- and -C(O)-; in particular **L^{b}** is a linker selected from the group consisting of a bond, -CH₂-, -(CH₂)₂-, - CH(CH₃)-, -CH₂-C(O)-, -(CH₂)₂-C(O)-, -(CH₂)₃-C(O)- and -C(O)-.

In one embodiment, the compounds for use of Formula I are those wherein **L^{b}** is a bond.

In one embodiment, the compounds for use of Formula I are those wherein **L^{b}** is a linker selected from the group consisting of a bond, -CH₂-, -(CH₂)₂-, -CH(CH₃)-, -CH₂-C(O)-, -(CH₂)₂-C(O)-, -(CH₂)₃-C(O)- and -C(O)-.

In one embodiment, the compounds for use of Formula I are those wherein **B** is selected from the group consisting of phenyl, naphthalenyl, 5 to 10-membered mono-cyclic or bi-cyclic heteroaryl and 3 to 6-membered cycloalkyl, said phenyl, naphthalenyl, 5 to 10-membered mono-cyclic or bi-cyclic heteroaryl and 3 to 6-membered cycloalkyl being optionally substituted with 1 to 5 substituents **R^{b}**, wherein **R^{b}** is as defined above; in particular the compounds of Formula I are those wherein **B** is selected from the group consisting of phenyl, naphthalenyl, pyridinyl, thiophenyl and cyclopropyl, said phenyl, naphthalenyl, pyridinyl, thiophenyl and cyclopropyl being optionally substituted with 1 or 2 substituents **R^{b}**, wherein **R^{b}** is as defined above.

In one embodiment, the compounds for use of Formula I are those wherein **B** and **R^{b}**, together are selected from phenyl, 4-methoxyphenyl, 2-methylphenyl, 3-methylphenyl, 4-methylphenyl, 2-bromophenyl, 2-(trifluoromethyl)phenyl, 2-chloro-6-(trifluoromethyl)phenyl, naphthalen-1-yl, naphthalen-2-yl, 4-flurophenyl, 4-(trifluoromethyl)phenyl, 4-chlorophenyl, 2-(trifluoromethoxy)phenyl, 2,5-diflurophenyl, isoquinolin-5-yl, pyridin-4-yl, 2-methoxyphenyl, 4-methoxyphenyl, thiophen-2-yl and cyclopropyl; in particular **B** and **R^{b}**, together are selected from phenyl, 4-methoxyphenyl, 2-methylphenyl, 4-methylphenyl, 4-(trifluoromethyl)phenyl and 3-methylphenyl.

In one embodiment, the compounds for use of Formula I are those of Formula II: 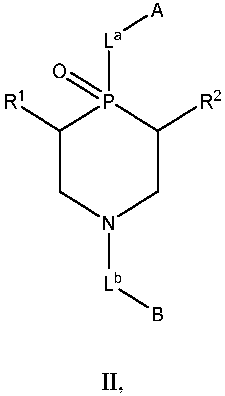 or pharmaceutically acceptable salts thereof,
wherein **A**, **L^{a}**, **R¹**, **R²**, **B** and **L^{b}** are as defined above with respect to Formula I and any of its embodiments.

In one embodiment, the compounds for use of Formula I are those of Formula III: 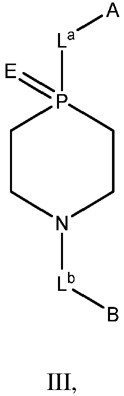 or pharmaceutically acceptable salts thereof,
wherein **A**, **L^{a}**, **B**, **L^{b}** and **E** are as defined above with respect to Formula I and any of its embodiments.

In one embodiment, the compounds for use of Formula I are those of Formula IV: 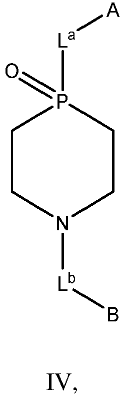 or pharmaceutically acceptable salts thereof,
wherein **A**, **L^{a}**, **B** and **L^{b}** are as defined above with respect to Formula I and any of its embodiments.

Particular compounds for use of Formula IV, or pharmaceutically acceptable salts thereof, are those wherein one or more of **A**, **L^{a}**, **B** and **L^{b}** are defined as follows:
A is selected from the group consisting of phenyl and naphthalenyl, said phenyl and naphthalenyl being optionally substituted with 1 to 5 substituents **R^{a}**, wherein **R^{a}** is as defined as above; in particular **A** is selected from the group consisting of phenyl and naphthalenyl, said phenyl and naphthalenyl being optionally substituted with 1 to 5 substituents **R^{a}**, wherein **R^{a}** is selected from the group consisting of trifluoromethyl, fluro and methyl;
**L^{a}** is a bond or is-CH₂-;
**B** is selected from the group consisting of phenyl, naphthalenyl, pyridinyl, thiophenyl and cyclopropyl, said phenyl, naphthalenyl, pyridinyl, thiophenyl and cyclopropyl being optionally substituted with 1 or 2 substituents **R^{b}**, wherein **R^{b}** is as defined above; in particular B is selected from the group consisting of phenyl, naphthalenyl, pyridinyl, thiophenyl and cyclopropyl, said phenyl, naphthalenyl, pyridinyl, thiophenyl and cyclopropyl being optionally substituted with 1 or 2 substituents **R^{b}**, wherein **R^{b}** is selected from the group consisting of methoxy, methyl and trifluromethyl; and
**L^{b}** is a bond or is -CH₂-.

In one embodiment, the compounds for use of Formula IV are those wherein **A** and **R^{a}**, together are selected from the group consisting of 4-(trifluoromethyl)phenyl, 3-(trifluoromethyl)phenyl, 4-fluorophenyl, phenyl, 4-methylphenyl, 3-methylphenyl, and naphthalen-1-yl.

In one embodiment, the compounds for use of Formula IV are those wherein **B** and **R^{b}**, together are selected from phenyl, 4-methoxyphenyl, 2-methylphenyl, 4-methylphenyl, 4-(trifluoromethyl)phenyl and 3-methylphenyl.

Particularly preferred compounds or use of the invention are those listed in Table 2 hereafter:

**Table 2**

| Compound | Structure | Name |
|---|---|---|
| 1 | 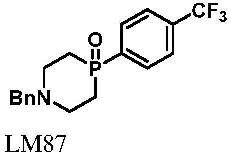 | 1-benzyl-4-(4-(trifluoromethyl)phenyl)-1,4-azaphosphinane 4-oxide |
| 2 | 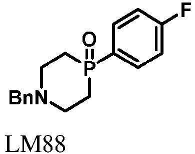 | 1-benzyl-4-(4-fluorophenyl)-1,4-azaphosphinane 4-oxide |
| 3 | 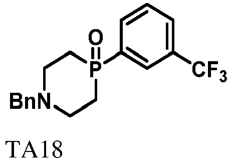 | 1-benzyl-4-(3-(trifluoromethyl)phenyl)-1,4-azaphosphinane 4-oxide |
| 4 | 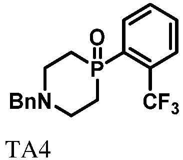 | 1-benzyl-4-(2-(trifluoromethyl)phenyl)-1,4-azaphosphinane 4-oxide |
| 5 | 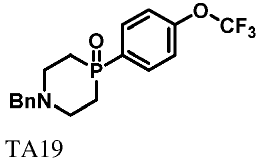 | 1-benzyl-4-(4-(trifluoromethoxy)phenyl)-1,4-azaphosphinane 4-oxide |
| 6 | 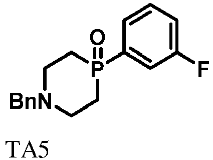 | 1-benzyl-4-(3-fluorophenyl)-1,4-azaphosphinane 4-oxide |
| 7 | 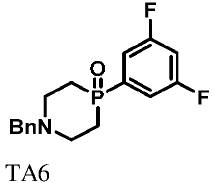 | 1-benzyl-4-(3,5-difluorophenyl)-1,4-azaphosphinane 4-oxide |
| 8 | 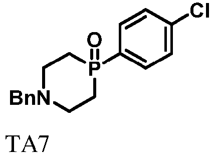 | 1-benzyl-4-(4-chlorophenyl)-1,4-azaphosphinane 4-oxide |
| 9 | 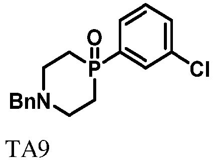 | 1-benzyl-4-(3-chlorophenyl)-1,4-azaphosphinane 4-oxide |
| 10 | 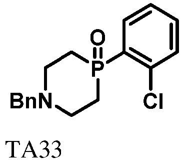 | 1-benzyl-4-(2-chlorophenyl)-1,4-azaphosphinane 4-oxide |
| 11 | 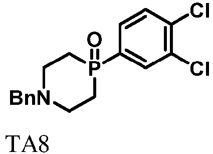 | 1-benzyl-4-(3,4-dichlorophenyl)-1,4-azaphosphinane 4-oxide |
| 12 | 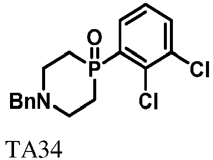 | 1-benzyl-4-(2,3-dichlorophenyl)-1,4-azaphosphinane 4-oxide |
| 13 | 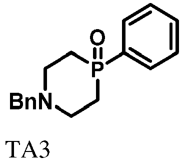 | 1-benzyl-4-phenyl-1,4-azaphosphinane 4-oxide |
| 14 | 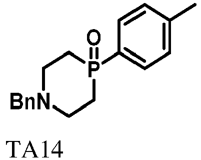 | 1-benzyl-4-(p-tolyl)-1,4-azaphosphinane 4-oxide |
| 15 | 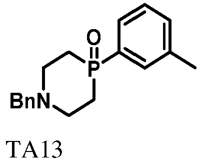 | 1-benzyl-4-(m-tolyl)-1,4-azaphosphinane 4-oxide |
| 16 | 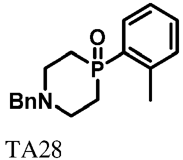 | 1-benzyl-4-(o-tolyl)-1,4-azaphosphinane 4-oxide |
| 17 | 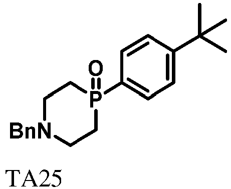 | 1-benzyl-4-(4-(tert-butyl)phenyl)-1,4-azaphosphinane 4-oxide |
| 18 | 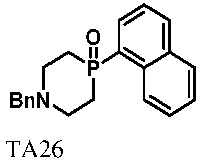 | 1-benzyl-4-(naphthalen-1-yl)-1,4-azaphosphinane 4-oxide |
| 19 | 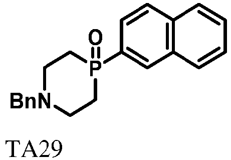 | 1-benzyl-4-(naphthalen-2-yl)-1,4-azaphosphinane 4-oxide |
| 20 | 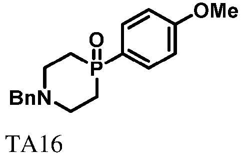 | 1-benzyl-4-(4-methoxyphenyl)-1,4-azaphosphinane 4-oxide |
| 21 | 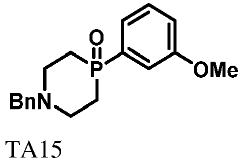 | 1-benzyl-4-(3-methoxyphenyl)-1,4-azaphosphinane 4-oxide |
| 22 | 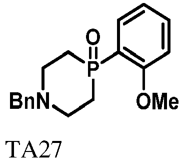 | 1-benzyl-4-(2-methoxyphenyl)-1,4-azaphosphinane 4-oxide |
| 23 | 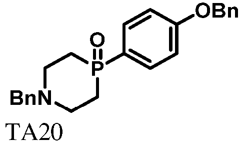 | 1-benzyl-4-(4-(benzyloxy)phenyl)-1,4-azaphosphinane 4-oxide |
| 24 | 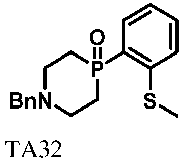 | 1-benzyl-4-(2-(methylthio)phenyl)-1,4-azaphosphinane 4-oxide |
| 25 | 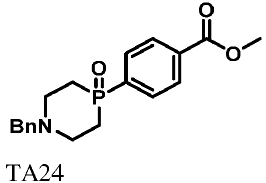 | methyl 4-(1-benzyl-4-oxido-1,4-azaphosphinan-4-yl)benzoate |
| 26 | 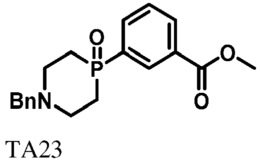 | methyl 3-(1-benzyl-4-oxido-1,4-azaphosphinan-4-yl)benzoate |
| 27 | 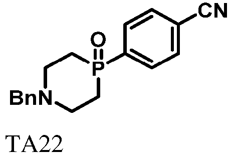 | 4-(1-benzyl-4-oxido-1,4-azaphosphinan-4-yl)benzonitrile |
| 28 | 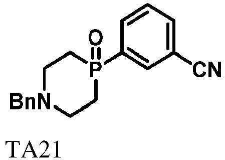 | 3-(1-benzyl-4-oxido-1,4-azaphosphinan-4-yl)benzonitrile |
| 29 | 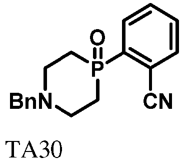 | 2-(1-benzyl-4-oxido-1,4-azaphosphinan-4-yl)benzonitrile |
| 30 | 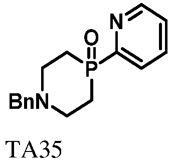 | 1-benzyl-4-(pyridin-2-yl)-1,4-azaphosphinane 4-oxide |
| 31 | 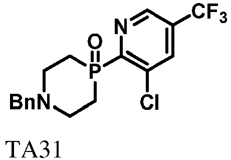 | 1-benzyl-4-(3-chloro-5-(trifluoromethyl)pyridin-2-yl)-1,4-azaphosphinane 4-oxide |
| 32 | 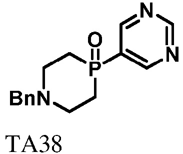 | 1-benzyl-4-(pyrimidin-5-yl)-1,4-azaphosphinane 4-oxide |
| 33 | 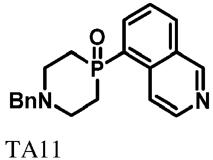 | 1-benzyl-4-(isoquinolin-5-yl)-1,4-azaphosphinane 4-oxide |
| 34 | 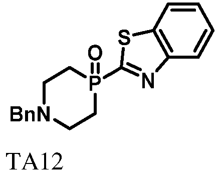 | 4-(benzo[d]thiazol-2-yl)-1-benzyl-1,4-azaphosphinane 4-oxide |
| 35 | 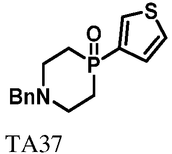 | 1-benzyl-4-(thiophen-3-yl)-1,4-azaphosphinane 4-oxide |
| 36 | 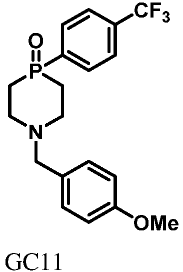 | 1-(4-methoxybenzyl)-4-(4-(trifluoromethyl)phenyl)-1,4-azaphosphinane 4-oxide |
| 37 | 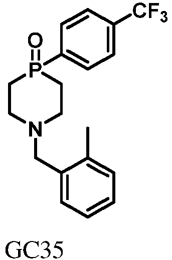 | 1-(2-methylbenzyl)-4-(4-(trifluoromethyl)phenyl)-1,4-azaphosphinane 4-oxide |
| 38 | 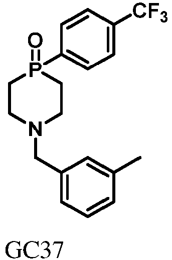 | 1-(3-methylbenzyl)-4-(4-(trifluoromethyl)phenyl)-1,4-azaphosphinane 4-oxide |
| 39 | 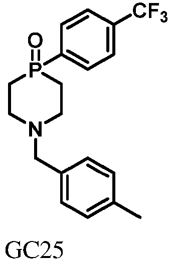 | 1-(4-methylbenzyl)-4-(4-(trifluoromethyl)phenyl)-1,4-azaphosphinane 4-oxide |
| 40 | 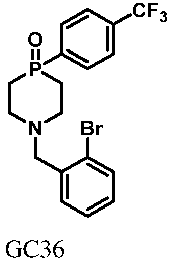 | 1-(2-bromobenzyl)-4-(4-(trifluoromethyl)phenyl)-1,4-azaphosphinane 4-oxide |
| 41 | 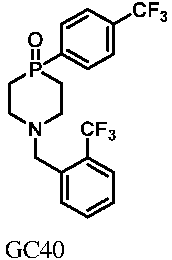 | 1-(2-(trifluoromethyl)benzyl)-4-(4-(trifluoromethyl)phenyl)-1,4-azaphosphinane 4-oxide |
| 42 | 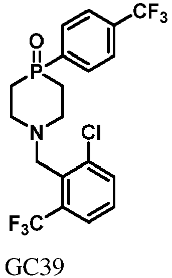 | 1-(2-chloro-6-(trifluoromethyl)benzyl)-4-(4-(trifluoromethyl)phenyl)-1,4-azaphosphinane 4-oxide |
| 43 | 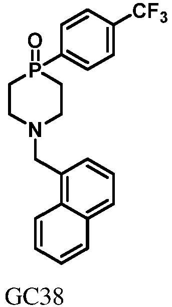 | 1-(naphthalen-1-ylmethyl)-4-(4-(trifluoromethyl)phenyl)-1,4-azaphosphinane 4-oxide |
| 44 | 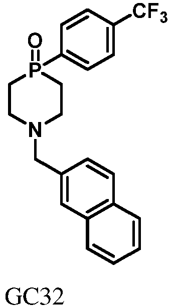 | 1-(naphthalen-2-ylmethyl)-4-(4-(trifluoromethyl)phenyl)-1,4-azaphosphinane 4-oxide |
| 45 | 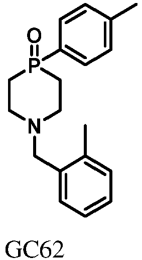 | 1-(2-methylbenzyl)-4-(p-tolyl)-1,4-azaphosphinane 4-oxide |
| 46 | 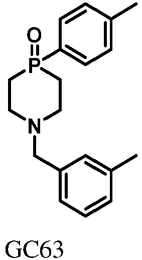 | 1-(3-methylbenzyl)-4-(p-tolyl)-1,4-azaphosphinane 4-oxide |
| 47 | 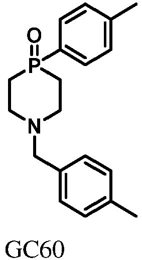 | 1-(4-methylbenzyl)-4-(p-tolyl)-1,4-azaphosphinane 4-oxide |
| 48 | 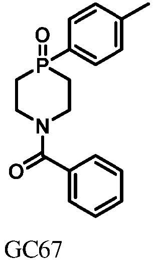 | (4-oxido-4-(*p*-tolyl)-1,4-azaphosphinan-1-yl)(phenyl)methanone |
| 49 | 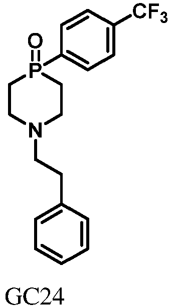 | 1-phenethyl-4-(4-(trifluoromethyl)phenyl)-1,4-azaphosphinane 4-oxide |
| 50 | 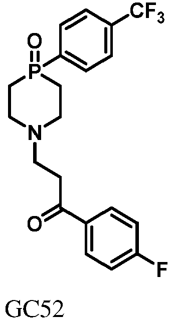 | 1-(4-fluorophenyl)-3-(4-oxido-4-(4-(trifluoromethyl)phenyl)-1,4-azaphosphinan-1-yl)propan-1-one |
| 51 | 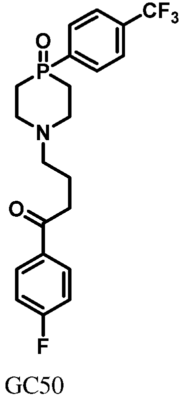 | 1-(4-fluorophenyl)-4-(4-oxido-4-(4-(trifluoromethyl)phenyl)-1,4-azaphosphinan-1-yl)butan-1-one |
| 52 | 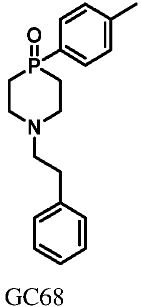 | 1-phenethyl-4-(*p*-tolyl)-1,4-azaphosphinane 4-oxide |
| 53 | 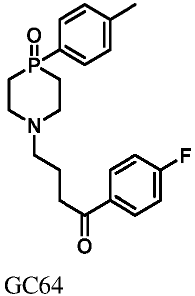 | 1-(4-fluorophenyl)-4-(4-oxido-4-(*p*-tolyl)-1,4-azaphosphinan-1-yl)butan-1-one |
| 54 | 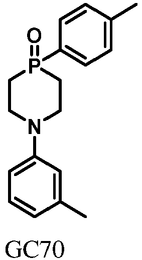 | 1-(*m*-tolyl)-4-(*p*-tolyl)-1,4-azaphosphinane 4-oxide |
| 55 | 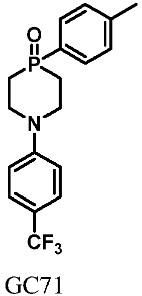 | 4-(*p*-tolyl)-1-(4-(trifluoromethyl)phenyl)-1,4-azaphosphinane 4-oxide |
| 56 | 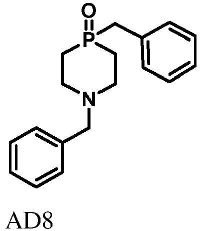 | 1,4-dibenzyl-1,4-azaphosphinane 4-oxide |
| 57 | 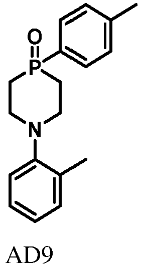 | 1-(*o*-tolyl)-4-(*p*-tolyl)-1,4-azaphosphinane 4-oxide |
| 58 | 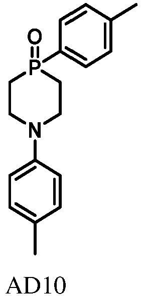 | 1,4-di-*p*-tolyl-1,4-azaphosphinane 4-oxide |
| 59 | 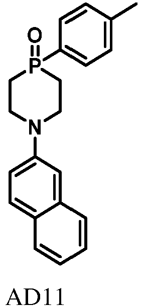 | 1-(naphthalen-2-yl)-4-(*p*-tolyl)-1,4-azaphosphinane 4-oxide |
| 60 | 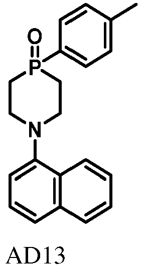 | 1-(naphthalen-1-yl)-4-(*p*-tolyl)-1,4-azaphosphinane 4-oxide |
| 61 | 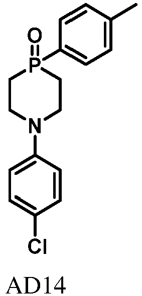 | 1-(4-chlorophenyl)-4-(*p*-tolyl)-1,4-azaphosphinane 4-oxide |
| 62 | 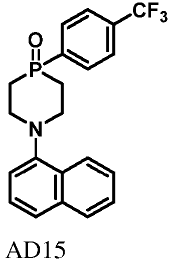 | 1-(naphthalen-1-yl)-4-(4-(trifluoromethyl)phenyl)-1,4-azaphosphinane 4-oxide |
| 63 | 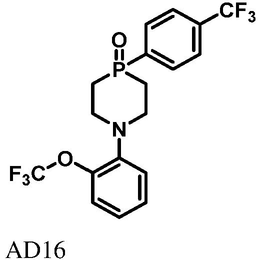 | 1-(2-(trifluoromethoxy)phenyl)-4-(4-(trifluoromethyl)phenyl)-1,4-azaphosphinane 4-oxide |
| 64 | 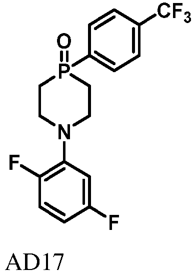 | 1-(2,5-difluorophenyl)-4-(4-(trifluoromethyl)phenyl)-1,4-azaphosphinane 4-oxide |
| 65 | 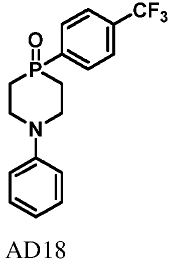 | 1-phenyl-4-(4-(trifluoromethyl)phenyl)-1,4-azaphosphinane 4-oxide |
| 66 | 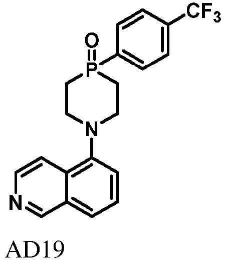 | 1-(isoquinolin-5-yl)-4-(4-(trifluoromethyl)phenyl)-1,4-azaphosphinane 4-oxide |
| 67 | 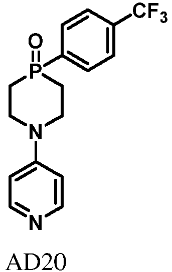 | 1-(pyridin-4-yl)-4-(4-(trifluoromethyl)phenyl)-1,4-azaphosphinane 4-oxide |
| 68 | 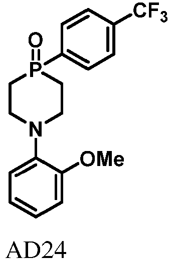 | 1-(2-methoxyphenyl)-4-(4-(trifluoromethyl)phenyl)-1,4-azaphosphinane 4-oxide |
| 69 | 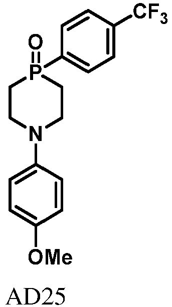 | 1-(4-methoxyphenyl)-4-(4-(trifluoromethyl)phenyl)-1,4-azaphosphinane 4-oxide |
| 70 | 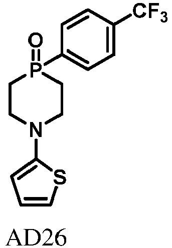 | 1-(thiophen-2-yl)-4-(4-(trifluoromethyl)phenyl)-1,4-azaphosphinane 4-oxide |
| 71 | 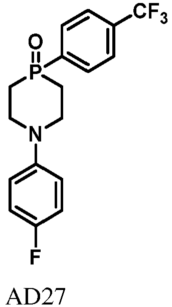 | 1-(4-fluorophenyl)-4-(4-(trifluoromethyl)phenyl)-1,4-azaphosphinane 4-oxide |
| 72 | 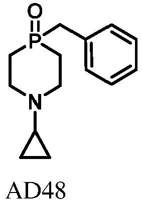 | 4-benzyl-1-cyclopropyl-1,4-azaphosphinane 4-oxide |
| 73 | 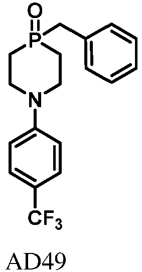 | 4-benzyl-1-(4-(trifluoromethyl)phenyl)-1,4-azaphosphinane 4-oxide |
| 74 | 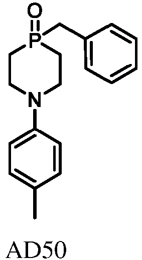 | 4-benzyl-1-(*p*-tolyl)-1,4-azaphosphinane 4-oxide |
| 75 | 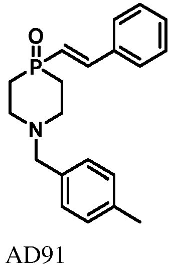 | (E)-1-(4-methylbenzyl)-4-styryl-1,4-azaphosphinane 4-oxide |
| 76 | 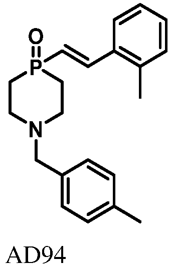 | (E)-1-(4-methylbenzyl)-4-(2-methylstyryl)-1,4-azaphosphinane 4-oxide |
| 77 | 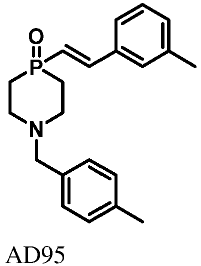 | (E)-1-(4-methylbenzyl)-4-(3-methylstyryl)-1,4-azaphosphinane 4-oxide |
| 78 | 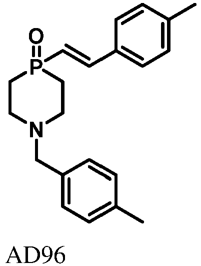 | (E)-1-(4-methylbenzyl)-4-(4-methylstyryl)-1,4-azaphosphinane 4-oxide |
| 79 | 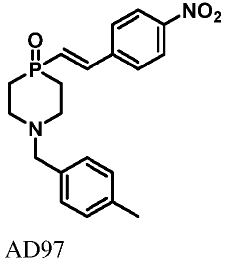 | (E)-1-(4-methylbenzyl)-4-(4-nitrostyryl)-1,4-azaphosphinane 4-oxide |
| 80 | 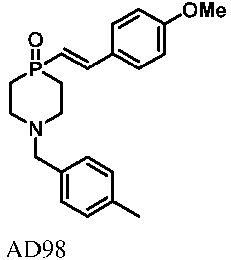 | (E)-4-(4-methoxystyryl)-1-(4-methylbenzyl)-1,4-azaphosphinane 4-oxide |
| 81 | 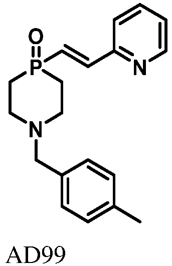 | (E)-1-(4-methylbenzyl)-4-(2-(pyridin-2-yl)vinyl)-1,4-azaphosphinane 4-oxide |
| 82 | 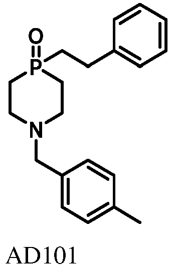 | 1-(4-methylbenzyl)-4-phenethyl-1,4-azaphosphinane 4-oxide |
| 83 | 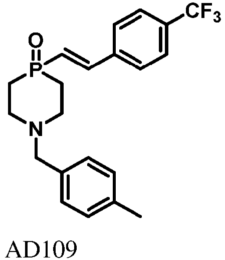 | (E)-1-(4-methylbenzyl)-4-(4-(trifluoromethyl)styryl)-1,4-azaphosphinane 4-oxide |
| 84 | 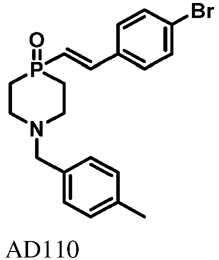 | (E)-4-(4-bromostyryl)-1-(4-methylbenzyl)-1,4-azaphosphinane 4-oxide |
| 85 | 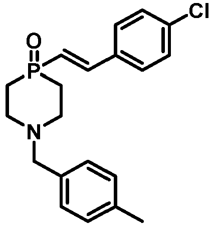 | (E)-4-(4-chlorostyryl)- 1-(4-methylbenzyl)-1,4-azaphosphinane 4-oxide |
| | 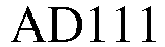 | |
| 86 | 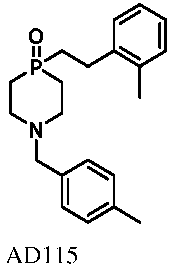 | 1-(4-methylbenzyl)-4-(2-methylphenethyl)-1,4-azaphosphinane 4-oxide |
| 87 | 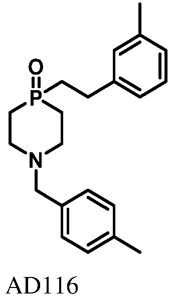 | 1-(4-methylbenzyl)-4-(3-methylphenethyl)-1,4-azaphosphinane 4-oxide |
| 88 | 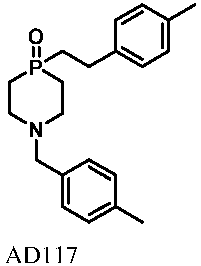 | 1-(4-methylbenzyl)-4-(4-methylphenethyl)-1,4-azaphosphinane 4-oxide |
| 89 | 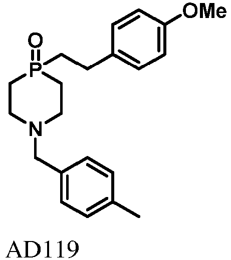 | 4-(4-methoxyphenethyl)-1-(4-methylbenzyl)-1,4-azaphosphinane 4-oxide |
| 90 | 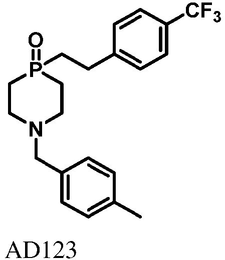 | 1-(4-methylbenzyl)-4-(4-(trifluoromethyl)phenethyl)-1,4-azaphosphinane 4-oxide |
| 91 | 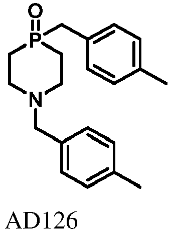 | 1,4-bis(4-methylbenzyl)-1,4-azaphosphinane 4-oxide |
| 92 | 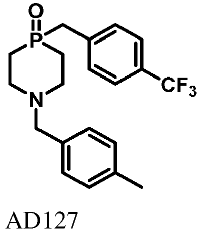 | 1-(4-methylbenzyl)-4-(4-(trifluoromethyl)benzyl)-1,4-azaphosphinane 4-oxide |
| 93 | 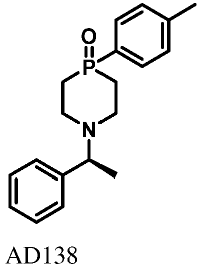 | (S)-1-(1-phenylethyl)-4-(p-tolyl)-1,4-azaphosphinane 4-oxide |
| 94 | 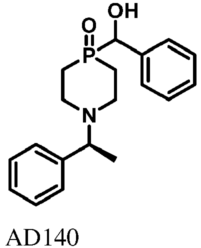 | 4-(hydroxy(phenyl)methyl)-1-((S)-1-phenylethyl)-1,4-azaphosphinane 4-oxide |
| 95 | 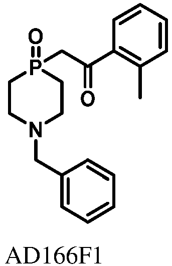 | 2-(1-benzyl-4-oxido-1,4-azaphosphinan-4-yl)-1-(o-tolyl)ethan-1-one |
| 96 | 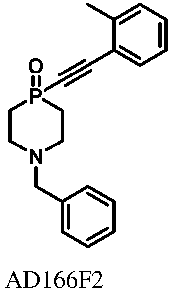 | 1-benzyl-4-(o-tolylethynyl)-1,4-azaphosphinane 4-oxide |
| 97 | 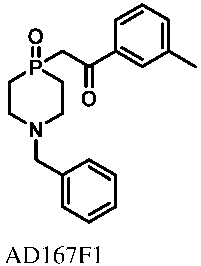 | 2-(1-benzyl-4-oxido-1,4-azaphosphinan-4-yl)-1-(m-tolyl)ethan-1-one |
| 98 | 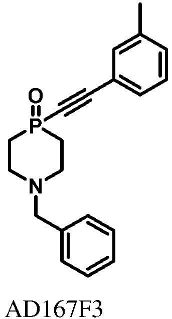 | 1-benzyl-4-(o-tolylethynyl)-1,4-azaphosphinane 4-oxide |
| 99 | 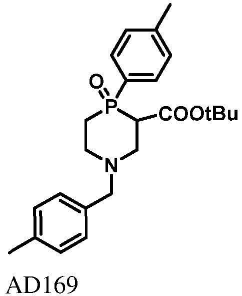 | tert-butyl 1-(4-methylbenzyl)-4-(p-tolyl)-1,4-azaphosphinane-3-carboxylate 4-oxide |
| 100 | 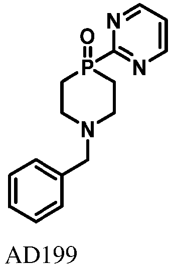 | 1-benzyl-4-(pyrimidin-2-yl)-1,4-azaphosphinane 4-oxide |
| 101 | 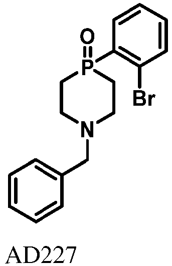 | 1-benzyl-4-(2-bromophenyl)-1,4-azaphosphinane 4-oxide |
| 102 | 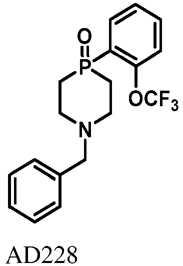 | 1-benzyl-4-(2-(trifluoromethoxy)phenyl)-1,4-azaphosphinane 4-oxide |
| 103 | 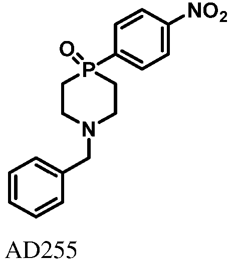 | 1-benzyl-4-(4-nitrophenyl)-1,4-azaphosphinane 4-oxide |
| 104 | 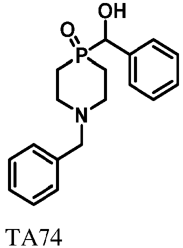 | 1-benzyl-4-(hydroxy(phenyl)methyl)-1,4-azaphosphinane 4-oxide |
| 105 | 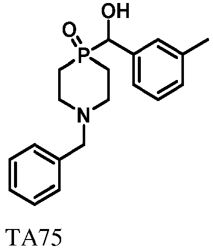 | 1-benzyl-4-(hydroxy(m-tolyl)methyl)-1,4-azaphosphinane 4-oxide |
| 106 | 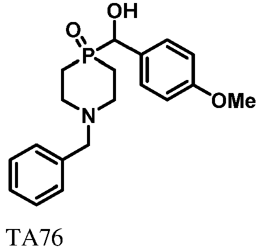 | 1-benzyl-4-(hydroxy(4-methoxyphenyl)methyl)-1,4-azaphosphinane 4-oxide |
| 107 | 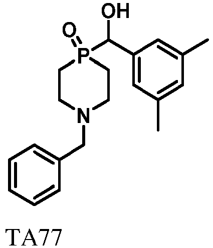 | 1-benzyl-4-((3,5-dimethylphenyl)(hydroxy)methyl)-1,4-azaphosphinane 4-oxide |
| 108 | 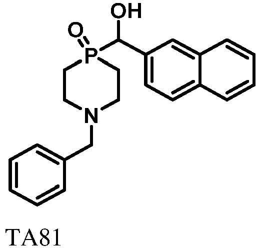 | 1-benzyl-4-(hydroxy(naphthalen-2-yl)methyl)-1,4-azaphosphinane 4-oxide |
| 109 | 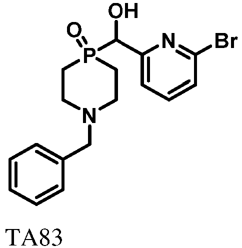 | 1-benzyl-4-((6-bromopyridin-2-yl)(hydroxy)methyl)-1,4-azaphosphinane 4-oxide |
| 110 | 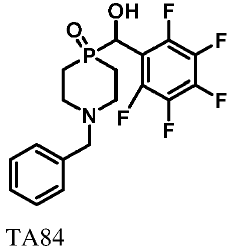 | 1-benzyl-4-(hydroxy(perfluorophenyl)methyl)-1,4-azaphosphinane 4-oxide |
| 111 | 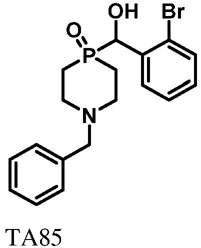 | 1-benzyl-4-((2-bromophenyl)(hydroxy)methyl)-1,4-azaphosphinane 4-oxide |
| 112 | 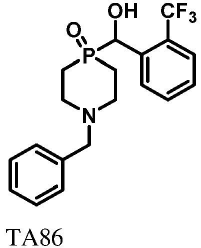 | 1-benzyl-4-(hydroxy(2-(trifluoromethyl)phenyl)methyl)-1,4-azaphosphinane 4-oxide |
| 113 | 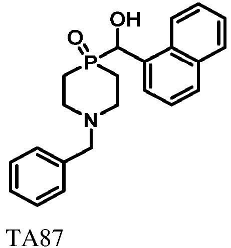 | 1-benzyl-4-(hydroxy(naphthalen-1-yl)methyl)-1,4-azaphosphinane 4-oxide |
| 114 | 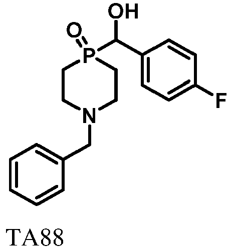 | 1-benzyl-4-((4-fluorophenyl)(hydroxy)methyl)-1,4-azaphosphinane 4-oxide |
| 115 | 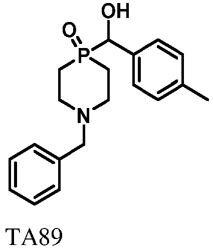 | 1-benzyl-4-(hydroxy(p-tolyl)methyl)-1,4-azaphosphinane 4-oxide |
| 116 | 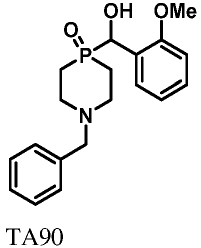 | 1-benzyl-4-(hydroxy(2-methoxyphenyl)methyl)-1,4-azaphosphinane 4-oxide |
| 117 | 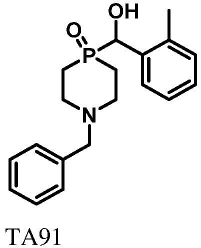 | 1-benzyl-4-(hydroxy(o-tolyl)methyl)-1,4-azaphosphinane 4-oxide |
| 118 | 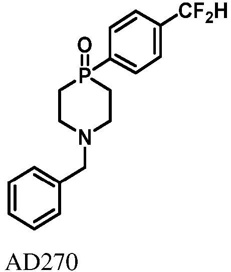 | 1-benzyl-4-(4-(difluoromethyl)phenyl)-1,4-azaphosphinane 4-oxide |
| 119 | 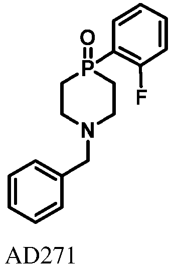 | 1-benzyl-4-(2-fluorophenyl)-1,4-azaphosphinane 4-oxide |
| 120 | 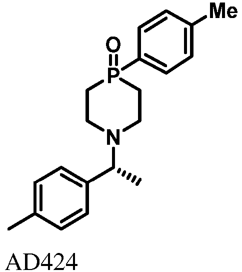 | (R)-4-(p-tolyl)-1-(1-(p-tolyl)ethyl)-1,4-azaphosphinane 4-oxide |
| 121 | 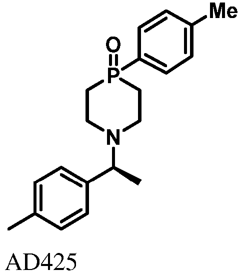 | (S)-4-(p-tolyl)-1-(1-(p-tolyl)ethyl)-1,4-azaphosphinane 4-oxide |

In one embodiment, the compounds for use of the invention, or pharmaceutically acceptable salts thereof, are selected from the group consisting of Compound 1 (LM87), Compound 2 (LM88), Compound 3 (TA18), Compound 13 (TA3), Compound 14 (TA14), Compound 15 (TA13), Compound 18 (TA26), Compound 36 (GC11), Compound 37 (GC35), Compound 39 (GC25), Compound 46 (GC63), Compound 47 (GC 60), Compound 49 (GC24), Compound 50 (GC52), Compound 52 (GC68), Compound 54 (GC70), Compound 56 (AD8), Compound 57 (AD9), Compound 61 (AD14), Compound 65 (AD18, Compound 65 (AD18), Compound 67 (AD20), Compound 72 (AD48), Compound 91 (AD126), Compound 93 (AD138), Compound 94 (AD140), Compound 104 (TA74), Compound 105 (TA75), Compound 120 (AD424) and Compound 121 (AD425). In particular, the compounds for use of the invention, or pharmaceutically acceptable salts thereof, are selected from the group consisting of Compound 3 (TA18), Compound 14 (TA14), Compound 47 (GC 60), Compound 54 (GC70), Compound 120 (AD424) and Compound 121.

In certain embodiments, the compounds for use of the invention, and/or that are comprised in a pharmaceutical composition of the invention, exclude one or more of the compounds of formula (I) when **E** is O and subject to any of the following provisos and:
(a) when both of **L^{a}** and **L^{b}** is a bond and **A** is either unsubstituted phenyl or 4-fluorophenyl, then **B** is not 2,6-difluoro-4-nitrophenyl or 2,6-difluoro-4-aminophenyl; and/or
(b) when **L^{a}** is a bond, **L^{b}** is -(CH₂)- and **B** is unsubstituted phenyl, then **A** is not unsubstituted phenyl, 4-bromophenyl, 4-fluorophenyl, 4-(trifluoromethyl)phenyl, 4-nitrophenyl or unsubstituted cyclopropanyl; and/or
(c) when **L^{a}** is a bond, **L^{b}** is -(CH₂)- and **A** is 4-fluorophenyl, then **B** is not 4-methoxyphenyl; and/or
(d) when **L^{a}** is -CH₂-, **L^{b}** is -(CH₂)- and **B** is unsubstituted phenyl, then **A** is not 4-fluorophenyl or unsubstituted cyclopropanyl; and/or
(e) when **L^{a}** is a bond, **L^{b}** is -(CH₂)- and **B** is 2,4-di-methoxyphenyl, then **A** is not 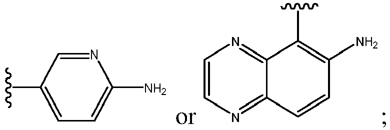 and/or
(f) when **L^{a}** is a bond and **A** is unsubstituted phenyl, then **L^{b}-B** is not -(CH₂)₃-(1-isoquinoline), -(CH₂)₂-(4-carboxyphenyl), -(CH₂)-(4-methylbenzoate) or 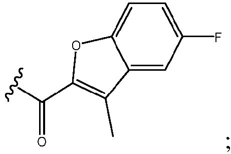 and/or
when **L^{a}** is a bond and **A** is 3-methoxy-4-aminophenyl or 3-methoxy-4-nitrophenyl, then **L^{b}**-**B** is not unsubstituted phenyl, unsubstituted cyclopropanyl or -(CO)-cyclopropanyl.

The compounds for use of the invention can be prepared by different ways with reactions known by the person skilled in the art. Reaction schemes as described in the example section illustrate by way of example different possible approaches.

As detailed above, the compounds for use of the invention are, in certain embodiments, ligands of sigma-1 receptor. In other words, such compounds for use of the invention have the ability to bind to S1R.

In particular, the compounds of the invention are agonists, or positive modulators, of S1R, thus increasing MAM functioning, as shown by differently affecting a biological response, in particular in a zebrafish model of Wolfram syndrome, between wfs1ab^{KO} larvae and wfs1^{WT} larvae. Compounds of the invention are surprisingly shown to have: (i) direct S1R activity, for example by having the ability to bind to S1R, in particular an activity as direct activators, more particularly agonists or positive modulators, of S1R, or (ii) to have a different mechanism of modulating, in particular increasing, MAM functioning because they can modulate, in particular activate, another MAM chaperone or another protein acting as an upstream regulator of S1R or another MAM chaperone.

The compounds for use of the invention are therefore useful in the treatment and/or prevention of a disease, disorder or condition that is associated with, caused by or a symptom of a mutation in WFS1 or WFS2 (CISD2) gene, in particular Wolfram syndrome, Wolfram-like syndrome, or disease, disorder or condition associated therewith, caused thereby or a symptom thereof, associated with mitochondrial dysfunction, and/or is treatable, ameliorable or preventable by activation or inhibition of S1R. In particular, the compounds for use of the invention are useful in the treatment and/or prevention of one or more neurodegenerative diseases, disorders or conditions.

In one embodiment, the compound for use of the invention is a ligand of sigma-1 receptor.

In one embodiment, the compound for use of the invention is an agonist of sigma-1 receptor.

In one embodiment, there is provided a compound of the invention, or a pharmaceutically acceptable salt thereof, for use in treating and/or preventing a disease, disorder or condition that is associated with, caused by or a symptom of a mutation in WFS1 or WFS2 (CISD2) gene.

Diseases, disorders or conditions that are associated with a mutation in WFS1 or WFS2 (CISD2) gene include within the context of the invention Wolfram syndrome or Wolfram-like syndrome, in particular Wolfram syndrome.

In one embodiment, there is provided a compound of the invention, or a pharmaceutically acceptable salt thereof, for use in treating and/or preventing a disease, disorder or condition that is associated with, caused by or a symptom of: (a) a mutation in WFS 1 or WFS2 (CISD2) gene, and/or (b) Wolfram syndrome or Wolfram-like syndrome, in particular Wolfram syndrome. In particular of such embodiments, such disease, disorder or condition is exhibited in an individual: (a) suffering from Wolfram syndrome or Wolfram-like syndrome; or (b) possessing a mutation in WFS1 or WFS2 (CISD2).

Diseases, disorders or conditions associated with, caused by or a symptom of: (a) a mutation in WFS1 or WFS2 (CISD2) gene include within the context of the invention Wolfram syndrome, Wolfram-like syndrome, diabetes insipidus (DI), diabetes mellitus (DM), an optic atrophy (OA), deafness, urinary disorders, pons atrophy, ataxia, and psychiatric disorders, including bipolar disorder, schizophrenia, suicide, or mania, in particular diabetes insipidus (DI), diabetes mellitus (DM), an optic atrophy (OA), and deafness.

In one embodiment, there is provided a compound of the invention, or a pharmaceutically acceptable salt thereof, for use in treating and/or preventing a disease, disorder or condition that is exhibited in an individual with Wolfram syndrome or Wolfram-like syndrome, in particular Wolfram syndrome. More particularly, the disease, disorder or condition exhibited in an individual with Wolfram syndrome or Wolfram-like syndrome is selected from the group consisting of diabetes insipidus (DI), diabetes mellitus (DM), an optic atrophy (OA) and deafness.

In one embodiment, there is provided a compound of the invention, or a pharmaceutically acceptable salt thereof, for use in treating and/or preventing a neurodegenerative disease, disorder or condition. In particular, the neurodegenerative disease, disorder or condition is selected from the group consisting of Alzheimer's disease (AD), frontotemporal dementia (FTD), Lewy body dementia (LBD), Parkinson's disease (PD), Huntington disease (HD), amyotrophic lateral sclerosis (ALS), multiple sclerosis (MS) and Charcot-Marie-Tooth 2A disease (CMT2A).

In one embodiment, there is provided a compound of the invention, or a pharmaceutically acceptable salt thereof, for use in treating and/or preventing a disease, disorder or condition selected from the group consisting of stroke, stroke-induced neuronal damage, depression, anxiety, addiction, epilepsy, multiple sclerosis, pain, neuropathic pain and cancer.

In one embodiment, there is provided a compound of the invention, or a pharmaceutically acceptable salt thereof, for use in treating and/or preventing a disease, disorder or condition that is associated with a mitochondrial dysfunction, endoplasmic reticulum (ER) stress, or disfunction of nucleocytoplasmic transport. In particular, the disease, disorder or condition that is associated with a mitochondrial dysfunction, endoplasmic reticulum (ER) stress, or disfunction of nucleocytoplasmic transport is selected from the list consisting of: (a) dysfunctional alterations in mitochondrial-associated endoplasmic reticulum (ER) membranes (MAMs); (b) dysregulation of calcium ion transfer between ER and mitochondria. In particular, the disease, disorder or condition that is associated with a mitochondrial dysfunction is selected from the group consisting of a mood disorder, a mitochondrial myopathy, a lysosomal storage disease, frontotemporal dementia (FTD), Charcot-Marie-Tooth 2A disease (CMT2A), stroke, hypoxia/ischemia, neonatal anoxia, spinal cord injuries, multiple sclerosis, glaucoma, diabetes mellitus, cardiomyopathy, ototoxicity and acoustic trauma.

In particular, said mitochondrial myopathy comprises, MELAS syndrome, MERRF syndrome, Leigh Disease, Chronic Progressive External Ophthalmoplegia (C/PEO), Diabetes mellitus and deafness (MIDD or DAD, Kearns-Sayre syndrome (KSS), Alpers Syndrome, Mitochondrial DNA depletion syndrome (MDS), Mitochondrial neurogastrointestinal encephalomyopathy (MNGIE), Neuropathy, ataxia and retinitis pigmentosa (NARP), Pearson syndrome, Lebers Hereditary Optic Neuropathy (LHON), Dominant Optic Atrophy (DOA), Pigmentary retinopathy, Wolfram Syndrome, Friedrich's Ataxia (FRDA), Mitochondrial neurogastrointestinal encephalomyopathy (MNGIE), or a combination thereof.

In particular, said lysosomal storage disease comprises Glycogenosis Type II (Pompe Disease), Multiple Sulphatase Deficiency (MSD), Mucopolysaccharidoses (MPS), Mucolipidoses (ML) Types I-III, G(M1)-Gangliosidosis, Fabry Disease, Farber Disease, Gaucher Disease, Niemann-Pick Disease, Mucolipidoses (ML) Type IV, Cystinosis, Neuronal Ceroid-Lipofuscinoses, or a combination thereof.

In one embodiment, there is provided a compound of the invention, or a pharmaceutically acceptable salt thereof, for use in treating and/or preventing a disease, disorder or condition treatable, ameliorable or preventable by activation or inhibition of S1R. In particular, the disease, disorder or condition treatable, ameliorable or preventable by activation or inhibition of S1R is selected from the list consisting of Alzheimer's disease (AD), amyotrophic lateral sclerosis (ALS), Parkinson's disease (PD), Wolfram syndrome, Huntington disease (HD), Rett Syndrome and Dravet Syndrome.

In one embodiment, there is provided a compound of the invention, or a pharmaceutically acceptable salt thereof, for use in treating and/or preventing a disease, disorder or condition selected from the group consisting of Wolfram syndrome, diabetes insipidus (DI), diabetes mellitus (DM), an optic atrophy (OA), deafness, urinary disorders, pons atrophy, ataxia, psychiatric disorders, including bipolar disorder, schizophrenia, suicide, mania, Alzheimer's disease (AD), frontotemporal dementia (FTD), Lewy body dementia (LBD), Parkinson's disease (PD), Huntington disease (HD), amyotrophic lateral sclerosis (ALS), stroke, stroke-induced neuronal damage, depression, anxiety, addiction, epilepsy, ataxia, multiple sclerosis (MS), pain, neuropathic pain, cancer, Charcot-Marie-Tooth 2A disease (CMT2A), a mood disorder, a mitochondrial myopathy, a lysosomal storage disease, hypoxia, ischemia, neonatal anoxia, spinal cord injuries, glaucoma, cardiomyopathy, ototoxicity and acoustic trauma, Rett Syndrome and Dravet Syndrome. In particular, the disease, disorder or condition is selected from the group consisting of Wolfram syndrome, diabetes insipidus (DI), diabetes mellitus (DM), an optic atrophy (OA), deafness, Alzheimer's disease (AD), frontotemporal dementia (FTD), Lewy body dementia (LBD), Parkinson's disease (PD), Huntington disease (HD), amyotrophic lateral sclerosis (ALS) stroke, depression, anxiety, addiction, epilepsy, multiple sclerosis (MS), pain, cancer and Charcot-Marie-Tooth 2A disease (CMT2A). More particularly, the disease, disorder or condition is selected from the group consisting of Wolfram syndrome, Alzheimer's disease (AD), frontotemporal dementia (FTD), Lewy body dementia (LBD), Parkinson's disease (PD), Huntington disease (HD), amyotrophic lateral sclerosis (ALS), multiple sclerosis (MS) and Charcot-Marie-Tooth 2A disease (CMT2A). Still more particularly, the disease, disorder or condition is Wolfram syndrome or Charcot-Marie-Tooth 2A disease (CMT2A). Even more particularly, the disease, disorder or condition is Wolfram syndrome.

In other terms, the invention also relates to a method of treating and/or preventing disease, disorder or condition that is associated with a mutation in WFS1 or WFS2 (CISD2) gene, in particular Wolfram syndrome, Wolfram-like syndrome, or disease, disorder or condition associated therewith, caused thereby or a symptom thereof, associated with mitochondrial dysfunction, and/or is treatable, ameliorable or preventable by activation or inhibition of S1R, comprising the administration of a therapeutically effective amount of a compound of the invention, or a pharmaceutically acceptable salt, to a patient in need thereof. Preferably the patient is a warm-blooded animal, more preferably a human. The diseases, disorders or conditions that are associated with, caused by or a symptom of a mutation in WFS1 or WFS2 (CISD2) gene are preferably those defined above. The diseases, disorders or conditions that are associated with mitochondrial dysfunction are preferably those defined above. The diseases, disorders or conditions that are treatable, ameliorable or preventable by activation or inhibition of S1R are preferably those defined above.

The invention further provides the use of a compound of the invention, or a pharmaceutically acceptable salt thereof, for the manufacture of a medicament for use in treating and/or preventing a disease, disorder or condition that is associated with caused by or a symptom of a mutation in WFS1 or WFS2 (CISD2) gene, in particular Wolfram syndrome, Wolfram-like syndrome, or disease, disorder or condition associated therewith, caused thereby or a symptom thereof, associated with mitochondrial dysfunction, and/or targeted/addressed/ameliorated by activation or inhibition of S1R. Preferably the patient is a warm-blooded animal, more preferably a human. The diseases, disorders or conditions that are associated with, caused by or a symptom of a mutation in WFS1 or WFS2 (CISD2) gene are preferably those defined above. The diseases, disorders or conditions that are associated with mitochondrial dysfunction are preferably those defined above. The diseases, disorders or conditions that are treatable, ameliorable or preventable by activation or inhibition of S1R are preferably those defined above.

According to a further feature of the present invention, there is provided a compound of the invention, or a pharmaceutically acceptable salt thereof, for use in modulating MAM functioning, in particular modulating S1R activity, in a patient in need of such treatment, comprising administering to said patient an effective amount of a compound of the present invention, a pharmaceutically acceptable salt thereof, or a pharmaceutical composition comprising such compound or pharmaceutically acceptable salt. In other terms, the invention also provides a method for modulating MAM functioning, in particular modulating S1R activity, in a patient in need of such treatment, which comprises administering to said patient an effective amount of a compound of the present invention, a pharmaceutically acceptable salt thereof, or a pharmaceutical composition comprising such compound or pharmaceutically acceptable salt. The invention further provides the use of a compound of the invention, or a pharmaceutically acceptable salt thereof, for the manufacture of a medicament for use in modulating MAM functioning, in particular modulating S1R activity, in a patient in need of such treatment. Preferably, the patient is a warm blooded animal, more preferably a human.

According to one embodiment, the compounds of the invention, or their pharmaceutical acceptable salts thereof, may be administered as part of a combination therapy. Thus, are included within the scope of the present invention embodiments comprising coadministration of, and compositions and medicaments which contain, in addition to a compound of the present invention, or a pharmaceutically acceptable salt thereof, as active ingredient, additional therapeutic agents and/or active ingredients. Such multiple drug regimens, often referred to as combination therapy, may be used in the treatment and/or prevention of any S1R related disease or disorder, particularly those defined above.

Thus, the methods of treatment and pharmaceutical compositions of the present invention may employ the compounds of the invention or their pharmaceutical acceptable salts thereof in the form of monotherapy, but said methods and compositions may also be used in the form of multiple therapy in which one or more compounds of Formula I, or their pharmaceutically acceptable salts, are coadministered in combination with one or more other therapeutic agents.

The invention also provides pharmaceutical compositions comprising a compound of the invention or a pharmaceutically acceptable salt thereof and at least one pharmaceutically acceptable carrier, diluent, excipient and/or adjuvant. As indicated above, the invention also covers pharmaceutical compositions which contain, in addition to a compound of the present invention, a pharmaceutically acceptable salt thereof as active ingredient, additional therapeutic agents and/or active ingredients.

Another object of this invention is a medicament comprising at least one compound of the invention, or a pharmaceutically acceptable salt thereof, as active ingredient.

Generally, for pharmaceutical use, the compounds of the invention may be formulated as a pharmaceutical preparation comprising at least one compound of the invention and at least one pharmaceutically acceptable carrier, diluent, excipient and/or adjuvant, and optionally one or more further pharmaceutically active compounds.

By means of non-limiting examples, such a formulation may be in a form suitable for oral administration, for parenteral administration (such as by intravenous, intramuscular or subcutaneous injection or intravenous infusion), for topical administration (including ocular), cerebral administration, for administration by inhalation, by a skin patch, by an implant, by a suppository, etc. Such suitable administration forms - which may be solid, semi-solid or liquid, depending on the manner of administration - as well as methods and carriers, diluents and excipients for use in the preparation thereof, will be clear to the skilled person; reference is made to the latest edition of Remington's Pharmaceutical Sciences.

For example, the compound of the invention or a pharmaceutical composition comprising a compound of the invention can be administered orally in the form of tablets, coated tablets, pills, capsules, soft gelatin capsules, oral powders, granules, ovules, elixirs, solutions or suspensions, which may contain flavouring or colouring agents, for immediate-, delayed-, modified-, sustained-, pulsed- or controlled-release applications.

The tablets may contain excipients such as microcrystalline cellulose, lactose, sodium citrate, calcium carbonate, dibasic calcium phosphate and glycine, a disintegrant such as starch (preferably corn, potato or tapioca starch), sodium starch glycollate, croscarmellose sodium and certain complex silicates, a binder such as polyvinylpyrrolidone, hydroxypropylmethylcellulose (HPMC), hydroxypropylcellulose (HPC), sucrose, gelatin and acacia, a lubricant such as magnesium stearate, stearic acid, glyceryl behenate. Solid compositions of a similar type may also be employed as fillers in hard gelatin capsules. Preferred excipients in this regard include lactose, saccharose, sorbitol, mannitol, potato starch, corn starch, amylopectin, cellulose derivatives or gelatin. Hard gelatin capsules may contain granules of the compound of the invention.

Soft gelatin capsules may be prepared with capsules containing the compound of the invention, vegetable oil, waxes, fat, or other suitable vehicle for soft gelatin capsules. As an example, the acceptable vehicle can be an oleaginous vehicle, such as a long chain triglyceride vegetable oil (e.g. corn oil).

Dispersible powders and granules suitable for preparation of an aqueous suspension by the addition of water may contain the active ingredient in a mixture with dispersing agents, wetting agents, and suspending agents and one or more preservatives. Additional excipients, for example sweetening, flavouring and colouring agents, may also be present. These compositions may be preserved by the addition of an anti-oxidant such as ascorbic acid.

Liquid dosage forms for oral administration may include pharmaceutically acceptable, solutions, emulsions, suspensions, syrups, and elixirs containing inert diluents commonly used in the art, such as water or an oleaginous vehicle. Liquid dosage form may be presented as a dry product for constitution with water or other suitable vehicle before use. Such compositions may also comprise adjuvants, such as wetting agents, emulsifying and suspending agents, complexing agents such as 2-hydroxypropyl-beta-cyclodextrin, sulfobutylether-beta-cylodextrin, and sweetening, flavouring, perfuming agents, colouring matter or dyes with diluents such as water, ethanol, propylene glycol and glycerin, and combinations thereof. These compositions may be preserved by the addition of an anti-oxidant such as butylated hydroxyanisol or alpha-tocopherol.

Finely divided powder of the compound of the invention may be prepared for example by micronisation or by processes known in the art. The compound of the invention may be milled using known milling procedures such as wet milling to obtain a particle size appropriate for tablet formation and for other formulation types.

If the compound of the present invention is administered parenterally, then examples of such administration include one or more of: intravenously, intraarterially, intraperitoneally, intrathecally, intraventricularly, intraurethrally, intrasternally, intracranially, intramuscularly or subcutaneously administering the agent; and/or by using infusion techniques.

The compound of the invention can be administered via the parenteral route with a readily available or a depot-type formulation.

The pharmaceutical compositions for the parenteral administration of a readily available formulation may be in the form of a sterile injectable aqueous or oleagenous solution or suspension in a non-toxic parenterally-acceptable diluent or solvent and may contain formulatory agents such as suspending, stabilising dispersing, wetting and/or complexing agents such as cyclodextrin e.g. 2-hydroxypropyl-beta-cyclodextrin, sulfobutylether-beta-cylodextrin.

The depot-type formulation for the parenteral administration may be prepared by conventional techniques with pharmaceutically acceptable excipient including without being limited to, biocompatible and biodegradable polymers (e.g. poly(β-caprolactone), poly(ethylene oxide), poly(glycolic acid), poly[(lactic acid)-co-(glycolic acid)...)], poly(lactic acid)...), non-biodegradable polymers (e.g. ethylene vinylacetate copolymer, polyurethane, polyester(amide), polyvinyl chloride...) aqueous and non-aqueous vehicles (e.g. water, sesame oil, cottonseed oil, soybean oil, castor oil, almond oil, oily esters, ethyl alcohol or fractionated vegetable oils, propylene glycol, DMSO, THF, 2-pyrrolidone, N-methylpyrrolidinone, N-vinylpyrrolidinone... ).

Alternatively, the active ingredient may be in dry form such as a powder, crystalline or freeze-dried solid for constitution with a suitable vehicle. The preparation of suitable parenteral formulations under sterile conditions is readily accomplished by standard pharmaceutical techniques well known to those skilled in the art.

As indicated, the compound of the present invention can be administered intranasally or by inhalation and is conveniently delivered in the form of a dry powder inhaler or an aerosol spray presentation from a pressurised container, pump, spray or nebuliser with the use of a suitable propellant, e.g. dichlorodifluoromethane, trichlorofluoromethane, dichlorotetrafluoroethane, (for example from Ineos Fluor), carbon dioxide or other suitable gas. In the case of a pressurised aerosol, the dosage unit may be determined by providing a valve to deliver a metered amount. The pressurised container, pump, spray or nebuliser may contain a solution or suspension of the active compound. Capsules and cartridges (made, for example, from gelatin) for use in an inhaler or insufflator may be formulated to contain a powder mix of the compound and a suitable powder base such as lactose or starch. For compositions suitable and/or adapted for inhaled administration, it is preferred that the compound or salt of formula I is in a particle-size-reduced form, and more preferably the size-reduced form is obtained or obtainable by micronisation. The preferable particle size of the size-reduced (e.g. micronised) compound or salt is defined by a D50 value of about 0.5 to about 50 microns (for example as measured using laser diffraction).

Alternatively, the compound of the present invention can be administered in the form of a suppository or pessary, or it may be applied topically in the form of a gel, hydrogel, lotion, solution, cream, ointment or dusting powder. The compound of the present invention may also be dermally or transdermally administered, for example, by the use of a skin patch. They may also be administered by the pulmonary or rectal routes. It may also be administered by the ocular route. For ophthalmic use, the compound can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, it may be formulated in an ointment such as petrolatum.

For topical application to the skin, the agent of the present invention can be formulated as a suitable ointment containing the active compound suspended or dissolved in, for example, a mixture with one or more of the following: mineral oil, liquid petrolatum, white petrolatum, propylene glycol, polyoxyethylene, polyoxypropylene compound, emulsifying wax and water. Alternatively, it can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

### DEFINITIONS

The definitions and explanations below are for the terms as used throughout the entire application, including both the specification and the claims.

Unless otherwise stated, any reference to compounds of the invention herein, means the compounds as such as well as their pharmaceutically acceptable salts thereof.

When describing the compounds of the invention, the terms used are to be construed in accordance with the following definitions, unless indicated otherwise.

The term "halo" or "halogen" means fluoro, chloro, bromo, or iodo. Preferred halo groups are fluoro and chloro, fluoro being particularly preferred.

The term "alkyl" by itself or as part of another substituent refers to a hydrocarbyl radical of Formula CₙH₂ₙ₊₁ wherein n is a number greater than or equal to 1.

The term "haloalkyl" alone or in combination, refers to an alkyl radical having the meaning as defined above wherein one or more hydrogens are replaced with a halogen as defined above. Non-limiting examples of such haloalkyl radicals include chloromethyl, 1-bromoethyl, fluoromethyl, difluoromethyl, trifluoromethyl, 1,1,1-trifluoroethyl and the like. A preferred haloalkyl radical is trifluoromethyl.

The term "cycloalkyl" as used herein is a monovalent, saturated, or unsaturated monocyclic or bicyclic hydrocarbyl group. Cycloalkyl groups may comprise 3 or more carbon atoms in the ring and generally, according to this invention comprise from 3 to 10, more preferably from 3 to 8 carbon atoms still more preferably from 3 to 6 carbon atoms. Examples of cycloalkyl groups include but are not limited to cyclopropyl, cyclobutyl, cyclopentyl, and cyclohexyl.

The terms "heterocyclyl", "heterocycloalkyl" or "heterocyclo" as used herein by itself or as part of another group refer to non-aromatic, fully saturated or partially unsaturated cyclic groups (for example, 3 to 7 member monocyclic, 7 to 11 member bicyclic, or containing a total of 3 to 10 ring atoms) which have at least one heteroatom in at least one carbon atom-containing ring. Each ring of the heterocyclic group containing a heteroatom may have 1, 2, 3 or 4 heteroatoms selected from nitrogen, oxygen and/or sulfur atoms, where the nitrogen and sulfur heteroatoms may optionally be oxidized and the nitrogen heteroatoms may optionally be quaternized. The heterocyclic group may be attached at any heteroatom or carbon atom of the ring or ring system, where valence allows.

The term "aryl" as used herein refers to a polyunsaturated, aromatic hydrocarbyl group having a single ring (*i.e.* phenyl) or multiple aromatic rings fused together (e.g. naphthyl), typically containing 5 to 12 atoms; preferably 6 to 10, wherein at least one ring is aromatic.

The term "heteroaryl" as used herein by itself or as part of another group refers but is not limited to 5 to 12 carbon-atom aromatic rings or ring systems containing 1 to 2 rings which are fused together, typically containing 5 to 6 atoms; at least one of which is aromatic, in which one or more carbon atoms in one or more of these rings is replaced by oxygen, nitrogen and/or sulfur atoms where the nitrogen and sulfur heteroatoms may optionally be oxidized and the nitrogen heteroatoms may optionally be quaternized.

The compounds of the invention containing a basic functional group may be in the form of pharmaceutically acceptable salts. Pharmaceutically acceptable salts of the compounds of the invention containing one or more basic functional groups include in particular the acid addition salts thereof. Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

Pharmaceutically acceptable salts of compounds of Formulae I and subformulae may for example be prepared as follows:
(i) reacting the compound of Formula I or any of its subformulae with the desired acid; or
(ii) converting one salt of the compound of Formula I or any of its subformulae to another by reaction with an appropriate acid or by means of a suitable ion exchange column.

All these reactions are typically carried out in solution. The salt may precipitate from solution and be collected by filtration or may be recovered by evaporation of the solvent.

The degree of ionization in the salt may vary from completely ionized to almost non-ionized.

The compounds of the invention include compounds of the invention as hereinbefore defined, including solvates thereof, all polymorphs and crystalline forms thereof, prodrugs and isomers thereof (including optical, geometric and tautomeric isomers) and isotopically labeled compounds of the invention.

In addition, although generally, with respect to the salts of the compounds of the invention, pharmaceutically acceptable salts are preferred, it should be noted that the invention in its broadest sense also includes non-pharmaceutically acceptable salts, which may for example be used in the isolation and/or purification of the compounds of the invention. For example, salts formed with optically active acids or bases may be used to form diastereoisomeric salts that can facilitate the separation of optically active isomers of the compounds of the invention.

The term "patient" refers to a warm-blooded animal, more preferably a human, who/which is awaiting or receiving medical care or is or will be the object of a medical procedure.

The term "human" refers to subjects of both genders and at any stage of development (*i.e.* neonate, infant, juvenile, adolescent, adult). In one embodiment, the human is an adolescent or adult, preferably an adult.

The terms "treat", "treating" and "treatment, as used herein, are meant to include alleviating or abrogating a condition or disease and/or its attendant symptoms.

The terms "prevent", "preventing" and "prevention", as used herein, refer to a method of delaying or precluding the onset of a condition or disease and/or its attendant symptoms, barring a patient from acquiring a condition or disease, or reducing a patient's risk of acquiring a condition or disease.

The term "therapeutically effective amount" (or more simply an "effective amount") as used herein means the amount of active agent or active ingredient (e.g. σ1R agonist) which is sufficient to achieve the desired therapeutic or prophylactic effect in the individual to which it is administered.

The term "administration", or a variant thereof (e.g.,"administering"), means providing the active agent or active ingredient (e. g. a S1R agonist), alone or as part of a pharmaceutically acceptable composition, to the patient in whom/which the condition, symptom, or disease is to be treated or prevented.

By "pharmaceutically acceptable" is meant that the ingredients of a pharmaceutical composition are compatible with each other and not deleterious to the patient thereof.

The term "agonist" as used herein means a ligand that activates an intracellular response when it binds specifically to a receptor.

The term "antagonist" as used herein means a ligand that blocks or dampens an intracellular response when it binds specifically to a receptor.

The term "excipient" as used herein means a substance formulated alongside the active agent or active ingredient in a pharmaceutical composition or medicament. Acceptable excipients for therapeutic use are well known in the pharmaceutical art, and are described, for example, in Remington's Pharmaceutical Sciences, 21^{st} Edition 2011. The choice of excipient can be selected with regard to the intended route of administration and standard pharmaceutical practice. The excipient must be acceptable in the sense of being not deleterious to the recipient thereof. The at least one pharmaceutically acceptable excipient may be for example, a binder, a diluent, a carrier, a lubricant, a disintegrator, a wetting agent, a dispersing agent, a suspending agent, and the like.

The term "pharmaceutical vehicle" as used herein means a carrier or inert medium used as solvent or diluent in which the pharmaceutically active agent is formulated and/or administered. Non-limiting examples of pharmaceutical vehicles include creams, gels, lotions, solutions, and liposomes.

The present invention will be better understood with reference to the following examples and figures. These examples are intended to representative of specific embodiments of the invention, and are not intended as limiting the scope of the invention.

### FIGURES

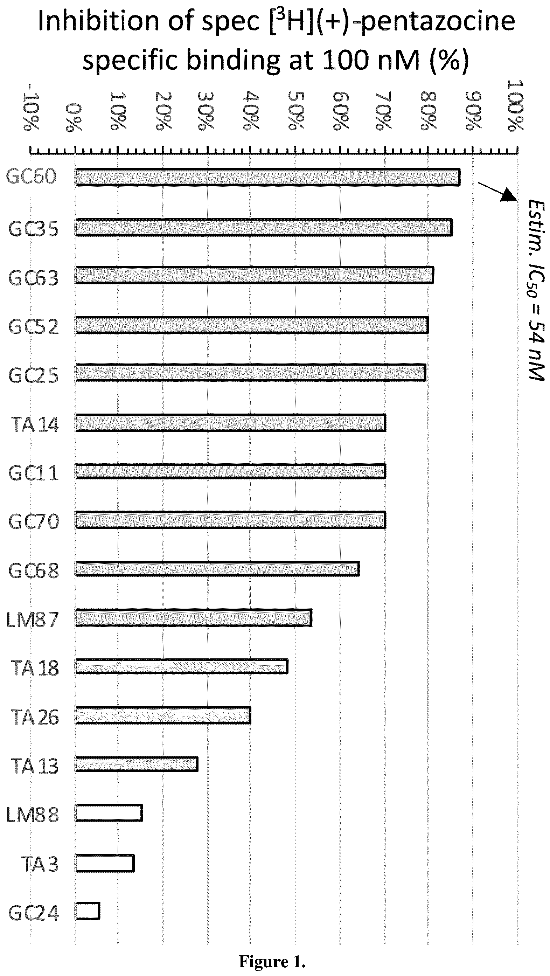

**Figure 1****.** Percentage of inhibition of [³H](+)-pentazocine specific binding to S1R by the compounds of the invention. Green: inhibition > 50%, yellow > 20%.

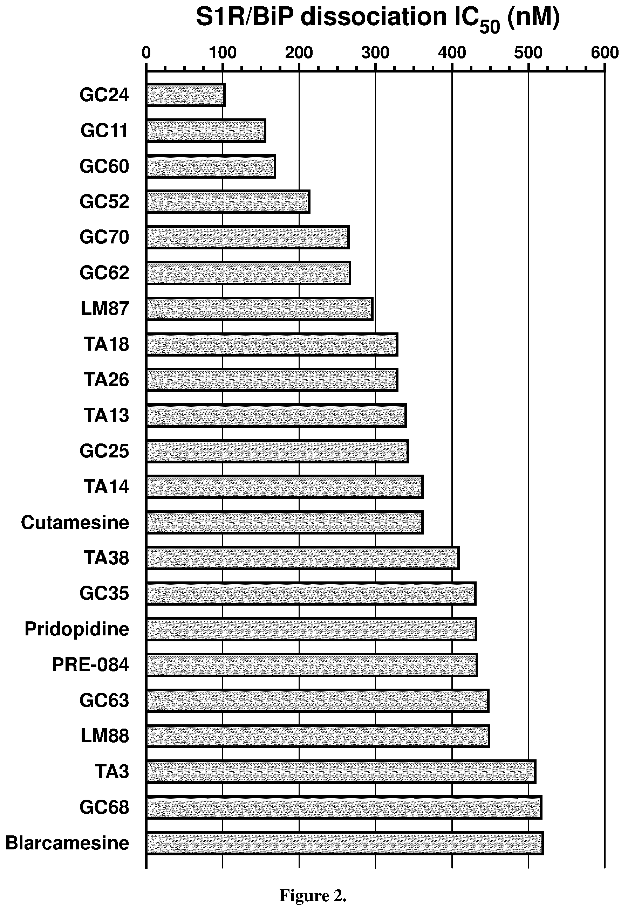

**Figure 2****.** Percentage of inhibition of [³H](+)-pentazocine specific binding to S1R by the compounds of the invention. Green: inhibition > 50%, yellow > 20%.

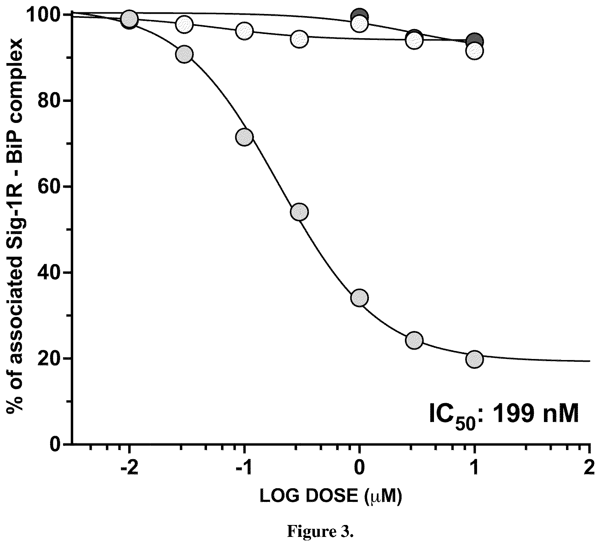

**Figure 3****.** Concentration-response effect of Compound 47 (GC60) (light grey dots) in the S1R/BiP dissociation assay. The reference S1R antagonist NE-100 (white dots) was tested in parallel and Compound 47 (GC60) was tested at the 3 highest concentrations with 3 µM NE-100 (dark grey dots) showing complete prevention of the drug effect.

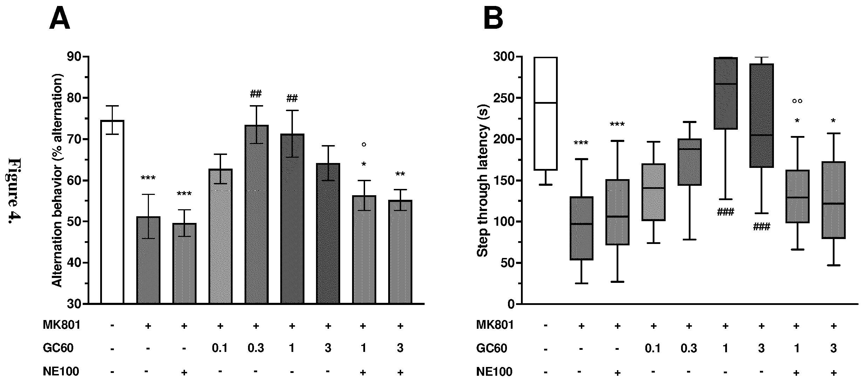

**Figure 4****.** Dose-response effect of Compound 47 (GC60) (0.1-30 mg/kg IP) on Dizocilpine-induced spontaneous alternation deficits in the Y-maze (**A**) and passive avoidance deficits (**B**) in mice. Doses are in mg/kg IP Data showed the mean ± SEM in (A) of n = 12 animals per group in (A) and median and interquartile range of n = 12 animals per group in (B). * p < 0.05, ** p < 0.01, *** p < 0.001 vs. (V+V)-treated group; ## p < 0.01, ### p < 0.001 vs. (Dizocilpine+V)-treated group; ° p < 0.05, °° p < 0.01 vs. (Dizocilpine+GC60)-treated group, Dunnett's test in (A), Dunn's test in (B).

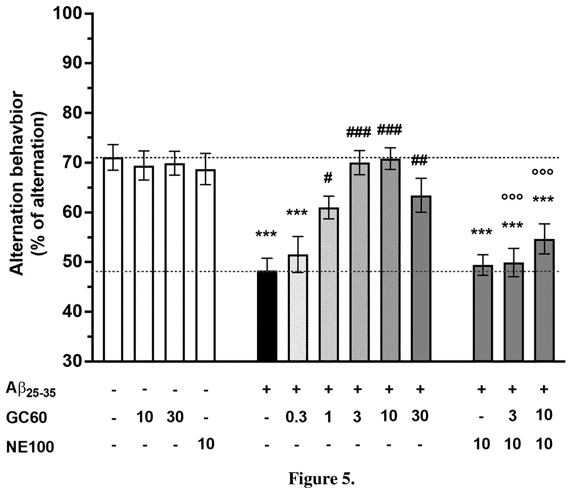

**Figure 5****.** Dose-response effect of Compound 47 (GC60) (0.3-30 mg/kg PO) on Aβ₂₅₋₃₅-induced spontaneous alternation deficits in the Y-maze in mice. Prevention of the GC60 effect by NE-100 (10 mg/kg PO).*** p < 0.001 vs. (V+V)-treated group; # p < 0.05, ## p < 0.01, ### p < 0.001 vs. (Aβ₂₅₋₃₅+V)-treated group; °°° p < 0.001 vs. (Aβ₂₅₋₃₅+GC60)-treated group, Dunnett's test.

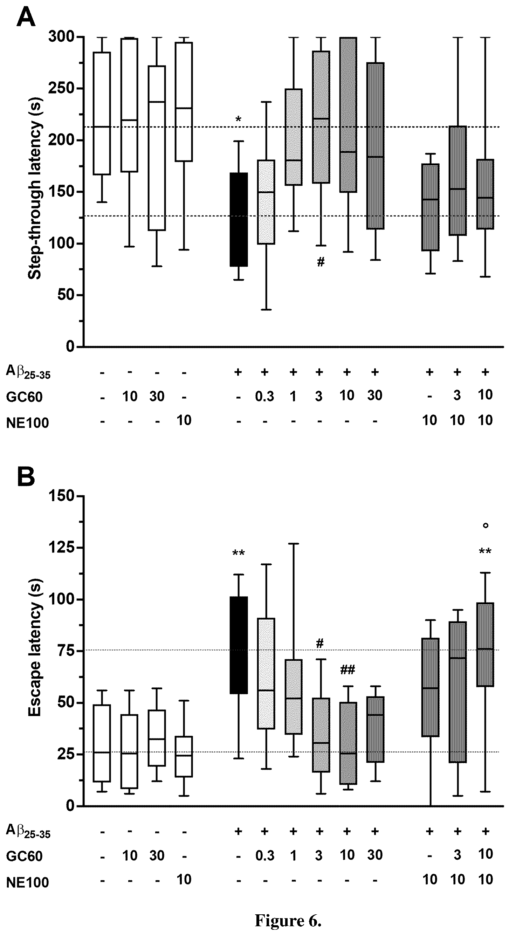

**Figure 6****.** Dose-response effect of Compound 47 (GC60) (0.3-30 mg/kg PO) on Aβ₂₅₋₃₅-induced passive avoidance deficits in mice: (**A**) step-through latency, (**B**) escape latency. Prevention of the GC60 effect by the S1R antagonist NE-100 (10 mg/kg PO). Data showed the median and interquartile range. * p < 0.05, ** p < 0.01 vs. (V+V)-treated group; # p < 0.05, ## p < 0.01 vs. (Aβ₂₅₋₃₅+V)-treated group; ° p < 0.05 vs. (Aβ₂₅₋₃₅+GC60)-treated group, Dunn's test.

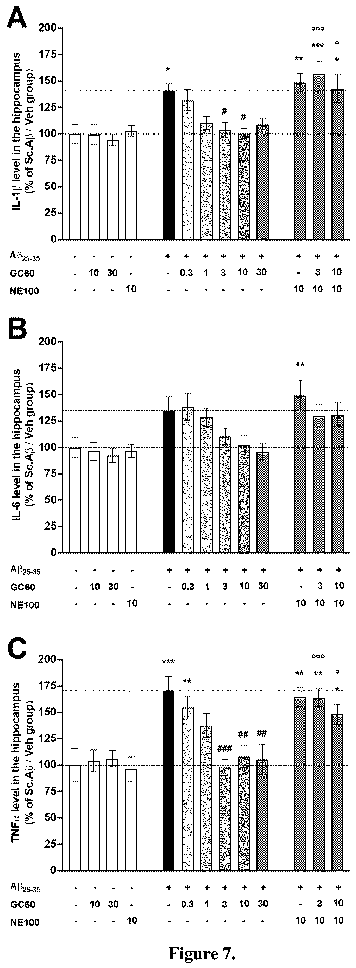

**Figure 7****.** Dose-response effect of Compound 47 (GC60) (0.3-30 mg/kg PO) on Aβ₂₅₋₃₅-induced cytokine levels in the mouse hippocampus: (**A**) IL-1β, (**B**) IL-6, (**C**) TNFa. Prevention of the GC60 effect by NE-100 (10 mg/kg PO).* p < 0.05, ** p < 0.01, *** p < 0.001 vs. (V+V)-treated group; # p < 0.05, ## p < 0.01, ### p < 0.001 vs. (Aβ₂₅₋₃₅+V)-treated group; ° p < 0.05, °°° p < 0.001 vs. (Aβ₂₅₋₃₅+GC60)-treated group, Dunnett's test.

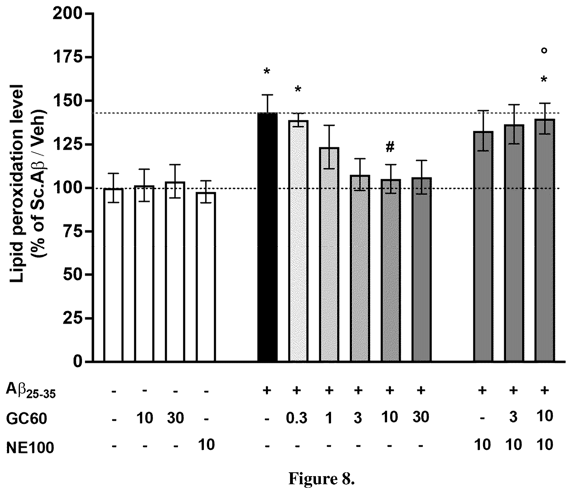

**Figure 8****.** Dose-response effect of Compound 47 (GC60) (0.3-30 mg/kg PO) on Aβ₂₅₋₃₅-induced lipid peroxidation in the mouse hippocampus. Prevention of the GC60 effect by NE-100 (10 mg/kg PO).* p < 0.05 vs. (V+V)-treated group; # p < 0.05 vs. (Aβ₂₅₋₃₅+V)-treated group; ° p < 0.05 vs. (Aβ₂₅₋₃₅+GC60)-treated group, Dunnett's test.

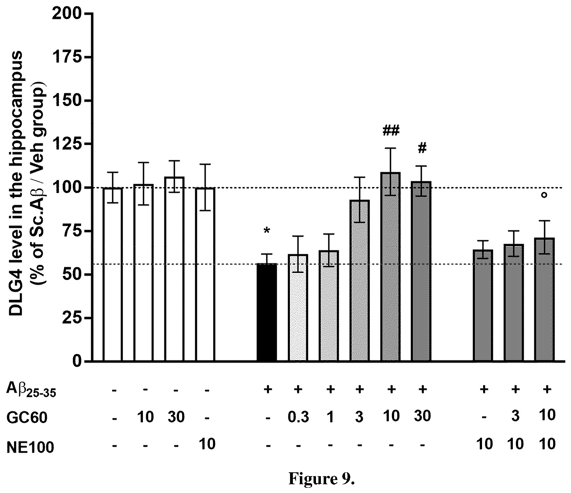

**Figure 9****.** Dose-response effect of Compound 47 (GC60) (0.3-30 mg/kg PO) on Aβ₂₅₋₃₅-induced DLG4 (PSD-95) level in the mouse hippocampus. Prevention of the GC60 effect by NE-100 (10 mg/kg PO).* p < 0.05 vs. (V+V)-treated group; # p < 0.05, ## p < 0.01 vs. (Aβ₂₅₋₃₅+V)-treated group; ° p < 0.05 vs. (Aβ₂₅₋₃₅+GC60)-treated group, Dunnett's test.

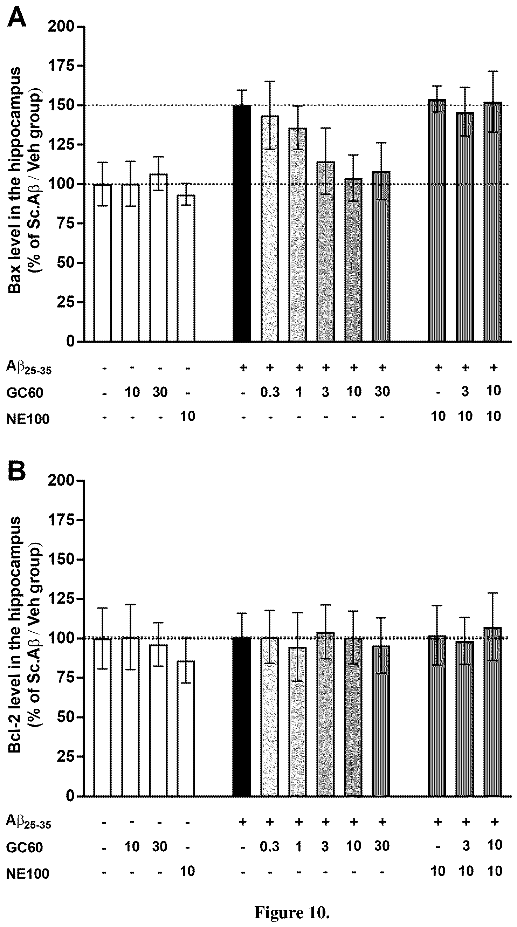

**Figure 10****.** Dose-response effect of Compound 47 (GC60) (0.3-30 mk/kg PO) on Aβ₂₅₋₃₅-induced apoptosis in the mouse hippocampus: (**A**) Bax level, (**B**) Bcl-2 level. Prevention of the GC60 effect by NE-100 (10 mg/kg PO).

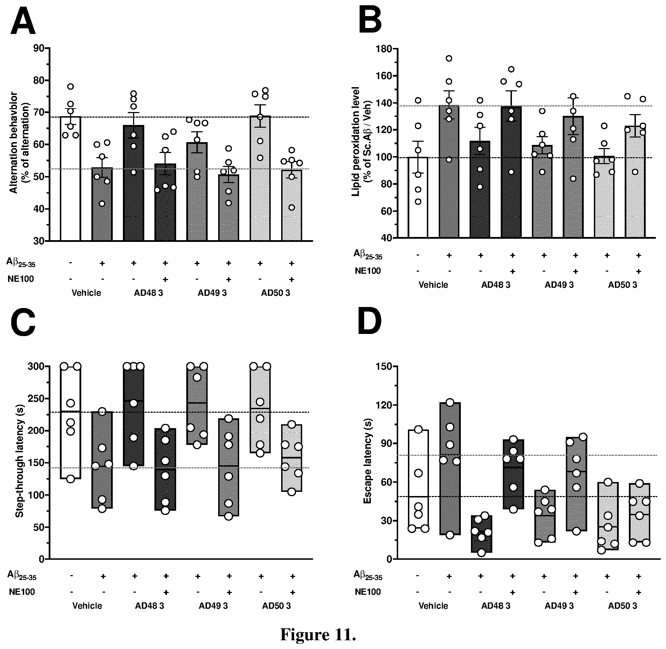

**Figure 11****.** Effect of Compound 72 (AD48), Compound 73 (AD49) and Compound 74 (AD50) on Aβ₂₅₋₃₅-induced spatial working memory in the Y-maze (A), lipid peroxidation in the hippocampus (B) and passive avoidance deficits in mice (C).

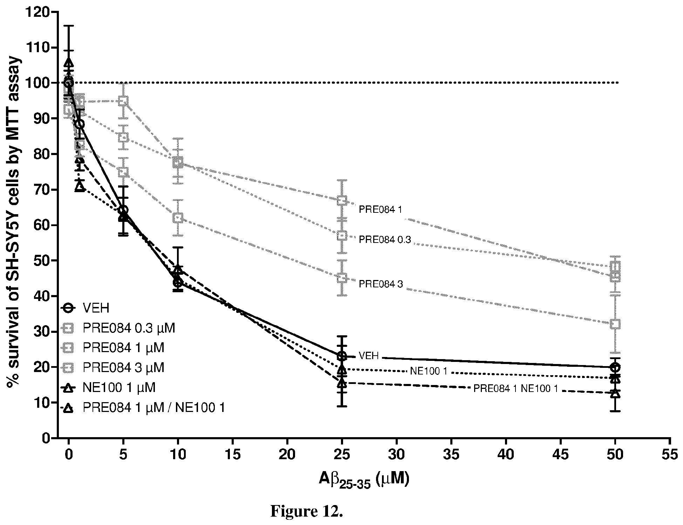

**Figure 12****.** Dose-response effect of PRE084 (0.3-3 µM) on Aβ₂₅₋₃₅-induced SH-SY5Y neuroblastoma cells toxicity.

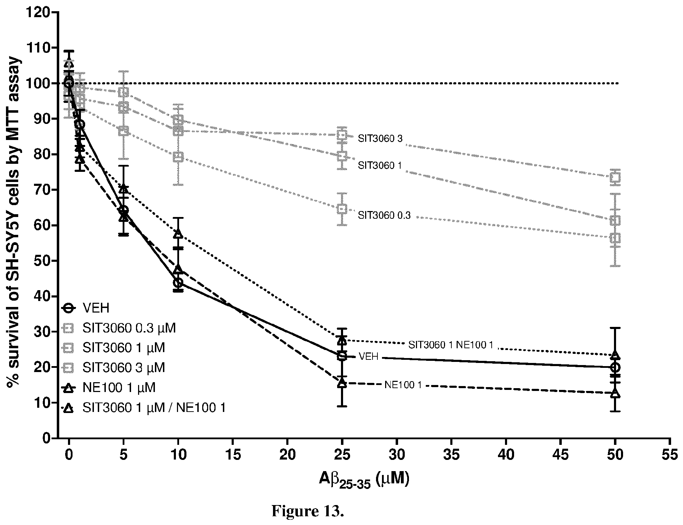

**Figure 13****.** Dose-response effect of Compound 47 (GC60) (0.3-3 µM) on Aβ₂₅₋₃₅-induced SH-SY5Y neuroblastoma cells toxicity.

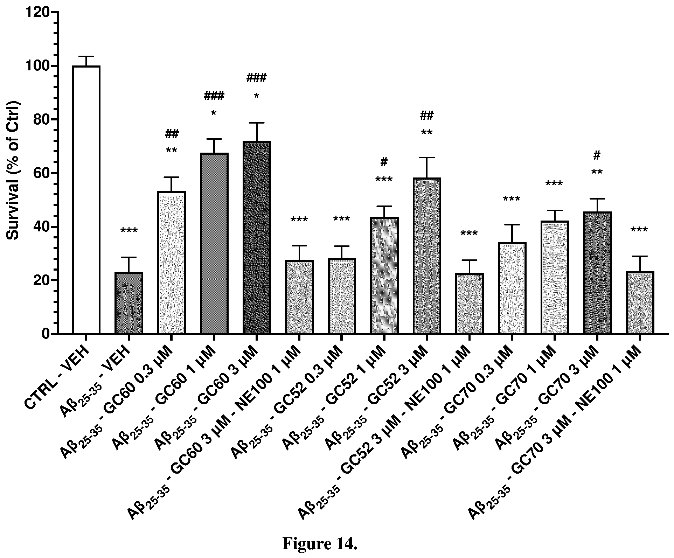

**Figure 14****.** Dose-response effect of various compounds (0.3-3 µM) on Aβ₂₅₋₃₅-induced SH-SY5Y neuroblastoma cells toxicity. * p < 0.05, ** p < 0.01, *** p < 0.001 vs. (CTRL-VEH)-treated group; # p < 0.05, ## p < 0.01, ### p < 0.001 vs. (Aβ₂₅₋₃₅+VEH)-treated group: Dunnett's test.

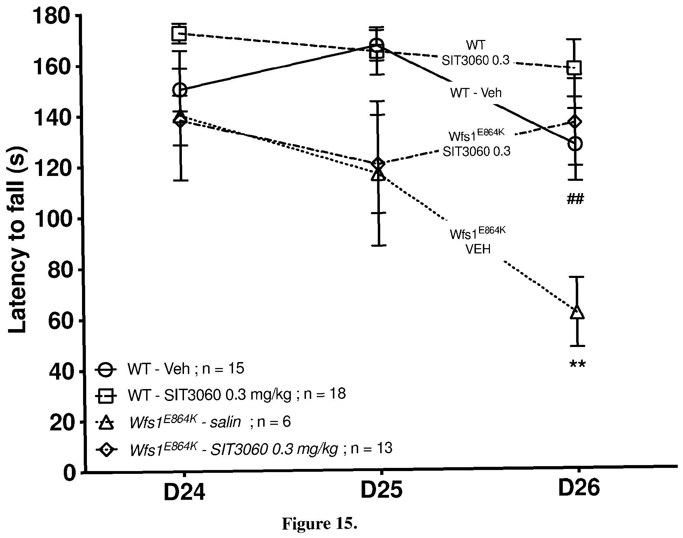

**Figure 15****.** Effect of Compound 47 (GC60) on vestibular deficits induced in Wfs1^{E864K} compared to Wfs1^{WT} mice. ** p < 0.01, vs. Wfs1^{WT} mice; ## p < 0.01 vs. Wfs1^{E864K} mice, Bonferroni's test.

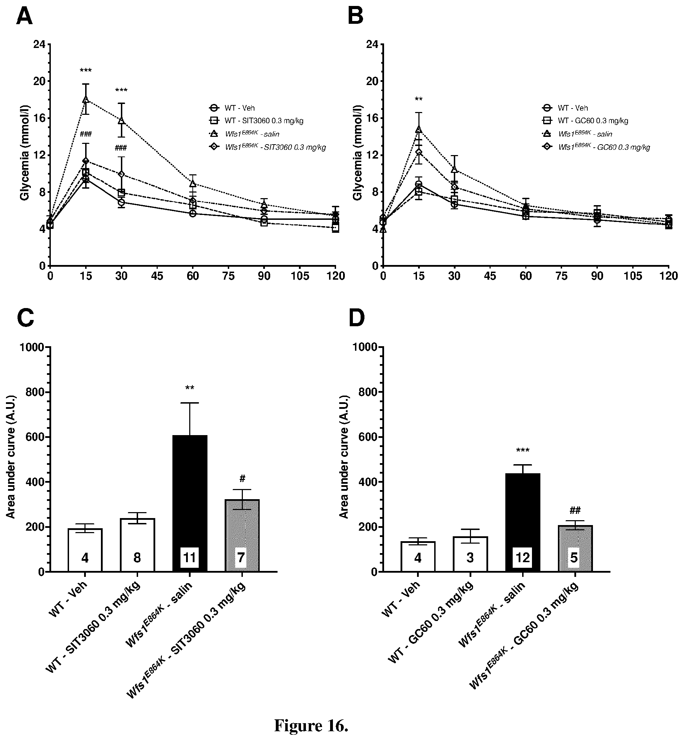

**Figure 16****.** Glycemia measurement in Wfs1^{E864K} compared to Wfs1^{WT} mice and effect of Compound 47 (GC60). Data were expressed in time-course curve (A-B), and also calculated by using area under curve procedure (C-D). ** p < 0.01, *** p < 0.001 vs. Wfs1^{WT} - Veh mice; # p < 0.05, ## p < 0.01 vs. Wfs1^{E864K} - Veh mice, Bonferroni's test (A-B) and Dunnett's test (C-D).

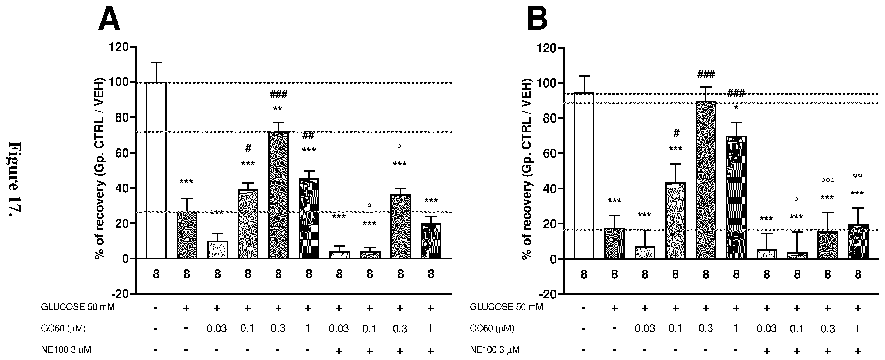

**Figure 17****.** Dose-response effect of Compound 47 (GC60) (0.03-1 µM) on glucose-induced toxicity in MIN6-Wfs1^{WT} (A) and MIN6-Wfs1^{KD70} (B). Data were expressed in percentage of survival. * p < 0.05, *** p < 0.001 vs. MIN6-Wfs1 non-treated cells; # p < 0.05, ## p < 0.01, ### p < 0.001 vs. MIN6-Wfs1 glucose-treated cells, ° p < 0.05, °° p < 0.01, °°° p < 0.001 vs. MIN6-Wfs1 GC60-treated cells. Dunnett's test.

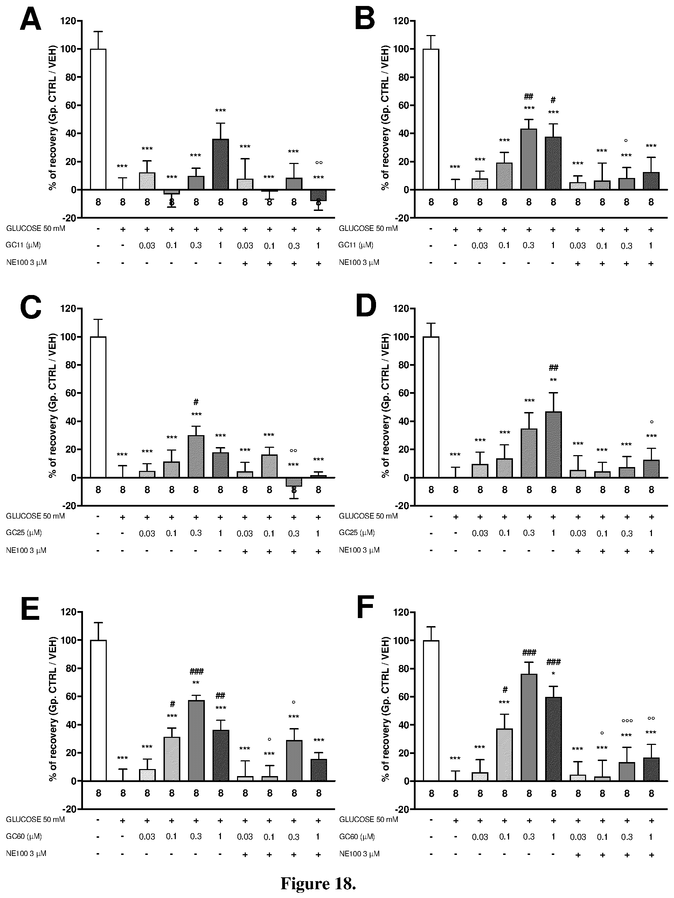

**Figure 18****.** Dose-response effect of Compound 36 (GC11) (0.03-1 µM, A-B), Compound 39 (GC25) (0.03-1 µM, C-D), and Compound 74 (AD50) (0.03-1 µM, E-F), on glucose-induced toxicity in MIN6-Wfs1^{WT} (A-C-E) & MIN6-Wfs1^{KD70} (B-D-F). Data were expressed in percentage of survival. * p < 0.05, ** p < 0.01, *** p < 0.001 vs. MIN6-Wfs1 non-treated cells; # p < 0.05, ## p < 0.01, ### p < 0.001 vs. MIN6-Wfs1 glucose-treated cells, ° p < 0.05, °° p < 0.01, °°° p < 0.001 vs. MIN6-Wfs1 compound treated cells. Dunnett's test.

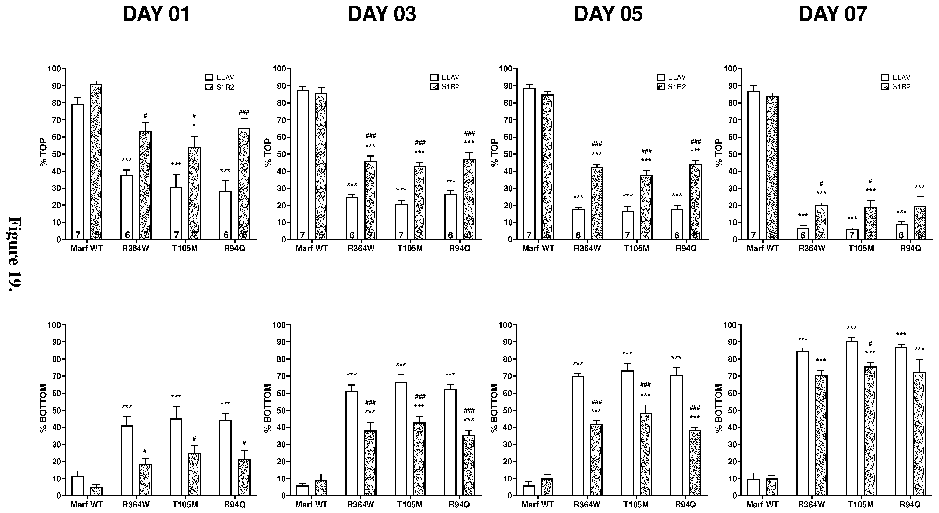

**Figure 19****.** Effect of various marf mutation in locomotion in drosophila. Data were represented in mean ± SEM of % of Drosophila which reach the top of the geotaxis apparatus (%TOP). * p < 0.05, *** p < 0.001 vs marf WT Drosophila ; # p < 0.05, ### p < 0.001 vs Elav::marf mutants of Drosophila ; n between 5 and 7 per group, 8 flies per point

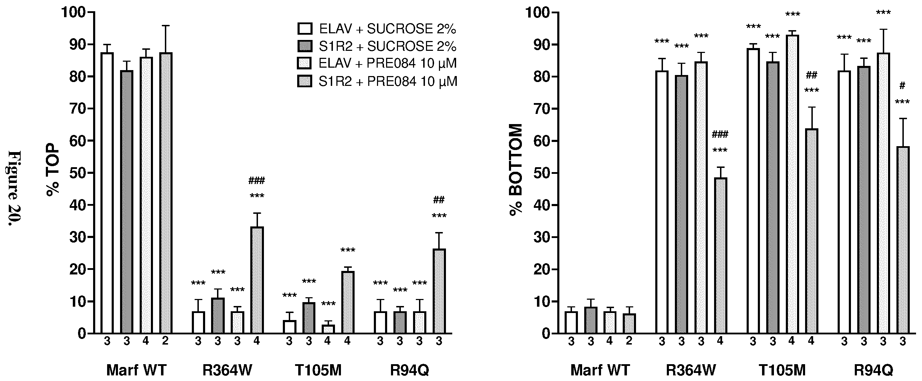

**Figure 20****.** Effect of S1R agonist PRE-084 in various marf mutation in locomotion in drosophila. After the test at day 7, each group of the first experiment (figure 19) were divided into two subgroups, either treated with sucrose 2% or treated with the S1R agonist PRE-084 at 10 µM. Data were represented in mean and SEM of % of Drosophila which reach the top of the geotaxis apparatus (%TOP). *** p < 0.001 vs marf^{WT} Drosophila; # p < 0.05, vs Elav::Marf mutation of Drosophila, n = 5-7 per group; *** p < 0.001 vs marf^{WT} treated with sucrose 2% Drosophila ; ## p < 0.01, ### p < 0.001 flies treated with PRE-084 vs treated with sucrose, n between 2-4 per group.

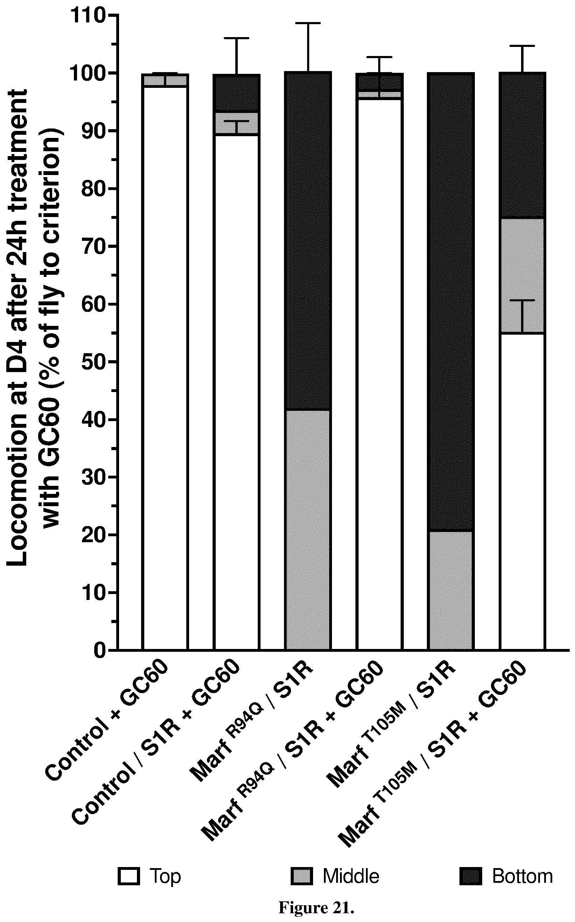

**Figure 21****.** Effect of S1R agonist Compound 47 (GC60) in various marf mutation in locomotion in drosophila. At D4, each group were treated with the S1R agonist Compound 47 (GC60) at 10 µM. Data were represented in mean of % of Drosophila which reach the top of the geotaxis apparatus (white part of histogram), which stay in the bottom of the apparatus (dark grey part of histogram), or between the two parts of the apparatus (light grey in the histogram). n between 2-4 per group.

### EXAMPLES

### ABBREVIATIONS

DCM: dichloromethane;
DIPEA: N, N-diisopropylethylamine;
DMAP: 4-dimethylaminopyridine;
DMEM: Dulbecco's Modified Eagle Medium;
DMF: N,N-dimethylformamide;
EDA: ethylenediamine;
DMSO: dimethylsufoxide;
eq. or equiv.: equivalent;
ESI: electrospray ionization;
HPLC: high-performance liquid chromatography;
HRMS: high resolution mass spectrometry;
LDA: lithium diisopropylamide;
Mp: melting point;
MS: mass spectrometry;
MW: molecular weight;
NMR: nuclear magnetic resonance;
ppm: parts per million;
RP-HPLC: reversed-phase high-performance liquid chromatography;
THF: tetrahydrofurane;
TLC: thin-layer chromatography;
t_{R}: retention time;
UV: ultraviolet.

### SYNTHESIS

### 1. Material and instrumentation

Unless specified, all of the reagents and starting materials were purchased from Fluorochem^{®}, TCI^{®},Alfa Aesar^{®} or Sigma-Aldrich^{®} and used as received.

All temperatures are expressed in °C and all reactions were carried out at room temperature (RT) unless otherwise stated.

All NMR spectroscopy measurements were performed with a Bruker AC 400 MHz spectrometer and are calibrated using the residual proton in the deuterated solvent (CHCl₃ at 7.26 ppm ¹H NMR, 77.16 ppm ¹³C NMR; DMSO at 2.50 ppm ¹H NMR, 39.52 ppm ¹³C NMR; MeOH at 3.31 ppm ¹H NMR, 49.00 ppm ¹³C NMR). The chemical shifts are reported in ppm, and the coupling constants (J) are reported in Hz. The following abbreviations were used to explain multiplicities: s = singlet, br s = broad singlet, d = doublet, t = triplet, q = quartet, p = pentuplet and m = multiplet. For ¹³C NMR (101 MHz) and ³¹P NMR (162 MHz) all spectra were decoupled from the proton unless stated otherwise.

High-resolution mass spectra (HRMS) were recorded on an Agilent 6210 ESI (electrospray ionization) TOF (time of flight) mass spectrometer.

TLC was performed on Merck^{®} TLC-plates with fluorescence indication (silica type 60, F254), spots were visualized using UV-light and stained using either potassium permanganate or phosphomolybdic acid solution.

HPLC analyses were performed with a Shimadzu machine equipped with a SPD-M20A PDA Detector at 25°C employing SunFire Waters, C₁₈ 5 µm, 4.6 x 150 mm. HPLC grade MeCN and H₂O were used as the eluting solvents.

### 2. Synthesis of synthetic intermediates

### Ethyl divinylphosphonate

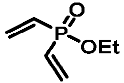

In a 500 mL flame dried round flask under N₂, a solution of freshly distilled ethyl phosphorodichloridate (8.26 g, 50.7 mmol, 1.0 equiv.) in THF (200 mL) was prepared. The reaction mixture was cooled down to -80 °C with a liquid nitrogen/acetone bath then, a solution of vinylmagnesium bromide in THF (1 M, 101.4 mmol, 2.0 equiv.) was added dropwise. The reaction was vigorously stirred and allowed to warm up to room temperature for 2 h until no starting material was detected (monitored by ³¹P NMR). The reaction was quenched with a limited amount of a saturated NH₄Cl aqueous solution (15 mL). The resulting mixture was filtered on Celite, triturated and rinsed with THF (200 mL). The filtrate was dried with MgSO₄, filtered and dried under vacuum to afford 5.0 g (67% yield) of the desired product that was further used without purification.

¹H NMR (400 MHz, CDCl₃): δ 6.45 - 6.07 (m, 6H), 4.07 (p, *J* = 7.2 Hz, 2H), 1.33 (t, *J =* 7.1 Hz, 3H).

³¹P NMR (162 MHz, CDCl₃): δ 28.83.

### 1-benzyl-4-ethoxy-1,4-azaphosphinane 4-oxide

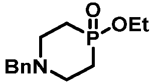

### Prepared according to General procedure 1. Column chromatography purification (DCM/MeOH 10/0 to 9/1) afforded the product as a yellow oil (6.1 g, 70%)

¹H NMR (400 MHz, CDCl₃): δ 7.34 - 7.20 (m, 5H), 4.06 (ddt, *J =* 7.6, 7.0, 0.7 Hz, 2H), 3.57 (d, *J* = 1.0 Hz, 2H), 3.05 - 2.89 (m, 2H), 2.67 - 2.52 (m, 2H), 2.02 - 1.75 (m, 4H), 1.33 (td, *J =* 7.0, 0.9 Hz, 3H).

¹³C NMR (101 MHz, CDCl₃): δ 138.3, 128.7, 128.4, 127.3, 61.7 (d, *J =* 1.0 Hz), 59.9 (d, *J* = 6.2 Hz), 50.8 (d, *J* = 7.0 Hz), 27.1 (d, *J =* 86.1 Hz), 16.7 (d, *J =* 5.9 Hz).

³¹P NMR (162 MHz, CDCl₃): δ 45.55.

HRMS (ESI-TOF): calc for C₁₃H₂₁NO₂P [M+H]⁺: 254.1304, measured: 254.1308.

HPLC: SunFire Waters, C₁₈ 5 µm, 4.6 × 150 mm; gradient 60% to 98% MeCN/H₂O, 1.0 mL/min, 214 nm; t_{R} = 3.8 min.

### 1-benzyl-1,4-azaphosphinane 4-oxide

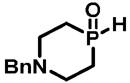

In a 250 mL flame dried round flask equipped with an addition funnel under N₂, 1-benzyl-4-ethoxy-1,4-azaphosphinane 4-oxide (2.9 g, 11.4 mmol, 1.0 equiv.) was weighted. Anhydrous THF (40 mL) was added and the solution was cooled down to 0 °C. A lithium aluminium hydride solution in THF (1 M, 9.12 mmol, 0.8 equiv.) was added dropwise. The reaction mixture was then stirred for 30 min until no more starting material was detected (monitored by ³¹P NMR). Then, water (424 µL), an aqueous solution of KOH (15%, 424 µL) and water (1.3 mL) were successively carefully added. The resulting suspension was filtered and 5 mL of water was added to the filtrate. The filtrate was then extracted with CH₂Cl₂ (2 × 40 mL). The combined organic phases were then dried over MgSO₄, filtered and concentrated. Then, the crude was dissolved in CH₂Cl₂ and a few drops of an H₂O₂ solution (30%) were added and the resulting mixture was stirred for 5 min. Then, Na₂S₂O₈ was added and the mixture was dried over MgSO₄, filtered and concentrated to afford 2.25 g (93% yield) of the desired product that was used without further purification.

¹H NMR (400 MHz, CDCl₃): δ 6.94 (dtt, *J* = 456.9, 5.1, 3.7 Hz, 1H), 7.34 - 7.24 (m, 5H), 3.58 (s, 2H), 3.12 - 2.98 (m, 2H), 2.57 - 2.43 (m, 2H), 2.16 - 2.08 (m, 4H).

¹³C NMR (101 MHz, CDCl₃): δ 137.9, 128.7, 128.5, 127.5, 62.0, 49.2 (d, *J =* 6.1 Hz), 27.0 (d, *J = 62.6* Hz).

³¹P NMR (162 MHz, CDCl₃): δ 25.34

HRMS (ESI-TOF): calc for C₁₁H₁₇NOP [M+H]⁺: 210.1042, measured: 210.1048.

### 3. General procedures

### General procedure 1: Formation of azaphosphinane

In a 250 mL flame dried round flask under N₂, a solution of ethyl divinylphosphonate (5.0 g, 34.2 mmol, 1.0 equiv.) in freshly distilled EtOH (75 mL) was prepared. Benzylamine derivatives (7.3 g, 68.4 mmol, 2.0 equiv.) was then added and the reaction was refluxed for 48 h until no starting material was detected (monitored by ³¹P NMR). Then, the reaction mixture was concentrated under reduced pressure. Purification by column chromatography (silica, Acetone/MeOH 100:0 to 90:10) afforded 6.1 g (70% yield) of the desired product.

### General procedure 2: Hirao coupling reactions

In a flame dried round bottom flask under N₂, Pd₂dba₃ (11 mg, 0.012 mmol, 2.5 mol%) and Xantphos (14 mg, 0.024 mmol, 5 mol%) were added. Then dry DMF (1 mL), 1-benzyl-1,4-azaphosphinane 4-oxide (100 mg, 0.48 mmol, 1.0 equiv.) the corresponding aryl halide (0.53 mmol, 1.1 equiv.) and DIPEA (310 mg, 2.40 mmol, 5.0 equiv.) were successively added. The reaction mixture was stirred for 16 h at 100°C until no starting material was detected (monitored by ³¹P NMR). Then the reaction was cooled down to room temperature, filtered on Celite pad and concentrated *in vacuo.* Purification by column chromatography (deactivated silica with 5% Et₃N, CH₂Cl₂/AcOEt/MeOH 8:2:0 to 8:2:1.5) followed, if necessary, by RP-HPLC (Water Sunfire C18, 65% MeCN/H²O) afforded the pure product.

### General procedure 3: N-benzyl hydrogenolysis

In a round bottom flask, the corresponding N-benzyl azaphosphinane (1.0 equiv.) was stirred in methanolic HCl (3 M, 3.0 equiv.) during 15 min. After precipitation of the amine hydrochloride as a colorless solid, the solvent was removed under reduced pressure and the product was dried *in vacuo*. In a second step, in a flame dried round bottom flask, the azaphosphinane hydrochloride previously made was added (1.0 equiv.). MeOH (0.05 M) and activated Pd/C (10 mol%, 10% wt) were added. Finally, nitrogen was replaced by H₂ thanks to a balloon and the mixture was stirred for 24 h at room temperature (monitored by ³¹P NMR). Then, the reaction mixture was concentrated under reduced pressure and the solid was triturated with Et₂O to afford the desired product that was used without further purification.

### General procedure 4: Reductive amination

In a flame dried round bottom flask under N₂, the corresponding azaphosphinane free amine salt or azaphosphinane hydrochloride (1.0 equiv.) and the corresponding aldehyde (2.0 equiv.) were added. Then, anhydrous CH₂Cl₂ (0.25 M) was added and the suspension was stirred for 5 min. Then, sodium acetoxyborohydride (2.4 equiv.) was added and the mixture was stirred at room temperature for 16 h until no starting material was detected (monitored by ³¹P NMR). Then, the reaction was quenched with MeOH (1 mL) and concentrated under reduced pressure. Purification by column chromatography (deactivated silica with 5% Et₃N, CH₂Cl₂/AcOEt/MeOH 8:2:0 to 8:2:1) followed, if necessary, by RP-HPLC (Water Sunfire C18, 65% MeCN/H₂O) afforded the pure product.

### General procedure 5: N-Alkylation A

In a flame dried round bottom flask under N₂, the corresponding azaphosphinane free amine salt or azaphosphinane hydrochloride (1.0 equiv.) was dissolved in acetonitrile (0.25 M). Then DIPEA (2.5 equiv.), NaI (10 mol%) and the corresponding alkyl halide (1.2 equiv.) were added. The mixture was refluxed for 18 h until no starting material was detected (monitored by ³¹P NMR). Then the reaction was diluted with CH₂Cl₂ and the resulting suspension was filtered through Celite. The filtrate was concentrated under reduced pressure. Purification by column chromatography (deactivated silica with 5% Et₃N, AcOEt/MeOH 100:0 to 75:25) afforded the pure product.

### General procedure 6: N-Alkylation B

In a flame dried round bottom flask under N₂, the corresponding azaphosphinane free amine salt or azaphosphinane hydrochloride (1.0 equiv.) was dissolved in toluene (0.3 M). Then the corresponding alkyl halide (1.1 equiv.), KI (10 mol%) and Na₂CO₃ (2.0 equiv.) were successively added. The mixture was refluxed for 24 h until no starting material was detected (monitored by ³¹P NMR). Then the reaction was diluted with CH₂Cl₂ and the resulting suspension was filtered through Celite. The filtrate was concentrated under reduced pressure. Purification by column chromatography (deactivated silica with 5% Et₃N, AcOEt/MeOH 100:0 to 75:25) afforded the pure product.

### General procedure 7: Buchwald-Hartwig coupling reactions

In a flame dried round bottom flask under N₂, the corresponding azaphosphinane free amine salt or azaphosphinane hydrochloride (1.0 equiv.) was added. Then, RuPhos (20 mol%), Pd₂dba₃ (10 mol%) and Cs₂CO₃ (3.0 equiv.) were added. Toluene (0.1 M) and the corresponding aryl iodide (1.1 equiv.) were added to the flask. The reaction mixture was stirred and heated at 100 °C for 24 h until completion (monitored by ³¹P NMR). Then, the reaction mixture was filtered through Celite and concentrated under reduced pressure. Purification by column chromatography (deactivated silica with 5% Et₃N, acetone/MeOH, gradient 0 to 25% MeOH) followed by preparative TLC (toluene/acetone/MeOH 55:40:5) afforded the pure product.

### General procedure 8: Pudovik reaction

In a flame dried Schlenk tube under N₂, 1-benzyl-1,4-azaphosphinane 4-oxide (105 mg, 0.5 mmol, 1 equiv.), benzaldehyde derivatives (0.6 mmol, 1.2 equiv.) were added. Then, DCM (1 mL) was added and the mixture was heated to 40°C for 1 hour until no starting material was detected (monitored by ³¹P NMR). The resulting solution was cooled to room temperature, concentrated under reduced pressure and purified by column chromatography (silica, DCM/MeOH 99:1 to 90;10) followed, if necessary, by RP-HPLC (Water Sunfire C₁₈, 65% MeCN/H₂O) afforded the pure product.

### General procedure 9: Kabachnik-Fields three-component reactions

In a flame dried Schlenk tube under N₂, 1-benzyl-1,4-azaphosphinane 4-oxide (105 mg, 0.5 mmol, 1 equiv.), benzaldehyde derivatives (0.6 mmol, 1.2 equiv.), aniline derivatives (0.6 mmol, 1.2 equiv.) were added. Then, DCM (1 mL) were added and the mixture was heated to 40°C for 1 hour until no starting material was detected (monitored by ³¹P NMR). The resulting solution was cooled to room temperature, concentrated under reduced pressure and purified by column chromatography (silica, DCM/MeOH 99:1 to 90;10) followed, if necessary, by RP-HPLC (Water Sunfire C₁₈, 65% MeCN/H₂O) afforded the pure product.

### General procedure 10: oxidation of Kwon derivatives

In a Schlenk tube, a solution of Kwon derivatives (0.3 mmol, 1 equiv.) in DCM (5 mL) was prepared. Then, H₂O₂ (30 % in H₂O, 1.5 equiv.) was added and the mixture was stirred for 15 min until no starting material was detected (monitored by ³¹P NMR). The reaction was then dried over anhydrous MgSO₄, filtrated and concentrated under reduced pressure to afford the pure product.

### General procedure 11: Formation of styryl-azaphosphinane

1-(4-methylbenzyl)-1,4-azaphosphinane 4-oxide (112 mg, 0.5 mmol, 1 equiv.), phenylacetylene derivatives (0.75 mmol, 1.5 equiv.), CuI (10 mg, 0.05 mmol, 10 mol%) and EDA (5 µL, 0.075 mmol, 15 mol%) were dissolved in 1 ml of DMSO under nitrogen and heated at 60°C for 16 h until no starting material was detected (monitored by ³¹P NMR). The resulting solution was cooled to room temperature, and then 10 ml of DCM and 10 ml of brine were added to the solution, the organic layer was washed with brine (10 ml × 2) and was dried over anhydrous MgSO₄. After filtration the crude product was then purified by column chromatography (silica, DCM/MeOH 99:1 to 90;10) followed, if necessary, by RP-HPLC (Water Sunfire C₁₈, 65% MeCN/H₂O) afforded the pure product.

### General procedure 12: Formation of phenethyl-azaphosphinane

In a flame dried Schlenk tube under N₂, styryl-azaphosphinane (0.25 mmol, 1 equiv.), B₂(OH)₄ (24 mg, 0.27 mmol, 1.1 equiv.) and Pd/C (10% w/w, 25 mg) were added. Then, DCM (1 mL) and H₂O (22 µL, 5 equiv.) were added and the solution was stirred for 16 h until no starting material was detected (monitored by ³¹P NMR). The mixture was dried over anhdrous MgSO₄, filtrated and purified by column chromatography (silica, DCM/MeOH 99:1 to 90;10) followed, if necessary, by RP-HPLC (Water Sunfire C₁₈, 65% MeCN/H₂O) afforded the pure product.

### General procedure 13: Formation of phenethynyl-azaphosphinane

Under N₂ atmosphere, 0.2 mmol diphenylphosphine oxide, 0.2 mmol phenylacetylene, 5 mol% Pd(OAc)₂, 0.4 mmol AgBF₄ and 0.5 mL THF were charged into a 10 mL schlenck tube, and the mixture was stirred at 60°C for 3 h. Then 1 mL Et₃N was added. After filtration the crude product was concentrated and then purified by column chromatography (silica, DCM/MeOH 99:1 to 90;10) followed, if necessary, by RP-HPLC (Water Sunfire C₁₈, 65% MeCN/H₂O) afforded the pure product.

### General procedure 14: Formation of α-Ester derivatives

In a flame dried Schlenk tube under N₂, LDA (1.5 eq.) was added to a solution of azaphosphinane (1 eq.) in THF at -78°C and under argon. Stirring was maintained for 20 minutes. A solution of dibenzy- or diallylcarbonate (1.4 eq.) in THF was then added dropwise. The mixture was stirred for 45 minutes and then a second addition of LDA (1.5 eq.) was carried out. The mixture was stirred for additionnal 2 hours at -78°C. A 10% NH₄Cl solution was then added dropwise. AcOEt was then added and he resulting solution was gradually brought to room temperature. The mixture was extracted with AcOEt, the organic layer was washed with brine and dried over anhydrous MgSO₄. After filtration the crude product was then purified by column chromatography (silica, DCM/MeOH 99:1 to 90;10) followed, if necessary, by RP-HPLC (Water Sunfire C₁₈, 65% MeCN/H₂O) afforded the pure product.

### EXAMPLE 1: 1-benzyl-4-(4-(trifluoromethyl)phenyl)-1,4-azaphosphinane 4-oxide (LM87)

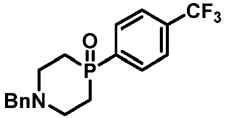

Compound 1 was prepared according to General procedure 2. Column chromatography purification (DCM/MeOH 10/0 to 9/1) afforded the product as a colorless solid (95 mg, 71%).

¹H NMR (400 MHz, CDCl₃): δ 7.94 (ddt, *J =* 11.0, 7.9, 0.8 Hz, 2H), 7.77 (ddd, *J =* 7.9, 2.0, 0.8 Hz, 2H), 7.39 - 7.25 (m, 5H), 3.67 (s, 2H), 3.18 - 2.89 (m, 4H), 2.31 - 2.15 (m, 2H), 2.08 (dddd, *J =* 20.3, 12.4, 5.5, 2.9 Hz, 2H).

¹³C NMR (101 MHz, CDCl₃): δ 138.1, 137.6 (d, *J =* 1.2 Hz), 136.6 (d, *J =* 1.3 Hz), 133.9 (qd, *J =* 32.8, 3.0 Hz), 131.0 (d, *J =* 9.6 Hz), 128.9, 128.5, 127.5, 125.7 (dq, *J =* 11.5, 3.7 Hz), 125.0 (d, *J* = 1.1 Hz), 122.3 (d, *J* = 1.1 Hz), 62.7 (d, *J =* 1.1 Hz), 49.1 (d, *J =* 7.3 Hz), 28.7 (d, *J* = 66.1 Hz).

³¹P NMR (162 MHz, CDCl₃): δ 27.70.

HRMS (ESI-TOF): calc for C18H₂₀F₃NOP [M+H]⁺: 354.1229, measured 354.1238.

HPLC: SunFire Waters, C₁₈ 5 µm, 4.6 × 150 mm; gradient 60% to 98% MeCN/H₂O, 1.0 mL/min, 214 nm; t_{R} = 5.9 min.

### EXAMPLE 2: 1-benzyl-4-(4-fluorophenyl)-1,4-azaphosphinane 4-oxide (LM88)

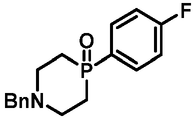

Compound 2 was prepared according to General procedure 2. Column chromatography purification (DCM/MeOH 10/0 to 9/1) afforded the product as a colorless solid (72 mg, 63%).

¹H NMR (400 MHz, CDCl₃): δ 7.79 (dddd, *J =* 10.9, 8.7, 5.5, 2.0 Hz, 2H), 7.39 - 7.25 (m, 5H), 7.20 (ddt, *J =* 8.7, 6.7, 2.1 Hz, 2H), 3.65 (s, 2H), 3.11 - 2.84 (m, 4H), 2.23 - 2.11 (m, 2H), 2.04 (ddt, *J =* 17.5, 15.1, 3.0 Hz, 2H).

¹³C NMR (101 MHz, CDCl₃): δ 166.4 (d, *J =* 3.4 Hz), 163.9 (d, *J =* 3.2 Hz), 138.2, 132.9 (dd, *J =* 10.7, 8.7 Hz), 128.9, 128.5, 127.4, 116.2 (dd, *J =* 21.4, 12.7 Hz), 62.7 (d, *J =* 1.1 Hz), 49.2 (d, *J* = 7.2 Hz), 28.8 (d, *J =* 66.3 Hz).

³¹P NMR (162 MHz, CDCl₃): δ 27.76.

HRMS (ESI-TOF): calc for C₁₇H₂₀FNOP [M+H]⁺: 304.1261, measured 304.1263.

HPLC: SunFire Waters, C₁₈ 5 µm, 4.6 × 150 mm; gradient 60% to 98% MeCN/H₂O, 1.0 mL/min, 214 nm; t_{R} (major) = 4.3 min, t_{R} (minor) = 4.0 min.

### EXAMPLE 3: 1-benzyl-4-(3-(trifluoromethyl)phenyl)-1,4-azaphosphinane 4-oxide (TA18)

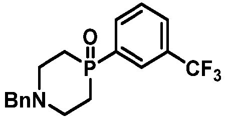

Compound 3 was prepared according to General procedure 2. Column chromatography purification (DCM/MeOH 10/0 to 9/1) afforded the product as a colorless solid (148 mg, 88%).

¹H NMR (400 MHz, CDCl₃): δ 8.10 - 8.00 (m, 2H), 7.86 - 7.81 (m, 1H), 7.69 (tdt, *J =* 7.8, 2.5, 0.8 Hz, 1H), 7.44 - 7.29 (m, 5H), 3.70 (s, 2H), 3.21 - 2.93 (m, 4H), 2.23 (ddt, *J =* 15.5, 10.2, 4.5 Hz, 2H), 2.17 - 2.01 (m, 2H).

¹³C NMR (101 MHz, CDCl₃): δ 138.0, 134.7, 133.9 - 133.5 (m), 129.4 (d, *J* = 11.3 Hz), 128.9, 128.8 - 128.7 (m), 128.5, 127.4, 127.3 - 127.0 (m), 62.7, 49.0 (d, *J =* 7.2 Hz), 28.7 (d, *J* = 66.3 Hz).

¹⁹F NMR (377 MHz, CDCl₃): δ -62.76 (d, *J =* 0.8 Hz).

³¹P NMR (162 MHz, CDCl₃): δ 27.77.

HRMS (ESI-TOF): calc for C₁₈H₂₀F₃NOP [M+H]⁺: 354.1229, measured 354.1237.

HPLC: SunFire Waters, C₁₈ 5 µm, 4.6 × 150 mm; gradient 60% to 98% MeCN/H₂O, 1.0 mL/min, 214 nm; t_{R} = 5.8 min.

### EXAMPLE 4: 1-benzyl-4-(2-(trifluoromethyl)phenyl)-1,4-azaphosphinane 4-oxide (TA4)

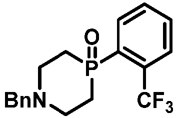

Compound 4 was prepared according to General procedure 2. Column chromatography purification (DCM/MeOH 10/0 to 9/1) afforded the product as a colorless solid (100 mg, 59%).

¹H NMR (400 MHz, CDCl₃): δ 8.26 - 8.08 (m, 1H), 7.92 - 7.85 (m, 1H), 7.78 - 7.62 (m, 2H), 7.41 - 7.22 (m, 5H), 3.69 (s, 2H), 3.21 - 2.94 (m, 4H), 2.50 - 2.32 (m, 2H), 2.31 - 2.08 (m, 2H).

¹³C NMR (101 MHz, CDCl₃): δ 138.2, 133.8 (d, *J =* 7.7 Hz), 132.01, 131.99, 131.9, 131.5 (d, *J =* 5.6 Hz), 131.0, 128.6 (d, *J =* 37.2 Hz), 127.6 (dq, *J =* 7.8, 5.9 Hz), 127.3, 123.9 (dq, *J =* 273.9, 2.9 Hz), 62.3 (d, *J =* 1.1 Hz), 49.2 (d, *J =* 7.4 Hz), 28.9 (dd, *J =* 67.1, 2.3 Hz).

¹⁹F NMR (377 MHz, CDCl₃): δ -56.21 (d, *J =* 1.1 Hz).

³¹P NMR (162 MHz, CDCl₃): δ 30.69.

HRMS (ESI-TOF): calc for C₁₈H₂₀F₃NOP [M+H]⁺: 354.1229, measured 354.1236.

HPLC: SunFire Waters, C₁₈ 5 µm, 4.6 × 150 mm; gradient 60% to 98% MeCN/H₂O, 1.0 mL/min, 214 nm; t_{R} = 5.3 min.

### EXAMPLE 5: 1-benzyl-4-(4-(trifluoromethoxy)phenyl)-1,4-azaphosphinane 4-oxide (TA19)

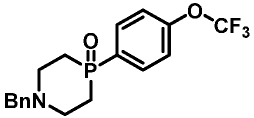

Compound 5 was prepared according to General procedure 2. Column chromatography purification (DCM/MeOH 10/0 to 9/1) afforded the product as a colorless solid (120 mg, 69%).

¹H NMR (400 MHz, CDCl₃): δ 7.89 - 7.77 (m, 2H), 7.39 - 7.26 (m, 7H), 3.66 (s, 2H), 3.11 - 2.85 (m, 4H), 2.31 - 1.96 (m, 4H).

¹³C NMR (101 MHz, CDCl₃): δ 152.0 (dq, *J =* 3.6, 1.8 Hz), 138.0, 132.4 (d, *J =* 10.4 Hz), 131.2 (d, *J* = 97.0 Hz), 128.9, 128.4, 127.4, 120.9 (dq, *J =* 12.2, 1.1 Hz), 120.4 (q, *J =* 258.7

Hz), 62.6 (d, *J =* 1.1 Hz), 49.1 (d, *J* = 7.2 Hz), 28.7 (d, *J =* 66.3 Hz).

¹⁹F NMR (377 MHz, CDCl₃): δ -57.63.

³¹P NMR (162 MHz, CDCl₃): δ 27.53.

HRMS (ESI-TOF): calc for C₁₈H₂₀F₃NO₂P [M+H]⁺: 370.1178, measured 370.1180.

HPLC: SunFire Waters, C₁₈ 5 µm, 4.6 × 150 mm; gradient 60% to 98% MeCN/H₂O, 1.0 mL/min, 214 nm; t_{R} (major) = 6.3 min, t_{R} (minor) = 6.9 min.

### EXAMPLE 6: 1-benzyl-4-(3-fluorophenyl)-1,4-azaphosphinane 4-oxide (TA5)

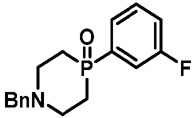

Compound 6 was prepared according to General procedure 2. Column chromatography purification (DCM/MeOH 10/0 to 9/1) afforded the product as a colorless solid (100 mg,69 %).

¹H NMR (400 MHz, CDCl₃): δ 7.62 - 7.48 (m, 3H), 7.40 - 7.22 (m, 6H), 3.68 (s, 2H), 3.18 - 2.91 (m, 4H), 2.26 - 2.12 (m, 2H), 2.15 - 1.99 (m, 2H).

¹³C NMR (101 MHz, CDCl₃): δ 162.7 (dd, *J =* 250.5, 16.1 Hz), 138.1, 135.4 (dd, *J =* 94.9, 5.4 Hz), 130.8 (dd, *J =* 13.2, 7.4 Hz), 128.8, 128.4, 127.4, 125.9 (dd, *J =* 8.8, 3.3 Hz), 119.1 (dd, *J =* 21.1, 2.6 Hz), 117.3 (dd, *J =* 22.2, 10.0 Hz), 62.5 (d, *J* = 1.1 Hz), 49.1 (d, *J* = 7.2 Hz), 28.6 (d, *J =* 66.3 Hz).

¹⁹F NMR (377 MHz, CDCl₃): δ -110.75 (d, *J =* 5.4 Hz).

³¹P NMR (162 MHz, CDCl₃): δ 27.75.

HRMS (ESI-TOF): calc for C₁₇H₂₀FNOP [M+H]⁺: 304.1261, measured 304.1271.

HPLC: SunFire Waters, C₁₈ 5 µm, 4.6 × 150 mm; gradient 60% to 98% MeCN/H₂O, 1.0 mL/min, 214 nm; t_{R} (major) = 4.4 min, t_{R} (minor) = 4.8 min.

### EXAMPLE 7: 1-benzyl-4-(3,5-difluorophenyl)-1,4-azaphosphinane 4-oxide (TA6)

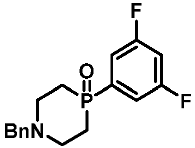

Compound 7 was prepared according to General procedure 2. Column chromatography purification (DCM/MeOH 10/0 to 9/1) afforded the product as a colorless solid (132 mg, 86%).

¹H NMR (400 MHz, CDCl₃): δ 7.40 - 7.26 (m, 7H), 7.03 - 6.95 (m, 1H), 3.67 (s, 2H), 3.17 - 2.89 (m, 4H), 2.27 - 2.00 (m, 4H).

¹³C NMR (101 MHz, CDCl₃): δ 163.1 (ddd, *J =* 253.9, 18.6, 11.2 Hz), 138.0, 137.0 (dt, *J* = 93.3, 6.6 Hz), 128.8, 128.5, 127.4, 113.3 (ddd, *J =* 18.6, 9.8, 7.4 Hz), 107.5 (td, *J =* 25.0, 1.9 Hz), 62.5 (d, *J* = 1.1 Hz), 49.0 (d, *J* = 7.2 Hz), 28.5 (d, *J =* 66.5 Hz).

¹⁹F NMR (377 MHz, CDCl₃): δ -106.78 (d, *J =* 6.0 Hz).

³¹P NMR (162 MHz, CDCl₃): δ 27.76.

HRMS (ESI-TOF): calc for C₁₇H₁₉F₂NOP [M+H]⁺: 322.1167, measured 322.1176.

HPLC: SunFire Waters, C₁₈ 5 µm, 4.6 × 150 mm; gradient 60% to 98% MeCN/H₂O, 1.0 mL/min, 214 nm; t_{R} = 4.8 min.

### EXAMPLE 8: 1-benzyl-4-(4-chlorophenyl)-1,4-azaphosphinane 4-oxide (TA7)

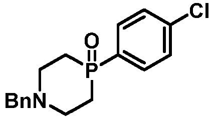

Compound 8 was prepared according to General procedure 2. Column chromatography purification (DCM/MeOH 10/0 to 9/1) afforded the product as a colorless solid (100 mg,65 %).

¹H NMR (400 MHz, CDCl₃): δ 7.78 - 7.70 (m, 2H), 7.54 - 7.48 (m, 2H), 7.40 - 7.27 (m, 5H), 3.67 (s, 2H), 3.13 - 2.89 (m, 4H), 2.16 (d, *J =* 10.5 Hz, 2H), 2.06 (ddt, *J =* 17.5, 15.1, 2.9 Hz, 2H).

¹³C NMR (101 MHz, CDCl₃): δ 138.5 (d, *J =* 3.4 Hz), 138.2, 131.8 (d, *J =* 10.1 Hz), 131.1 (d, *J* = 97.2 Hz), 129.1 (d, *J =* 12.2 Hz), 128.8, 128.4, 127.4, 62.6 (d, *J =* 1.1 Hz), 49.1 (d, *J* = 7.3 Hz), 28.7 (d, *J =* 66.3 Hz).

³¹P NMR (162 MHz, CDCl₃): δ 27.74.

HRMS (ESI-TOF): calc for C₁₇H₂₀ClNOP [M+H]⁺: 320.0966, measured 320.0974.

HPLC: SunFire Waters, C₁₈ 5 µm, 4.6 × 150 mm; gradient 60% to 98% MeCN/H₂O, 1.0 mL/min, 214 nm; t_{R} = 5.2 min.

### EXAMPLE 9: 1-benzyl-4-(3-chlorophenyl)-1,4-azaphosphinane 4-oxide (TA9)

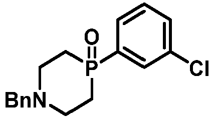

Compound 9 was prepared according to General procedure 2. Column chromatography purification (DCM/MeOH 10/0 to 9/1) afforded the product as a red solid (130 mg,85 %).

¹H NMR (400 MHz, CDCl₃): δ 7.76 (dt, *J =* 11.5, 1.7 Hz, 1H), 7.68 (ddt, *J =* 10.9, 7.4, 1.3 Hz, 1H), 7.55 - 7.51 (m, 1H), 7.46 (td, *J =* 7.7, 3.1 Hz, 1H), 7.39 - 7.25 (m, 5H), 3.67 (s, 2H), 3.15 - 2.89 (m, 4H), 2.27 - 1.91 (m, 4H).

¹³C NMR (101 MHz, CDCl₃): δ 138.1, 135.2 (d, *J =* 14.8 Hz), 135.0 (d, *J =* 94.1 Hz), 132.1 (d, *J =* 2.6 Hz), 130.3, 130.2 (d, *J =* 2.3 Hz), 128.9, 128.44, 128.37 (d, *J =* 8.9 Hz), 127.4, 62.5 (d, *J* = 1.1 Hz), 49.1 (d, *J =* 7.3 Hz), 28.6 (d, *J =* 66.1 Hz).

³¹P NMR (162 MHz, CDCl₃): δ 27.71.

HRMS (ESI-TOF): calc for C₁₇H₂₀ClNOP [M+H]⁺: 320.0966, measured 320.0973.

HPLC: SunFire Waters, C₁₈ 5 µm, 4.6 × 150 mm; gradient 60% to 98% MeCN/H₂O, 1.0 mL/min, 214 nm; t_{R} = 5.1 min.

### EXAMPLE 10: 1-benzyl-4-(2-chlorophenyl)-1,4-azaphosphinane 4-oxide (TA33)

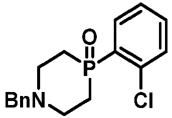

Compound 10 was prepared according to General procedure 2. Column chromatography purification (DCM/MeOH 10/0 to 9/1) afforded the product as a colorless solid (110 mg,72 %).

¹H NMR (400 MHz, CDCl₃): δ 8.05 (dddd, *J* = 12.1, 7.3, 1.8, 0.7 Hz, 1H), 7.51 - 7.39 (m, 3H), 7.38 - 7.30 (m, 4H), 7.29 - 7.21 (m, 1H), 3.67 (s, 2H), 3.20 - 2.95 (m, 4H), 2.77 - 2.66 (m, 2H), 2.03 - 1.89 (m, 2H).

¹³C NMR (101 MHz, CDCl₃): δ 138.4, 135.6 (d, *J =* 5.2 Hz), 135.1 (d, *J =* 6.7 Hz), 133.5 (d, *J =* 2.5 Hz), 130.9 (d, *J* = 7.1 Hz), 130.5 (d, *J =* 93.7 Hz), 128.8, 128.4, 127.3, 127.1 (d, *J* = 9.9 Hz), 62.1 (d, *J =* 1.1 Hz), 49.0 (d, *J =* 7.5 Hz), 27.1 (d, *J* = 67.0 Hz).

³¹P NMR (162 MHz, CDCl₃): δ 28.90.

HRMS (ESI-TOF): calc for C₁₇H₂₀ClNOP [M+H]⁺: 320.0966, measured 320.0966.

HPLC: SunFire Waters, C₁₈ 5 µm, 4.6 × 150 mm; gradient 60% to 98% MeCN/H₂O, 1.0 mL/min, 214 nm; t_{R} = 5.0 min.

### EXAMPLE 11: 1-benzyl-4-(3,4-dichlorophenyl)-1,4-azaphosphinane 4-oxide (TA8)

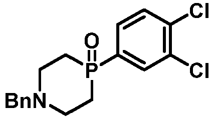

Compound 11 was prepared according to General procedure 2. Column chromatography purification (DCM/MeOH 10/0 to 9/1) afforded the product as a colorless solid (136 mg, 96%).

¹H NMR (400 MHz, CDCl₃): δ 7.90 - 7.85 (m, 1H), 7.65 - 7.59 (m, 2H), 7.39 - 7.26 (m, 5H), 3.67 (s, 2H), 3.15 - 2.85 (m, 4H), 2.23 - 2.01 (m, 4H).

¹³C NMR (101 MHz, CDCl₃): δ 138.0, 136.7 (d, *J =* 3.1 Hz), 133.6 (d, *J =* 15.3 Hz), 133.1 (d, *J* = 94.3 Hz), 132.3 (d, *J* = 10.8 Hz), 131.1 (d, *J =* 12.4 Hz), 129.4 (d, *J =* 9.3 Hz), 128.8, 128.5, 127.4, 62.6 (d, *J =* 1.1 Hz), 49.0 (d, *J* = 7.2 Hz), 28.7 (d, *J =* 66.3 Hz).

³¹P NMR (162 MHz, CDCl₃): δ 27.35.

HRMS (ESI-TOF): calc for C₁₇H₁₉Cl₂NOP [M+H]⁺: 354.0576, measured 354.0577.

HPLC: SunFire Waters, C₁₈ 5 µm, 4.6 × 150 mm; gradient 60% to 98% MeCN/H₂O, 1.0 mL/min, 214 nm; t_{R} (major) = 6.7 min, t_{R} (minor) = 8.6 min.

### EXAMPLE 12: 1-benzyl-4-(2,3-dichlorophenyl)-1,4-azaphosphinane 4-oxide (TA34)

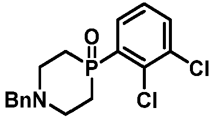

Compound 12 was prepared according to General procedure 2. Column chromatography purification (DCM/MeOH 10/0 to 9/1) afforded the product as a yellow liquid (70 mg, 41%).

¹H NMR (400 MHz, CDCl₃): δ 8.02 (ddd, *J =* 11.8, 7.7, 1.6 Hz, 1H), 7.69 (ddd, *J =* 8.0, 1.6, 0.7 Hz, 1H), 7.46 - 7.42 (m, 1H), 7.41 - 7.35 (m, 4H), 7.34 - 7.30 (m, 1H), 3.71 (s, 2H), 3.25 - 3.01 (m, 4H), 2.84 - 2.69 (m, 2H), 2.10 - 1.94 (m, 2H).

¹³C NMR (101 MHz, CDCl₃): δ 138.3, 134.7 (d, *J =* 9.6 Hz), 134.2 (d, *J =* 2.4 Hz), 133.5 (d, *J =* 6.2 Hz), 133.4 (d, *J* = 6.8 Hz), 133.0 (d, *J* = 91.3 Hz), 128.8, 128.5, 127.9 (d, *J =* 11.3 Hz), 127.3, 62.2, 49.0 (d, *J* = 7.4 Hz), 27.1 (d, *J =* 67.2 Hz).

³¹P NMR (162 MHz, CDCl₃): δ 29.71.

HRMS (ESI-TOF): calc for C₁₇H₁₉Cl₂NOP [M+H]⁺: 354.0576, measured 354.0572.

HPLC: SunFire Waters, C₁₈ 5 µm, 4.6 × 150 mm; gradient 60% to 98% MeCN/H₂O, 1.0 mL/min, 214 nm; t_{R} = 6.5 min.

### EXAMPLE 13: 1-benzyl-4-phenyl-1,4-azaphosphinane 4-oxide (TA3)

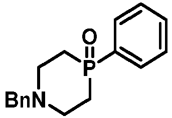

Compound 13 was prepared according to General procedure 2. Column chromatography purification (DCM/MeOH 10/0 to 9/1) afforded the product as a colorless solid (110 mg, 81%).

¹H NMR (400 MHz, CDCl₃): δ 7.84 - 7.78 (m, 2H), 7.61 - 7.49 (m, 3H), 7.41 - 7.27 (m, 5H), 3.68 (s, 2H), 3.14 - 2.89 (m, 4H), 2.33 - 2.15 (m, 2H), 2.12 - 2.01 (m, 2H).

¹³C NMR (101 MHz, CDCl₃): δ 138.2, 132.6 (d, *J =* 97.0 Hz), 132.0 (d, *J =* 2.8 Hz), 130.2 (d, *J =* 9.4 Hz), 128.9, 128.8 (d, *J =* 11.7 Hz), 128.4, 127.3, 62.5 (d, *J* = 1.1 Hz), 49.2 (d, *J* = 7.1 Hz), 28.5 (d, *J =* 65.9 Hz).

³¹P NMR (162 MHz, CDCl₃): δ 28.03.

HRMS (ESI-TOF): calc for C₁₇H₂₁NOP [M+H]⁺: 286.1355, measured 286.1361.

HPLC: SunFire Waters, C₁₈ 5 µm, 4.6 × 150 mm; gradient 60% to 98% MeCN/H₂O, 1.0 mL/min, 214 nm; t_{R} = 4.1 min.

### EXAMPLE 14: 1-benzyl-4-(p-tolyl)-1,4-azaphosphinane 4-oxide (TA14)

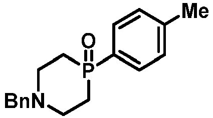

Compound 14 was prepared according to General procedure 2. Column chromatography purification (DCM/MeOH 10/0 to 9/1) afforded the product as a colorless solid (142 mg, 99%).

¹H NMR (400 MHz, CDCl₃): δ 7.69 - 7.62 (m, 2H), 7.39 - 7.22 (m, 7H), 3.64 (s, 2H), 3.05 - 2.90 (m, 4H), 2.41 (s, 3H), 2.23 - 2.11 (m, 2H), 2.08 - 1.95 (m, 2H).

¹³C NMR (101 MHz, CDCl₃): δ 142.5 (d, *J* = 2.9 Hz), 138.3, 130.3 (d, *J* = 9.7 Hz), 129.5 (d, *J* = 12.0 Hz), 129.2 (d, *J =* 99.6 Hz), 128.9, 128.4, 127.3, 62.6, 49.3 (d, *J* = 7.1 Hz), 28.6 (d, *J =* 66.0 Hz), 21.6 (d, *J =* 1.3 Hz).

³¹P NMR (162 MHz, CDCl₃): δ 28.10.

HRMS (ESI-TOF): calc for C₁₈H₂₃NOP [M+H]⁺: 300.1512, measured 300.1520.

HPLC: SunFire Waters, C₁₈ 5 µm, 4.6 × 150 mm; gradient 60% to 98% MeCN/H₂O, 1.0 mL/min, 214 nm; t_{R} (major) = 4.7 min, t_{R} (minor) = 4.1 min.

### EXAMPLE 15: 1-benzyl-4-(m-tolyl)-1,4-azaphosphinane 4-oxide (TA13)

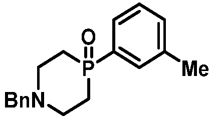

Compound 15 was prepared according to General procedure 2. Column chromatography purification (DCM/MeOH 10/0 to 9/1) afforded the product as a colorless solid (118 mg, 83%).

¹H NMR (400 MHz, CDCl₃): δ 7.60 (dtt, *J =* 11.8, 1.5, 0.7 Hz, 1H), 7.58 - 7.52 (m, 1H), 7.42 - 7.31 (m, 6H), 7.30 - 7.25 (m, 1H), 3.65 (s, 2H), 3.11 - 2.88 (m, 4H), 2.41 (s, 3H), 2.27 - 2.12 (m, 2H), 2.09 - 1.97 (m, 2H).

¹³C NMR (101 MHz, CDCl₃): δ 138.7 (d, *J =* 11.4 Hz), 138.3, 132.5 (d, *J =* 96.3 Hz), 132.8 (d, *J =* 3.0 Hz), 130.9 (d, *J =* 9.2 Hz), 128.9, 128.7 (d, *J =* 12.2 Hz), 128.4, 127.3, 127.2 (d, *J =* 9.5 Hz), 62.6, 49.2 (d, *J* = 7.2 Hz), 28.6 (d, *J =* 65.8 Hz), 21.4.

³¹P NMR (162 MHz, CDCl₃): δ 28.12.

HRMS (ESI-TOF): calc for C₁₈H₂₃NOP [M+H]⁺: 300.1512, measured 300.1519.

HPLC: SunFire Waters, C₁₈ 5 µm, 4.6 × 150 mm; gradient 60% to 98% MeCN/H₂O, 1.0 mL/min, 214 nm; t_{R} = 4.8 min.

**EXAMPLE 16:** 1-benzyl-4-(o-tolyl)-1,4-azaphosphinane 4-oxide (TA28)

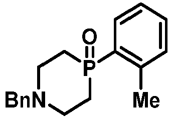

Compound 16 was prepared according to General procedure 2. Column chromatography purification (DCM/MeOH 10/0 to 9/1) afforded the product as a colorless solid (142 mg, 99%).

¹H NMR (400 MHz, CDCl₃): δ 7.55 (ddd, *J =* 12.6, 8.0, 1.5 Hz, 1H), 7.41 (tt, *J* = 7.5, 1.5 Hz, 1H), 7.35 - 7.23 (m, 7H), 3.63 (s, 2H), 3.05 - 2.93 (m, 4H), 2.71 (s, 3H), 2.34 - 2.12 (m, 4H).

¹³C NMR (101 MHz, CDCl₃): δ 142.1 (d, *J =* 7.8 Hz), 138.4, 132.1 (d, *J =* 10.3 Hz), 131.9 (d, *J =* 2.7 Hz), 130.6 (d, *J* = 95.7 Hz), 130.5 (d, *J* = 11.1 Hz), 128.8, 128.4, 127.3, 125.7 (d, *J =* 11.9 Hz), 62.5 (d, *J =* 1.1 Hz), 49.3 (d, *J =* 7.0 Hz), 28.4 (d, *J =* 65.7 Hz), 21.4 (d, *J =* 4.0 Hz).

³¹P NMR (162 MHz, CDCl₃): δ 30.08.

HRMS (ESI-TOF): calc for C₁₈H₂₃NOP [M+H]⁺: 300.1512, measured 300.1518.

HPLC: SunFire Waters, C₁₈ 5 µm, 4.6 × 150 mm; gradient 60% to 98% MeCN/H₂O, 1.0 mL/min, 214 nm; t_{R} (major) = 4.6 min, t_{R} (minor) = 4.1 min.

### EXAMPLE 17: 1-benzyl-4-(4-(tert-butyl)phenyl)-1,4-azaphosphinane 4-oxide (TA25)

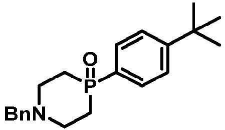

Compound 17 was prepared according to General procedure 2. Column chromatography purification (DCM/MeOH 10/0 to 9/1) afforded the product as a colorless solid (146 mg, 90%).

¹H NMR (400 MHz, CDCl₃): δ 7.73 - 7.65 (m, 2H), 7.54 - 7.48 (m, 2H), 7.37 - 7.29 (m, 4H), 7.28 - 7.21 (m, 1H), 3.64 (s, 2H), 3.09 - 2.90 (m, 4H), 2.25 - 2.12 (m, 2H), 2.10 - 1.96 (m, 2H), 1.32 (s, 9H).

¹³C NMR (101 MHz, CDCl₃): δ 155.5 (d, *J* = 2.9 Hz), 138.2, 130.2 (d, *J* = 9.7 Hz), 129.2 (d, *J* = 99.8 Hz), 128.9, 128.4, 127.3, 125.8 (d, *J =* 11.8 Hz), 62.5 (d, *J =* 1.1 Hz), 49.2 (d, *J =* 7.1 Hz), 35.0, 31.1, 28.6 (d, *J =* 65.9 Hz).

³¹P NMR (162 MHz, CDCl₃): δ 27.80.

HRMS (ESI-TOF): calc for C₂₁H₂₉NOP [M+H]⁺: 342.1981, measured 342.1982.

HPLC: SunFire Waters, C₁₈ 5 µm, 4.6 × 150 mm; gradient 60% to 98% MeCN/H₂O, 1.0 mL/min, 214 nm; t_{R} (major) = 8.4 min, t_{R} (minor) = 4.1 min.

### EXAMPLE 18: 1-benzyl-4-(naphthalen-1-yl)-1,4-azaphosphinane 4-oxide (TA26)

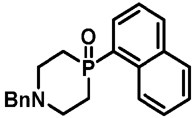

Compound 18 was prepared according to General procedure 2. Column chromatography purification (DCM/MeOH 10/0 to 9/1) afforded the product as a colorless solid (138 mg, 86%).

¹H NMR (400 MHz, CDCl₃): δ 8.79 (dt, *J =* 8.5, 1.0 Hz, 1H), 8.00 (dq, *J =* 8.3, 1.3 Hz, 1H), 7.91 (dt, *J =* 8.1, 1.7 Hz, 1H), 7.85 (ddd, *J =* 14.4, 7.1, 1.3 Hz, 1H), 7.62 (ddd, *J =* 8.5, 6.9, 1.5 Hz, 1H), 7.59 - 7.47 (m, 2H), 7.38 - 7.24 (m, 5H), 3.66 (s, 2H), 3.18 - 2.89 (m, 4H), 2.53 - 2.40 (m, 2H), 2.31 (dddt, *J =* 17.7, 14.9, 5.6, 2.5 Hz, 2H).

¹³C NMR (101 MHz, CDCl₃): δ 143.7, 138.2, 133.9 (d, *J =* 8.9 Hz), 133.2 (d, *J =* 8.4 Hz), 133.1 (d, *J* = 2.9 Hz), 130.5 (d, *J* = 9.6 Hz), 129.3 (d, *J =* 1.3 Hz), 128.9, 128.8 (d, *J =* 94.3 Hz), 128.5, 128.4, 127.5, 127.4, 126.5, 126.2 (d, *J =* 5.0 Hz), 124.5 (d, *J =* 13.2 Hz), 62.5 (d, *J =* 1.0 Hz), 49.5 (d, *J* = 7.0 Hz), 28.9 (d, *J =* 65.8 Hz).

³¹P NMR (162 MHz, CDCl₃): δ 29.71.

HRMS (ESI-TOF): calc for C₂₁H₂₃NOP [M+H]⁺: 336.1512, measured 336.1514.

HPLC: SunFire Waters, C₁₈ 5 µm, 4.6 × 150 mm; gradient 60% to 98% MeCN/H₂O, 1.0 mL/min, 214 nm; t_{R} (major) = 5.9 min, t_{R} (minor) = 8.6 min.

### EXAMPLE 19: 1-benzyl-4-(naphthalen-2-yl)-1,4-azaphosphinane 4-oxide (TA29)

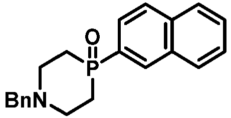

Compound 19 was prepared according to General procedure 2. Column chromatography purification (DCM/MeOH 10/0 to 9/1) afforded the product as a colorless solid (152 mg, 85%).

¹H NMR (400 MHz, CDCl₃): δ 8.39 (dt, *J =* 13.8, 1.2 Hz, 1H), 7.97 - 7.92 (m, 2H), 7.90 - 7.84 (m, 1H), 7.77 - 7.70 (m, 1H), 7.63 - 7.54 (m, 2H), 7.40 - 7.32 (m, 4H), 7.30 - 7.25 (m, 1H), 3.68 (s, 2H), 3.15 - 2.92 (m, 4H), 2.30 (s, 2H), 2.09 (ddt, *J =* 17.7, 15.0, 2.9 Hz, 2H).

¹³C NMR (101 MHz, CDCl₃): δ 138.1, 134.7 (d, *J =* 2.5 Hz), 132.6 (d, *J =* 12.6 Hz), 132.5 (d, *J* = 8.6 Hz), 130.0, 128.9, 128.8, 128.7 (d, *J* = 11.5 Hz), 128.5, 128.2 (d, *J* = 0.7 Hz), 127.9 (d, *J* = 1.1 Hz), 127.4, 127.1 (d, *J =* 0.9 Hz), 124.9 (d, *J =* 10.6 Hz), 62.6 (d, *J =* 1.1 Hz), 49.2 (d, *J =* 7.2 Hz), 28.6 (d, *J =* 65.9 Hz).

³¹P NMR (162 MHz, CDCl₃): δ 28.12.

HRMS (ESI-TOF): calc for C₂₁H₂₃NOP [M+H]⁺: 336.1512, measured 336.1501.

HPLC: SunFire Waters, C₁₈ 5 µm, 4.6 × 150 mm; gradient 60% to 98% MeCN/H₂O, 1.0 mL/min, 214 nm; t_{R} = 5.9 min.

### EXAMPLE 20: 1-benzyl-4-(4-methoxyphenyl)-1,4-azaphosphinane 4-oxide (TA16)

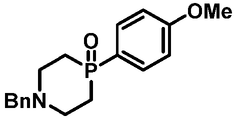

Compound 20 was prepared according to General procedure 2. Column chromatography purification (DCM/MeOH 10/0 to 9/1) afforded the product as a colorless solid (62 mg, 41%).

¹H NMR (400 MHz, CDCl₃): δ 7.72 - 7.65 (m, 2H), 7.36 - 7.21 (m, 5H), 7.02 - 6.96 (m, 2H), 3.84 (s, 3H), 3.63 (s, 2H), 3.07 - 2.87 (m, 4H), 2.14 (ddt, *J =* 15.1, 10.6, 5.0 Hz, 2H), 2.06 - 1.94 (m, 2H).

¹³C NMR (101 MHz, CDCl₃): δ 162.5 (d, *J* = 2.9 Hz), 138.3, 132.1 (d, *J =* 10.6 Hz), 128.9, 128.4, 127.3, 123.6 (d, *J=* 103.1 Hz), 114.4 (d, *J =* 12.5 Hz), 62.6, 55.4, 49.3 (d, *J* = 7.1 Hz), 28.8 (d, *J* = 66.4 Hz).

³¹P NMR (162 MHz, CDCl₃): δ 27.80.

HRMS (ESI-TOF): calc for C₁₈H₂₃NO₂P [M+H]⁺: 316.1461, measured 316.1466.

HPLC: SunFire Waters, C₁₈ 5 µm, 4.6 × 150 mm; gradient 60% to 98% MeCN/H₂O, 1.0 mL/min, 214 nm; t_{R} = 4.2 min.

### EXAMPLE 21: 1-benzyl-4-(3-methoxyphenyl)-1,4-azaphosphinane 4-oxide (TA15)

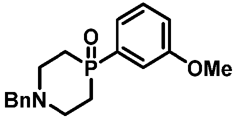

Compound 21 was prepared according to General procedure 2. Column chromatography purification (DCM/MeOH 10/0 to 9/1) afforded the product as a colorless solid (112 mg, 74%).

¹H NMR (400 MHz, CDCl₃): δ 7.39 (ddd, J = 8.2, 7.4, 3.8 Hz, 1H), 7.36 - 7.29 (m, 6H), 7.29 - 7.23 (m, 1H), 7.05 (ddt, *J =* 8.2, 2.7, 1.0 Hz, 1H), 3.83 (s, 3H), 3.63 (s, 2H), 3.08 - 2.87 (m, 4H), 2.17 (ddt, *J =* 15.4, 10.5, 4.7 Hz, 2H), 2.02 (ddt, *J =* 17.5, 14.9, 2.9 Hz, 2H).

¹³C NMR (101 MHz, CDCl₃): δ 159.8 (d, *J =* 14.2 Hz), 138.2, 134.0 (d, *J =* 96.0 Hz), 130.0 (d, *J* = 13.8 Hz), 128.8, 128.4, 127.3, 122.1 (d, *J =* 9.6 Hz), 118.0 (d, *J =* 2.7 Hz), 115.4 (d, *J =* 10.1 Hz), 62.5 (d, *J =* 1.0 Hz), 55.5, 49.2 (d, *J =* 7.1 Hz), 28.6 (d, *J =* 65.9 Hz).

³¹P NMR (162 MHz, CDCl₃): δ 28.42.

HRMS (ESI-TOF): calc for C₁₈H₂₃NO₂P [M+H]⁺: 316.1461, measured 316.1470.

HPLC: SunFire Waters, C₁₈ 5 µm, 4.6 × 150 mm; gradient 60% to 98% MeCN/H₂O, 1.0 mL/min, 214 nm; t_{R} (major) = 4.4 min, t_{R} (minor) = 4.1 min, t_{R} (minor) = 8.6 min.

### EXAMPLE 22: 1-benzyl-4-(2-methoxyphenyl)-1,4-azaphosphinane 4-oxide (TA27)

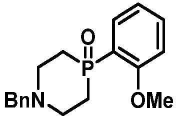

Compound 22 was prepared according to General procedure 2. Column chromatography purification (DCM/MeOH 10/0 to 9/1) afforded the product as a colorless solid (137 mg, 91%).

¹H NMR (400 MHz, CDCl₃): δ 7.89 (ddd, *J* = 12.9, 7.5, 1.8 Hz, 1H), 7.51 (dddd, *J* = 8.3, 7.4, 1.8, 0.9 Hz, 1H), 7.40 - 7.30 (m, 4H), 7.29 - 7.22 (m, 1H), 7.09 (tdd, *J* = 7.5, 1.8, 0.9 Hz, 1H), 6.93 (ddd, *J =* 8.5, 5.2, 0.9 Hz, 1H), 3.93 (s, 3H), 3.67 (s, 2H), 3.10 - 2.92 (m, 4H), 2.62 - 2.45 (m, 2H), 1.88 (tdd, *J =* 15.6, 5.1, 2.9 Hz, 2H).

¹³C NMR (101 MHz, CDCl₃): δ 160.2 (d, *J =* 4.3 Hz), 138.6, 134.1 (d, *J =* 5.8 Hz), 134.0 (d, *J =* 2.2 Hz), 128.8, 128.4, 127.2, 121.0 (d, *J =* 10.8 Hz), 119.7 (d, *J =* 95.2 Hz), 110.8 (d, *J* = 6.6 Hz), 61.8 (d, *J* = 1.1 Hz), 55.5, 48.9 (d, *J* = 7.4 Hz), 27.1 (d, *J =* 66.8 Hz).

³¹P NMR (162 MHz, CDCl₃): δ 27.63.

HRMS (ESI-TOF): calc for C₁₈H₂₃NO₂P [M+H]⁺: 316.1461, measured 316.1459.

HPLC: SunFire Waters, C₁₈ 5 µm, 4.6 × 150 mm; gradient 60% to 98% MeCN/H₂O, 1.0 mL/min, 214 nm; t_{R} (major) = 4.4 min, t_{R} (minor) = 4.1 min.

### EXAMPLE 23: 1-benzyl-4-(4-(benzyloxy)phenyl)-1,4-azaphosphinane 4-oxide (TA20)

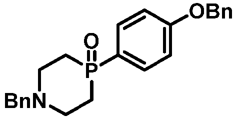

Compound 23 was prepared according to General procedure 2. Column chromatography purification (DCM/MeOH 10/0 to 9/1) afforded the product as a colorless solid (140 mg, 75%).

¹H NMR (400 MHz, CDCl₃): δ 7.73 - 7.66 (m, 2H), 7.46 - 7.25 (m, 10H), 7.11 - 7.05 (m, 2H), 5.11 (s, 2H), 3.64 (s, 2H), 3.10 - 2.87 (m, 4H), 2.15 (ddt, *J =* 15.1, 10.7, 5.0 Hz, 2H), 2.08 - 1.95 (m, 2H).

¹³C NMR (101 MHz, CDCl₃): δ 161.7 (d, *J =* 2.9 Hz), 138.3, 136.2, 132.1 (d, *J =* 10.6 Hz), 128.9, 128.7, 128.4, 128.3, 127.5, 127.3, 124.0 (d, *J* = 102.7 Hz), 115.2 (d, *J* = 12.5 Hz), 70.1, 62.6 (d, *J* = 1.1 Hz), 49.3 (d, *J* = 7.1 Hz), 28.8 (d, *J =* 66.3 Hz).

³¹P NMR (162 MHz, CDCl₃): δ 27.66.

HRMS (ESI-TOF): calc for C₂₄H₂₇NO₂P [M+H]⁺: 397.1774, measured 397.1773.

HPLC: SunFire Waters, C₁₈ 5 µm, 4.6 × 150 mm; gradient 60% to 98% MeCN/H₂O, 1.0 mL/min, 214 nm; t_{R} = 7.7 min.

### EXAMPLE 24: 1-benzyl-4-(2-(methylthio)phenyl)-1,4-azaphosphinane 4-oxide (TA32)

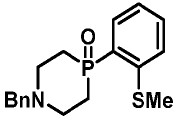

Compound 24 was prepared according to General procedure 2. Column chromatography purification (DCM/MeOH 10/0 to 9/1) afforded the product as an orange oil (78 mg,49 %).

¹H NMR (400 MHz, CDCl₃): δ 8.87 (tq, *J* = 1.5, 0.8 Hz, 1H), 8.01 (ddq, *J =* 3.2, 2.0, 0.6 Hz, 1H), 7.44 - 7.21 (m, 5H), 3.59 (s, 2H), 3.22 - 2.98 (m, 2H), 2.90 - 2.55 (m, 4H), 2.48 - 2.30 (m, 2H).

¹³C NMR (101 MHz, CDCl₃): 141.8 (d, *J =* 8.1 Hz), 138.2, 133.1 (d, *J =* 8.7 Hz), 132.4 (d, *J =* 2.5 Hz), 130.9 (d, *J =* 95.3 Hz), 128.9, 128.4, 128.3 (d, *J* = 9.1 Hz), 127.3, 125.4 (d, *J =* 10.8 Hz), 62.0 (d, *J =* 1.0 Hz), 49.1 (d, *J =* 7.3 Hz), 27.3 (d, *J =* 66.1 Hz), 17.6.

³¹P NMR (162 MHz, CDCl₃): δ 29.18.

HRMS (ESI-TOF): calc for C₁₈H₂₃NOPS [M+H]⁺: 332.1232, measured 332.1231.

HPLC: SunFire Waters, C₁₈ 5 µm, 4.6 × 150 mm; gradient 60% to 98% MeCN/H₂O, 1.0 mL/min, 214 nm; t_{R} (major) = 5.0 min, t_{R} (minor) = 4.1 min, t_{R} (minor) = 8.6 min.

### EXAMPLE 25: methyl 4-(1-benzyl-4-oxido-1,4-azaphosphinan-4-yl)benzoate(TA24)

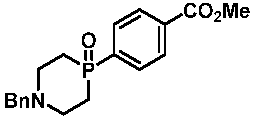

Compound 25 was prepared according to General procedure 2. Column chromatography purification (DCM/MeOH 10/0 to 9/1) afforded the product as a colorless solid (130 mg, 79%).

¹H NMR (400 MHz, CDCl₃): δ 8.17 - 8.11 (m, 2H), 7.91 - 7.81 (m, 2H), 7.39 - 7.20 (m, 5H), 3.93 (s, 3H), 3.64 (s, 2H), 3.12 - 2.87 (m, 4H), 2.28 - 2.13 (m, 2H), 2.13 - 1.96 (m, 2H).

¹³C NMR (101 MHz, CDCl₃): δ 166.1 (d, *J =* 0.9 Hz), 138.0, 137.5 (d, *J =* 99.7 Hz), 133.2 (d, *J =* 2.9 Hz), 130.4 (d, *J* = 9.5 Hz), 129.7 (d, *J =* 11.6 Hz), 128.8, 128.4, 127.3, 62.5 (d, *J* = 1.1 Hz), 52.4, 49.0 (d, *J* = 7.2 Hz), 28.4 (d, *J =* 65.9 Hz).

³¹P NMR (162 MHz, CDCl₃): δ 27.98.

HRMS (ESI-TOF): calc for C₁₉H₂₃NO₃P [M+H]⁺: 344.1410, measured 344.1411.

HPLC: SunFire Waters, C₁₈ 5 µm, 4.6 × 150 mm; gradient 60% to 98% MeCN/H₂O, 1.0 mL/min, 214 nm; t_{R} = 4.2 min.

### EXAMPLE 26: methyl 3-(1-benzyl-4-oxido-1,4-azaphosphinan-4-yl)benzoate (TA23)

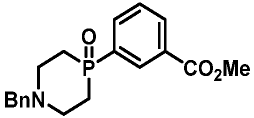

Compound 26 was prepared according to General procedure 2. Column chromatography purification (DCM/MeOH 10/0 to 9/1) afforded the product as a colorless oil (122 mg, 74%).

¹H NMR (400 MHz, CDCl₃): δ 8.36 (dtd, *J* = 11.6, 1.6, 0.6 Hz, 1H), 8.21 - 8.16 (m, 1H), 8.02 (ddt, *J =* 11.1, 7.6, 1.4 Hz, 1H), 7.58 (tdd, *J* = 7.8, 2.5, 0.6 Hz, 1H), 7.38 - 7.19 (m, 5H), 3.92 (s, 3H), 3.63 (s, 2H), 3.11 - 2.89 (m, 4H), 2.28 - 2.15 (m, 2H), 2.11 - 1.98 (m, 2H).

¹³C NMR (101 MHz, CDCl₃): δ 166.1 (d, *J =* 1.4 Hz), 138.0, 134.9 (d, *J =* 8.9 Hz), 133.9, 132.9 (d, *J* = 2.6 Hz), 131.1 (d, *J =* 10.8 Hz), 130.7 (d, *J =* 11.6 Hz), 129.1 (d, *J =* 11.2 Hz), 128.9, 128.4, 127.1, 62.6 (d, *J =* 1.1 Hz), 52.4, 49.1 (d, *J =* 7.2 Hz), 28.5 (d, *J =* 66.1 Hz).

³¹P NMR (162 MHz, CDCl₃): δ 28.03.

HRMS (ESI-TOF): calc for C₁₉H₂₃NO₃P [M+H]⁺: 344.1410, measured 344.1411.

HPLC: SunFire Waters, C₁₈ 5 µm, 4.6 × 150 mm; gradient 60% to 98% MeCN/H₂O, 1.0 mL/min, 214 nm; t_{R} = 4.2 min.

### EXAMPLE 27: 4-(1-benzyl-4-oxido-1,4-azaphosphinan-4-yl)benzonitrile (TA22)

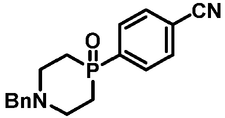

Compound 27 was prepared according to General procedure 2. Column chromatography purification (DCM/MeOH 10/0 to 9/1) afforded the product as a colorless solid (128 mg, 86%).

¹H NMR (400 MHz, CDCl₃): δ 7.94 - 7.86 (m, 2H), 7.83 - 7.75 (m, 2H), 7.38 - 7.22 (m, 5H), 3.64 (s, 2H), 3.17 - 2.88 (m, 4H), 2.29 - 1.96 (m, 4H).

¹³C NMR (101 MHz, CDCl₃): δ 138.2 (d, *J* = 91.5 Hz), 137.9, 132.3 (d, *J =* 11.3 Hz), 131.1 (d, *J* = 9.4 Hz), 128.8, 128.5, 127.4, 117.8 (d, *J* = 1.6 Hz), 115.7 (d, *J* = 3.2 Hz), 62.5 (d,*J* = 1.1 Hz), 48.9 (d, *J =* 7.2 Hz), 28.4 (d, *J =* 66.2 Hz).

³¹P NMR (162 MHz, CDCl₃): δ 27.74.

HRMS (ESI-TOF): calc for C₁₈H₂₀N₂OP [M+H]⁺: 311.1308, measured 311.1318.

HPLC: SunFire Waters, C₁₈ 5 µm, 4.6 × 150 mm; gradient 60% to 98% MeCN/H₂O, 1.0 mL/min, 214 nm; t_{R} = 3.9 min.

### EXAMPLE 28: 3-(1-benzyl-4-oxido-1,4-azaphosphinan-4-yl)benzonitrile (TA21)

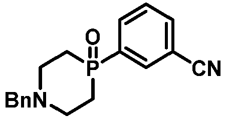

Compound 28 was prepared according to General procedure 2. Column chromatography purification (DCM/MeOH 10/0 to 9/1) afforded the product as a colorless solid (146 mg, 99%).

¹H NMR (400 MHz, CDCl₃): δ 8.09 -7.99 (m, 2H), 7.80 (dq, *J =* 7.8, 1.4 Hz, 1H), 7.63 (tdd, *J =* 7.7, 2.5, 0.7 Hz, 1H), 7.36 - 7.20 (m, 5H), 3.65 (s, 2H), 3.15 - 2.87 (m, 4H), 2.17 (q, *J* = 10.6 Hz, 2H), 2.12 - 2.00 (m, 2H).

¹³C NMR (101 MHz, CDCl₃): δ 137.7, 135.2 (d, *J =* 2.7 Hz), 134.8 (d, *J =* 92.0 Hz), 134.5 (d, *J* = 8.7 Hz), 134.0 (d, *J =* 10.3 Hz), 129.7 (d, *J =* 11.0 Hz), 128.9, 128.46, 127.5, 117.8 (d, *J =* 1.3 Hz), 113.4 (d, *J =* 13.1 Hz), 62.5 (d, *J =* 1.0 Hz), 48.9 (d, *J =* 7.2 Hz), 28.5 (d, *J* = 66.3 Hz).

³¹P NMR (162 MHz, CDCl₃): δ 27.51.

HRMS (ESI-TOF): calc for C₁₈H₂₀N₂OP [M+H]⁺: 311.1308, measured 311.1312.

HPLC: SunFire Waters, C₁₈ 5 µm, 4.6 × 150 mm; gradient 60% to 98% MeCN/H₂O, 1.0 mL/min, 214 nm; t_{R} = 3.9 min.

### EXAMPLE 29: 2-(1-benzyl-4-oxido-1,4-azaphosphinan-4-yl)benzonitrile (TA30)

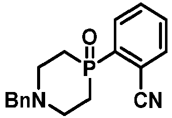

Compound 29 was prepared according to General procedure 2. Column chromatography purification (DCM/MeOH 10/0 to 9/1) afforded the product as a colorless solid (110 mg, 74%).

¹H NMR (400 MHz, CDCl₃): δ 8.24 (dddd, *J =* 11.9, 7.7, 1.3, 0.6 Hz, 1H), 7.84 (dddd, *J* = 7.6, 3.6, 1.3, 0.6 Hz, 1H), 7.78 (tt, *J* = 7.7, 1.5 Hz, 1H), 7.67 (tt, *J =* 7.6, 1.4 Hz, 1H), 7.42 - 7.37 (m, 2H), 7.36 - 7.31 (m, 2H), 7.29 - 7.23 (m, 1H), 3.73 (s, 2H), 3.27 - 3.02 (m, 4H), 2.77 - 2.61 (m, 2H), 2.06 - 1.88 (m, 2H).

¹³C NMR (101 MHz, CDCl₃): δ 138.4, 136.0 (d, *J =* 86.5 Hz), 134.7 (d, *J =* 8.0 Hz), 134.1 (d, *J =* 6.1 Hz), 132.9 (d, *J* = 9.9 Hz), 132.2 (d, *J =* 2.4 Hz), 128.7, 128.5, 127.3, 117.8 (d, *J* = 4.6 Hz), 112.9 (d, *J =* 6.7 Hz), 61.6 (d, *J =* 1.1 Hz), 48.5 (d, *J* = 7.4 Hz), 27.1 (d, *J =* 66.4 Hz).

³¹P NMR (162 MHz, CDCl₃): δ 27.94.

HRMS (ESI-TOF): calc for C₁₈H₂₀N₂OP [M+H]⁺: 311.1308, measured 311.1304.

HPLC: SunFire Waters, C₁₈ 5 µm, 4.6 × 150 mm; gradient 60% to 98% MeCN/H₂O, 1.0 mL/min, 214 nm; t_{R} = 4.2 min.

### EXAMPLE 30: 1-benzyl-4-(pyridin-2-yl)-1,4-azaphosphinane 4-oxide (TA35)

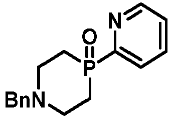

Compound 30 was prepared according to General procedure 2. Column chromatography purification (DCM/MeOH 10/0 to 9/1) afforded the product as a colorless solid (86 mg, 63%).

¹H NMR (400 MHz, CDCl₃): δ 8.77 (ddd, *J* = 4.8, 1.7, 1.0 Hz, 1H), 8.19 - 8.07 (m, 1H), 7.84 (tdd, *J =* 7.7, 3.6, 1.7 Hz, 1H), 7.44 - 7.31 (m, 5H), 7.30 - 7.24 (m, 1H), 3.69 (s, 2H), 3.21 - 2.91 (m, 4H), 2.56 - 2.40 (m, 2H), 1.98 (dddt, *J =* 17.6, 14.8, 5.6, 2.5 Hz, 2H).

¹³C NMR (101 MHz, CDCl₃): δ 156.2 (d, *J =* 124.3 Hz), 150.1 (d, *J =* 18.7 Hz), 138.2, 136.2 (d, *J* = 8.9 Hz), 128.8, 128.4, 127.6 (d, *J =* 19.6 Hz), 127.3, 125.6 (d, *J =* 3.1 Hz), 62.0 (d, *J* = 1.0 Hz), 49.0 (d, *J =* 7.0 Hz), 26.8 (d, *J =* 65.3 Hz).

³¹P NMR (162 MHz, CDCl₃): δ 29.01.

HRMS (ESI-TOF): calc for C₁₆H₂₀N₂OP [M+H]⁺: 287.1308, measured 287.1302.

HPLC: SunFire Waters, C₁₈ 5 µm, 4.6 × 150 mm; gradient 60% to 98% MeCN/H₂O, 1.0 mL/min, 214 nm; t_{R} = 3.6 min.

### EXAMPLE 31: 1-benzyl-4-(3-chloro-5-(trifluoromethyl)pyridin-2-yl)-1,4-azaphosphinane 4-oxide (TA31)

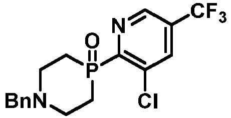

Compound 31 was prepared according to General procedure 2. Column chromatography purification (DCM/MeOH 10/0 to 9/1) afforded the product as a colorless solid (160 mg, 86%).

¹H NMR (400 MHz, CDCl₃): δ 8.87 (tq, *J* = 1.5, 0.8 Hz, 1H), 8.01 (ddq, *J =* 3.2, 2.0, 0.6 Hz, 1H), 7.44 - 7.21 (m, 5H), 3.59 (s, 2H), 3.22 - 2.98 (m, 2H), 2.90 - 2.55 (m, 4H), 2.48 - 2.30 (m, 2H).

¹³C NMR (101 MHz, CDCl₃): δ 156.7 (dd, *J =* 118.1, 1.4 Hz), 143.9 (dq, *J =* 16.9, 3.9 Hz), 138.0, 136.6 (d, *J =* 16.4 Hz), 135.2 (dq, *J =* 5.3, 3.6 Hz), 129.0 (dq, *J =* 34.0, 2.8 Hz), 128.7, 128.4, 127.4, 122.7 (dq, *J =* 274.0, 1.3 Hz), 61.8 (d, *J =* 0.8 Hz), 49.7 (d, *J =* 6.7 Hz), 27.3 (d, *J =* 67.2 Hz).

¹⁹F NMR (377 MHz, CDCl₃): δ -62.71 (d, *J =* 1.1 Hz).

³¹P NMR (162 MHz, CDCl₃): δ 34.04.

HRMS (ESI-TOF): calc for C₁₇H₁₈ClF₃N₂OP [M+H]⁺: 389.0792, measured 389.0787.

HPLC: SunFire Waters, C₁₈ 5 µm, 4.6 × 150 mm; gradient 60% to 98% MeCN/H₂O, 1.0 mL/min, 214 nm; t_{R} (major) = 6.3 min, t_{R} (minor) = 8.6 min.

### EXAMPLE 32: 1-benzyl-4-(pyrimidin-5-yl)-1,4-azaphosphinane 4-oxide (TA38)

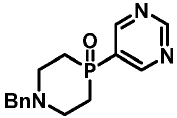

Compound 32 was prepared according to General procedure 2. Column chromatography purification (DCM/MeOH 10/0 to 9/1) afforded the product as a colorless solid (60 mg, 43%).

¹H NMR (400 MHz, CDCl₃): δ 9.37 (d, *J* = 3.2 Hz, 1H), 9.09 (d, *J =* 5.6 Hz, 2H), 7.45 - 7.22 (m, 5H), 3.67 (s, 2H), 3.19 - 2.93 (m, 4H), 2.19 (d, *J =* 15.3 Hz, 4H).

¹³C NMR (101 MHz, CDCl₃): δ 161.0 (d, *J =* 0.9 Hz), 158.8 (d, *J =* 9.2 Hz), 137.7, 128.9, 128.5, 127.6, 127.2 (d, *J =* 89.5 Hz), 62.5 (d, *J =* 1.1 Hz), 48.7 (d, *J =* 7.3 Hz), 28.9 (d, *J* = 67.0 Hz).

³¹P NMR (162 MHz, CDCl₃): δ 24.89.

HRMS (ESI-TOF): calc for C₁₅H₁₉N₃OP [M+H]⁺: 288.1260, measured 288.1250.

HPLC: SunFire Waters, C₁₈ 5 µm, 4.6 × 150 mm; 60% MeCN/H₂O, 1.0 mL/min, 214 nm; t_{R} = 3.0 min.

### EXAMPLE 33: 1-benzyl-4-(isoquinolin-5-yl)-1,4-azaphosphinane 4-oxide (TA11)

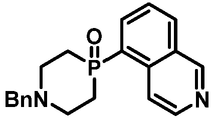

Compound 33 was prepared according to General procedure 2. Column chromatography purification (DCM/MeOH 10/0 to 9/1) afforded the product as a colorless solid (140 mg, 87%).

¹H NMR (400 MHz, CDCl₃): δ 9.32 (dd, *J =* 1.7, 1.0 Hz, 1H), 8.65 (d, *J =* 6.1 Hz, 1H), 8.59 (dq, *J* = 6.1, 0.9 Hz, 1H), 8.15 (dq, *J* = 8.3, 1.1 Hz, 1H), 8.08 (ddd, *J* = 13.8, 7.1, 1.2 Hz, 1H), 7.68 (ddd, *J* = 8.2, 7.1, 2.2 Hz, 1H), 7.38 - 7.26 (m, 5H), 3.68 (s, 2H), 3.15 - 2.99 (m, 4H), 2.52 - 2.39 (m, 2H), 2.37 - 2.23 (m, 2H).

¹³C NMR (101 MHz, CDCl₃): δ 153.7 (d, *J =* 1.0 Hz), 144.4 (d, *J* = 0.7 Hz), 138.0, 136.0 (d, *J* = 8.4 Hz), 134.6 (d, *J* = 9.5 Hz), 132.5 (d, *J* = 2.8 Hz), 128.82, 128.75 (d, *J* = 7.2 Hz), 128.5, 127.9, 127.4, 126.1 (d, *J =* 12.4 Hz), 118.8 (d, *J =* 4.4 Hz), 62.5 (d, *J =* 1.1 Hz), 49.3 (d, *J* = 7.1 Hz), 28.9 (d, *J =* 66.1 Hz).

³¹P NMR (162 MHz, CDCl₃): δ 29.18.

HRMS (ESI-TOF): calc for C₂₀H₂₂N₂OP [M+H]⁺: 337.1464, measured 337.1471.

HPLC: SunFire Waters, C₁₈ 5 µm, 4.6 × 150 mm; gradient 60% to 98% MeCN/H₂O, 1.0 mL/min, 214 nm; t_{R} = 3.5 min.

### EXAMPLE 34: 4-(benzo[d]thiazol-2-yl)-1-benzyl-1,4-azaphosphinane 4-oxide (TA12)

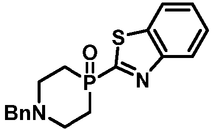

Compound 34 was prepared according to General procedure 2. Column chromatography purification (DCM/MeOH 10/0 to 9/1) afforded the product as a colorless solid (22 mg, 13%).

¹H NMR (400 MHz, CDCl₃): δ 8.19 (ddd, *J =* 8.2, 1.3, 0.7 Hz, 1H), 8.03 (ddd, *J =* 8.0, 1.4, 0.7 Hz, 1H), 7.61 - 7.55 (m, 1H), 7.52 (dddd, *J =* 7.8, 7.2, 1.3, 0.6 Hz, 1H), 7.39 - 7.26 (m, 5H), 3.68 (s, 2H), 3.13 - 3.03 (m, 4H), 2.55 - 2.44 (m, 2H), 2.38 - 2.23 (m, 2H).

¹³C NMR (101 MHz, CDCl₃): δ 166.9, 165.7, 154.8 (d, *J =* 20.7 Hz), 138.3, 136.6, 128.8, 128.5, 127.1 (d, *J =* 58.9 Hz), 126.8, 124.6, 122.2, 61.7, 49.2 (d, *J =* 6.9 Hz), 28.6 (d, *J =* 66.7 Hz).

³¹P NMR (162 MHz, CDCl₃): δ 28.23.

HRMS (ESI-TOF): calc for C₁₈H₂₀N₂OPS [M+H]⁺: 343.1028, measured 343.1038.

HPLC: SunFire Waters, C₁₈ 5 µm, 4.6 × 150 mm; gradient 60% to 98% MeCN/H₂O, 1.0 mL/min, 214 nm; t_{R} = 6.1 min.

### EXAMPLE 35: 1-benzyl-4-(thiophen-3-yl)-1,4-azaphosphinane 4-oxide (TA37)

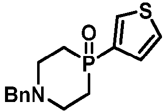

Compound 35 was prepared according to General procedure 2. Column chromatography purification (DCM/MeOH 10/0 to 9/1) afforded the product as a colorless solid (80 mg, 58%).

¹H NMR (400 MHz, CDCl₃): δ 7.95 - 7.87 (m, 1H), 7.44 (qt, *J* = 3.1, 1.6 Hz, 1H), 7.34 - 7.19 (m, 6H), 3.60 (s, 2H), 3.04 - 2.73 (m, 4H), 2.24 - 1.99 (m, 4H).

¹³C NMR (101 MHz, CDCl₃): δ 137.9, 133.8 (d, *J =* 100.7 Hz), 133.5 (d, *J =* 13.0 Hz), 130.3 (d, *J =* 9.4 Hz), 128.9, 128.5, 127.9 (d, *J =* 14.4 Hz), 127.7 (d, *J =* 14.9 Hz), 127.4, 62.4 (d, *J =* 1.0 Hz), 49.3 (d, *J =* 7.1 Hz), 29.2 (d, *J =* 67.5 Hz).

³¹P NMR (162 MHz, CDCl₃): δ 23.24.

HRMS (ESI-TOF): calc for C₁₅H₁₉NOPS [M+H]⁺: 292.0919, measured 292.0919.

HPLC: SunFire Waters, C₁₈ 5 µm, 4.6 × 150 mm; 60% MeCN/H₂O, 1.0 mL/min, 214 nm; t_{R} = 3.8 min.

### EXAMPLE 36: 1-(4-methoxybenzyl)-4-(4-(trifluoromethyl)phenyl)-1,4-azaphosphinane 4-oxide (GC11)

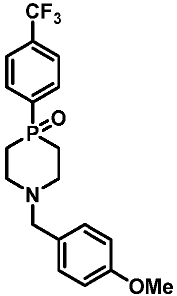

Compound 36 was prepared according to General procedure 4. Column chromatography purification (DCM/MeOH 10/0 to 9/1) afforded the product as a colorless solid (935 mg, 53%).

¹H NMR (400 MHz, CDCl₃): δ 7.92 (dd, *J =* 10.6, 8.4 Hz, 2H), 7.76 (dd, *J =* 8.4, 1.7 Hz, 2H), 7.28 - 7.20 (m, 2H), 6.91 - 6.82 (m, 2H), 3.81 (s, 3H), 3.59 (s, 2H), 3.13 - 2.83 (m, 4H), 2.30 - 1.97 (m, 4H).

¹³C NMR (101 MHz, CDCl₃): δ 159.0, 137.0 (d, *J =* 93.6 Hz), 133.9 (dd, *J =* 31.5, 3.0 Hz), 130.9 (d, *J =* 9.6 Hz), 130.1, 123.0, 125.6 (dq, *J =* 11.6, 3.7 Hz), 123.6 (dq, *J =* 1.2, 272.9 Hz), 113.8, 62.0, 55.3, 48.8 (d, *J* = 7.3 Hz), 28.6 (d, *J =* 66.2 Hz).

³¹P NMR (162 MHz, CDCl₃): δ 28.13.

¹⁹F NMR (377 MHz, CDCl₃): δ -63.20.

HRMS (ESI-TOF): calc for C₁₉H₂₂F₃NO₂P [M+H]⁺: 384.1335 measured 384.1336.

HPLC: SunFire Waters, C₁₈ 5 µm, 4.6 × 150 mm; gradient 60% to 98% MeCN/H₂O, 1.0 mL/min, 214 nm; t_{R} = 5.6 min.

### EXAMPLE 37: 1-(2-methylbenzyl)-4-(4-(trifluoromethyl)phenyl)-1,4-azaphosphinane 4-oxide (GC35)

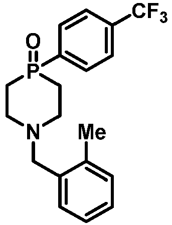

Compound 37 was prepared according to General procedure 4. Column chromatography purification (DCM/MeOH 10/0 to 9/1) afforded the product as a colorless solid (98 mg, 78%).

¹H NMR (400 MHz, CDCl₃): δ 7.96 - 7.87 (m, 2H), 7.76 (dd, *J* = 8.0, 2.2 Hz, 2H), 7.30 - 7.25 (m, 1H), 7.22 - 7.14 (m, 3H), 3.61 (s, 2H), 3.12 - 2.91 (m, 4H), 2.38 (s, 3H), 2.23 - 2.00 (m, 4H).

¹³C NMR (101 MHz, CDCl₃): δ 137.5, 137.0 (d, *J =* 92.7 Hz), 136.1, 133.9 (d, *J =* 35.0 Hz), 130.9 (d, *J* = 9.6 Hz), 130.5, 129.7, 127.4, 125.8, 125.6 (dq, *J* = 11.6, 3.8 Hz), 123.5 (q, *J* = 272.8 Hz), 60.4, 49.1 (d, *J =* 7.3 Hz), 28.6 (d, *J =* 66.1 Hz), 19.3.

³¹P NMR (162 MHz, CDCl₃): δ 28.10.

¹⁹F NMR (377 MHz, CDCl₃): δ -63.19 (d, *J =* 0.9 Hz).

HRMS (ESI-TOF): calc for C₁₉H₂₂F₃NOP [M+H]⁺: 368.1386 measured 368.1392.

HPLC: SunFire Waters, C₁₈ 5 µm, 4.6 × 150 mm; gradient 60% to 98% MeCN/H₂O, 1.0 mL/min, 214 nm; t_{R} = 8.4 min.

### EXAMPLE 38: 1-(3-methylbenzyl)-4-(4-(trifluoromethyl)phenyl)-1,4-azaphosphinane 4-oxide (GC37)

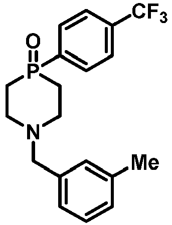

Compound 38 was prepared according to General procedure 4. Column chromatography purification (DCM/MeOH 10/0 to 9/1) afforded the product as a colorless solid (97 mg, 78%).

¹H NMR (400 MHz, CDCl₃): δ 7.92 (ddd, *J =* 11.0, 8.6, 0.7 Hz, 2H), 7.76 (ddd, *J =* 8.6, 2.3, 0.6 Hz, 2H), 7.22 (t, *J =* 7.4 Hz, 1H), 7.18 - 7.13 (m, 2H), 7.13 - 7.07 (m, 1H), 3.62 (s, 2H), 3.16 - 2.85 (m, 4H), 2.36 (s, 3H), 2.26 - 2.00 (m, 4H).

¹³C NMR (101 MHz, CDCl₃): δ 138.1, 137.9, 137.0 (d, *J* = 93.5 Hz), 133.8 (dq, *J* = 32.9, 3.1 Hz), 130.9 (d, *J* = 9.6 Hz), 129.7, 128.3, 128.2, 126.0, 125.6 (dq, *J* = 11.4, 3.8 Hz), 123.6 (dq, *J =* 1.1, 272.6 Hz), 62.6 (d, *J* = 1.1 Hz), 49.0 (d, *J* = 7.2 Hz), 28.6 (d, *J =* 66.1 Hz), 21.4.

³¹P NMR (162 MHz, CDCl₃): δ 27.76.

¹⁹F NMR (377 MHz, CDCl₃): δ -63.19.

HRMS (ESI-TOF): calc for C₁₉H₂₂F₃NOP [M+H]⁺: 368.1386 measured 368.1397.

HPLC: SunFire Waters, C₁₈ 5 µm, 4.6 × 150 mm; gradient 60% to 98% MeCN/H₂O, 1.0 mL/min, 214 nm; t_{R} = 7.4 min.

### EXAMPLE 39: 1-(4-methylbenzyl)-4-(4-(trifluoromethyl)phenyl)-1,4-azaphosphinane 4-oxide (GC25)

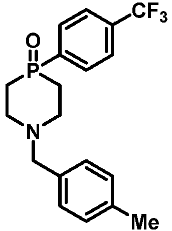

Compound 39 was prepared according to General procedure 4. Column chromatography purification (DCM/MeOH 10/0 to 9/1) afforded the product as a colorless solid (30 mg, 39%).

¹H NMR (400 MHz, CDCl₃): δ 7.92 (ddp, *J =* 10.2, 7.5, 0.8 Hz, 2H), 7.75 (ddt, *J =* 7.8, 2.5, 0.7 Hz, 2H), 7.24 - 7.20 (m, 2H), 7.18 - 7.11 (m, 2H), 3.62 (s, 2H), 3.14 - 2.87 (m, 4H), 2.34 (s, 3H), 2.26 - 2.13 (m, 2H), 2.12 - 1.99 (m, 2H).

¹³C NMR (101 MHz, CDCl₃): δ 137.2, 137.1 (d, *J =* 94.0 Hz), 134.9, 134.0 (dq, *J =* 3.0, 32.6 Hz), 131.0 (d, *J* = 9.7 Hz), 129.2, 129.0, 125.7 (dq, *J =* 11.6, 3.8 Hz), 123.6 (d, *J =* 272.8 Hz), 62.4, 49.0 (d, *J* = 7.3 Hz), 28.6 (d, *J =* 66.1 Hz), 21.2.

³¹P NMR (162 MHz, CDCl₃): δ 28.19.

¹⁹F NMR (377 MHz, CDCl₃): δ -63.20.

HRMS (ESI-TOF): calc for C₁₉H₂₂F₃NOP [M+H]⁺: 368.1386 measured 368.1391.

HPLC: SunFire Waters, C₁₈ 5 µm, 4.6 × 150 mm; gradient 60% to 98% MeCN/H₂O, 1.0 mL/min, 214 nm; t_{R} = 7.4 min.

### EXAMPLE 40: 1-(2-bromobenzyl)-4-(4-(trifluoromethyl)phenyl)-1,4-azaphosphinane 4-oxide (GC36)

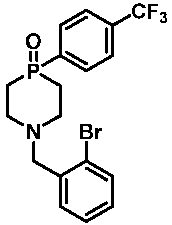

Compound 40 was prepared according to General procedure 4. Column chromatography purification (DCM/MeOH 10/0 to 9/1) afforded the product as a colorless solid (117 mg, 82%).

¹H NMR (400 MHz, CDCl₃): δ 7.93 (dd, *J* = 10.4, 8.5 Hz, 2H), 7.80 - 7.72 (m, 2H), 7.56 (dd, *J* = 8.0, 1.2 Hz, 1H), 7.46 (d, *J* = 7.4 Hz, 1H), 7.31 (t, *J =* 7.1 Hz, 1H), 7.14 (td, *J =* 7.8, 1.6 Hz, 1H), 3.75 (s, 2H), 3.17 - 2.96 (m, 4H), 2.31 - 2.01 (m, 4H).

¹³C NMR (101 MHz, CDCl₃): δ 137.4, 136.9 (d, *J* = 91.9 Hz), 133.9 (d, *J =* 34.4 Hz), 133.1, 130.9 (d, *J* = 9.5 Hz), 130.6, 128.8, 127.4, 125.7 (dq, *J =* 11.7, 3.8 Hz), 124.8, 123.6 (q, *J =* 272.2 Hz), 61.4, 49.0 (d, *J* = 7.2 Hz), 28.5 (d, *J =* 66.2 Hz).

³¹P NMR (162 MHz, CDCl₃): δ 27.61.

¹⁹F NMR (377 MHz, CDCl₃): δ -63.19.

HRMS (ESI-TOF): calc for C₁₈H₁₉BrF₃NOP [M+H]⁺: 432.0334 measured 432.0344.

HPLC: SunFire Waters, C₁₈ 5 µm, 4.6 × 150 mm; gradient 60% to 98% MeCN/H₂O, 1.0 mL/min, 214 nm; t_{R} = 8.8 min.

### EXAMPLE 41: 1-(2-(trifluoromethyl)benzyl)-4-(4-(trifluoromethyl)phenyl)-1,4-azaphosphinane 4-oxide (GC40)

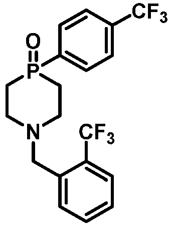

Compound 41 was prepared according to General procedure 4. Column chromatography purification (DCM/MeOH 10/0 to 9/1) afforded the product as a colorless solid (47 mg, 70%).

¹H NMR (400 MHz, CDCl₃): δ 7.97 - 7.88 (m, 2H), 7.81 - 7.72 (m, 3H), 7.65 (d, *J* = 7.9 Hz, 1H), 7.54 (t, *J =* 7.6 Hz, 1H), 7.37 (t, *J =* 7.3 Hz, 1H), 3.81 (s, 2H), 3.15 - 2.90 (m, 4H), 2.32 - 1.89 (m, 4H).

¹³C NMR (101 MHz, CDCl₃): δ 137.4, 136.8 (d, *J =* 96.9 Hz), 133.9 (d, *J =* 35.8 Hz), 131.9, 130.9 (d, *J =* 9.6 Hz), 130.2, 129.0, 127.3, 126.2 (q, *J =* 5.8 Hz), 125.7 (dq, *J =* 11.5, 3.8 Hz), 124.3 (q, *J =* 253.5 Hz), 123.6 (q, *J =* 270.3 Hz), 58.1, 49.2 (d, *J =* 7.3 Hz), 28.5 (d, *J =* 66.2 Hz).

³¹P NMR (162 MHz, CDCl₃): δ 27.70.

¹⁹F NMR (377 MHz, CDCl₃): δ -60.14 (s), -63.21 (d, *J =* 1.0 Hz).

HRMS (ESI-TOF): calc for C₁₉H₁₉F₆NOP [M+H]⁺: 422.1103 measured 422.1104.

HPLC: SunFire Waters, C₁₈ 5 µm, 4.6 × 150 mm; gradient 60% to 98% MeCN/H₂O, 1.0 mL/min, 214 nm; t_{R} = 9.7 min.

### EXAMPLE 42: 1-(2-chloro-6-(trifluoromethyl)benzyl)-4-(4-(trifluoromethyl)phenyl)-1,4-azaphosphinane 4-oxide (GC39)

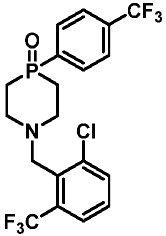

Compound 42 was prepared according to General procedure 4. Column chromatography purification (DCM/MeOH 10/0 to 9/1) afforded the product as a colorless solid (38 mg, 51%).

¹H NMR (400 MHz, CDCl₃): δ 7.94 (dd, *J =* 11.0, 8.0 Hz, 2H), 7.82 - 7.73 (m, 3H), 7.53 - 7.44 (m, 2H), 3.80 (s, 2H), 3.20 - 2.93 (m, 4H), 2.28 - 2.02 (m, 4H).

¹³C NMR (101 MHz, CDCl₃): δ 138.0 (d, *J* = 1.5 Hz), 137.0, 136.7 (dq, *J =* 94.8, 1.4 Hz), 134.0 (dd, *J =* 32.9, 3.0 Hz), 130.9 (d, *J =* 9.7 Hz), 130.2, 129.4 (q, *J =* 32.9 Hz), 126.2 (dq, *J =* 120.0, 3.7 Hz), 125.4 (dq, *J =* 41.1, 4.0 Hz), 123.8 (q, *J =* 272.2 Hz), 123.5 (dq, *J =* 1.2, 273.1 Hz), 58.6, 49.2 (d, *J =* 7.2 Hz), 28.6 (d, *J =* 66.2 Hz).

³¹P NMR (162 MHz, CDCl₃): δ 27.14.

¹⁹F NMR (377 MHz, CDCl₃): δ -62.44 (s), -63.22 (d, *J =* 1.0 Hz).

HRMS (ESI-TOF): calc for C₁₉H₁₈ClF₆NOP [M+H]⁺: 456.0713 measured 456.0709.

HPLC: SunFire Waters, C₁₈ 5 µm, 4.6 × 150 mm; gradient 60% to 98% MeCN/H₂O, 1.0 mL/min, 214 nm; t_{R} = 10.9 min.

### EXAMPLE 43: 1-(naphthalen-1-ylmethyl)-4-(4-(trifluoromethyl)phenyl)-1,4-azaphosphinane 4-oxide (GC38)

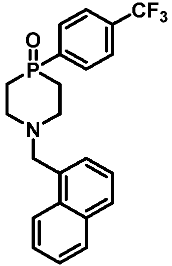

Compound 43 was prepared according to General procedure 4. Column chromatography purification (DCM/MeOH 10/0 to 9/1) afforded the product as a colorless solid (67 mg, 64%).

¹H NMR (400 MHz, CDCl₃): δ 8.31 - 8.26 (m, 1H), 7.89 (ddd, *J* = 7.8, 7.0, 5.0 Hz, 3H), 7.81 (dd, *J* = 7.1, 2.1 Hz, 1H), 7.78 - 7.72 (m, 2H), 7.58 - 7.48 (m, 2H), 7.46 - 7.39 (m, 2H), 4.06 (s, 2H), 3.24 - 2.98 (m, 4H), 2.14 - 1.99 (m, 4H).

¹³C NMR (101 MHz, CDCl₃): δ 136.9 (d, *J* = 94.0 Hz), 134.0, 133.7, 132.5, 130.9 (d, *J =* 9.6 Hz), 128.6, 128.4, 127.6, 126.0, 125.8, 125.6 (dq, *J =* 11.5, 3.8 Hz), 125.2, 123.5 (q, *J =* 276.5 Hz), 124.6, 60.8, 49.2 (d, *J =* 7.2 Hz), 28.5 (d, *J =* 66.2 Hz).

³¹P NMR (162 MHz, CDCl₃): δ 27.99.

¹⁹F NMR (377 MHz, CDCl₃): δ -63.20.

HRMS (ESI-TOF): calc for C₂₂H₂₂F₃NOP [M+H]⁺: 404.1386 measured 404.1393.

HPLC: SunFire Waters, C₁₈ 5 µm, 4.6 × 150 mm; gradient 60% to 98% MeCN/H₂O, 1.0 mL/min, 214 nm; t_{R} = 9.8 min.

### EXAMPLE 44: 1-(naphthalen-2-ylmethyl)-4-(4-(trifluoromethyl)phenyl)-1,4-azaphosphinane 4-oxide (GC32)

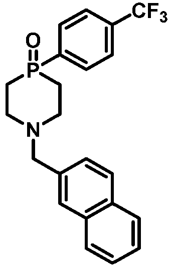

Compound 44 was prepared according to General procedure 4. Column chromatography purification (DCM/MeOH 10/0 to 9/1) afforded the product as a colorless solid (44 mg, 58%).

¹H NMR (400 MHz, CDCl₃): δ 7.93 (dd, *J =* 11.0, 8.0 Hz, 2H), 7.87 - 7.73 (m, 6H), 7.57 - 7.42 (m, 3H), 3.82 (s, 2H), 3.19 - 2.90 (m, 4H), 2.17 - 2.00 (m, 4H).

¹³C NMR (162 MHz, CDCl₃): δ 114.4 (d, *J =* 93.0 Hz), 112.1, 111.9, 110.6 (d, *J =* 9.7 Hz), 109.4, 109.1, 109.0, 108.62 (d, *J =* 1.6 Hz), 108.57, 108.2, 107.7, 107.5, 107.3 (dq, *J =* 11.7, 3.9 Hz), 106.0 (q, *J =* 273.2 Hz), 68.3, 59.8 (d, *J =* 7.2 Hz), 48.5, 47.0 (d, *J =* 66.1 Hz).

³¹P NMR (162 MHz, CDCl₃): δ 27.83.

¹⁹F NMR (377 MHz, CDCl₃): δ -63.18 (d, *J* = 0.7 Hz).

HRMS (ESI-TOF): calc for C₂₂H₂₂F₃NOP [M+H]⁺: 404.1386 measured 404.1388.

HPLC: SunFire Waters, C₁₈ 5 µm, 4.6 × 150 mm; gradient 60% to 98% MeCN/H₂O, 1.0 mL/min, 214 nm; t_{R} = 9.1 min.

### EXAMPLE 45: 1-(2-methylbenzyl)-4-(p-tolyl)-1,4-azaphosphinane 4-oxide (GC62)

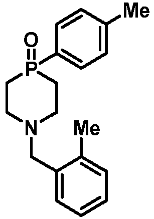

Compound 45 was prepared according to General procedure 4. Column chromatography purification (DCM/MeOH 10/0 to 9/1) afforded the product as a colorless solid (30 mg, 46%).

¹H NMR (400 MHz, CDCl₃): δ 7.64 (dd, *J =* 11.3, 8.1 Hz, 2H), 7.32 - 7.26 (m, 3H), 7.19 - 7.11 (m, 3H), 3.58 (s, 2H), 3.08 - 2.83 (m, 4H), 2.40 (s, 3H), 2.37 (s, 3H), 2.22 - 1.92 (m, 4H).

¹³C NMR (101 MHz, CDCl₃): δ 142.5 (d, *J =* 2.9 Hz), 137.5, 136.3, 130.3, 130.2, 129.9 (d, *J =* 87.3 Hz), 129.7, 129.5, 127.3, 125.7, 60.4, 49.3 (d, *J =* 7.1 Hz), 28.6 (d, *J =* 66.1 Hz), 21.6, 19.3.

³¹P NMR (162 MHz, CDCl₃): δ 28.48.

HRMS (ESI-TOF): calc for C₁₉H₂₅NOP [M+H]⁺: 314.1668 measured 314.1668.

HPLC: SunFire Waters, C₁₈ 5 µm, 4.6 × 150 mm; gradient 60% to 98% MeCN/H₂O, 1.0 mL/min, 214 nm; t_{R} = 6.8 min.

### EXAMPLE 46: 1-(3-methylbenzyl)-4-(p-tolyl)-1,4-azaphosphinane 4-oxide (GC63)

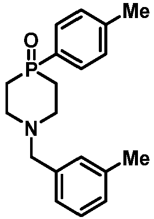

Compound 46 was prepared according to General procedure 4. Column chromatography purification (DCM/MeOH 10/0 to 9/1) afforded the product as a colorless solid (58 mg, 88%).

¹H NMR (400 MHz, CDCl₃): δ 7.65 (dd, *J =* 11.3, 8.1 Hz, 2H), 7.35 - 7.27 (m, 2H), 7.21 (t, *J =* 7.5 Hz, 1H), 7.16 - 7.05 (m, 3H), 3.60 (s, 2H), 3.08 - 2.84 (m, 4H), 2.39 (s, 3H), 2.34 (s, 3H), 2.26 - 1.92 (m, 4H).

¹³C NMR (101 MHz, CDCl₃): δ 142.5 (d, *J =* 2.8 Hz), 138.1, 130.2, 129.9 (d, *J =* 76.6 Hz), 129.7, 129.5, 128.4, 128.3, 128.1, 126.0, 62.6, 49.3 (d, *J =* 7.1 Hz), 28.5 (d, *J =* 66.0 Hz), 21.6 (d, *J =* 1.2 Hz), 21.4.

³¹P NMR (162 MHz, CDCl₃): δ 28.06.

HRMS (ESI-TOF): calc for C₁₉H₂₅NOP [M+H]⁺: 314.1668 measured 314.1671.

HPLC: SunFire Waters, C₁₈ 5 µm, 4.6 × 150 mm; gradient 60% to 98% MeCN/H₂O, 1.0 mL/min, 214 nm; t_{R} = 6.8 min.

### EXAMPLE 47: 1-(4-methylbenzyl)-4-(p-tolyl)-1,4-azaphosphinane 4-oxide (GC60)

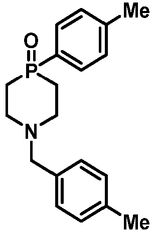

Compound 47 was prepared according to General procedure 4. Column chromatography purification (DCM/MeOH 10/0 to 9/1) afforded the product as a colorless solid (75 mg, 78%).

¹H NMR (400 MHz, CDCl₃): δ 7.65 (dd, *J* = 11.3, 8.1 Hz, 2H), 7.36 - 7.28 (m, 2H), 7.18 (dd, *J* = 35.9, 7.9 Hz, 4H), 3.61 (s, 2H), 3.12 - 2.82 (m, 4H), 2.40 (s, 3H), 2.34 (s, 3H), 2.25 - 1.93 (m, 4H).

¹³C NMR (101 MHz, CDCl₃): δ 142.5, 137.0, 130.3, 129.9 (d, *J =* 75.8 Hz), 129.5, 129.1, 128.9, 128.4, 62.3, 49.2 (d, *J =* 7.2 Hz), 28.6 (d, *J =* 65.8 Hz), 21.4 (d, *J =* 46.5 Hz).

³¹P NMR (162 MHz, CDCl₃): δ 28.25.

HPLC: SunFire Waters, C₁₈ 5 µm, 4.6 × 150 mm; gradient 60% to 98% MeCN/H₂O, 1.0 mL/min, 214 nm; t_{R} = 6.2 min.

### EXAMPLE 48: (4-oxido-4-(p-tolyl)-1,4-azaphosphinan-1-yl)(phenyl)methanone (GC67)

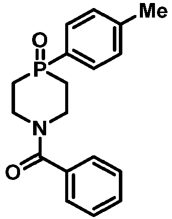

In a flame dried round bottom flask, under nitrogen atmosphere, 4-(p-tolyl)-1,4-azaphosphinan-1-ium 4-oxide chloride 4.85 (49 mg, 0.20 mmol, 1.0 equiv.) and CH₂Cl₂ (0.1 M, 2 mL) were added. Then, benzoyl chloride (28 mg, 0.20 mmol, 1.0 equiv.) and NEt₃ (41 mg, 0.40 mmol, 2.0 equiv.) were added to the mixture. The reaction mixture was stirred at room temperature for 24 h (monitored by ³¹P NMR). Then, the reaction mixture was concentrated under reduced pressure. Purification by column chromatography (silica deactivated with 5% NEt₃, acetone/MeOH gradient 0% to 15% MeOH) the desired product (56 mg, 89% yield).

¹H NMR (400 MHz, CDCl₃): δ 7.69 - 7.56 (m, 2H), 7.45 - 7.39 (m, 5H), 7.36 - 7.27 (m, 2H), 4.82 - 4.57 (m, 1H), 4.09 - 3.56 (m, 3H), 3.05 - 2.86 (m, 1H), 2.45 - 2.33 (m, 3H), 2.34 - 1.79 (m, 3H).

¹³C NMR (101 MHz, CDCl₃): δ 171.1, 143.2 (d, *J* = 2.8 Hz), 135.3, 130.3 (d, *J =* 9.9 Hz), 130.2, 130.1 (d, *J =* 8.2 Hz), 129.8 (d, *J =* 12.3 Hz), 129.5 (d, *J =* 12.0 Hz), 128.8, 128.1 (d, *J =* 101.7 Hz), 126.7, 44.2, 39.0, 28.8, 28.1, 21.6 (d, *J =* 1.3 Hz).

³¹P NMR (162 MHz, CDCl₃): δ 28.71.

HRMS (ESI-TOF): calc for C₁₈H₂₁NO₂P [M+H]⁺: 314.1304 measured 314.1313.

HPLC: SunFire Waters, C₁₈ 5 µm, 4.6 × 150 mm; gradient 60% to 98% MeCN/H₂O, 1.0 mL/min, 214 nm; t_{R} = 3.4 min.

### EXAMPLE 49: 1-phenethyl-4-(4-(trifluoromethyl)phenyl)-1,4-azaphosphinane 4-oxide (GC24)

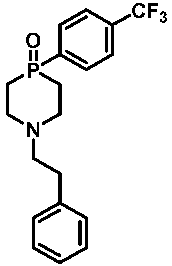

Compound 49 was prepared according to General procedure 5. Column chromatography purification (DCM/MeOH 10/0 to 9/1) afforded the product as a colorless solid (10 mg, 13%).

¹H NMR (400 MHz, CDCl₃): δ 7.93 (dd, *J* = 10.6, 8.5 Hz, 2H), 7.77 (d, *J* = 6.6 Hz, 2H), 7.31 (t, *J =* 7.2 Hz, 2H), 7.25 - 7.14 (m, 3H), 3.11 (dd, *J =* 24.7, 18.0 Hz, 4H), 2.83 (s, 4H), 2.29 (dd, *J =* 22.3, 14.7 Hz, 2H), 2.10 (dd, *J =* 17.0, 15.6 Hz, 2H).

¹³C NMR (101 MHz, CDCl₃): δ 131.0 (d, *J =* 9.7 Hz), 128.7, 128.6, 128.3, 126.4, 127.7 (dq, *J* = 11.6, 3.5 Hz), 123.5 (q, *J* = 274.0 Hz), 60.0, 49.1 (d, *J* = 7.3 Hz), 33.6, 28.4 (d, *J* = 67.1 Hz).

³¹P NMR (162 MHz, CDCl₃): δ 27.87.

¹⁹F NMR (377 MHz, CDCl₃): δ -63.21.

HRMS (ESI-TOF): calc for C₁₉H₂₂F₃NOP [M+H]⁺: 368.1386 measured 368.1387.

HPLC: SunFire Waters, C₁₈ 5 µm, 4.6 × 150 mm; gradient 60% to 98% MeCN/H₂O, 1.0 mL/min, 214 nm; t_{R} = 6.1 min.

### EXAMPLE 50: 1-(4-fluorophenyl)-3-(4-oxido-4-(4-(trifluoromethyl)phenyl)-1,4-azaphosphinan-1-yl)propan-1-one (GC52)

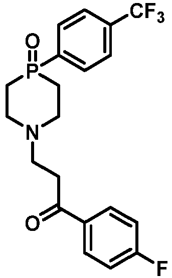

Compound 50 was prepared according to General procedure 6. Column chromatography purification (DCM/MeOH 10/0 to 9/1) afforded the product as a colorless solid (22 mg, 32%).

¹H NMR (400 MHz, CDCl₃): δ 8.02 - 7.96 (m, 2H), 7.94 - 7.86 (m, 2H), 7.75 (dtt, *J =* 8.0, 1.5, 0.7 Hz, 2H), 7.18 - 7.10 (m, 2H), 3.27 - 3.01 (m, 8H), 2.35 - 2.17 (m, 2H), 2.13 - 2.00 (m, 2H).

¹³C NMR (101 MHz, CDCl₃): δ 196.9, 165.9 (d, *J =* 255.3 Hz), 136.4 (d, *J =* 95.3 Hz), 134.0 (dd, *J* = 30.1, 3.1 Hz), 133.2 (d, *J* = 3.1 Hz), 131.0 (d, *J* = 9.7 Hz), 130.7 (d, *J* = 9.4 Hz), 125.7 (dq, *J =* 11.5, 3.7 Hz), 123.5 (q, *J =* 272.6 Hz), 115.9 (d, *J =* 21.9 Hz), 52.7, 49.1 (d, *J* = 7.4 Hz), 35.8, 28.2 (d, *J =* 66.0 Hz).

³¹P NMR (162 MHz, CDCl₃): δ 27.17.

¹⁹F NMR (377 MHz, CDCl₃): δ -63.22 (d, *J =* 0.9 Hz), -104.62.

HRMS (ESI-TOF): calc for C₂₀H₂₁F₄NO₂P [M+H]⁺: 414.1241 measured 414.1247.

HPLC: SunFire Waters, C₁₈ 5 µm, 4.6 × 150 mm; gradient 60% to 98% MeCN/H₂O, 1.0 mL/min, 214 nm; t_{R} = 5.2 min.

### EXAMPLE 51: 1-(4-fluorophenyl)-4-(4-oxido-4-(4-(trifluoromethyl)phenyl)-1,4-azaphosphinan-1-yl)butan-1-one (GC50)

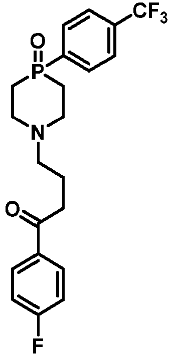

Compound 51 was prepared according to General procedure 6. Column chromatography purification (DCM/MeOH 10/0 to 9/1) afforded the product as a colorless solid (20 mg, 27%).

¹H NMR (400 MHz, CDCl₃): δ 8.06 - 7.97 (m, 2H), 7.79 (dd, *J =* 10.9, 8.1 Hz, 2H), 7.76 - 7.69 (m, 2H), 7.21 - 7.07 (m, 2H), 3.14 - 2.89 (m, 6H), 2.59 (t, *J =* 6.9 Hz, 2H), 2.13 - 1.90 (m, 6H).

¹³C NMR (101 MHz, CDCl₃): δ 198.0, 165.7 (d, *J =* 254.8 Hz), 136.7 (d, J= 94.1 Hz), 134.1, 133.7 (d, *J =* 3.0 Hz), 131.0, 130.9, 130.7 (d, *J =* 9.2 Hz), 125.6 (dq, *J =* 11.5, 3.6 Hz), 123.5 (q, *J =* 272.9 Hz), 115.7 (d, *J =* 21.8 Hz), 57.4, 49.1 (d, *J =* 7.2 Hz), 35.9, 28.2 (d, *J =* 66.1 Hz), 21.9.

³¹P NMR (162 MHz, CDCl₃): δ 27.64.

¹⁹F NMR (377 MHz, CDCl₃): δ -63.20 (d, *J =* 1.0 Hz), -105.27.

HRMS (ESI-TOF): calc for C₂₁H₂₃F₄NO₂P [M+H]⁺: 428.1397 measured 428.1401.

HPLC: SunFire Waters, C₁₈ 5 µm, 4.6 × 150 mm; gradient 60% to 98% MeCN/H₂O, 1.0 mL/min, 214 nm; t_{R} = 6.3 min.

### EXAMPLE 52: 1-phenethyl-4-(p-tolyl)-1,4-azaphosphinane 4-oxide (GC68)

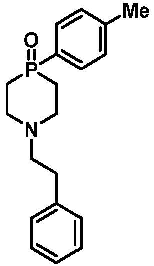

Compound 52 was prepared according to General procedure 5. Column chromatography purification (DCM/MeOH 10/0 to 9/1) afforded the product as a colorless solid (23 mg, 18%).

¹H NMR (400 MHz, CDCl₃): δ 7.68 (dd, *J =* 11.5, 8.0 Hz, 2H), 7.31 (dd, *J =* 13.5, 4.9 Hz, 4H), 7.22 (dd, *J =* 10.3, 4.3 Hz, 3H), 3.31 - 3.06 (m, 4H), 2.88 (br s, 4H), 2.40 (br s, 4H), 2.19 - 1.86 (m, 3H).

¹³C NMR (101 MHz, CDCl₃): δ 142.8, 130.3, 130.0 (d, *J =* 75.0 Hz), 129.6, 128.7, 128.6, 128.5, 126.4, 59.9, 49.3 (d, *J =* 7.2 Hz), 33.1, 28.2 (d, *J =* 65.6 Hz), 21.6 (d, *J =* 1.3 Hz).

³¹P NMR (162 MHz, CDCl₃): δ 27.35.

HRMS (ESI-TOF): calc for C₁₉H₂₅NOP [M+H]⁺: 314.1668 measured 314.1672.

HPLC: SunFire Waters, C₁₈ 5 µm, 4.6 × 150 mm; gradient 60% to 98% MeCN/H₂O, 1.0 mL/min, 214 nm; t_{R} = 4.9 min.

### EXAMPLE 53: 1-(4-fluorophenyl)-3-(4-oxido-4-(4-(trifluoromethyl)phenyl)-1,4-azaphosphinan-1-yl)propan-1-one (GC64)

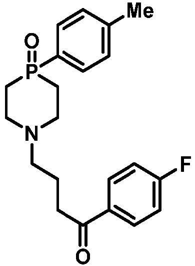

Compound 53 was prepared according to General procedure 6. Column chromatography purification (DCM/MeOH 10/0 to 9/1) afforded the product as a colorless solid (20 mg, 25%).

¹H NMR (400 MHz, CDCl₃): δ 8.04 - 7.98 (m, 2H), 7.56 - 7.49 (m, 2H), 7.30 - 7.24 (m, 2H), 7.17 - 7.10 (m, 2H), 3.06 - 2.85 (m, 6H), 2.56 (t, *J =* 6.9 Hz, 2H), 2.39 (s, 3H), 2.06 - 1.87 (m, 6H).

¹³C NMR (101 MHz, CDCl₃): δ 198.2, 165.7 (d, *J =* 254.6 Hz), 142.5 (d, *J =* 2.8 Hz), 133.8 (d, *J* = 3.1 Hz), 130.7 (d, *J* = 9.2 Hz), 130.2 (d, *J =* 9.7 Hz), 129.5 (d, *J =* 12.0 Hz), 129.0 (d, *J = 98.6* Hz), 115.7 (d, *J =* 21.8 Hz), 57.3, 49.3 (d, *J =* 7.1 Hz), 36.0, 28.3 (d, *J =* 66.0 Hz), 22.1, 21.6.

³¹P NMR (162 MHz, CDCl₃): δ 28.08.

¹⁹F NMR (377 MHz, CDCl₃): δ -105.47.

HRMS (ESI-TOF): calc for C₂₁H₂₆FNO₂P [M+H]⁺: 374.1680 measured 374.1681.

HPLC: SunFire Waters, C₁₈ 5 µm, 4.6 × 150 mm; gradient 60% to 98% MeCN/H₂O, 1.0 mL/min, 214 nm; t_{R} = 4.8 min.

### EXAMPLE 54: 1-(m-tolyl)-4-(p-tolyl)-1,4-azaphosphinane 4-oxide (GC70)

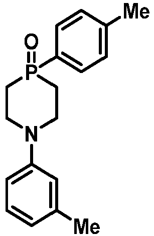

Compound 54 was prepared according to General procedure 7. Column chromatography purification (DCM/MeOH 10/0 to 9/1) afforded the product as a colorless solid (34 mg, 57%).

¹H NMR (400 MHz, CDCl₃): δ 7.62 - 7.53 (m, 2H), 7.31 - 7.25 (m, 2H), 7.20 - 7.13 (m, 1H), 6.79 - 6.72 (m, 2H), 6.67 (d, *J =* 7.6 Hz, 1H), 4.03 - 3.77 (m, 4H), 2.38 (s, 3H), 2.32 (s, 3H), 2.20 (ddd, *J =* 19.4, 10.3, 4.6 Hz, 2H), 2.08 - 1.92 (m, 2H).

¹³C NMR (101 MHz, CDCl₃): δ 146.3, 140.8 (d, *J =* 2.9 Hz), 137.5, 128.2 (d, *J =* 9.8 Hz), 127.8, 127.6 (d, *J =* 3.3 Hz), 127.1 (d, *J =* 100.5 Hz), 118.2, 114.2, 110.5, 43.2 (d, *J =* 6.5 Hz), 24.8 (d, *J =* 66.3 Hz), 20.0, 19.7.

³¹P NMR (162 MHz, CDCl₃): δ 30.07.

HRMS (ESI-TOF): calc for C₁₈H₂₃NOP [M+H]⁺: 300.1512 measured 300.1509.

HPLC: SunFire Waters, C₁₈ 5 µm, 4.6 × 150 mm; gradient 60% to 98% MeCN/H₂O, 1.0 mL/min, 214 nm; t_{R} = 5.9 min.

### EXAMPLE 55: 4-(p-tolyl)-1-(4-(trifluoromethyl)phenyl)-1,4-azaphosphinane 4-oxide (GC71)

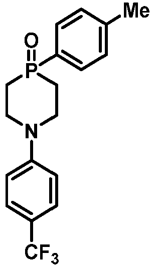

Compound 55 was prepared according to General procedure 7. Column chromatography purification (DCM/MeOH 10/0 to 9/1) afforded the product as a colorless solid (21 mg, 30%).

¹H NMR (400 MHz, CDCl₃): δ 7.59 - 7.49 (m, 4H), 7.32 - 7.27 (m, 2H), 6.95 (d, *J* = 8.7 Hz, 2H), 4.16 - 3.89 (m, 4H), 2.39 (s, 3H), 2.16 (ddt, *J =* 15.5, 11.4, 4.4 Hz, 2H), 2.10 1.95 (m, 2H).

¹³C NMR (101 MHz, CDCl₃): δ 149.8, 143.0 (d, *J =* 2.8 Hz), 130.0 (d, *J =* 9.9 Hz), 129.7 (d, *J =* 12.3 Hz), 128.4 (d, *J =* 101.5 Hz), 127.1 (q, *J =* 3.7 Hz), 124.7 (q, *J =* 270.5 Hz), 120.1 (q, *J =* 32.8 Hz), 113.5, 44.2 (d, *J =* 6.5 Hz), 26.4 (d, *J =* 66.4 Hz), 21.6.

³¹P NMR (162 MHz, CDCl₃): δ 30.02.

¹⁹F NMR (377 MHz, CDCl₃): δ -61.33.

HRMS (ESI-TOF): calc for C₁₈H₂₀F₃NOP [M+H]⁺: 354.1229 measured 354.1232.

HPLC: SunFire Waters, C₁₈ 5 µm, 4.6 × 150 mm; gradient 60% to 98% MeCN/H₂O, 1.0 mL/min, 214 nm; t_{R} = 7.1 min.

### EXAMPLE 56: 1,4-dibenzyl-1,4-azaphosphinane 4-oxide (AD8)

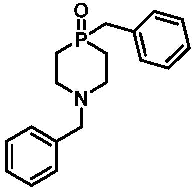

Compound 56 was prepared according to General procedure 2. Column chromatography purification (DCM/MeOH 10/0 to 9/1) afforded the product as a yellowish solid (1.1g 85%)

¹H NMR (400 MHz, CDCl₃) δ 7.40 - 7.24 (m, 10H), 3.61 (s, 2H), 3.22 (d, *J =* 14.2 Hz, 2H), 3.02 - 2.87 (m, 2H), 2.85 - 2.70 (m, 2H), 1.99 - 1.77 (m, 3H).

¹³C NMR (101 MHz, CDCl₃) δ 138.1, 131.4 (d, *J =* 7.8 Hz), 129.6 (d, *J =* 5.1 Hz), 128.9 (d, *J =* 2.7 Hz), 128.7, 128.4, 127.3, 127.0 (d, *J =* 3.1 Hz), 62.1, 49.5 (d, *J =* 6.7 Hz), 36.2 (d, *J* = 61.4 Hz), 26.6 (d, *J =* 63.0 Hz).

³¹P NMR (162 MHz, CDCl₃) δ 34.48.

HRMS (ESI-TOF): calc for C₁₈H₂₃NOP [M+H]+ 300.1517, measured 300.1519
HPLC: SunFire Waters, C₁₈ 5 µm, 4.6 × 150 mm; gradient 60% to 98% MeCN/H₂O, 1.0 mL/min, 214 nm; t_{R} = 4.6 min.

### EXAMPLE 57: 1-(o-tolyl)-4-(p-tolyl)-1,4-azaphosphinane 4-oxide (AD9)

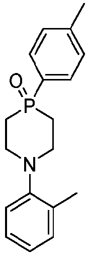

Compound 57 was prepared according to General procedure 7. Column chromatography purification (DCM/MeOH 10/0 to 9/1) afforded the product as a yellowish solid (32 mg, 475%)

¹H NMR (400 MHz, CDCl₃) δ 7.71 (dd, *J =* 11.4, 8.1 Hz, 2H), 7.35 (dd, *J =* 8.0, 2.3 Hz, 2H), 7.24 - 7.13 (m, 2H), 7.08 (d, *J =* 7.1 Hz, 1H), 7.02 (td, *J =* 7.4, 1.1 Hz, 1H), 3.59 - 3.46 (m, 2H), 3.36 - 3.20 (m, 2H), 2.43 (s, 3H), 2.35 (s, 3H), 2.33 - 2.24 (m, 2H), 2.24 - 2.07 (m, 2H).

¹³C NMR (101 MHz, CDCl₃) δ 152.0, 142.6, 132.6, 131.0, 130.3 (d, *J =* 9.7 Hz), 129.6 (d, *J* = 12.0 Hz), 128.6, 126.7, 123.9, 120.2, 49.1 (d, *J =* 7.2 Hz), 29.7 (d, *J =* 66.0 Hz), 21.6, 17.8.

³¹P NMR (162 MHz, CDCl₃) δ 27.52.

### HRMS (ESI-TOF): calc for C₁₈H₂₃NOP [M+H]+ 300.1517, measured 300.1519

HPLC: SunFire Waters, C₁₈ 5 µm, 4.6 × 150 mm; gradient 60% to 98% MeCN/H₂O, 1.0 mL/min, 214 nm; t_{R} = 5.9 min.

### EXAMPLE 58: 1,4-di-p-tolyl-1,4-azaphosphinane 4-oxide (AD10)

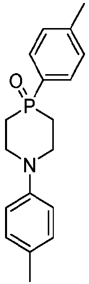

Compound 58 was prepared according to General procedure 7. Column chromatography purification (DCM/MeOH 10/0 to 9/1) afforded the product as a yellowish solid (40 mg, 59%)

¹H NMR (400 MHz, CDCl₃) δ 7.65 - 7.56 (m, 2H), 7.31 (dd, *J =* 8.0, 2.7 Hz, 2H), 7.12 (d, *J* = 8.3 Hz, 2H), 6.89 (d, 2H), 3.98 - 3.77 (m, 4H), 2.41 (s, 3H), 2.30 (s, 3H), 2.28 - 2.15 (m, 2H), 2.14 - 1.92 (m, 2H).

¹³C NMR (101 MHz, CDCl₃) δ 146.2, 142.6, 130.1, 130.0, 129.6, 129.5, 128.8, 115.8, 45.6 (d, *J =* 6.4 Hz), 26.6 (d, *J =* 66.4 Hz), 21.5, 20.3.

³¹P NMR (162 MHz, CDCl₃) δ 29.65.

HRMS (ESI-TOF): calc for C₁₈H₂₃NOP [M+H]+ 300.1517, measured 300.1519
HPLC: SunFire Waters, C₁₈ 5 µm, 4.6 × 150 mm; gradient 60% to 98% MeCN/H₂O, 1.0 mL/min, 214 nm; t_{R} = 5.2 min.

### EXAMPLE 59: 1-(naphthalen-2-yl)-4-(p-tolyl)-1,4-azaphosphinane 4-oxide (AD11)

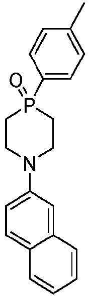

Compound 59 was prepared according to General procedure 7. Column chromatography purification (DCM/MeOH 10/0 to 9/1) afforded the product as a colorless solid (43 mg, 57%)

¹H NMR (400 MHz, CDCl₃) δ 7.77 (d, *J =* 9.0 Hz, 1H), 7.74 (d, *J =* 8.3 Hz, 1H), 7.68 (dd, *J* = 8.5, 1.2 Hz, 1H), 7.62 - 7.52 (m, 2H), 7.46 - 7.38 (m, 1H), 7.34 - 7.27 (m, 4H), 7.19 (d, *J* = 2.6 Hz, 1H), 4.27 - 4.04 (m, 2H), 3.97 (dtd, *J =* 13.8, 10.9, 2.3 Hz, 2H), 2.38 (s, 3H), 2.27 (ddq, *J =* 15.8, 12.9, 4.3 Hz, 2H), 2.06 (ddt, *J* = 17.3, 14.8, 2.5 Hz, 2H).

¹³C NMR (101 MHz, CDCl₃) δ 146.0, 142.9, 134.9, 130.2 (d, *J =* 9.8 Hz), 129.7, 129.6 (d, *J* = 2.8 Hz), 129.5, 128.5, 128.1, 127.6, 126.6 (d, *J =* 5.5 Hz), 123.4, 118.0, 109.8, 45.4 (d, *J* = 6.4 Hz), 26.8 (d, *J =* 66.3 Hz), 21.7.

³¹P NMR (162 MHz, CDCl₃) δ 29.70.

HRMS (ESI-TOF): calc for C₂₁H₂₃NOP [M+H]+ 336.1517, measured 336.1521

HPLC: SunFire Waters, C₁₈ 5 µm, 4.6 × 150 mm; gradient 60% to 98% MeCN/H₂O, 1.0 mL/min, 214 nm; t_{R} = 6.7 min.

### EXAMPLE 60: 1-(naphthalen-1-yl)-4-(p-tolyl)-1,4-azaphosphinane 4-oxide (AD13)

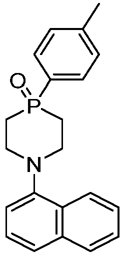

Compound 61 was prepared according to General procedure 7. Column chromatography purification (DCM/MeOH 10/0 to 9/1) afforded the product as a colorless solid (38 mg, 58%)

¹H NMR (400 MHz, CDCl₃) δ 8.21 (d, *J =* 8.0 Hz, 1H), 7.83 (dd, *J =* 9.5, 8.0 Hz, 1H), 7.77 (dd, *J=* 11.4, 8.0 Hz, 2H), 7.58 (t, *J =* 8.8 Hz, 1H), 7.56 - 7.44 (m, 2H), 7.41 (t, *J =* 6.1 Hz, 3H), 7.40 - 7.35 (m, 2H), 7.18 (d, *J* = 7.2 Hz, 1H), 3.67 (td, *J =* 12.9, 1.9 Hz, 2H), 3.62 - 3.45 (m, 2H), 2.55 - 2.45 (m, 2H), 2.44 (s, 3H), 2.25 (dd, *J =* 33.2, 17.8 Hz, 2H).

¹³C NMR (101 MHz, CDCl₃) δ 150.0, 142.8, 134.8, 130.4 (d, J= 9.9 Hz), 129.7 (d, *J =* 11.9 Hz), 129.0 (d, *J =* 100.6 Hz), 129.0, 128.6, 126.1, 125.9, 125.9, 124.3, 123.0, 116.2, 50.1 (d, *J =* 7.2 Hz), 29.8 (d, *J =* 65.9 Hz), 21.7.

³¹P NMR (162 MHz, CDCl₃) δ 27.72.

HRMS (ESI-TOF): calc for C₂₁H₂₃NOP [M+H]+ 336.1517, measured 336.1526
HPLC: SunFire Waters, C₁₈ 5 µm, 4.6 × 150 mm; gradient 60% to 98% MeCN/H₂O, 1.0 mL/min, 214 nm; t_{R} = 7.5 min.

### EXAMPLE 61: 1-(4-chlorophenyl)-4-(p-tolyl)-1,4-azaphosphinane 4-oxide (AD14)

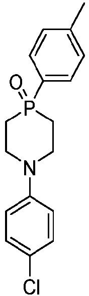

Compound 61 was prepared according to General procedure 7. Column chromatography purification (DCM/MeOH 10/0 to 9/1) afforded the product as a colorless solid (26 mg, 35%).

¹H NMR (400 MHz, CDCl₃) δ 7.56 (dd, *J =* 11.6, 8.1 Hz, 2H), 7.29 (dd, *J =* 8.0, 2.7 Hz, 2H), 7.25 - 7.20 (m, 2H), 6.95 - 6.84 (m, 2H), 3.98 - 3.76 (m, 4H), 2.39 (s, 3H), 2.26 - 2.13 (m, 2H), 2.10 - 1.91 (m, 2H).

¹³C NMR (101 MHz, CDCl₃) δ 146.6, 142.9, 130.0 (d, *J =* 10.0 Hz), 129.6 (d, *J =* 12.3 Hz), 129.5, 128.6 (d, *J =* 101.1 Hz), 124.0, 116.4, 45.1 (d, *J =* 6.5 Hz), 26.5 (d, *J =* 66.3 Hz), 21.6.

³¹P NMR (162 MHz, CDCl₃) δ 29.89.

HRMS (ESI-TOF): calc for C₁₇H₂₀NOPCl [M+H]+ 320.0971, measured 320.0975
HPLC: SunFire Waters, C₁₈ 5 µm, 4.6 × 150 mm; gradient 60% to 98% MeCN/H₂O, 1.0 mL/min, 214 nm; t_{R} = 5.5 min.

### EXAMPLE 62: 1-(naphthalen-1-yl)-4-(4-(trifluoromethyl)phenyl)-1,4-azaphosphinane 4-oxide (AD15)

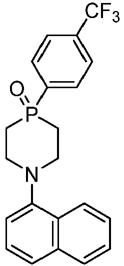

Compound 62 was prepared according to General procedure 7. Column chromatography purification (DCM/MeOH 10/0 to 9/1) afforded the product as a colorless solid (99 mg, 100%).

¹H NMR (400 MHz, CDCl₃) δ 8.33 - 8.15 (m, 1H), 8.10 - 7.97 (m, 2H), 7.85 (ddd, *J =* 11.0, 8.1, 1.8 Hz, 3H), 7.62 (dd, *J* = 8.2, 1.1 Hz, 1H), 7.58 - 7.47 (m, 2H), 7.47 - 7.37 (m, 1H), 7.20 (dd, *J* = 7.4, 1.1 Hz, 1H), 3.84 - 3.46 (m, 4H), 2.60 - 2.43 (m, 2H), 2.38 - 2.24 (m, 2H).

¹³C NMR (101 MHz, CDCl₃) δ 149.7, 136.9 (d, *J =* 94.6 Hz), 134.8, 134.2 (d, *J =* 33.6 Hz), 131.1 (d, *J =* 9.7 Hz), 128.9, 128.7, 126.2, 125.9 (d, *J =* 6.4 Hz), 125.8, 124.6, 123.6 (d, *J* = 272.8 Hz), 122.8, 116.3, 49.9 (d, *J =* 7.5 Hz), 29.8 (d, *J =* 66.1 Hz).

¹⁹F NMR (377 MHz, CDCl₃) δ -64.18.

³¹P NMR (162 MHz, CDCl₃) δ 27.52.

HRMS (ESI-TOF): calc for C₂₁H₂₀NOPF₃ [M+H]+ 390.1235, measured 390.1236
HPLC: SunFire Waters, C₁₈ 5 µm, 4.6 × 150 mm; gradient 60% to 98% MeCN/H₂O, 1.0 mL/min, 214 nm; t_{R} = 7.4 min.

### EXAMPLE 63: 1-(2-(trifluoromethoxy)phenyl)-4-(4-(trifluoromethyl)phenyl)-1,4-azaphosphinane 4-oxide (AD16)

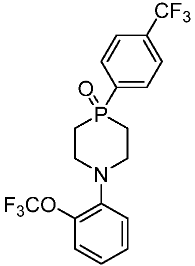

Compound 63 was prepared according to General procedure 7. Column chromatography purification (DCM/MeOH 10/0 to 9/1) afforded the product as a colorless solid (70 mg, 82%).

¹H NMR (400 MHz, CDCl₃) δ 7.88 - 7.78 (m, 2H), 7.78 - 7.71 (m, 2H), 7.15 (d, *J =* 8.6 Hz, 2H), 6.96 - 6.87 (m, 2H), 4.04 - 3.77 (m, 4H), 2.29 - 2.17 (m, 2H), 2.14 - 1.99 (m, 2H).

¹³C NMR (101 MHz, CDCl₃) δ 146.8, 141.9, 136.4 (d, *J =* 95.2 Hz), 134.1 (q, *J =* 33.2 Hz), 130.7 (d, *J =* 9.8 Hz), 126.0 - 125.3 (m), 124.7 (d, *J =* 274.0 Hz), 122.6, 120.6 (q, *J =* 256.2 Hz), 116.1, 45.2 (d, *J =* 6.6 Hz), 26.5 (d, *J =* 66.5 Hz).

¹⁹F NMR (377 MHz, CDCl₃) δ -59.32, -64.29.

³¹P NMR (162 MHz, CDCl₃) δ 29.12.

HRMS (ESI-TOF): calc for C₁₈H₁₇NO₂PF₆ [M+H]+ 424.0901, measured 424.0901.

HPLC: SunFire Waters, C₁₈ 5 µm, 4.6 × 150 mm; gradient 60% to 98% MeCN/H₂O, 1.0 mL/min, 214 nm; t_{R} = 6.3 min.

### EXAMPLE 64: 1-(2,5-difluorophenyl)-4-(4-(trifluoromethyl)phenyl)-1,4-azaphosphinane 4-oxide (AD17)

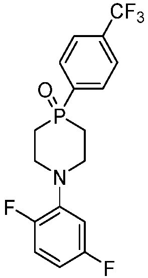

Compound 64 was prepared according to General procedure 7. Column chromatography purification (DCM/MeOH 10/0 to 9/1) afforded the product as a colorless solid (70 mg, 93%).

¹H NMR (400 MHz, CDCl₃) δ 7.94 (d, *J =* 8.8 Hz, 1H), 7.77 (dd, *J =* 8.3, 2.8 Hz, 2H), 6.98 (ddddd, J= 11.6, 8.9, 5.3, 2.6, 1.1 Hz, 1H), 6.72 (ddd, *J =* 9.8, 6.8, 2.8 Hz, 1H), 6.63 (ddtd, J= 9.6, 6.4, 3.2, 1.6 Hz, 1H), 3.75 - 3.59 (m, 4H), 2.41 - 2.27 (m, 2H), 2.16 (t, *J =* 16.3 Hz, 2H).

¹³C NMR (101 MHz, CDCl₃) δ 159.0 (d, *J =* 242.8 Hz), 151.6 (dd, *J =* 240.7, 3.0 Hz), 140.8 (t, *J =* 10.0 Hz), 136.5 (d, *J =* 95.2 Hz), 134.2 (q, *J =* 32.6 Hz), 131.0 (d, *J =* 9.7 Hz), 125.8 (dt, *J* = 12.0, 3.9 Hz), 123.5 (q, *J* = 272.8 Hz), 116.8 (dd, *J* = 23.6, 9.9 Hz), 108.9 (dd, *J =* 23.9, 8.2 Hz), 107.4 (dd, *J =* 25.7, 2.8 Hz), 47.4 (dd, *J =* 7.4, 3.5 Hz), 29.0 (d, *J =* 66.3 Hz).

¹⁹F NMR (377 MHz, CDCl₃) δ -64.24(s, 3F), -117.86 - -118.08 (m, 1F), -129.71 - -129.88 (m, 1F).

³¹P NMR (162 MHz, CDCl₃) δ 27.16.

HRMS (ESI-TOF): calc for C₁₇H₁₆NOPF₅ [M+H]+ 376.0890, measured 376.0887
HPLC: SunFire Waters, C₁₈ 5 µm, 4.6 × 150 mm; gradient 60% to 98% MeCN/H₂O, 1.0 mL/min, 214 nm; t_{R} = 7.0 min.

### EXAMPLE 65: 1-phenyl-4-(4-(trifluoromethyl)phenyl)-1,4-azaphosphinane 4-oxide (AD18)

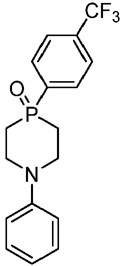

Compound 65 was prepared according to General procedure 7. Column chromatography purification (DCM/MeOH 10/0 to 9/1) afforded the product as a colorless solid (62 mg, 91%).

¹H NMR (400 MHz, CDCl₃) δ 7.84 (t, *J =* 9.5 Hz, 2H), 7.74 (d, *J =* 6.0 Hz, 2H), 7.31 (d, *J* = 8.0 Hz, 2H), 6.97 (d, *J =* 8.1 Hz, 2H), 6.88 (t, *J =* 7.3 Hz, 1H), 4.12 - 3.80 (m, 4H), 2.25 (ddt, *J =* 15.4, 11.5, 4.3 Hz, 2H), 2.15 - 1.98 (m, 2H).

¹³C NMR (101 MHz, CDCl₃) δ 147.8, 136.7 (d, *J =* 94.7 Hz), 134.0 (dq, J = 32.8, 3.3 Hz)), 130.7 (d, J = 9.8 Hz), 129.7, 125.7 (dq, J = 11.6, 3.8 Hz), 123.9 (q, *J =* 271.2 Hz)), 119.5, 115.3, 44.9 (d, J = 6.6 Hz), 26.4 (d, J = 66.5 Hz).

¹⁹F NMR (377 MHz, CDCl₃) δ -64.25 (s, 3F).

³¹P NMR (162 MHz, CDCl₃) δ 29.26.

HRMS (ESI-TOF): calc for C₁₇H₁₈NOPF₃ [M+H]+ 340.1078, measured 340.1078
HPLC: SunFire Waters, C₁₈ 5 µm, 4.6 × 150 mm; gradient 60% to 98% MeCN/H₂O, 1.0 mL/min, 214 nm; t_{R} = 5.8 min.

### EXAMPLE 66: 1-(isoquinolin-5-yl)-4-(4-(trifluoromethyl)phenyl)-1,4-azaphosphinane 4-oxide (AD19)

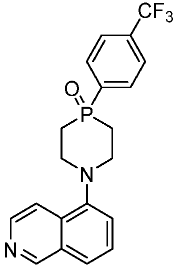

Compound 66 was prepared according to General procedure 7. Column chromatography purification (DCM/MeOH 10/0 to 9/1) afforded the product as a colorless solid (82 mg, 100%).

¹H NMR (400 MHz, CDCl₃) δ 9.20 (s, 1H), 8.53 (s, 1H), 8.00 (t, *J* = 9.7 Hz, 2H), 7.89 - 7.86 (m, 1H), 7.79 (s, 2H), 7.70 - 7.64 (m, 1H), 7.54 - 7.46 (m, 1H), 7.38 - 7.30 (m, 1H), 3.78 - 3.43 (m, 4H), 2.55 - 2.38 (m, 2H), 2.37 - 2.17 (m, 2H).

¹³C NMR (101 MHz, CDCl₃) δ 153.0, 148.6, 143.1, 136.5 (d, *J* = 95.0 Hz), 134.1 (q, *J* = 32.7 Hz), 131.6, 131.0 (d, *J* = 9.7 Hz), 129.8, 127.3, 125.7 (dq, *J* = 11.5, 3.8 Hz), 123.6, 123.4 (q, *J =* 272.9 Hz), 120.0, 115.7, 49.6 (d, *J =* 7.4 Hz), 29.5 (d, *J =* 66.0 Hz).

¹⁹F NMR (377 MHz, CDCl₃) δ -63.81 (s, 3F).

³¹P NMR (162 MHz, CDCl₃) δ 26.75.

HRMS (ESI-TOF): calc for C₂₀H₁₉N₂OPF₃ [M+H]+ 391.1187, measured 391.1186
HPLC: SunFire Waters, C₁₈ 5 µm, 4.6 × 150 mm; gradient 60% to 98% MeCN/H₂O, 1.0 mL/min, 214 nm; t_{R} = 4.4 min.

### EXAMPLE 67: 1-(pyridin-4-yl)-4-(4-(trifluoromethyl)phenyl)-1,4-azaphosphinane 4-oxide (AD20)

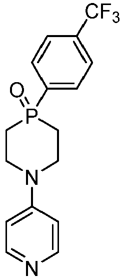

Compound 67 was prepared according to General procedure 7. Column chromatography purification (DCM/MeOH 10/0 to 9/1) afforded the product as a colorless solid (63 mg, 93%).

¹H NMR (400 MHz, CDCl₃) δ 8.28 (d, *J =* 5.7 Hz, 2H), 7.82 - 7.63 (m, 4H), 6.69 (d, *J =* 5.5 Hz, 2H), 4.24 - 4.01 (m, 2H), 3.98 - 3.78 (m, 2H), 2.23 - 1.87 (m, 4H).

¹³C NMR (101 MHz, CDCl₃) δ 151.9, 150.8, 135.9 (d, *J =* 95.9 Hz), 134.1 (dd, *J =* 32.7, 3.1 Hz), 130.7 (d, *J =* 10.0 Hz), 125.8 (dq, *J* = 11.7, 3.7 Hz), 123.3 (q, *J =* 272.5 Hz), 108.0, 42.7 (d, *J =* 6.7 Hz), 26.3 (d, *J =* 66.4 Hz).

¹⁹F NMR (377 MHz, CDCl₃) δ -63.31 (s, 3F).

³¹P NMR (162 MHz, CDCl₃) δ 29.29.

HRMS (ESI-TOF): calc for C₁₆H₁₇N₂OPF₃ [M+H]+ 341.1031, measured 341.1034
HPLC: SunFire Waters, C₁₈ 5 µm, 4.6 × 150 mm; gradient 60% to 98% MeCN/H₂O, 1.0 mL/min, 214 nm; t_{R} = 5.7 min.

### EXAMPLE 68: 1-(2-methoxyphenyl)-4-(4-(trifluoromethyl)phenyl)-1,4-azaphosphinane 4-oxide (AD 24)

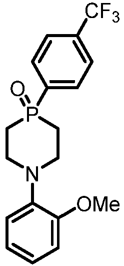

Compound 68 was prepared according to General procedure 7. Column chromatography purification (DCM/MeOH 10/0 to 9/1) afforded the product as a colorless solid (18 mg, 24%).

¹H NMR (400 MHz, CDCl₃) δ 7.99 (dd, *J =* 10.6, 8.1 Hz, 2H), 7.79 (dd, *J =* 8.4, 2.6 Hz, 2H), 7.05 (ddd, *J* = 16.3, 8.1, 1.7 Hz, 2H), 6.99 - 6.86 (m, 2H), 3.90 (s, 3H), 3.75 - 3.45 (m, 4H), 2.56 - 2.32 (m, 2H), 2.28 - 2.09 (m, 2H).

¹³C NMR (101 MHz, CDCl₃) δ 152.3, 141.4, 137.1 (d, *J =* 94.1 Hz), 134.2 (d, *J =* 33.5 Hz), 131.1 (d, *J* = 9.7 Hz), 125.7 (d, *J* = 7.6 Hz), 125.0 (q, *J* = 272.7 Hz), 124.0, 121.2, 119.8, 111.44, 55.6, 47.8 (d, *J =* 7.5 Hz), 29.4 (d, *J =* 66.1 Hz).

¹⁹F NMR (377 MHz, CDCl₃) δ -64.19 (s, 3F).

³¹P NMR (162 MHz, CDCl₃) δ 27.81.

HRMS (ESI-TOF): calc for C₁₈H₂₀NO₂PF₃ [M+H]+ 370.1184, measured 370.1184
HPLC: SunFire Waters, C₁₈ 5 µm, 4.6 × 150 mm; gradient 60% to 98% MeCN/H₂O, 1.0 mL/min, 214 nm; t_{R} = 5.7 min.

### EXAMPLE 69: 1-(4-methoxyphenyl)-4-(4-(trifluoromethyl)phenyl)-1,4-azaphosphinane 4-oxide (AD25)

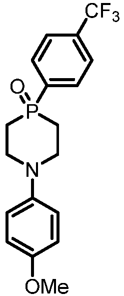

Compound 69 was prepared according to General procedure 7. Column chromatography purification (DCM/MeOH 10/0 to 9/1) afforded the product as a colorless solid (8 mg, 10%).

¹H NMR (400 MHz, CDCl₃) δ 7.88 (dd, *J =* 11.1, 8.1 Hz, 2H), 7.76 (dd, *J =* 8.5, 2.3 Hz, 2H), 6.99 - 6.92 (m, 2H), 6.91 - 6.84 (m, 2H), 3.84 - 3.71 (m, 7H), 2.29 (qd, *J =* 9.8, 4.2 Hz, 2H), 2.09 (t, *J =* 16.5 Hz, 2H).

¹³C NMR (101 MHz, CDCl₃) δ 154.1, 143.5, 136.9 (d, *J* = 94.2 Hz), 130.9 (d, *J =* 9.7 Hz), 128.8 (d, *J* = 57.9 Hz), 125.8 (d, *J* = 8.2 Hz), 123.6 (q, *J* = 273.2 Hz), 118.6, 115.0, 55.7, 47.0 (d, *J =* 6.7 Hz), 27.4 (d, *J* = 66.5 Hz).

¹⁹F NMR (377 MHz, CDCl₃) δ -64.23.

³¹P NMR (162 MHz, CDCl₃) δ 28.66.

HRMS (ESI-TOF): calc for C₁₈H₂₀NO₂PF₃ [M+H]+ 370.1184, measured 370.1184
HPLC: SunFire Waters, C₁₈ 5 µm, 4.6 × 150 mm; gradient 60% to 98% MeCN/H₂O, 1.0 mL/min, 214 nm; t_{R} = 5.3 min.

### EXAMPLE 70: 1-(thiophen-2-yl)-4-(4-(trifluoromethyl)phenyl)-1,4-azaphosphinane 4-oxide (AD26)

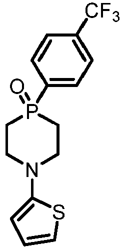

Compound 70 was prepared according to General procedure 7. Column chromatography purification (DCM/MeOH 10/0 to 9/1) afforded the product as a colorless solid (9 mg, 13%).

¹H NMR (400 MHz, CDCl₃) δ 7.90 - 7.81 (m, 2H), 7.79 - 7.72 (m, 2H), 7.29 (dd, *J =* 5.3, 3.1 Hz, 1H), 6.86 (dd, *J =* 5.2, 1.6 Hz, 1H), 6.26 (dd, *J* = 3.1, 1.6 Hz, 1H), 3.91 - 3.74 (m, 4H), 2.39 - 2.21 (m, 2H), 2.06 (ddq, *J =* 16.7, 12.3, 2.2 Hz, 2H).

¹³C NMR (101 MHz, CDCl₃) δ 149.2, 136.7 (d, *J =* 94.9 Hz), 134.1 (d, *J =* 35.2 Hz), 130.9 (d, *J =* 9.7 Hz), 126.4, 125.8 (dq, *J* = 11.7, 4.1 Hz), 123.5 (d, *J =* 272.9 Hz), 119.9, 101.0, 46.7 (d, *J =* 6.6 Hz), 26.3 (d, *J =* 66.5 Hz).

¹⁹F NMR (377 MHz, CDCl₃) δ -64.25.

³¹P NMR (162 MHz, CDCl₃) δ 28.74.

HRMS (ESI-TOF): calc for C₁₅H₁₆NOPF₃S [M+H]+ 346.0642, measured 346.0641
HPLC: SunFire Waters, C₁₈ 5 µm, 4.6 × 150 mm; gradient 60% to 98% MeCN/H₂O, 1.0 mL/min, 214 nm; t_{R} = 5.2 min.

### EXAMPLE 71: 1-(4-fluorophenyl)-4-(4-(trifluoromethyl)phenyl)-1,4-azaphosphinane 4-oxide (AD27)

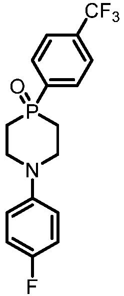

Compound 71 was prepared according to General procedure 7. Column chromatography purification (DCM/MeOH 10/0 to 9/1) afforded the product as a colorless solid (20 mg, 28%).

¹H NMR (400 MHz, CDCl₃) δ 7.90 - 7.83 (m, 2H), 7.79 - 7.73 (m, 2H), 7.04 - 6.97 (m, 2H), 6.95 - 6.88 (m, 2H), 3.94 - 3.75 (m, 4H), 2.38 - 2.20 (m, 2H), 2.08 (ddq, J= 16.8, 12.4, 2.6 Hz, 2H).

¹³C NMR (101 MHz, CDCl₃) δ 157.3 (d, *J =* 239.6 Hz), 145.5, 136.6 (d, *J =* 95.0 Hz), 134.0 (d, *J =* 31.9 Hz), 130.9 (d, *J =* 9.7 Hz), 125.8 (dq, *J* = 11.7, 3.8 Hz), 123.5 (q, *J =* 272.8 Hz), 118.0 (d, *J =* 7.6 Hz), 116.2 (d, *J =* 22.2 Hz), 46.5 (d, *J =* 6.8 Hz), 27.1 (d, *J =* 66.5 Hz).

¹⁹F NMR (377 MHz, CDCl₃) δ -64.25, -125.02 (dt, *J =* 8.3, 4.0 Hz).

³¹P NMR (162 MHz, CDCl₃) δ 28.75.

HPLC: SunFire Waters, C₁₈ 5 µm, 4.6 × 150 mm; gradient 60% to 98% MeCN/H₂O, 1.0 mL/min, 214 nm; t_{R} = 6.0 min.

### EXAMPLE 72: 4-benzyl-1-cyclopropyl-1,4-azaphosphinane 4-oxide (AD48)

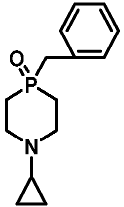

Compound 72 was prepared according to General procedure 7. Column chromatography purification (DCM/MeOH 10/0 to 9/1) afforded the product as a colorless solid (43 mg, 43%).

¹H NMR (400 MHz, CDCl₃) δ 7.30 (t, *J =* 7.5 Hz, 2H), 7.25 - 7.21 (m, 3H), 3.16 (d, *J =* 14.3 Hz, 2H), 3.05 (dddd, *J =* 16.7, 12.4, 8.7, 3.5 Hz, 2H), 2.90 (tq, *J =* 11.7, 5.1 Hz, 2H), 1.92 - 1.66 (m, 5H), 0.46 (d, *J =* 6.3 Hz, 2H), 0.38 - 0.24 (m, 2H).

¹³C NMR (101 MHz, CDCl₃) δ 131.5 (d, *J =* 7.6 Hz), 129.6 (d, *J =* 5.1 Hz), 128.9 (d, *J =* 2.6 Hz), 127.0 (d, *J =* 3.1 Hz), 49.8 (d, *J =* 6.7 Hz), 37.8 (d, *J =* 1.4 Hz), 36.4 (d, *J =* 61.3 Hz), 26.5 (d, *J =* 62.9 Hz), 7.1.

³¹P NMR (162 MHz, CDCl₃) δ 34.78.

HRMS (ESI-TOF): calc for C₁₄H₂₁NOP [M+H]+ 250.1355, measured 250.1358
HPLC: SunFire Waters, C₁₈ 5 µm, 4.6 × 150 mm; gradient 60% to 98% MeCN/H₂O, 1.0 mL/min, 214 nm; t_{R} = 3.4 min.

### EXAMPLE 73: 4-benzyl-1-(4-(trifluoromethyl)phenyl)-1,4-azaphosphinane 4-oxide (AD49)

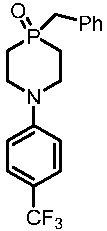

Compound 73 was prepared according to General procedure 7. Column chromatography purification (DCM/MeOH 10/0 to 9/1) afforded the product as a colorless solid (143 mg, 100%).

¹H NMR (400 MHz, CDCl₃) δ 7.52 - 7.44 (m, 2H), 7.31 (dddd, *J =* 7.3, 6.5, 1.9, 1.2 Hz, 2H), 7.28 - 7.21 (m, 3H), 6.88 - 6.82 (m, 2H), 3.96 - 3.72 (m, 4H), 3.18 (d, *J =* 14.1 Hz, 2H), 1.86 (dt, *J =* 11.7, 5.5 Hz, 4H).

¹³C NMR (101 MHz, CDCl₃) δ 149.7, 130.8 (d, *J* = 8.0 Hz), 129.6 (d, *J* = 5.2 Hz), 129.1 (d, *J =* 2.8 Hz), 127.4 (d, *J =* 3.2 Hz), 127.1 (d, *J =* 3.8 Hz), 124.7 (q, *J =* 270.6 Hz), 120.3 (d, *J* = 32.8 Hz), 113.6, 44.3 (d, *J =* 6.2 Hz), 37.3 (d, *J =* 62.4 Hz), 24.9 (d, *J =* 63.1 Hz).

³¹P NMR (162 MHz, CDCl₃) δ 35.54.

HRMS (ESI-TOF): calc for C₁₈H₂₀NOPF₃ [M+H]+ 354.1229, measured 354.1231
HPLC: SunFire Waters, C₁₈ 5 µm, 4.6 × 150 mm; gradient 60% to 98% MeCN/H₂O, 1.0 mL/min, 214 nm; t_{R} = 15.3 min.

### EXAMPLE 74: 4-benzyl-1-(p-tolyl)-1,4-azaphosphinane 4-oxide (AD50)

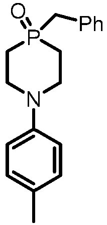

Compound 74 was prepared according to General procedure 7. Column chromatography purification (DCM/MeOH 10/0 to 9/1) afforded the product as a colorless solid (95 mg, 77%).

¹H NMR (400 MHz, CDCl₃) δ 7.29 - 7.20 (m, 2H), 7.21 - 7.14 (m, 3H), 6.99 (d, *J =* 8.1 Hz, 2H), 6.71 (dd, *J* = 8.6, 1.8 Hz, 2H), 3.69 (ddt, *J* = 15.9, 13.0, 5.1 Hz, 2H), 3.52 (ddd, *J =* 16.4, 12.3, 7.6 Hz, 2H), 3.09 (dd, *J =* 13.9, 2.0 Hz, 2H), 2.19 (s, 3H), 1.80 (dt, *J =* 12.9, 5.8 Hz, 4H).

¹³C NMR (101 MHz, CDCl₃) δ 145.9, 131.0 (d, *J =* 8.0 Hz), 130.0, 129.5 (d, *J =* 5.2 Hz), 128.9 (d, *J = 2.6* Hz), 128.8, 127.0 (d, *J =* 3.0 Hz), 115.7, 45.7 (d, *J =* 6.2 Hz), 36.7 (d, *J* = 61.8 Hz), 25.1 (d, *J =* 63.1 Hz), 20.3.

³¹P NMR (162 MHz, CDCl₃) δ 35.36.

HRMS (ESI-TOF): calc for C₁₈H₂₃NOP [M+H]+ 300.1511, measured 300.1514
HPLC: SunFire Waters, C₁₈ 5 µm, 4.6 × 150 mm; gradient 60% to 98% MeCN/H₂O, 1.0 mL/min, 214 nm; t_{R} = 5.1 min.

### EXAMPLE 75: (E)-1-(4-methylbenzyl)-4-styryl-1,4-azaphosphinane 4-oxide (91)

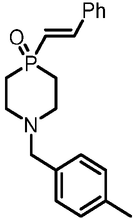

Compound 75 was prepared according to General procedure 11. Column chromatography purification (DCM/MeOH 10/0 to 9/1) afforded the product as a colorless solid (135 mg, 83%).

¹H NMR (400 MHz, CDCl₃) δ 7.57 - 7.43 (m, 3H), 7.40 - 7.30 (m, 3H), 7.22 - 7.16 (m, 2H), 7.12 (d, *J =* 7.7 Hz, 2H), 6.57 - 6.40 (m, 1H), 3.56 (s, 2H), 2.95 (dtd, *J =* 17.3, 10.4, 3.4 Hz, 2H), 2.78 (dddd, *J =* 16.8, 12.1, 7.7, 4.0 Hz, 2H), 2.32 (s, 3H), 2.14 - 1.87 (m, 4H).

¹³C NMR (101 MHz, CDCl₃) δ 147.5 (d, *J =* 2.8 Hz), 137.0, 135.2 (d, *J =* 17.0 Hz), 135.2, 130.1, 129.1, 128.9, 128.8, 127.6 (d, *J =* 1.1 Hz), 118.3 (d, *J =* 96.4 Hz), 62.0 (d, *J =* 1.0 Hz), 49.6 (d, *J =* 6.7 Hz), 28.8 (d, *J =* 67.2 Hz), 21.2.

³¹P NMR (162 MHz, CDCl₃) δ 25.81.

HRMS (ESI-TOF): calc for C₂₀H₂₅NOP [M+H]+ 326.1668, measured 326.1664
HPLC: SunFire Waters, C₁₈ 5 µm, 4.6 × 150 mm; gradient 60% to 98% MeCN/H₂O, 1.0 mL/min, 214 nm; t_{R} = 6.3 min.

### EXAMPLE 76: (E)-1-(4-methylbenzyl)-4-(2-methylstyryl)-1,4-azaphosphinane 4-oxide (AD94)

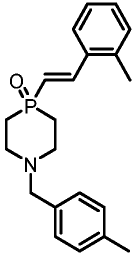

Compound 76 was prepared according to General procedure 11. Column chromatography purification (DCM/MeOH 10/0 to 9/1) afforded the product as a colorless solid (96 mg, 95%).

¹H NMR (400 MHz, CDCl₃) δ 7.86 - 7.73 (m, 1H), 7.53 (dd, *J* = 7.6, 1.6 Hz, 1H), 7.30 - 7.18 (m, 5H), 7.14 (d, *J =* 7.5 Hz, 2H), 6.41 (m, 1H), 3.60 (s, 2H), 3.06 - 2.91 (m, 2H), 2.88 - 2.73 (m, 2H), 2.43 (s, 3H), 2.35 (s, 3H), 2.16 - 1.91 (m, 4H).

¹³C NMR (101 MHz, CDCl₃) δ 145.3 (d, *J =* 3.1 Hz), 137.2 (d, *J =* 16.5 Hz), 135.2, 134.5, 134.3, 130.9, 129.9, 129.2, 128.9, 126.4, 126.1 (d, *J =* 1.5 Hz), 119.8 (d, *J =* 96.1 Hz), 62.1, 49.7 (d, *J =* 6.7 Hz), 28.9 (d, *J =* 67.1 Hz), 21.2, 19.9.

³¹P NMR (162 MHz, CDCl₃) δ 25.67.

HRMS (ESI-TOF): calc for C₂₁H₂₇NOP [M+H]+ 340.1824, measured 340.1821
HPLC: SunFire Waters, C₁₈ 5 µm, 4.6 × 150 mm; gradient 60% to 98% MeCN/H₂O, 1.0 mL/min, 214 nm; t_{R} = 7.5 min.

### EXAMPLE 77: (E)-1-(4-methylbenzyl)-4-(3-methylstyryl)-1,4-azaphosphinane 4-oxide (AD95)

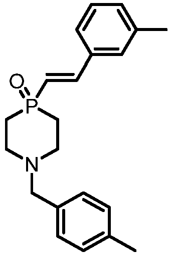

Compound 77 was prepared according to General procedure 11. Column chromatography purification (DCM/MeOH 10/0 to 9/1) afforded the product as a colorless solid (96 mg, 95%).

¹H NMR (400 MHz, CDCl₃) δ 7.57 - 7.42 (m, 1H), 7.35 - 7.08 (m, 8H), 6.54 - 6.40 (m, 1H), 3.57 (s, 2H), 3.03 - 2.88 (m, 2H), 2.78 (dddd, *J =* 16.8, 12.0, 7.7, 4.0 Hz, 2H), 2.35 (s, 3H), 2.32 (s, 3H), 2.15 - 1.87 (m, 4H).

¹³C NMR (101 MHz, CDCl₃) δ 147.5 (d, *J =* 2.8 Hz), 138.4, 136.8, 135.1, 135.0 (d, *J =* 17.0 Hz), 130.8, 129.0, 128.7, 128.7, 128.1 (d, *J* = 1.1 Hz), 124.8 (d, *J* = 1.1 Hz), 117.9 (d, *J* = 96.5 Hz), 61.9 (d, *J =* 0.9 Hz), 49.5 (d, *J =* 6.8 Hz), 28.7 (d, *J =* 67.2 Hz), 21.3, 21.1.

³¹P NMR (162 MHz, CDCl₃) δ 25.78.

HRMS (ESI-TOF): calc for C₂₁H₂₇NOP [M+H]+ 340.1824, measured 340.1826
HPLC: SunFire Waters, C₁₈ 5 µm, 4.6 × 150 mm; gradient 60% to 98% MeCN/H₂O, 1.0 mL/min, 214 nm; t_{R} = 8.0 min.

### EXAMPLE 78: (E)-1-(4-methylbenzyl)-4-(4-methylstyryl)-1,4-azaphosphinane 4-oxide (AD96)

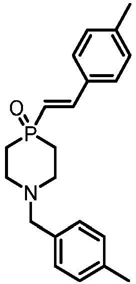

Compound 78 was prepared according to General procedure 11. Column chromatography purification (DCM/MeOH 10/0 to 9/1) afforded the product as a colorless solid (46 mg, 46%).

¹H NMR (400 MHz, CDCl₃) δ 7.55 - 7.41 (m, 1H), 7.38 (d, *J =* 8.0 Hz, 2H), 7.23 - 7.13 (m, 4H), 7.12 (d, *J* = 7.8 Hz, 2H), 6.48 - 6.33 (m, 1H), 3.56 (s, 2H), 3.03 - 2.87 (m, 2H), 2.85 - 2.70 (m, 2H), 2.34 (s, 3H), 2.32 (s, 3H), 2.12 - 1.88 (m, 4H).

¹³C NMR (101 MHz, CDCl₃) δ 147.2 (d, *J =* 2.9 Hz), 140.3, 136.9, 135.1, 132.4 (d, *J =* 17.2 Hz), 129.5, 129.0, 128.7, 127.5 (d, *J =* 1.1 Hz), 116.8 (d, *J =* 97.3 Hz), 61.9, 49.5 (d, *J =* 6.7 Hz), 28.7 (d, *J =* 67.1 Hz), 21.4, 21.1.

³¹P NMR (162 MHz, CDCl₃) δ 25.89.

HRMS (ESI-TOF): calc for C₂₁H₂₇NOP [M+H]+ 340.1824, measured 340.1823
HPLC: SunFire Waters, C₁₈ 5 µm, 4.6 × 150 mm; gradient 60% to 98% MeCN/H₂O, 1.0 mL/min, 214 nm; t_{R} = 8.0 min.

### EXAMPLE 79: (E)-1-(4-methylbenzyl)-4-(4-nitrostyryl)-1,4-azaphosphinane 4-oxide (AD97)

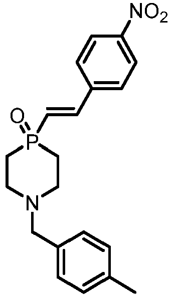

Compound 79 was prepared according to General procedure 11. Column chromatography purification (DCM/MeOH 10/0 to 9/1) afforded the product as a colorless solid (47 mg, 42%).

¹H NMR (400 MHz, CDCl₃) δ 8.21 (dt, *J =* 8.7, 1.3 Hz, 2H), 7.63 (d, *J =* 8.7 Hz, 2H), 7.61 - 7.51 (m, 1H), 7.19 (d, *J =* 7.8 Hz, 2H), 7.11 (d, *J =* 7.8 Hz, 2H), 6.78 - 6.60 (m, 1H), 3.58 (s, 2H), 3.03 - 2.90 (m, 2H), 2.88 - 2.73 (m, 2H), 2.31 (s, 3H), 2.16 - 1.91 (m, 4H).

¹³C NMR (101 MHz, CDCl₃) δ 148.3, 144.9 (d, *J =* 2.9 Hz), 141.0 (d, *J =* 17.0 Hz), 137.0, 134.9, 129.1, 128.7, 128.3 (d, *J =* 1.0 Hz), 124.1, 123.7 (d, *J =* 92.3 Hz), 62.0, 49.4 (d, *J =* 6.9 Hz), 28.6 (d, *J =* 67.3 Hz), 21.1.

³¹P NMR (162 MHz, CDCl₃) δ 25.56.

HRMS (ESI-TOF): calc for C₂₀H₂₄N₂O₃P [M+H]+ 371.1519, measured 371.1513
HPLC: SunFire Waters, C₁₈ 5 µm, 4.6 × 150 mm; gradient 60% to 98% MeCN/H₂O, 1.0 mL/min, 214 nm; t_{R} = 6.1 min.

### EXAMPLE 80: (E)-4-(4-methoxystyryl)-1-(4-methylbenzyl)-1,4-azaphosphinane 4-oxide (AD98)

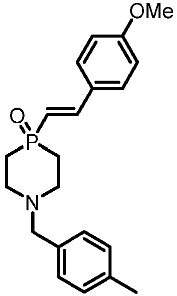

Compound 80 was prepared according to General procedure 11. Column chromatography purification (DCM/MeOH 10/0 to 9/1) afforded the product as a colorless solid (43 mg, 41%).

¹H NMR (400 MHz, CDCl₃) δ 7.50 - 7.37 (m, 3H), 7.24 - 7.17 (m, 2H), 7.17 - 7.09 (m, 2H), 6.93 - 6.85 (m, 2H), 6.37 - 6.22 (m, 1H), 3.82 (s, 3H), 3.58 (s, 2H), 3.03 - 2.88 (m, 2H), 2.88 - 2.72 (m, 2H), 2.33 (s, 3H), 2.12 - 1.88 (m, 4H).

¹³C NMR (101 MHz, CDCl₃) δ 161.2, 147.0 (d, *J* = 3.0 Hz), 137.0, 135.2, 129.2, 129.1, 128.8, 128.0 (d, *J =* 17.4 Hz), 115.2 (d, *J =* 98.5 Hz), 114.3, 62.1, 55.4, 49.6 (d, *J =* 6.8 Hz), 28.9 (d, *J =* 67.2 Hz), 21.2.

³¹P NMR (162 MHz, CDCl₃) δ 26.31 (d, *J =* 1.4 Hz).

HRMS (ESI-TOF): calc for C₂₁H₂₇NO₂P [M+H]+ 356.1773, measured 356.1768
HPLC: SunFire Waters, C₁₈ 5 µm, 4.6 × 150 mm; gradient 60% to 98% MeCN/H₂O, 1.0 mL/min, 214 nm; t_{R} = 6.2 min.

### EXAMPLE 81: (E)-1-(4-methylbenzyl)-4-(2-(pyridin-2-yl)vinyl)-1,4-azaphosphinane 4-oxide (AD99)

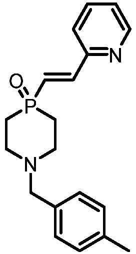

Compound 81 was prepared according to General procedure 11. Column chromatography purification (DCM/MeOH 10/0 to 9/1) afforded the product as a colorless solid (60 mg, 61%).

¹H NMR (400 MHz, CDCl₃) δ 8.60 (dd, *J =* 4.8, 1.7 Hz, 1H), 7.67 (td, *J =* 7.7, 1.8 Hz, 1H), 7.60 - 7.44 (m, 1H), 7.31 (d, *J =* 7.7 Hz, 1H), 7.25 - 7.19 (m, 1H), 7.19 - 7.11 (m, 3H), 7.09 (d, *J =* 7.8 Hz, 2H), 3.54 (s, 2H), 3.02 - 2.87 (m, 2H), 2.85 - 2.70 (m, 2H), 2.29 (s, 3H), 2.13 - 1.89 (m, 4H).

¹³C NMR (101 MHz, CDCl₃) δ 152.7 (d, *J =* 17.0 Hz), 149.9, 146.1 (d, *J =* 3.2 Hz), 137.0, 136.8, 135.0, 129.0, 128.7, 124.5, 124.2, 123.0 (d, *J =* 93.5 Hz), 61.8, 49.4 (d, *J =* 6.7 Hz), 28.6 (d, *J =* 67.2 Hz), 21.0.

³¹P NMR (162 MHz, CDCl₃) δ 26.02.

HRMS (ESI-TOF): calc for C₁₉H₂₄N₂OP [M+H]+ 327.1621, measured 327.1621
HPLC: SunFire Waters, C₁₈ 5 µm, 4.6 × 150 mm; gradient 60% to 98% MeCN/H₂O, 1.0 mL/min, 214 nm; t_{R} = 4.2 min.

### EXAMPLE 82: 1-(4-methylbenzyl)-4-phenethyl-1,4-azaphosphinane 4-oxide (AD101)

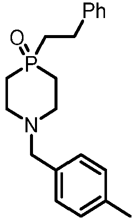

Compound 82 was prepared according to General procedure 12. Column chromatography purification (DCM/MeOH 10/0 to 9/1) afforded the product as a colorless solid (43 mg, 54%).

¹H NMR (400 MHz, CDCl₃) δ 7.28 (t, *J =* 7.5 Hz, 2H), 7.24 - 7.18 (m, 3H), 7.16 (d, *J =* 8.0 Hz, 2H), 7.10 (d, *J* = 7.8 Hz, 2H), 3.52 (s, 2H), 3.03 - 2.92 (m, 2H), 2.92 - 2.81 (m, 2H), 2.72 - 2.55 (m, 2H), 2.31 (s, 3H), 2.12 - 1.99 (m, 2H), 1.98 - 1.85 (m, 2H), 1.76 (dtd, *J* = 12.6, 8.2, 3.7 Hz, 2H).

¹³C NMR (101 MHz, CDCl₃) δ 140.9 (d, *J* = 12.6 Hz), 136.9, 135.0, 129.0, 128.7, 128.7, 128.1, 126.5, 61.9 (d, *J* = 0.9 Hz), 49.5 (d, *J* = 6.7 Hz), 29.9 (d, *J* = 65.1 Hz), 27.5 (d, *J* = 62.3 Hz), 27.1 (d, *J* = 3.7 Hz), 21.1.

³¹P NMR (162 MHz, CDCl₃) δ 35.50.

HRMS (ESI-TOF): calc for C₂₀H₂₇NOP [M+H]+ 328.1824, measured 328.1824
HPLC: SunFire Waters, C₁₈ 5 µm, 4.6 × 150 mm; gradient 60% to 98% MeCN/H₂O, 1.0 mL/min, 214 nm; t_{R} = 6.2 min.

### EXAMPLE 83: (E)-1-(4-methylbenzyl)-4-(4-(trifluoromethyl)styryl)-1,4-azaphosphinane 4-oxide (AD109)

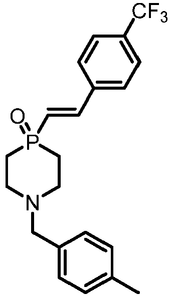

Compound 83 was prepared according to General procedure 11. Column chromatography purification (DCM/MeOH 10/0 to 9/1) afforded the product as a colorless solid (118 mg, 100%).

¹H NMR (400 MHz, CDCl₃) δ 7.60 (q, *J =* 8.2 Hz, 4H), 7.52 (d, *J =* 17.9 Hz, 1H), 7.19 (d, *J* = 8.0 Hz, 2H), 7.12 (d, *J =* 8.4 Hz, 2H), 6.70 - 6.52 (m, 1H), 3.58 (s, 2H), 3.04 - 2.89 (m, 2H), 2.88 - 2.72 (m, 2H), 2.32 (s, 3H), 2.15 - 1.90 (m, 4H).

¹³C NMR (101 MHz, CDCl₃) δ 145.9 (d, *J* = 2.7 Hz), 138.4 (dd, *J* = 17.0, 1.4 Hz), 137.0, 135.0, 131.5 (q, *J =* 32.8 Hz), 129.1, 128.8, 127.8, 125.8 (q, *J =* 3.8 Hz), 123.8 (q, *J =* 272.2 Hz), 121.5 (d, *J =* 93.9 Hz), 62.0, 49.5 (d, *J =* 6.8 Hz), 28.7 (d, *J =* 67.3 Hz), 21.1.

¹⁹F NMR (377 MHz, CDCl₃) δ -62.75.

³¹P NMR (162 MHz, CDCl₃) δ 25.59.

HRMS (ESI-TOF): calc for C₂₁H₂₄NOPF₃ [M+H]+ 394.1542, measured 394.1540
HPLC: SunFire Waters, C₁₈ 5 µm, 4.6 × 150 mm; gradient 60% to 98% MeCN/H₂O, 1.0 mL/min, 214 nm; t_{R} = 9.3 min.

### EXAMPLE 84: (E)-4-(4-bromostyryl)-1-(4-methylbenzyl)-1,4-azaphosphinane 4-oxide (AD110)

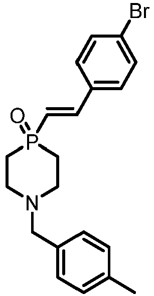

Compound 84 was prepared according to General procedure 11. Column chromatography purification (DCM/MeOH 10/0 to 9/1) afforded the product as a colorless solid (101 mg, 83%).

¹H NMR (400 MHz, CDCl₃) δ 7.50 - 7.45 (m, 2H), 7.44 - 7.38 (m, 1H), 7.36 - 7.31 (m, 2H), 7.18 (d, *J =* 7.8 Hz, 2H), 7.11 (d, *J =* 7.8 Hz, 2H), 6.53 - 6.39 (m, 1H), 3.56 (s, 2H), 3.01 - 2.87 (m, 2H), 2.85 - 2.70 (m, 2H), 2.31 (s, 3H), 2.11 - 1.86 (m, 4H).

¹³C NMR (101 MHz, CDCl₃) δ 146.1 (d, *J =* 2.9 Hz), 136.9, 134.9, 134.0 (d, *J =* 17.2 Hz), 132.0, 129.1, 129.0 (d, *J =* 0.9 Hz), 128.7, 124.2, 119.1 (d, *J =* 95.5 Hz), 61.9, 49.4 (d, *J* = 6.7 Hz), 28.6 (d, *J =* 67.2 Hz), 21.1.

³¹P NMR (162 MHz, CDCl₃) δ 25.84.

HRMS (ESI-TOF): calc for C₂₀H₂₄NOPBr [M+H]+ 404.0773, measured 404.0767
HPLC: SunFire Waters, C₁₈ 5 µm, 4.6 × 150 mm; gradient 60% to 98% MeCN/H₂O, 1.0 mL/min, 214 nm; t_{R} = 9.0 min.

### EXAMPLE 85: (E)-4-(4-chlorostyryl)-1-(4-methylbenzyl)-1,4-azaphosphinane 4-oxide (AD111)

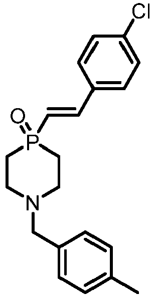

Compound 85 was prepared according to General procedure 11. Column chromatography purification (DCM/MeOH 10/0 to 9/1) afforded the product as a colorless solid (85 mg, 79%).

¹H NMR (400 MHz, CDCl₃) δ 7.52 - 7.45 (m, 1H), 7.42 (d, *J =* 8.2 Hz, 2H), 7.33 (d, *J =* 8.2 Hz, 2H), 7.20 (d, *J =* 7.8 Hz, 2H), 7.12 (d, *J =* 7.7 Hz, 2H), 6.57 - 6.35 (m, 1H), 3.58 (s, 2H), 3.04 - 2.88 (m, 2H), 2.87 - 2.72 (m, 2H), 2.33 (s, 3H), 2.11 - 1.86 (m, 4H).

¹³C NMR (101 MHz, CDCl₃) δ 146.1 (d, *J =* 2.9 Hz), 137.0, 135.9, 135.0, 133.6 (d, *J =* 17.2 Hz), 129.1, 129.0, 128.9, 128.8, 119.0 (d, *J =* 95.6 Hz), 62.0, 49.5 (d, *J =* 6.8 Hz), 28.7 (d, *J* = 67.1 Hz), 21.1.

³¹P NMR (162 MHz, CDCl₃) δ 25.71.

HRMS (ESI-TOF): calc for C₂₀H₂₄NOPC1 [M+H]+ 360.1279, measured 360.1273
HPLC: SunFire Waters, C₁₈ 5 µm, 4.6 × 150 mm; gradient 60% to 98% MeCN/H₂O, 1.0 mL/min, 214 nm; t_{R} = 8.4 min.

### EXAMPLE 86: 1-(4-methylbenzyl)-4-(2-methylphenethyl)-1,4-azaphosphinane 4-oxide (AD115)

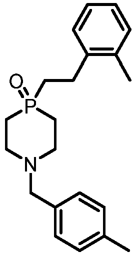

Compound 86 was prepared according to General procedure 12. Column chromatography purification (DCM/MeOH 10/0 to 9/1) afforded the product as a colorless solid (24 mg, 71%).

¹H NMR (400 MHz, CDCl₃) δ 7.21 - 7.16 (m, 3H), 7.16 - 7.11 (m, 5H), 3.55 (s, 2H), 3.01 - 2.85 (m, 4H), 2.75 - 2.58 (m, 2H), 2.34 (s, 3H), 2.33 (s, 3H), 2.08 - 1.91 (m, 4H), 1.83 (ddq, *J =* 12.5, 8.2, 3.7 Hz, 2H).

¹³C NMR (101 MHz, CDCl₃) δ 139.2 (d, *J* = 12.9 Hz), 137.0, 135.8, 130.5, 129.1, 128.8, 128.5, 128.54 (d, *J =* 51.7 Hz), 126.7, 126.4, 62.0 (d, *J* = 0*.*9 Hz), 49.6 (d, *J =* 6.7 Hz), 28.6 (d, *J* = 64.8 Hz), 27.5 (d, *J* = 62.3 Hz), 24.6 (d, *J =* 3.5 Hz), 21.2, 19.3.

³¹P NMR (162 MHz, CDCl₃) δ 35.65.

HRMS (ESI-TOF): calc for C₂₁H₂₉NOP [M+H]+ 342.1981, measured 342.1984
HPLC: SunFire Waters, C₁₈ 5 µm, 4.6 × 150 mm; gradient 60% to 98% MeCN/H₂O, 1.0 mL/min, 214 nm; t_{R} = 7.6 min.

### EXAMPLE 87: 1-(4-methylbenzyl)-4-(3-methylphenethyl)-1,4-azaphosphinane 4-oxide (AD116)

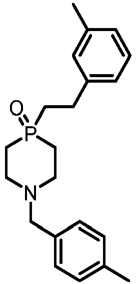

Compound 87 was prepared according to General procedure 12. Column chromatography purification (DCM/MeOH 10/0 to 9/1) afforded the product as a colorless solid (26 mg, 76%).

¹H NMR (400 MHz, CDCl₃) δ 7.22 - 7.16 (m, 3H), 7.15 - 7.10 (m, 2H), 7.07 - 6.99 (m, 3H), 3.54 (s, 2H), 3.01 - 2.82 (m, 4H), 2.75 - 2.60 (m, 2H), 2.33 (s, 3H), 2.33 (s, 3H), 2.15 - 2.01 (m, 2H), 2.00 - 1.86 (m, 2H), 1.87 - 1.72 (m, 2H).

¹³C NMR (101 MHz, CDCl₃) δ 141.0 (d, *J* = 12.6 Hz), 138.4, 137.0, 135.1, 129.1, 129.0, 128.8, 128.6, 127.3, 125.2, 62.0 (d, *J =* 0.9 Hz), 49.6 (d, *J =* 6.6 Hz), 30.0 (d, *J =* 65.1 Hz), 27.5 (d, *J =* 62.3 Hz), 27.1 (d, *J =* 3.7 Hz), 21.4, 21.2.

³¹P NMR (162 MHz, CDCl₃) δ 35.67.

HRMS (ESI-TOF): calc for C₂₁H₂₉NOP [M+H]+ 342.1981, measured 342.1989
HPLC: SunFire Waters, C₁₈ 5 µm, 4.6 × 150 mm; gradient 60% to 98% MeCN/H₂O, 1.0 mL/min, 214 nm; t_{R} = 7.9 min.

### EXAMPLE 88: 1-(4-methylbenzyl)-4-(4-methylphenethyl)-1,4-azaphosphinane 4-oxide (AD117)

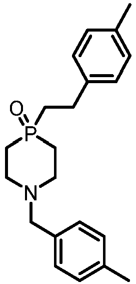

Compound 88 was prepared according to General procedure 12. Column chromatography purification (DCM/MeOH 10/0 to 9/1) afforded the product as a colorless solid (33 mg, 100%).

¹H NMR (400 MHz, CDCl₃) δ 7.18 (d, *J =* 8.1 Hz, 2H), 7.12 (dd, *J =* 8.5, 1.1 Hz, 6H), 3.54 (s, 2H), 3.04 - 2.81 (m, 4H), 2.76 - 2.56 (m, 2H), 2.33 (s, 3H), 2.31 (s, 3H), 2.11 - 1.99 (m, 2H), 1.97 - 1.86 (m, 2H), 1.85 - 1.71 (m, 2H).

¹³C NMR (101 MHz, CDCl₃) δ 137.9 (d, *J =* 12.6 Hz), 137.0, 136.1, 135.1, 129.4, 129.1, 128.8, 128.1, 62.0 (d, *J* = 0.9 Hz), 49.5 (d, *J =* 6.7 Hz), 30.1 (d, *J =* 65.1 Hz), 27.5 (d, *J* = 62.3 Hz), 26.8 (d, *J* = 3.7 Hz), 21.2, 21.1.

³¹P NMR (162 MHz, CDCl₃) δ 35.70.

HRMS (ESI-TOF): calc for C₂₁H₂₉NOP [M+H]+ 342.1981, measured 342.1988
HPLC: SunFire Waters, C₁₈ 5 µm, 4.6 × 150 mm; gradient 60% to 98% MeCN/H₂O, 1.0 mL/min, 214 nm; t_{R} = 7.9 min.

### EXAMPLE 89: 4-(4-methoxyphenethyl)-1-(4-methylbenzyl)-1,4-azaphosphinane 4-oxide (AD119)

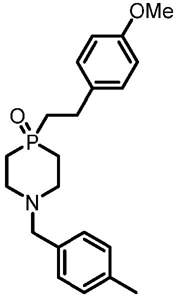

Compound 89 was prepared according to General procedure 12. Column chromatography purification (DCM/MeOH 10/0 to 9/1) afforded the product as a colorless solid (14 mg, 58%).

¹H NMR (400 MHz, CDCl₃) δ 7.19 (d, *J =* 7.9 Hz, 2H), 7.17 - 7.08 (m, 4H), 6.83 (d, *J =* 8.3 Hz, 2H), 3.78 (s, 3H), 3.59 (s, 2H), 3.00 - 2.85 (m, 4H), 2.82 - 2.65 (m, 2H), 2.34 (s, 3H), 2.10 - 1.99 (m, 2H), 1.95 - 1.79 (m, 4H).

¹³C NMR (101 MHz, CDCl₃) δ 158.3, 137.4, 132.9 (d, *J =* 12.3 Hz), 129.3, 129.1, 127.2, 124.3, 114.2, 62.0, 55.4, 49.4 (d, *J =* 6.9 Hz), 30.3 (d, *J =* 65.3 Hz), 27.3 (d, *J =* 62.5 Hz), 26.4 (d, *J =* 3.8 Hz), 21.2.

³¹P NMR (162 MHz, CDCl₃) δ 35.36.

HRMS (ESI-TOF): calc for C₂₁H₂₉NO₂P [M+H]+ 358.1930, measured 358.1935
HPLC: SunFire Waters, C₁₈ 5 µm, 4.6 × 150 mm; gradient 60% to 98% MeCN/H₂O, 1.0 mL/min, 214 nm; t_{R} = 6.0 min.

### EXAMPLE 90: 1-(4-methylbenzyl)-4-(4-(trifluoromethyl)phenethyl)-1,4-azaphosphinane 4-oxide (AD123)

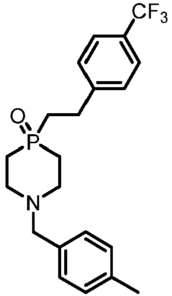

Compound 90 was prepared according to General procedure 12. Column chromatography purification (DCM/MeOH 10/0 to 9/1) afforded the product as a colorless solid (30 mg, 76%).

¹H NMR (400 MHz, CDCl₃) δ 7.55 (d, *J =* 8.0 Hz, 2H), 7.35 (d, *J =* 8.0 Hz, 2H), 7.19 (d, *J* = 8.0 Hz, 2H), 7.13 (d, *J =* 7.8 Hz, 2H), 3.57 (s, 2H), 3.03 (q, *J =* 9.2 Hz, 2H), 2.98 - 2.83 (m, 2H), 2.80 - 2.62 (m, 2H), 2.33 (s, 3H), 2.15 - 2.02 (m, 2H), 2.01 - 1.75 (m, 4H).

¹³C NMR (101 MHz, CDCl₃) δ 145.1 (d, *J* = 12.8 Hz), 137.3, 129.2, 129.2, 128.9, 128.9, 128.6, 125.7 (q, *J =* 3.7 Hz), 124.2 (q, *J =* 272.1 Hz), 62.0, 49.5 (d, *J* = 6.7 Hz), 29.7 (d, *J* = 65.0 Hz), 27.5 (d, *J* = 62.9 Hz), 27.1 (d, *J* = 3.7 Hz), 21.2.

³¹P NMR (162 MHz, CDCl₃) δ 35.33.

HRMS (ESI-TOF): calc for C₂₁H₂₆NOPF₃ [M+H]+ 396.1699, measured 396.1703
HPLC: SunFire Waters, C₁₈ 5 µm, 4.6 × 150 mm; gradient 60% to 98% MeCN/H₂O, 1.0 mL/min, 214 nm; t_{R} = 8.6 min.

### EXAMPLE 91: 1,4-bis(4-methylbenzyl)-1,4-azaphosphinane 4-oxide (AD126)

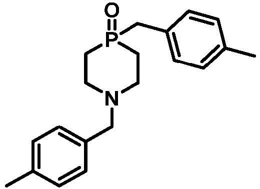

### Compound 91 was prepared according to General procedures

General **procedure 1.** Column chromatography purification (DCM/MeOH 10/0 to 9/1) afforded the product as a colorless solid (122 mg, 83 %).

¹H NMR (400 MHz, CDCl₃) δ 7.18 - 7.07 (m, 8H), 3.53 (s, 2H), 3.13 (d, *J =* 14.1 Hz, 2H), 2.97 - 2.81 (m, 2H), 2.81 - 2.64 (m, 2H), 2.32 (s, 3H), 2.31 (s, 3H), 1.95 - 1.72 (m, 4H).

¹³C NMR (101 MHz, CDCl₃) δ 137.0, 136.7 (d, *J =* 3.2 Hz), 135.0, 129.6 (d, *J =* 2.7 Hz), 129.5 (d, *J =* 5.1 Hz), 129.1, 128.8, 128.3 (d, *J =* 7.8 Hz), 61.9 (d, *J =* 0.9 Hz), 49.5 (d, *J* = 6.6 Hz), 35.8 (d, *J =* 61.8 Hz), 26.5 (d, *J =* 62.8 Hz), 21.1, 21.1 (d, *J =* 1.1 Hz).

³¹P NMR (162 MHz, CDCl₃) δ 34.56.

HRMS (ESI-TOF): calc for C₂₀H₂₇NOP [M+H]+ 328.1825, measured 328.1826
HPLC: SunFire Waters, C₁₈ 5 µm, 4.6 × 150 mm; gradient 60% to 98% MeCN/H₂O, 1.0 mL/min, 214 nm; t_{R} = 6.6 min.

### EXAMPLE 92: 1-(4-methylbenzyl)-4-(4-(trifluoromethyl)benzyl)-1,4-azaphosphinane 4-oxide (AD127)

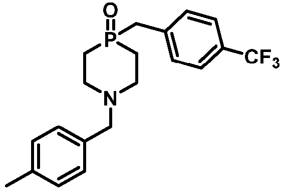

### 4. Compound 92 was prepared according to General procedure 2General procedures

### General procedure 1: Formation of azaphosphinane

In a 250 mL flame dried round flask under N2, a solution of ethyl divinylphosphonate (5.0 g, 34.2 mmol, 1.0 equiv.) in freshly distilled EtOH (75 mL) was prepared. Benzylamine derivatives (7.3 g, 68.4 mmol, 2.0 equiv.) was then added and the reaction was refluxed for 48 h until no starting material was detected (monitored by ^{31P} NMR). Then, the reaction mixture was concentrated under reduced pressure. Purification by column chromatography (silica, Acetone/MeOH 100:0 to 90:10) afforded 6.1 g (70% yield) of the desired product.

**General procedure 2.** Column chromatography purification (DCM/MeOH 10/0 to 9/1) afforded the product as a colorless solid (82 mg, 48%).

¹H NMR (400 MHz, CDCl₃) δ 7.58 (d, *J =* 8.1 Hz, 2H), 7.40 (d, *J =* 8.4 Hz, 2H), 7.17 (d, *J* = 8.1 Hz, 2H), 7.13 (d, *J =* 8.0 Hz, 2H), 3.56 (s, 2H), 3.23 (d, *J =* 13.8 Hz, 2H), 3.00 - 2.85 (m, 2H), 2.81 - 2.66 (m, 2H), 2.33 (s, 3H), 2.07 - 1.87 (m, 2H), 1.86 - 1.73 (m, 2H).

¹³C NMR (101 MHz, CDCl₃) δ 137.2, 135.8 (d, *J =* 7.7 Hz), 134.9, 130.1 (d, *J =* 5.0 Hz), 129.2, 128.9, 125.9 (t, *J =* 3.2 Hz), 124.2 (q, *J =* 272.6 Hz), 62.0, 49.6 (d, *J =* 6.7 Hz), 36.1 (d, *J* = 59.8 Hz), 27.0 (d, *J =* 63.2 Hz), 21.2.

³¹P NMR (162 MHz, CDCl₃) δ 33.98.

HRMS (ESI-TOF): calc for C₂₀H₂₄NOPF₃ [M+H]+ 382.1542, measured 382.1542
HPLC: SunFire Waters, C₁₈ 5 µm, 4.6 × 150 mm; gradient 60% to 98% MeCN/H₂O, 1.0 mL/min, 214 nm; t_{R} = 7.4 min.

### EXAMPLE 93: (S)-1-(1-phenylethyl)-4-(p-tolyl)-1,4-azaphosphinane 4-oxide (AD138)

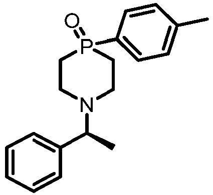

Compound 93 was prepared according to General procedure 2. Column chromatography purification (DCM/MeOH 10/0 to 9/1) afforded the product as a colorless solid (3 mg, 10%).

¹H NMR (400 MHz, CDCl₃) δ 7.65 (dd, *J* = 11.2, 7.8 Hz, 2H), 7.41 - 7.22 (m, 7H), 3.78 (q, *J* = 6.8 Hz, 1H), 2.98 (qd, *J* = 12.8, 6.1 Hz, 4H), 2.41 (s, 3H), 2.20 - 1.93 (m, 4H), 1.41 (d, *J =* 6.7 Hz, 3H).

¹³C NMR (126 MHz, CDCl₃) δ 143.4, 142.5, 130.4 (d, *J =* 9.7 Hz), 129.9, 129.6 (d, *J =* 12.0 Hz), 128.4, 127.5, 127.2, 63.3, 46.1 (dd, *J* = 132.2, 7.0 Hz), 28.9 (dd, *J* = 66.0, 9.6 Hz), 21.7, 17.6.

³¹P NMR (162 MHz, CDCl₃) δ 28.70.

HRMS (ESI-TOF): calc for C₁₉H₂₅NOP [M+H]+ 314.1668, measured 314.1674
HPLC: SunFire Waters, C₁₈ 5 µm, 4.6 × 150 mm; gradient 60% to 98% MeCN/H₂O, 1.0 mL/min, 214 nm; t_{R} = 4.7 min.

### EXAMPLE 94: 4-(hydroxy(phenyl)methyl)-1-((S)-1-phenylethyl)-1,4-azaphosphinane 4-oxide (AD140)

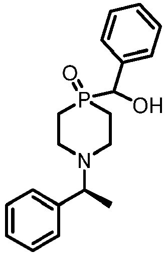

Compound 94 was prepared according to General procedure 8. Column chromatography purification (DCM/MeOH 10/0 to 9/1) afforded the product as a colorless solid (28 mg, 97%).

¹H NMR (400 MHz, CDCl₃) δ 7.41 (d, *J =* 7.9 Hz, 2H), 7.35 (t, *J =* 7.4 Hz, 2H), 7.33 - 7.21 (m, 6H), 4.97 (dd, *J* = 6.4, 2.0 Hz, 1H), 3.67 (q, *J =* 6.7 Hz, 1H), 2.97 - 2.71 (m, 4H), 1.93 (dd, *J* = 14.8, 7.3 Hz, 2H), 1.76 (t, *J =* 16.9 Hz, 2H), 1.34 (d, *J =* 6.7 Hz, 3H).

¹³C NMR (101 MHz, CDCl₃) δ 142.9, 137.0 (d, *J =* 4.0 Hz), 128.7 (d, *J =* 2.2 Hz), 128.5 (d, *J =* 1.9 Hz), 128.3 (d, *J =* 2.4 Hz), 127.4, 127.3, 126.8 (t, *J* = 4.8 Hz), 72.2 (d, *J =* 73.3 Hz), 62.9 (d, *J* = 4.0 Hz), 45.7 (ddd, *J =* 48.7, 6.5, 2.6 Hz), 24.0 (ddd, *J* = 58.3, 27.8, 20.5 Hz), 17.9.

³¹P NMR (162 MHz, CDCl₃) δ 39.90 (d, *J =* 34.4 Hz).

HRMS (ESI-TOF): calc for C₁₉H₂₅NO₂P [M+H]+ 330.1617, measured 330.1612
HPLC: SunFire Waters, C₁₈ 5 µm, 4.6 × 150 mm; gradient 60% to 98% MeCN/H₂O, 1.0 mL/min, 214 nm; t_{R} = 4.1 min.

### EXAMPLE 95: 2-(1-benzyl-4-oxido-1,4-azaphosphinan-4-yl)-1-(o-tolyl)ethan-1-one (AD166F1)

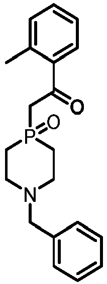

Compound 95 was prepared according to General procedure 13. Column chromatography purification (DCM/MeOH 10/0 to 9/1) afforded the product as a colorless solid (8 mg, 12%).

¹H NMR (400 MHz, CDCl₃) δ 7.94 (dd, *J* = 7.8, 1.4 Hz, 1H), 7.41 (td, *J =* 7.5, 1.4 Hz, 1H), 7.35 - 7.23 (m, 7H), 3.61 (s, 2H), 3.60 (d, *J =* 14.8 Hz, 2H), 3.08 - 2.90 (m, 2H), 2.87 - 2.73 (m, 2H), 2.54 (s, 3H), 2.13 - 1.99 (m, 4H).

¹³C NMR (101 MHz, CDCl₃) δ 196.5 (d, *J* = 6.0 Hz), 139.3, 138.2, 137.3, 132.4, 132.2, 130.6, 128.8, 128.5, 127.5, 126.2, 62.2, 49.6 (d, *J =* 7.0 Hz), 43.2 (d, *J =* 52.8 Hz), 28.2 (d, *J* = 64.1 Hz), 21.7.

³¹P NMR (162 MHz, CDCl₃) δ 33.87.

HRMS (ESI-TOF): calc for C₂₀H₂₅NO₂P [M+H]+ 342.1617, measured 342.1622
HPLC: SunFire Waters, C₁₈ 5 µm, 4.6 × 150 mm; gradient 60% to 98% MeCN/H₂O, 1.0 mL/min, 214 nm; t_{R} = 7.0 min.

### EXAMPLE 96: 1-benzyl-4-(o-tolylethynyl)-1,4-azaphosphinane 4-oxide (AD166F2)

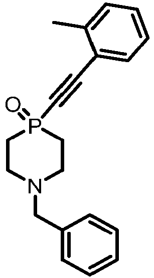

Compound 96 was prepared according to General procedure 13. Column chromatography purification (DCM/MeOH 10/0 to 9/1) afforded the product as a colorless solid (14 mg, 22%).

¹H NMR (400 MHz, CDCl₃) δ 7.51 (dd, *J* = 7.8, 1.3 Hz, 1H), 7.37 - 7.27 (m, 6H), 7.26 - 7.23 (m, 1H), 7.22 - 7.17 (m, 1H), 3.63 (s, 2H), 3.22 - 3.13 (m, 1H), 3.13 - 3.01 (m, 1H), 2.81 - 2.65 (m, 2H), 2.46 (s, 3H), 2.32 - 2.10 (m, 4H).

¹³C NMR (101 MHz, CDCl₃) δ 141.6 (d, *J* = 1.7 Hz), 138.1, 133.1 (d, *J* = 2.0 Hz), 130.7, 129.9, 128.9, 128.6, 127.5, 126.0, 119.9, 103.1 (d, *J =* 26.6 Hz), 85.7 (d, *J =* 152.1 Hz), 61.9, 50.4 (d, *J* = 6.7 Hz), 30.3 (d, *J* = 76.6 Hz), 20.9.

³¹P NMR (162 MHz, CDCl₃) δ 14.98.

HRMS (ESI-TOF): calc for C₂₀H₂₃NOP [M+H]+ 324.1512, measured 324.1519
HPLC: SunFire Waters, C₁₈ 5 µm, 4.6 × 150 mm; gradient 60% to 98% MeCN/H₂O, 1.0 mL/min, 214 nm; t_{R} = 11.6 min.

### EXAMPLE 97: 2-(1-benzyl-4-oxido-1,4-azaphosphinan-4-yl)-1-(m-tolyl)ethan-1-one (AD167F1)

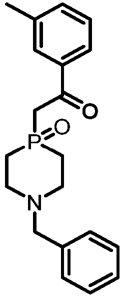

Compound 97 was prepared according to General procedure 13. Column chromatography purification (DCM/MeOH 10/0 to 9/1) afforded the product as a colorless solid (6 mg, 9%).

¹H NMR (400 MHz, CDCl₃) δ 7.85 (d, *J* = 7.6 Hz, 2H), 7.46 - 7.27 (m, 7H), 3.64 (d, *J =* 15.3 Hz, 2H), 3.60 (s, 2H), 3.04 - 2.88 (m, 2H), 2.81 (tq, *J =* 12.3, 5.8 Hz, 2H), 2.42 (s, 3H), 2.13 - 1.99 (m, 4H).

¹³C NMR (126 MHz, CDCl₃) δ 193.7 (d, *J =* 5.5 Hz), 138.8, 138.2, 137.0, 134.9, 132.3 (d, *J* = 24.2 Hz), 130.6, 129.7, 128.7 (d, *J* = 41.0 Hz), 127.4, 126.6, 62.1, 49.5 (d, *J* = 7.0 Hz), 40.9 (d, *J* = 52.2 Hz), 28.0 (d, *J =* 64.2 Hz), 21.4.

³¹P NMR (162 MHz, CDCl₃) δ 33.25.

HRMS (ESI-TOF): calc for C₂₀H₂₄NO₂P [M+H]+ 342.1617, measured 342.1617
HPLC: SunFire Waters, C₁₈ 5 µm, 4.6 × 150 mm; gradient 60% to 98% MeCN/H₂O, 1.0 mL/min, 214 nm; t_{R} = 7.0 min.

### EXAMPLE 98: 1-benzyl-4-(o-tolylethynyl)-1,4-azaphosphinane 4-oxide (AD167F3)

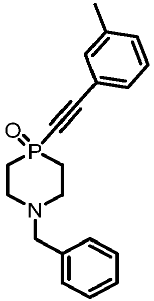

Compound 98 was prepared according to General procedure 13. Column chromatography purification (DCM/MeOH 10/0 to 9/1) afforded the product as a colorless solid (18 mg, 28%).

¹H NMR (400 MHz, CDCl₃) δ 7.43 - 7.30 (m, 9H), 3.66 (s, 2H), 3.22 - 3.15 (m, 1H), 3.14 - 3.03 (m, 1H), 2.86 - 2.67 (m, 2H), 2.39 (s, 3H), 2.32 - 2.11 (m, 4H).

¹³C NMR (126 MHz, CDCl₃) δ 138.5, 138.2, 133.0, 131.6, 129.7, 128.8, 128.6, 128.6, 127.5, 119.8 (d, *J* = 4.2 Hz), 104.4 (d, *J* = 26.8 Hz), 81.6 (d, *J* = 152.4 Hz), 61.8, 50.4 (d, *J =* 6.9 Hz), 30.2 (d, *J =* 76.7 Hz), 21.3.

³¹P NMR (162 MHz, CDCl₃) δ 14.98.

HRMS (ESI-TOF): calc for C₂₀H₂₃NOP [M+H]+ 324.1512, measured 324.1512
HPLC: SunFire Waters, C₁₈ 5 µm, 4.6 × 150 mm; gradient 60% to 98% MeCN/H₂O, 1.0 mL/min, 214 nm; t_{R} = 11.7 min.

### EXAMPLE 99: tert-butyl 1-(4-methylbenzyl)-4-(p-tolyl)-1,4-azaphosphinane-3-carboxylate 4-oxide (AD169)

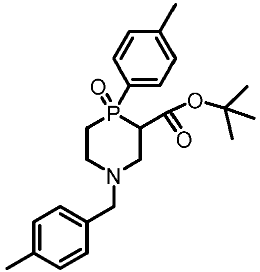

Compound 99 was prepared according to General procedure 13. Column chromatography purification (DCM/MeOH 10/0 to 9/1) afforded the product as a colorless solid (39 mg, 47%).

¹H NMR (400 MHz, CDCl₃) δ 7.70 (dd, *J =* 11.7, 8.1 Hz, 2H), 7.31 (dd, *J =* 7.8, 2.9 Hz, 2H), 7.23 (d, *J* = 8.0 Hz, 2H), 7.14 (d, *J* = 7.6 Hz, 2H), 3.65 (dd, *J* = 13.4, 3.9 Hz, 2H), 3.36 - 3.22 (m, 1H), 3.22 - 2.97 (m, 3H), 2.85 (tdd, *J* = 12.2, 6.4, 2.3 Hz, 1H), 2.41 (s, 3H), 2.34 (s, 3H), 2.16 - 1.90 (m, 2H), 1.10 (s, 9H).

¹³C NMR (101 MHz, CDCl₃) δ 166.4 (d, *J =* 7.7 Hz), 142.4 (d, *J =* 2.9 Hz), 137.0, 135.1, 131.0 (d, *J* = 9.8 Hz), 129.3, 129.2, 129.2, 129.0, 81.7, 62.6, 51.21 (d, *J =* 5.5 Hz), 48.6 (d, *J =* 7.7 Hz), 46.4 (d, *J =* 55.7 Hz), 30.1, 29.6 (d, *J =* 34.2 Hz), 27.8, 21.7 (d, *J =* 1.3 Hz), 21.2.

³¹P NMR (162 MHz, CDCl₃) δ 28.54.

HRMS (ESI-TOF): calc for C₂₄H₃₃NO₃P [M+H]+ 414.2193, measured 414.2189
HPLC: SunFire Waters, C₁₈ 5 µm, 4.6 × 150 mm; gradient 60% to 98% MeCN/H₂O, 1.0 mL/min, 214 nm; t_{R} = 11.1 min.

### EXAMPLE 100: 1-benzyl-4-(pyrimidin-2-yl)-1,4-azaphosphinane 4-oxide (AD199)

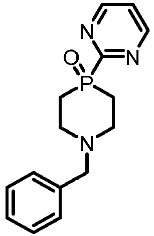

Compound 100 was prepared according to General procedure 2. Column chromatography purification (DCM/MeOH 10/0 to 9/1) afforded the product as a colorless solid (94 mg, 82%).

¹H NMR (400 MHz, CDCl₃) δ 8.89 (dt, *J =* 5.0, 0.5 Hz, 2H), 7.41 (td, *J =* 5.0, 3.3 Hz, 1H), 7.34 - 7.27 (m, 5H), 3.58 (s, 2H), 3.15 - 2.97 (m, 2H), 2.69 - 2.52 (m, 4H), 2.35 - 2.20 (m, 2H).

¹³C NMR (101 MHz, CDCl₃) δ 167.3 (d, *J =* 149.4 Hz), 157.1 (d, *J =* 13.0 Hz), 138.3, 128.8, 128.5, 127.4, 122.3 (d, *J =* 2.7 Hz), 68.0, 61.8 (d, *J =* 0.9 Hz), 49.8 (d, *J =* 6.5 Hz), 26.2 (d, *J* = 65.7 Hz), 25.7.

³¹P NMR (162 MHz, CDCl₃) δ 30.49.

HRMS (ESI-TOF): calc for C₁₅H₁₉N₃OP [M+H]+ 288.1260, measured 288.1260
HPLC: SunFire Waters, C₁₈ 5 µm, 4.6 × 150 mm; gradient 2% to 98% MeCN/H₂O, 1.0 mL/min, 214 nm; t_{R} = 11.8 min.

### EXAMPLE 101: 1-benzyl-4-(2-bromophenyl)-1,4-azaphosphinane 4-oxide (AD227)

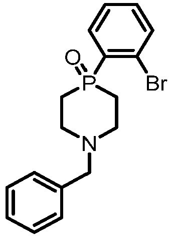

Compound 101 was prepared according to General procedure 2. Column chromatography purification (DCM/MeOH 10/0 to 9/1) afforded the product as a colorless solid (143 mg, 79%).

¹H NMR (400 MHz, CDCl₃) δ 8.04 (ddd, *J =* 12.1, 7.6, 1.8 Hz, 1H), 7.65 (ddd, *J =* 7.9, 3.9, 1.2 Hz, 1H), 7.47 (tt, *J* = 7.5, 1.4 Hz, 1H), 7.41 - 7.26 (m, 6H), 3.67 (s, 2H), 3.18 - 2.95 (m, 4H), 2.86 - 2.73 (m, 2H), 2.07 - 1.91 (m, 2H).

¹³C NMR (101 MHz, CDCl₃) δ 138.4, 135.4 (d, *J =* 7.4 Hz), 134.5 (d, *J =* 7.7 Hz), 133.5 (d, *J* = 2.6 Hz), 132.4 (d, *J* = 94.4 Hz), 128.8, 128.5, 127.6 (d, *J =* 10.0 Hz), 127.3, 124.4 (d, *J* = 6.0 Hz), 62.1 (d, *J* = 0.9 Hz), 49.1 (d, *J* = 7.6 Hz), 27.0 (d, *J =* 67.1 Hz).

³¹P NMR (162 MHz, CDCl₃) δ 30.37.

HRMS (ESI-TOF): calc for C₁₇H₂₀NOPBr [M+H]+ 364.0460, measured 364.0461
HPLC: SunFire Waters, C₁₈ 5 µm, 4.6 × 150 mm; gradient 60% to 98% MeCN/H₂O, 1.0 mL/min, 214 nm; t_{R} = 5.3 min.

### EXAMPLE 102: 1-benzyl-4-(2-(trifluoromethoxy)phenyl)-1,4-azaphosphinane 4-oxide (AD228)

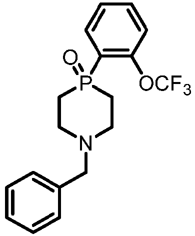

Compound 102 was prepared according to General procedure 2. Column chromatography purification (DCM/MeOH 10/0 to 9/1) afforded the product as a colorless solid (120 mg, 65%).

¹H NMR (400 MHz, CDCl₃) δ 8.05 (ddd, *J =* 12.4, 7.6, 1.8 Hz, 1H), 7.66 - 7.54 (m, 1H), 7.42 (t, *J =* 7.6 Hz, 1H), 7.38 - 7.26 (m, 6H), 3.67 (s, 2H), 3.20 - 2.94 (m, 4H), 2.49 - 2.35 (m, 2H), 2.08 - 1.91 (m, 2H).

¹³C NMR (101 MHz, CDCl₃) δ 149.9 (dq, *J =* 3.0, 1.4 Hz), 138.6, 135.0 (d, *J =* 5.5 Hz), 134.1 (d, *J* = 2.1 Hz), 128.7, 128.5, 127.3, 126.5 (d, *J =* 10.1 Hz), 123.7 (d, *J =* 90.1 Hz), 120.4 (d, *J =* 260.8 Hz), 117.9 (dq, *J =* 5.7, 2.3 Hz), 61.9 (d, *J =* 1.1 Hz), 48.9 (d, *J =* 7.5 Hz), 28.0 (d, *J =* 67.4 Hz).

¹⁹F NMR (377 MHz, CDCl₃) δ -55.80.

³¹P NMR (162 MHz, CDCl₃) δ 27.12.

HRMS (ESI-TOF): calc for C₁₈H₂₄NO₂PF₃ [M+H]+ 370.1178, measured 370.1178
HPLC: SunFire Waters, C₁₈ 5 µm, 4.6 × 150 mm; gradient 60% to 98% MeCN/H₂O, 1.0 mL/min, 214 nm; t_{R} = 6.7 min.

### EXAMPLE 103: 1-benzyl-4-(4-nitrophenyl)-1,4-azaphosphinane 4-oxide (AD255)

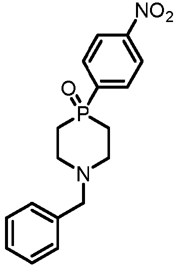

Compound 103 was prepared according to General procedure 2. Column chromatography purification (DCM/MeOH 10/0 to 9/1) afforded the product as a colorless solid (67 mg, 81%).

¹H NMR (400 MHz, CDCl₃) δ 8.34 (dd, *J =* 8.8, 2.0 Hz, 2H), 7.99 (dd, *J =* 10.6, 8.9 Hz, 2H), 7.34 (d, *J* = 4.4 Hz, 4H), 7.31 - 7.26 (m, 1H), 3.66 (s, 2H), 3.15 - 2.89 (m, 4H), 2.27 - 2.02 (m, 4H).

¹³C NMR (101 MHz, CDCl₃) δ 150.2 (d, *J* = 3.0 Hz), 140.1 (d, *J =* 90.8 Hz), 137.9, 131.8 (d, *J* = 10.1 Hz), 128.9, 128.6, 127.6, 123.7 (d, *J =* 11.7 Hz), 62.7 (d, *J =* 1.1 Hz), 49.0 (d, *J* = 7.4 Hz), 28.7 (d, *J =* 66.2 Hz).

³¹P NMR (162 MHz, CDCl₃) δ 27.89.

HPLC: SunFire Waters, C₁₈ 5 µm, 4.6 × 150 mm; gradient 60% to 98% MeCN/H₂O, 1.0 mL/min, 214 nm; t_{R} = 4.4 min.

### EXAMPLE 104: 1-benzyl-4-(hydroxy(phenyl)methyl)-1,4-azaphosphinane 4-oxide (TA74)

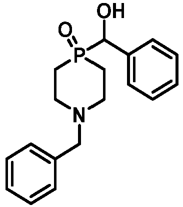

Compound 104 was prepared according to General procedure 8. Column chromatography purification (DCM/MeOH 10/0 to 9/1) afforded the product as a colorless solid (142 mg, 90%).

¹H NMR (400 MHz, CDCl₃) δ 7.40 (d, *J =* 8.7 Hz, 2H), 7.38 - 7.22 (m, 8H), 4.99 (d, *J =* 6.3 Hz, 1H), 3.53 (s, 2H), 2.92 - 2.67 (m, 4H), 2.02 - 1.89 (m, 2H), 1.84 - 1.64 (m, 2H).

¹³C NMR (101 MHz, CDCl₃) δ 137.9, 136.9, 129.0, 128.7, 128.5, 128.3, 127.4, 126.8 (d, *J* = 4.4 Hz), 72.2 (d, *J* = 75.5 Hz), 62.1, 48.8 (t, *J =* 6.4 Hz), 23.6 (dd, *J =* 59.3, 13.1 Hz).

³¹P NMR (162 MHz, CDCl₃) δ 40.31.

HRMS (ESI-TOF): calc for C₁₈H₂₃NO₂P [M+H]+ 316.1461, measured 316.1461
HPLC: SunFire Waters, C₁₈ 5 µm, 4.6 × 150 mm; gradient 60% to 98% MeCN/H₂O, 1.0 mL/min, 214 nm; t_{R} = 3.8 min.

### EXAMPLE 105: 1-benzyl-4-(hydroxy(m-tolyl)methyl)-1,4-azaphosphinane 4-oxide (TA75)

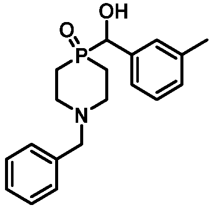

Compound 105 was prepared according to General procedure 8. Column chromatography purification (DCM/MeOH 10/0 to 9/1) afforded the product as a colorless solid (145 mg, 92%).

¹H NMR (400 MHz, CDCl₃) δ 7.39 - 7.15 (m, 8H), 7.10 (d, *J =* 7.4 Hz, 1H), 4.96 (d, *J =* 6.2 Hz, 1H), 3.53 (s, 2H), 2.92 - 2.68 (m, 4H), 2.33 (s, 3H), 1.93 (d, *J* = 15.1 Hz, 2H), 1.84 - 1.65 (m, 2H).

¹³C NMR (101 MHz, CDCl₃) δ 138.4, 137.9, 136.8, 129.1, 128.5, 128.5, 127.4, 123.9 (d, *J* = 4.6 Hz), 72.2 (d, *J* = 75.3 Hz), 62.1, 48.8 (t, *J =* 7.4 Hz), 23.7 (d, *J =* 60.2 Hz), 21.6.

³¹P NMR (162 MHz, CDCl₃) δ 42.02.

HRMS (ESI-TOF): calc for C₁₉H₂₅NO₂P [M+H]+ 330.1617, measured 330.1617
HPLC: SunFire Waters, C₁₈ 5 µm, 4.6 × 150 mm; gradient 60% to 98% MeCN/H₂O, 1.0 mL/min, 214 nm; t_{R} = 4.2 min.

### EXAMPLE 106: 1-benzyl-4-(hydroxy(4-methoxyphenyl)methyl)-1,4-azaphosphinane 4-oxide (TA76)

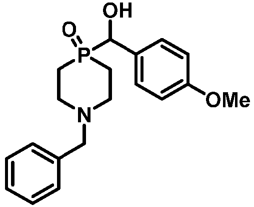

Compound 106 was prepared according to General procedure 8. Column chromatography purification (DCM/MeOH 10/0 to 9/1) afforded the product as a colorless solid (165 mg, 96%).

¹H NMR (400 MHz, CDCl₃) δ 7.38 - 7.22 (m, 7H), 6.89 (d, *J =* 8.6 Hz, 2H), 4.93 (d, *J =* 4.7 Hz, 1H), 3.80 (s, 3H), 3.54 (s, 2H), 2.92 - 2.68 (m, 4H), 2.04 - 1.84 (m, 2H), 1.84 - 1.66 (m, 2H).

¹³C NMR (101 MHz, CDCl₃) δ 159.6, 137.8, 128.8, 128.7, 128.4, 128.1 (d, *J* = 4.7 Hz), 127.3, 114.1, 71.6 (d, *J =* 76.4 Hz), 62.0, 55.3, 48.8 (d, *J =* 6.7 Hz), 23.7 (d, *J =* 59.3 Hz).

³¹P NMR (162 MHz, CDCl₃) δ 39.88.

HRMS (ESI-TOF): calc for C₁₉H₂₅NO₃P [M+H]+ 346.1566 measured 346.1567
HPLC: SunFire Waters, C₁₈ 5 µm, 4.6 × 150 mm; gradient 60% to 98% MeCN/H₂O, 1.0 mL/min, 214 nm; t_{R} = 3.8 min.

### EXAMPLE 107: 1-benzyl-4-((3,5-dimethylphenyl)(hydroxy)methyl)-1,4-azaphosphinane 4-oxide (TA77)

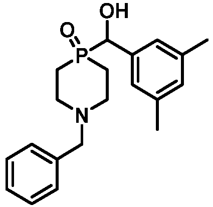

Compound 107 was prepared according to General procedure 8. Column chromatography purification (DCM/MeOH 10/0 to 9/1) afforded the product as a colorless solid (168 mg, 98%).

¹H NMR (400 MHz, CDCl₃) δ 7.35 - 7.21 (m, 5H), 7.00 (d, *J =* 2.0 Hz, 2H), 6.92 (s, 1H), 4.92 (d, *J* = 6.0 Hz, 1H), 3.54 (s, 2H), 2.91 - 2.68 (m, 4H), 2.29 (s, 6H), 1.95 (dd, *J =* 10.6, 5.8 Hz, 2H), 1.83 - 1.67 (m, 2H).

¹³C NMR (101 MHz, CDCl₃) δ 138.2, 138.0, 136.7, 130.0, 129.0, 128.5, 127.4, 124.6 (d, *J* = 4.6 Hz), 72.2 (d, *J* = 75.1 Hz), 62.2, 48.9 (t, *J =* 6.9 Hz), 23.8 (d, *J =* 59.4 Hz), 21.5.

³¹P NMR (162 MHz, CDCl₃) δ 39.93.

HRMS (ESI-TOF): calc for C₂₀H₂₇NO₂P [M+H]+ 344.1776, measured 344.1773
HPLC: SunFire Waters, C₁₈ 5 µm, 4.6 × 150 mm; gradient 60% to 98% MeCN/H₂O, 1.0 mL/min, 214 nm; t_{R} = 4.9 min.

### EXAMPLE 108: 1-benzyl-4-(hydroxy(naphthalen-2-yl)methyl)-1,4-azaphosphinane 4-oxide (TA81)

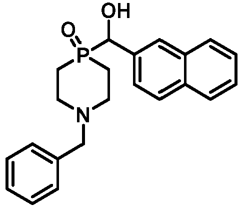

Compound 108 was prepared according to General procedure 8. Column chromatography purification (DCM/MeOH 10/0 to 9/1) afforded the product as a colorless solid (166 mg, 91%).

¹H NMR (400 MHz, CDCl₃) δ 7.86 - 7.73 (m, 4H), 7.53 - 7.42 (m, 3H), 7.32 - 7.17 (m, 5H), 5.17 (d, *J* = 7.1 Hz, 1H), 3.48 (s, 2H), 2.91 - 2.75 (m, 2H), 2.73 - 2.63 (m, 2H), 2.05 - 1.89 (m, 2H), 1.87 - 1.58 (m, 2H).

¹³C NMR (101 MHz, CDCl₃) δ 137.9, 134.6, 133.3 (dd, *J* = 4.1, 2.1 Hz), 129.0, 128.4, 128.4, 128.1, 127.8, 127.4, 126.4, 126.3, 125.8, 125.7, 124.7 (d, *J =* 2.8 Hz), 72.3 (d, *J =* 75.6 Hz), 62.1, 48.7 (dd, *J =* 13.6, 6.9 Hz), 23.6 (dd, *J =* 58.8, 19.8 Hz).

³¹P NMR (162 MHz, CDCl₃) δ 40.18.

HRMS (ESI-TOF): calc for C₂₂H₂₅NO₂P [M+H]+ 366.1617, measured 366.1617.
HPLC: SunFire Waters, C₁₈ 5 µm, 4.6 × 150 mm; gradient 60% to 98% MeCN/H₂O, 1.0 mL/min, 214 nm; t_{R} = 4.7 min.

### EXAMPLE 109: 1-benzyl-4-((6-bromopyridin-2-yl)(hydroxy)methyl)-1,4-azaphosphinane 4-oxide (TA83)

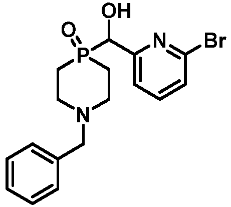

Compound 109 was prepared according to General procedure 8. Column chromatography purification (DCM/MeOH 10/0 to 9/1) afforded the product as a colorless solid (135 mg, 83%).

¹H NMR (400 MHz, CDCl₃) δ 8.60 (d, *J =* 2.3 Hz, 1H), 7.85 (dd, *J =* 8.4, 2.3 Hz, 1H), 7.62 - 7.48 (m, 1H), 7.34 - 7.22 (m, 5H), 5.06 (d, *J* = 7.3 Hz, 1H), 3.59 (s, 2H), 3.11 - 2.68 (m, 4H), 2.35 - 2.22 (m, 1H), 2.04 - 1.82 (m, 2H), 1.38 (dddt, *J =* 17.5, 14.6, 5.7, 2.9 Hz, 1H).

¹³C NMR (101 MHz, CDCl₃) δ 153.3 (d, *J* = 2.1 Hz), 149.4 (d, *J* = 2.1 Hz), 139.7 (d, *J =* 2.1 Hz), 138.2, 128.8, 128.5, 127.4, 123.5 (d, *J* = 2.8 Hz), 120.2, 71.3 (d, *J* = 74.3 Hz), 62.0, 48.6 (dd, *J =* 9.5, 7.1 Hz), 23.4 (dd, *J =* 217.2, 60.4 Hz).

³¹P NMR (162 MHz, CDCl₃) δ 40.90.

HRMS (ESI-TOF): calc for C₁₇H₂₁N₂O₂PBr [M+H]+ 395.0519, measured 395.0518.
HPLC: SunFire Waters, C₁₈ 5 µm, 4.6 × 150 mm; gradient 60% to 98% MeCN/H₂O, 1.0 mL/min, 214 nm; t_{R} = 4.0 min.

### EXAMPLE 110: 1-benzyl-4-(hydroxy(perfluorophenyl)methyl)-1,4-azaphosphinane 4-oxide (TA84)

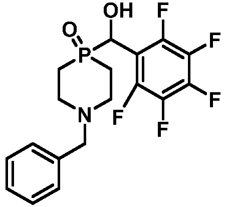

Compound 110 was prepared according to General procedure 8. Column chromatography purification (DCM/MeOH 10/0 to 9/1) afforded the product as a colorless solid (135 mg, 83%).

¹H NMR (400 MHz, CDCl₃) δ 7.37 - 7.23 (m, 5H), 5.36 (d, *J* = 7.6 Hz, 1H), 3.59 (s, 2H), 3.01 - 2.73 (m, 4H), 2.29 - 2.10 (m, 1H), 2.10 - 1.89 (m, 3H).

¹³C NMR (101 MHz, CDCl₃) δ 137.5, 129.0, 128.6, 127.7, 64.6 (d, *J =* 74.5 Hz), 62.3, 48.9 (t, *J =* 8.2 Hz), 24.7 (dd, *J =* 59.7, 16.8 Hz).

¹⁹F NMR (376 MHz, CDCl₃) δ -139.80 (d, *J* = 21.1 Hz), -152.82 (t, *J* = 20.9 Hz), -160.75 (td, *J =* 22.6, 8.1 Hz).

³¹P NMR (162 MHz, CDCl₃) δ 40.66.

HRMS (ESI-TOF): calc for C₁₈H₁₈NO₂PF₅ [M+H]+ 406.0988, measured 406.0988.

HPLC: SunFire Waters, C₁₈ 5 µm, 4.6 × 150 mm; gradient 60% to 98% MeCN/H₂O, 1.0 mL/min, 214 nm; t_{R} = 4.8 min.

### EXAMPLE 111: 1-benzyl-4-((2-bromophenyl)(hydroxy)methyl)-1,4-azaphosphinane 4-oxide (TA85)

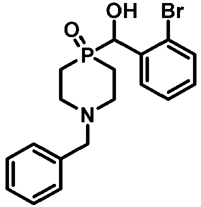

Compound 111 was prepared according to General procedure 8. Column chromatography purification (DCM/MeOH 10/0 to 9/1) afforded the product as a colorless solid (174 mg, 88%).

¹H NMR (400 MHz, CDCl₃) δ 7.75 (dt, *J* = 7.9, 2.1 Hz, 1H), 7.57 (dt, *J =* 8.0, 1.1 Hz, 1H), 7.41 (t, *J* = 7.7 Hz, 1H), 7.34 - 7.24 (m, 5H), 7.20 (tt, *J =* 7.6, 1.7 Hz, 1H), 5.60 (d, *J =* 4.9 Hz, 1H), 3.56 (d, *J* = 1.9 Hz, 2H), 2.99 - 2.84 (m, 2H), 2.84 - 2.70 (m, 2H), 2.15 - 1.93 (m, 2H), 1.92 - 1.74 (m, 2H).

¹³C NMR (101 MHz, CDCl₃) δ 137.9, 136.8, 132.9 (d, *J* = 1.9 Hz), 129.7, 129.6, 129.0, 128.5, 128.1, 127.4, 122.7 (d, *J =* 6.3 Hz), 71.2 (d, *J =* 73.6 Hz), 62.2, 48.7 (t, *J =* 8.2 Hz), 24.3 (dd, *J =* 226.1, 56.0 Hz).

³¹P NMR (162 MHz, CDCl₃) δ 41.34.

HRMS (ESI-TOF): calc for C₁₈H₂₂NO₂PBr [M+H]+ 394.0566, measured 394.0565
HPLC: SunFire Waters, C₁₈ 5 µm, 4.6 × 150 mm; gradient 60% to 98% MeCN/H₂O, 1.0 mL/min, 214 nm; t_{R} = 4.5 min.

### EXAMPLE 112: 1-benzyl-4-(hydroxy(2-(trifluoromethyl)phenyl)methyl)-1,4-azaphosphinane 4-oxide (TA86)

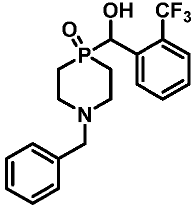

Compound 112 was prepared according to General procedure 8. Column chromatography purification (DCM/MeOH 10/0 to 9/1) afforded the product as a colorless solid (186 mg, 97%).

¹H NMR (400 MHz, CDCl₃) δ 8.04 (d, *J =* 7.9 Hz, 1H), 7.67 (d, *J =* 8.0 Hz, 1H), 7.62 (t, *J* = 7.7 Hz, 1H), 7.44 (t, *J =* 7.7 Hz, 1H), 7.34 - 7.21 (m, 5H), 5.34 (d, *J =* 3.4 Hz, 1H), 3.53 (s, 2H), 3.00 - 2.64 (m, 4H), 2.09 - 1.95 (m, 1H), 1.93 - 1.81 (m, 1H), 1.81 - 1.58 (m, 2H).

¹³C NMR (101 MHz, CDCl₃) δ 138.0, 136.3, 132.6, 130.2 (d, *J* = 4.0 Hz), 128.9, 128.5, 128.0 (d, *J =* 6.2 Hz), 127.7 (d, *J* = 6.3 Hz), 127.4, 126.0 (d, *J* = 5.9 Hz), 124.2 (q, *J* = 274.1 Hz), 67.9 (d, *J* = 72.8 Hz), 62.2, 48.6 (dd, *J* = 20.9, 7.1 Hz), 24.8 (dd, *J* = 166.0, 59.7 Hz).

¹⁹F NMR (376 MHz, CDCl₃) δ -56.90.

³¹P NMR (162 MHz, CDCl₃) δ 40.83.

HRMS (ESI-TOF): calc for C₁₉H₂₂NO₂PF₃ [M+H]+ 384.1334, measured 384.1332
HPLC: SunFire Waters, C₁₈ 5 µm, 4.6 × 150 mm; gradient 60% to 98% MeCN/H₂O, 1.0 mL/min, 214 nm; t_{R} = 4.6 min.

### EXAMPLE 113: 1-benzyl-4-(hydroxy(naphthalen-1-yl)methyl)-1,4-azaphosphinane 4-oxide (TA87)

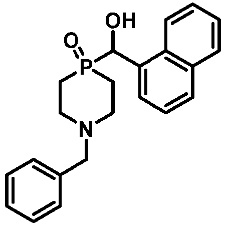

Compound 113 was prepared according to General procedure 8. Column chromatography purification (DCM/MeOH 10/0 to 9/1) afforded the product as a colorless solid (1162 mg, 89%).

¹H NMR (400 MHz, CDCl₃) δ 8.08 - 8.01 (m, 1H), 7.90 - 7.83 (m, 1H), 7.79 (t, *J =* 7.9 Hz, 2H), 7.54 - 7.42 (m, 3H), 7.25 - 7.19 (m, 3H), 7.16 (dd, *J* = 7.7, 1.8 Hz, 2H), 5.91 (d, *J =* 7.2 Hz, 1H), 3.41 (s, 2H), 2.91 - 2.53 (m, 4H), 2.00 - 1.87 (m, 1H), 1.80 (t, *J* = 16.7 Hz, 1H), 1.73 - 1.61 (m, 2H).

¹³C NMR (101 MHz, CDCl₃) δ 137.9, 133.8 (d, *J* = 1.8 Hz), 133.2, 130.8 (d, *J* = 4.2 Hz), 129.0, 129.0, 128.7, 128.4, 127.3, 126.4, 125.9, 125.5 (d, *J =* 3.0 Hz), 125.2 (d, *J =* 5.4 Hz), 123.5, 69.1 (d, *J =* 76.3 Hz), 62.1, 48.6 (dd, *J =* 15.1, 7.0 Hz), 23.2 (d, *J =* 60.2 Hz).

³¹P NMR (162 MHz, CDCl₃) δ 42.41.

HRMS (ESI-TOF): calc for C₂₂H₂₅NO₂P [M+H]+ 366.1617, measured 366.1617.

HPLC: SunFire Waters, C₁₈ 5 µm, 4.6 × 150 mm; gradient 60% to 98% MeCN/H₂O, 1.0 mL/min, 214 nm; t_{R} = 4.9 min.

### EXAMPLE 114: 1-benzyl-4-((4-fluorophenyl)(hydroxy)methyl)-1,4-azaphosphinane 4-oxide (TA88)

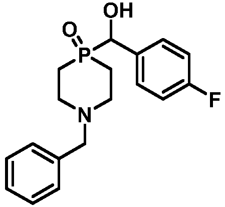

Compound 114 was prepared according to General procedure 8. Column chromatography purification (DCM/MeOH 10/0 to 9/1) afforded the product as a colorless solid (165 mg, 99%).

¹H NMR (400 MHz, CDCl₃) δ 7.40 (dd, *J* = 6.8, 5.2 Hz, 2H), 7.35 - 7.20 (m, 5H), 7.05 (t, *J* = 8.5 Hz, 2H), 4.99 (d, *J* = 5.7 Hz, 1H), 3.56 (s, 2H), 2.97 - 2.69 (m, 4H), 1.94 (tt, *J* = 14.0, 7.6 Hz, 2H), 1.80 (d, *J* = 17.2 Hz, 2H).

¹³C NMR (101 MHz, CDCl₃) δ 162.8 (d, *J* = 248.5 Hz), 137.7, 132.7, 129.0, 128.6, 128.5, 127.6, 115.7 (d, *J* = 21.5 Hz), 71.5 (d, *J* = 75.3 Hz), 62.2, 48.8 (d, *J =* 6.5 Hz), 23.7 (dd, *J* = 59.3, 23.5 Hz).

¹⁹F NMR (376 MHz, CDCl₃) δ -113.66.

³¹P NMR (162 MHz, CDCl₃) δ 39.98 (d, *J =* 4.6 Hz).

HRMS (ESI-TOF): calc for C₁₈H₂₂NO₂PF [M+H]+ 334.1367, measured 334.1366.

HPLC: SunFire Waters, C₁₈ 5 µm, 4.6 × 150 mm; gradient 60% to 98% MeCN/H₂O, 1.0 mL/min, 214 nm; t_{R} = 4.0 min.

### EXAMPLE 115: 1-benzyl-4-(hydroxy(p-tolyl)methyl)-1,4-azaphosphinane 4-oxide (TA89)

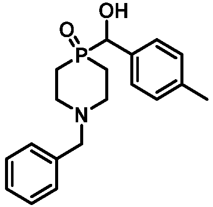

Compound 115 was prepared according to General procedure 8. Column chromatography purification (DCM/MeOH 10/0 to 9/1) afforded the product as a colorless solid (160 mg, 97%).

¹H NMR (400 MHz, CDCl₃) δ 7.34 - 7.21 (m, 7H), 7.15 (d, *J* = 8.3 Hz, 2H), 4.96 (d, *J =* 5.6 Hz, 1H), 3.53 (s, 2H), 2.90 - 2.67 (m, 4H), 2.33 (s, 3H), 2.03 - 1.86 (m, 2H), 1.83 - 1.71 (m, 2H).

¹³C NMR (101 MHz, CDCl₃) δ 138.0 (d, *J* = 2.9 Hz), 137.9, 133.7, 129.4, 128.9, 128.5, 127.4, 126.8 (d, *J* = 4.6 Hz), 72.0 (d, *J* = 75.9 Hz), 62.1, 48.8 (dd, *J* = 6.9, 3.1 Hz), 23.6 (dd, *J =* 59.3, 4.9 Hz), 21.3.

³¹P NMR (162 MHz, CDCl₃) δ 39.88.

HRMS (ESI-TOF): calc for C₁₉H₂₅NO₂P [M+H]+ 330.1617, measured 330.1617.

HPLC: SunFire Waters, C₁₈ 5 µm, 4.6 × 150 mm; gradient 60% to 98% MeCN/H₂O, 1.0 mL/min, 214 nm; t_{R} = 4.2 min.

### EXAMPLE 116: 1-benzyl-4-(hydroxy(2-methoxyphenyl)methyl)-1,4-azaphosphinane 4-oxide (TA90)

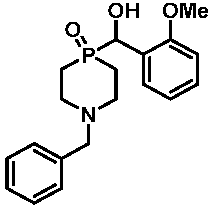

Compound 116 was prepared according to General procedure 8. Column chromatography purification (DCM/MeOH 10/0 to 9/1) afforded the product as a colorless solid (159 mg, 92%).

¹H NMR (400 MHz, CDCl₃) δ 7.56 (dd, *J* = 7.6, 2.0 Hz, 1H), 7.31 - 7.20 (m, 7H), 7.01 (t, *J* = 7.5 Hz, 1H), 6.86 (d, *J* = 8.2 Hz, 1H), 5.51 (d, *J =* 5.6 Hz, 1H), 3.81 (d, *J =* 0.8 Hz, 3H), 3.52 (s, 2H), 2.89 - 2.69 (m, 4H), 2.00 - 1.89 (m, 2H), 1.83 - 1.73 (m, 2H).

¹³C NMR (101 MHz, CDCl₃) δ 156.0 (d, *J =* 4.5 Hz), 138.0, 129.1 (d, *J* = 2.9 Hz), 129.0, 128.4, 128.1 (d, *J* = 4.2 Hz), 127.4, 125.3, 121.3 (d, *J* = 2.5 Hz), 110.3, 66.8 (d, *J* = 75.2 Hz), 62.1, 55.3, 48.9 (t, *J* = 6.7 Hz), 23.9 (dd, *J* = 200.6, 58.7 Hz).

³¹P NMR (162 MHz, CDCl3) δ 40.55.

HRMS (ESI-TOF): calc for C₁₉H₂₅NO₃P [M+H]+ 346.1567, measured 346.1565.

HPLC: SunFire Waters, C₁₈ 5 µm, 4.6 × 150 mm; gradient 60% to 98% MeCN/H₂O, 1.0 mL/min, 214 nm; t_{R} = 4.0 min.

### EXAMPLE 117: 1-benzyl-4-(hydroxy(o-tolyl)methyl)-1,4-azaphosphinane 4-oxide (TA91)

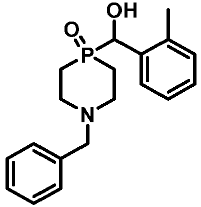

Compound 117 was prepared according to General procedure 8. Column chromatography purification (DCM/MeOH 10/0 to 9/1) afforded the product as a colorless solid (153 mg, 93%).

¹H NMR (400 MHz, CDCl₃) δ 7.53 (dt, *J =* 7.1, 2.1 Hz, 1H), 7.33 - 7.12 (m, 8H), 5.29 (d, *J* = 6.6 Hz, 1H), 3.54 (s, 2H), 2.98 - 2.84 (m, 2H), 2.73 (p, *J =* 9.5 Hz, 2H), 2.38 (s, 3H), 2.14 - 1.90 (m, 2H), 1.90 - 1.65 (m, 2H).

¹³C NMR (101 MHz, CDCl₃) δ 137.8, 135.8 (d, *J* = 5.5 Hz), 135.3, 130.9, 129.0, 128.5, 128.2 (d, *J* = 2.7 Hz), 127.5, 126.9 (d, *J* = 3.9 Hz), 126.4 (d, *J =* 2.5 Hz), 68.7 (d, *J =* 76.8 Hz), 62.2, 48.6 (dd, *J* = 9.2, 6.9 Hz), 23.7 (dd, *J* = 60.0, 16.8 Hz), 20.0.

³¹P NMR (162 MHz, CDCl₃) δ 42.02.

HRMS (ESI-TOF): calc for C₁₉H₂₅NO₂P [M+H]+ 330.1617, measured 330.1617.

HPLC: SunFire Waters, C₁₈ 5 µm, 4.6 × 150 mm; gradient 60% to 98% MeCN/H₂O, 1.0 mL/min, 214 nm; t_{R} = 4.2 min.

### EXAMPLE 118: 1-benzyl-4-(4-(difluoromethyl)phenyl)-1,4-azaphosphinane 4-oxide (AD270)

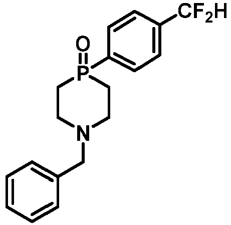

Compound 118 was prepared according to General procedure 2. Column chromatography purification (DCM/MeOH 10/0 to 9/1) afforded the product as a colorless solid (51 mg, 76%).

¹H NMR (400 MHz, CDCl₃) δ 7.88 (dd, *J =* 10.9, 8.0 Hz, 2H), 7.64 (d, *J =* 7.2 Hz, 2H), 7.37 - 7.21 (m, 5H), 6.67 (t, *J =* 56.1 Hz, 1H), 3.64 (s, 2H), 3.10 - 2.87 (m, 4H), 2.25 - 2.13 (m, 2H), 2.11 - 1.96 (m, 2H).

¹³C NMR (101 MHz, CDCl₃) δ 138.18, 137.86 (td, *J* = 22.7, 2.9 Hz), 135.61 (dt, *J =* 94.7, 1.9 Hz), 130.90 (d, J= 9.6 Hz), 128.94, 128.53, 127.46, 126.03 (dt, *J* = 11.9, 6.1 Hz), 113.99 (dd, *J* = 239.9, 1.0 Hz), 62.68 (d, *J* = 1.2 Hz), 49.14 (d, *J =* 7.2 Hz), 28.65 (d, *J =* 66.1 Hz).

¹⁹F NMR (376 MHz, CDCl₃) δ -112.30.

³¹P NMR (162 MHz, CDCl₃) δ 27.98.

HRMS (ESI-TOF): calc for C₁₈H₂₀F₂NOP [M+H]+ 336.1323, measured 336.1324
HPLC: SunFire Waters, C₁₈ 5 µm, 4.6 x 150 mm; gradient 60% to 98% MeCN/H₂O, 1.0 mL/min, 214 nm; t_{R} = 6.7 min.

### EXAMPLE 119: 1-benzyl-4-(2-fluorophenyl)-1,4-azaphosphinane 4-oxide (AD271)

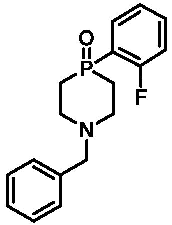

Compound 119 was prepared according to General procedure 2. Column chromatography purification (DCM/MeOH 10/0 to 9/1) afforded the product as a colorless solid (43 mg, 70%).

¹H NMR (400 MHz, CDCl₃δ 7.97 - 7.86 (m, 1H), 7.60 - 7.49 (m, 1H), 7.39 - 7.22 (m, 6H), 7.17 - 7.06 (m, 1H), 3.66 (s, 2H), 3.14 - 2.90 (m, 4H), 2.45 - 2.32 (m, 2H), 2.09 - 1.92 (m, 2H).

¹³C NMR (101 MHz, CDCl₃) δ 162.66 (dd, *J* = 248.2, 3.0 Hz), 138.37, 134.68 (dd, *J* = 8.6, 2.2 Hz), 134.18 (t, *J* = 4.6 Hz), 128.87, 128.48, 127.36, 124.92 (dd, *J =* 10.0, 3.3 Hz), 119.66 (dd, *J =* 92.7, 19.2 Hz), 116.03 (dd, *J =* 23.4, 5.7 Hz), 62.24 (d, *J* = 1.2 Hz), 48.88 (d, *J* = 7.5 Hz), 28.37 (dd, *J* = 67.3, 3.0 Hz).

¹⁹F NMR (376 MHz, CDCl₃) δ -104.37 (d, J = 5.6 Hz).

³¹P NMR (162 MHz, CDCl₃) δ 26.06.

HRMS (ESI-TOF): calc for C₁₇H₁₇FNOP [M+H]+ 304.1261, measured 304.1265
HPLC: SunFire Waters, C₁₈ 5 µm, 4.6 x 150 mm; gradient 60% to 98% MeCN/H₂O, 1.0 mL/min, 214 nm; t_{R} = 6.1 min.

### EXAMPLE 120: (R)-4-(p-tolyl)-1-(1-(p-tolyl)ethyl)-1,4-azaphosphinane 4-oxide (AD424)

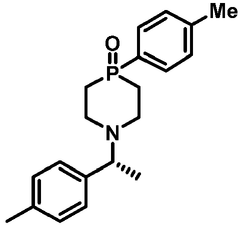

Compound 120 was prepared according to General procedure 2. Column chromatography purification (DCM/MeOH 10/0 to 9/1) afforded the product as a colorless solid (214 mg, 78%).

¹H NMR (400 MHz, CDCl₃) δ 7.64 (dd, *J =* 11.3, 8.1 Hz, 2H), 7.29 (dd, *J* = 8.1, 2.4 Hz, 2H), 7.24 (d, *J* = 8.1 Hz, 2H), 7.13 (d, *J =* 8.4 Hz, 2H), 3.75 (q, *J* = 6.7 Hz, 1H), 3.08 - 2.88 (m, 4H), 2.39 (s, 3H), 2.33 (s, 3H), 2.19 - 2.05 (m, 2H), 2.05 - 1.91 (m, 2H), 1.38 (d, *J =* 6.8 Hz, 3H).

¹³C NMR (101 MHz, CDCl₃) δ 142.41 (d, *J =* 3.0 Hz), 140.33, 136.72, 130.33 (d, *J =* 9.7 Hz), 130.10, 129.54 (d, *J =* 11.9 Hz), 129.08, 127.40, 62.97, 46.05 (dd, *J* = 87.3, 7.1 Hz), 28.92 (dd, *J* = 66.0, 7.1 Hz), 21.67, 21.13, 17.65.

³¹P NMR (162 MHz, CDCl₃) δ 28.72.

HRMS (ESI-TOF): calc for C₂₀H₂₆NOP [M+H]+ 328.1825, measured 328.1827
HPLC: SunFire Waters, C₁₈ 5 µm, 4.6 x 150 mm; gradient 60% to 98% MeCN/H₂O, 1.0 mL/min, 214 nm; t_{R} = 7.2 min.

### EXAMPLE 121: (S)-4-(p-tolyl)-1-(1-(p-tolyl)ethyl)-1,4-azaphosphinane 4-oxide

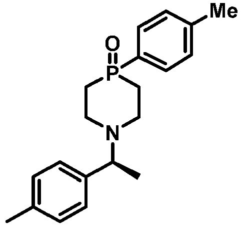

Compound 121 was prepared according to General procedure 2. Column chromatography purification (DCM/MeOH 10/0 to 9/1) afforded the product as a colorless solid (208 mg, 76%).

¹H NMR (400 MHz, CDCl₃) δ 7.65 (dd, *J* = 11.3, 8.1 Hz, 2H), 7.30 (dd, *J* = 8.4, 2.6 Hz, 2H), 7.24 (d, *J* = 8.1 Hz, 2H), 7.14 (d, *J =* 7.9 Hz, 2H), 3.75 (q, *J =* 6.7 Hz, 1H), 3.09 - 2.84 (m, 4H), 2.40 (s, 3H), 2.34 (s, 3H), 2.20 - 2.06 (m, 2H), 2.06 - 1.93 (m, 2H), 1.39 (d, *J =* 6.8 Hz, 3H).

¹³C NMR (101 MHz, CDCl₃) δ 142.46 (d, *J =* 3.0 Hz), 140.38, 136.78, 130.38 (d, *J =* 9.7 Hz), 130.17, 129.59 (d, *J* = 11.8 Hz), 129.13, 127.45, 63.03 (d, *J =* 1.0 Hz), 46.10 (dd, *J* = 88.2, 7.1 Hz), 28.97 (dd, *J* = 66.1, 7.2 Hz), 21.71, 21.18, 17.70.

³¹P NMR (162 MHz, CDCl₃) δ 28.74.

HRMS (ESI-TOF): calc for C₂₀H₂₆NOP [M+H]+ 328.1825, measured 328.1827
HPLC: SunFire Waters, C₁₈ 5 µm, 4.6 x 150 mm; gradient 60% to 98% MeCN/H₂O, 1.0 mL/min, 214 nm; t_{R} = 6.9 min.

### BIOLOGICAL EVALUATION

### 1. Material and methods

### 1.1. Drugs

4-methoxy-3-(2-phenylethoxy)-N,N-dipropylbenzeneethanamine (NE-100) was obtained from Tocris (Tebu-Bio). 2-(4-morpholinethyl)-1-phenylcyclohexane carboxylate hydrochloride (PRE-084) and (5S,10R)-(+)-5-methyl-10,11-dihydro-5Hdibenzo[a,d] cyclohepten-5,10-imine hydrogen maleate ((+)-MK-801, Dizocilpine) were from Sigma-Aldrich (Saint-Quentin-Fallavier, France). Amyloid β-[25-35] fragment (Aβ₂₅₋₃₅) was from Genepep (#1387-007, Saint-Jean-de-Védas, France) and dissolved in distilled water at 3 mg/ml and stored at -20°C until use.

### 1.2. Cellular S1R activity test

The activity of the ligands as S1R agonists was evaluated using their ability to dissociate the ER stress chaperone BiP from the S1R protein in S1R-overexpressing cells. Green fluorescent protein (GFP)-tagged S1R-overexpressing chinese hamster ovarian (CHO) cells were expanded in minimum essential medium culture medium supplemented with 10% heat-inactivated fetal bovine serum and 2 mM Glutamax (Invitrogen, Carlsbad, CA). After plating the cells, different concentrations of each compound were added for 30 min (37°C) and then the culture medium was replaced by phosphate buffered saline (3 mL). The cells were harvested and suspended in 50 mM Hepes pH 7.4, then cross-linked with 50 µg/mL of dithiobissuccinimidyl propionate (Thermoscientific, Waltham, MA). The reaction was terminated by adding Tris/HCl 50 mM pH 8.8. After incubating 15 min on ice, the cells were lysed in 50 mM Tris pH 7.4 buffer, containing 150 mM NaCl, 1% Triton X-100, 0.3% sodium deoxycholate, 0.1% sodium dodecylsulfate, and protease inhibitor cocktail (Roche Applied Science, Basel, Switzerland). After centrifugation at 16,000 g for 1 min, the supernatant was incubated with Chromotek GFP-trap agarose (Proteintech, United Kingdom) overnight at 4°C. After centrifugation at 16,000 g for 1 min, the supernatant was discarded and the pellet was suspended in 0.5 mL buffer (50 mM Tris (pH 7.4), 150 mM NaCl, 1% Triton X/100, 0.3% sodium deoxycholate, 0.1% SDS), rinsed twice, and analyzed by Heat Shock 70 KDa Protein 5 ELISA assay (#CL-SEC343Mu, Euromedex, Souffelweyersheim, France).

### 1.3. Cytotoxicity induced by Aβ₂₅₋₃₅ in neuroblastoma cells

Human neuroblastoma SH-SY5Y cells were routinely cultured in DMEM medium (Gibco, France), supplemented with 10% fetal bovine serum (Gibco, France). Aliquots of Aβ₂₅₋₃₅ solubilized in water were incubated at 37°C for 4 days to allow complete oligomerization of the peptide. The compounds were dissolved in dimethylsulfoxide (DMSO) and further diluted in cell growth medium. To initiate the experiments, the cells were seeded in 96 wells-plates at a density of 10,000 cells/well and were incubated at 37°C and 5% CO₂ for 24 h. Then, different concentrations of the compounds were added to the cells simultaneously with aggregated Aβ₂₅₋₃₅, with or without the presence of the σ₁ antagonist NE-100. After 24 h of incubation, the cell viability was indirectly determined by the MTT assay (CyQuant MTT cell viability assay, ThermoFisher, France), following the manufacturer recommendations.

### 1.4. Pancreatic MIN6 β-cells culture and glucose toxicity experiment procedure

Mouse beta-cell-derived MIN6 cells expressing interfering RNAs to provide reduction in *Wfs1* expression (MIN6-Wfs1^{WT} or MIN6-Wfs1^{KD70}) were cultured in Dulbecco's modified Eagle's medium (Sigma) containing 4.5 g/l glucose, 2 mmol/l pyruvate, supplemented with 10% (vol/vol) foetal bovine serum, 100 units/ml penicillin, 100 µg/ml streptomycin.

MIN6 cells (5 × 10⁵ cells/well) were seeded in 6-well microplates with 3 mM glucose for 16 h. Cells were then washed in phosphate-buffered saline and incubated in KRB medium with different dose-range glucose concentration. The cell viability was indirectly determined by the MTT assay (CyQuant MTT cell viability assay, ThermoFisher, France), following the manufacturer recommendations.

For insulin secretion, incubations were performed for 30 min at 37 °C. Total insulin was extracted in acidified ethanol. Total and secreted insulin were measured using ELISA assay according to the manufacturer's instructions.

### 1.5. Lipid peroxidation measurement procedure

The quantification of lipid peroxidation in tissue or cell extracts is based on Fe(III)xylenol orange complex formation. Mice were sacrificed by decapitation, without anesthesia nor sedation, after last behavioral procedure. The brains were rapidly removed, weighed and kept in liquid nitrogen until assayed. After thawing, one hippocampus per mouse or harvested cells were homogenized in cold methanol (1/10 w/v), centrifuged at 1,000 g for 5 min and the supernatant placed in an Eppendorf tube. The reaction volume of each homogenate was added to FeSO₄ 1 mM, H₂SO₄ 0.25 M, xylenol orange 1 mM and incubated for 30 min at room temperature. After reading the absorbance at 580 nm (A₅₈₀1), 10 µL of cumene hydroperoxide (CHP) 1 mM was added to the sample and incubated for 30 min at room temperature, to determine the maximal oxidation level. The absorbance was measured at 580 nm (A₅₈₀2).

The level of lipid peroxidation was determined as CHP equivalents according to: CHPE = A₅₈₀1/A₅₈₀2 × [CHP (nmol)] and expressed as CHP equivalents per mg of tissue and as a percentage of the control group data (V-treated Sc.Aβ-administered mice / control cell group).

### 1.6. Drosophila stock and breeding

*marf* coding sequence was amplified from w1118 fly, cloned into pDonr221 vector and checked by sequencing. *marf* 5' and 3' UTRs were not included, and only a kozak CAAA sequence was added in front of ATG. The R404W (R364Wlike), L118P (L76Plike), R135Q (R94Qlike), and T146M (T105Mlike) were made using quick change PCR (Agilent) that cloned (Gateway) into pUAST-w+-attB. Vectors carrying wild-type or mutant marf were inserted by phiC31-mediated transgenesis (injection into white flies carrying attP40 insertion site).

Drosophila containing marf mutation were crossed with drosophila containing S1R (which have no homologous gene in drosophila genome to generate drosophila which expressed marf mutated and S1R).

### 1.7. Drosophila treatment and negative geotaxis test

The locomotor performance of Drosophila was determined using a negative geotaxis test. Eight flies were placed in a clean plastic column (25 cm tall × internal diameter 1.5 cm) and gently tapped to the bottom. After 30 s, the flies at the top of the column (top) and the flies remaining at the bottom (bottom) were counted. Three trials were performed at 30-s intervals. Data were expressed in percentage of drosophila which are in top and percentage of Drosophila which stayed in bottom for each experimental group.

Animals were tested every two days from Day 1 to Day 9. Animals received PRE-084 at Day 8 for 24 h before Day 9 test by compound incorporated in sucrose solution 2% at 10 µM concentration, and food was removed to force drosophila to intake the compound solution. For GC60 experiment, Drosophila were tested at 1, and D3, treated with GC60 after Day 3 test for 24 h, and tested at Day 4 to determine the GC60 effect on locomotor performance.

### 1.8. Zebrafish breeding and drug treatments

Mutant *wfs1ab^{KO}* and wild-type *wfs1ab^{WT}* zebrafish lines were generated and maintained as previously described. Briefly, zebrafish lines were maintained in an automated fish tank system (ZebTEC, Tecniplast) at 28°C, pH 7, conductivity around 500 µS and with a 14 h:10 h light:dark cycle. They were fed with standard diet. Embryos were collected from natural breeding, raised at 28.5°C in zebrafish water.

At 4 dpf, larvae were transferred in 6 wells-plates, and treatment were administered in water to 3 µM concentration. At 5 dpf, treatment were freshly administered in water, and individual larvae are transferred in wells of 96-well plate (Whatman #7701-1651 square and flat bottom wells) with 300 µL embryo medium and placed in the incubator at 28°C. The *wfs1ab^{WT}* larvae were placed in even wells and *wfs1ab^{KO}* in odd wells. Larvae that did not have swim bladders or presented visible physiological deformities were not used for the experiment. The compounds were dissolved in DMSO and was applied at the concentration of 3 µM for screening.

### 1.9. Visual motor response

The visual motor response (VMR) assay quantifies the locomotor activity of zebrafish larvae in response to light changes using infrared (IR) tracking system. The experiments were performed as described. Briefly, at 5 dpf, the locomotor behavior of larvae was monitored by using an automated video tracking device (Zebrabox, ViewPoint), employing a DinionXF 1/3-inch Monochrome camera (model BFLY-PGE-13H2M, FLIR) fitted with a fixed-angle megapixel lens (SR5014MP-5C, Seiko Optical). In the device, larvae in 96-well plate are isolated from environmental surrounding noise. The box floor emitted by a light-controlling unit a white light (69-83 µW/cm² measured at λ 495 nm). The response to stimuli was recorded by an IR camera (25 frames/s) under IR light illumination at 850 nm, which the animals could not perceive.

The experiment started at 10:00 am and consisted in acclimating larvae to darkness (0% brightness) for 30 min, then switching light ON (100% brightness) for 10 min, then OFF (0% brightness) during 10 min. This was repeated twice, giving a total experiment duration of 70 min. The brightness changes were immediate (<< 1 s). The detection sensitivity was set to 31, activity burst threshold to 30 and activity freeze threshold to 10. Locomotor activity between the two threshold values was considered as normal activity. The distance covered was measured in mm/s. The values obtained in OFF were subtracted for each larvae to their ON values in order to normalize with the basic locomotion for each larvae.

### 1.10. Mice breeding and drug treatments

Male Swiss mice of 3 months old of age were maintained in the animal facility of the University of Montpellier (CECEMA) for experiments. Animals were housed in group, allowed food and water *ad libitum* access except during experiments. They were maintained in a controlled environment (22 ± 1°C, 55 ± 5% humidity) with a 12 h:12 h light/dark cycle, lights on at 7:00 h. Behavioral testing was performed between 10:00 and 16:00 h. All animal procedures were conducted in strict adherence to the European Union Directive 2010/63 and ARRIVE guidelines and authorized by the National Ethic Committee (Paris) (APAFIS # 35307-2022020909546393).

PRE-084 and NE-100 were solubilized in physiological saline (vehicle solution). GC60, LM91 and other compounds tested were solubilized in stock solution at 10 mM in 100% DMSO, then diluted for further experiment. It was administered intraperitoneally (IP) or, *per os* by gavage (PO), in accordance with the design of each experiment, at the indicated doses in a volume of 5 ml/kg body weight, 30 min before the behavioral tests for dizocilpine experiment, or 20 minutes prior to Aβ₂₅₋₃₅ intracerebroventricular (icv) injection. Before administration, Aβ₂₅₋₃₅ oligomerization was performed by incubating the peptide at 37°C for 4 days. Intracerebroventricular (ICV) injections were performed as previously described and control injections were included with the vehicle solution (distilled sterile water).

### 1.11. Spontaneous alternation in the Y-maze

The spatial working memory of the mice was evaluated as from the spontaneous alternation in the Y maze. The maze was made of grey polyvinylchloride. Each arm was 40 cm long, 13 cm high, 3 cm wide at the bottom, 10 cm wide at the top, and converging at an equal angle. Each mouse was placed at the end of one arm and allowed to move freely through the maze during an 8 min session. The series of arm entries, including possible returns into the same arm, was recorded visually. An alternation was defined as entries into all three arms on consecutive occasions. The number of maximum alternations was therefore the total number of arm entries minus two and the percentage of alternation was calculated as (actual alternations/maximum alternations) x 100. Animals that failed to explore more than 8 arms in 8 minutes were removed from the calculations.

### 1.12. Step-through passive avoidance

The contextual non-spatial long-term memory was studied by the step-through passive avoidance test. The apparatus is a two compartment (15 × 20 × 15 cm high) box with one illuminated with white polyvinylchloride walls and the other darkened with black polyvinylchloride walls and a grid floor. A guillotine door separates each compartment. A 60 W lamp positioned 40 cm above the apparatus lights up the white compartment during the experiment. Scrambled foot shocks (0.3 mA for 3 s) could be delivered to the grid floor using a shock generator scrambler (Lafayette Instruments, Lafayette, USA). The guillotine door was initially closed during the training session. Each mouse was placed into the white compartment. After 5 s, the door was raised. When the mouse entered the darkened compartment and placed all its paws on the grid floor, the door was closed, and the foot shocks delivered for 3 s. The step-through latency, that is the latency spent to enter the darkened compartment, and the shock sensitivity (estimated as 0 = no reaction, 1 = flinching reactions, 2 = flinchings and vocalizations) were recorded. None of the treatment affected shock sensitivity (data not shown). The retention test was performed 24 h after training. Each mouse was placed again into the white compartment. After 5 s, the door was raised. The step-through latency was recorded up to 300 s each. Results were expressed as median and interquartile (25%-75%) range because the data are nonparametric with an established upper limit.

### 1.13. Protein level measurement by ELISA assay

One hippocampus per mouse was homogenized in 50 mM Tris-150 mM NaCl buffer, pH 7.5, and sonicated for 20 s. After centrifugation (16,100 g for 15 min, 4°C), supernatants were used for ELISA assays according to the manufacturer instructions (USCN, ref. SEB343Mu for Bax, SEA778Mu for Bcl2, SEG168Mu for PSD-95, SEA563Mu for IL-1β, SEA079Mu for IL-6, and SEA133Mu for TNFα quantification). Results were expressed in percent of control (Sc.Aβ + Vehicle solution).

### 1.14. Rotarod procedure

Rotarod apparatus (Bioseb, Chaville, France) was used to test the balance and motor coordination of *Wfs1^{E864K}* mice. Familiarization of the mice with the rotating rod was immediately followed by a training session on day 1 (P24). The mice were trained to stay on the rod, with a progressively increased speed (from 4 rpm up to 10 rpm). Following each fall, the mice were replaced on the rotating rod (total of 4 trials on day 1). On day 2 (P25) and 3 (P26), the mice were tested with a fixed speed of 10 rpm. During these trials, the latency to fall was measured up to 180 s. Each animal underwent 4 trials per day. For each training and test day, the data were averaged per mouse and subsequently per.

### 1.15. Intraperitoneal glucose intolerance test

One-month old mice were fasted for 16 h before the experiment. 25% (w/v) glucose solution was prepared in 0,9% NaCl and administered intraperitoneally at a dose of 2 g/kg of body weight. Blood glucose levels were measured at 0 min, 15 min, 30 min, 60 min, 90 min and 120 min, respectively, from the tail vein using a glucometer (Accu-Chek).

### 1.16. Statistical analyses

Analyses were done using Prism v10.0. Data were analyzed using one-way analysis of variance (ANOVA, *F* value) or two-way ANOVA with genotype and treatment as independent variables, followed by a Dunnett's test, or Bonferroni's test, depending on the data representation. Passive avoidance latencies, expressed as median and interquartile range and represented as box-and-whiskers, were analyzed using a nonparametric Kruskal-Wallis ANOVA (*H* value) and *post-hoc* comparisons done using the Dunn's test.

### 2. Pharmacology - Results

### 2.1. Phenotypic screening in zebrafish

All neo-synthetized compounds were tested in zebrafish larvae using the VMR test to assess their effect on (1) the basal locomotion in *wfs1^{WT}* larvae and (2) the hyperlocomotor response observed in *wfs1^{KO}* larvae. Data were expressed as, respectively, the drug-induced percentage of locomotor response compared to untreated *wfs1^{WT}* larvae, LocWT% in (1), and the drug-induced percentage of locomotor increase observed in *wfs1^{KO}* larvae as compared to untreated *wfs1^{WT}* larvae, LocKO% in (2). From these data, a protection index was calculated as: PI = (100 - LocKO% + LocWT%) / 100. The PI was equal to 1.0 when a drug did not alter locomotion in *wfs1^{WT}* larvae and fully prevented the locomotor increase in *wfs1^{KO}* larvae. The three readouts were determined for each drug, tested at the concentration of 3 µM.

The reference drugs were tested at the same concentration of 3 µM as used in the phenotypic screening and data are detailed in Table 3. At this concentration, the pharmacological references were highly active (igmesine with a PI close to 1) or moderately active (PRE-084, cutamesine with PI close to 0.5). The two drugs in clinical development for other indication (blarcamesine, pridopidine) did not appear active in the test, with PI close to 0.

**Table 3. VMR results of the pharmacological or clinical S1R reference drugs in the VMR test in wfs1^{KO} zebrafish larvae. ^{a} variation from basal locomotion in wfs1^{WT} larvae less than 20%; ^{b}variation less than 50%.**

| Compound | LocWT% | LocKO% | PI |
|---|---|---|---|
| PRE-084 | 2^{a} | 48^{b} | 0.54^{b} |
| Igmesine | -15^{a} | -13^{a} | 0.98^{a} |
| Cutamesine | -2^{a} | 52 | 0.46 |
| Blarcamesine | -5^{a} | 73 | 0.22 |
| Pridopidine | 0^{a} | 139 | -0.39 |

The results for the compounds of the invention are presented in Table 4 below.

**Table 4.**

| Compound | Structure | LocWT% | LocKO% | PI |
|---|---|---|---|---|
| 1 | 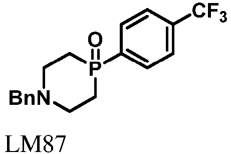 | 9 | 18.49 | 0.91 |
| 2 | 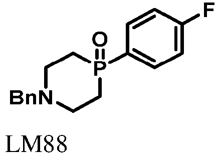 | -3.7 | 10.92 | 0.85 |
| 3 | 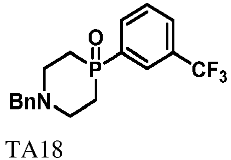 | -10 | 10 | 0.8 |
| 4 | 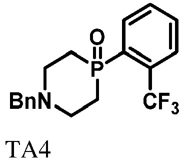 | -60 | -53 | 0.93 |
| 5 | 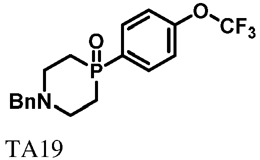 | 3 | 67 | 0.36 |
| 6 | 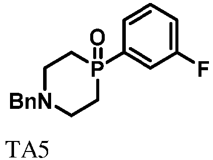 | -58 | 112 | -0.7 |
| 7 | 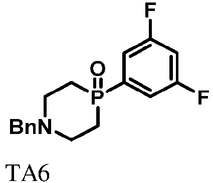 | -19 | 111 | -0.3 |
| 8 | 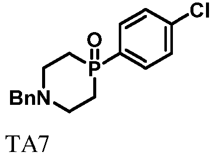 | -11 | 84 | 0.05 |
| 9 | 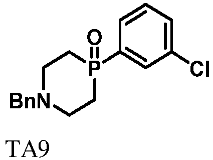 | 30 | 148 | -0.18 |
| 10 | 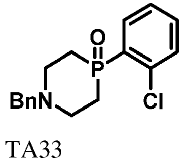 | 6 | 40 | 0.66 |
| 11 | 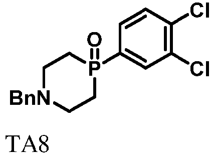 | -60 | -11 | 0.51 |
| 12 | 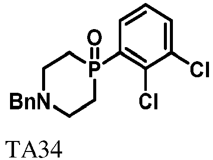 | -17 | -101 | 1.84 |
| 13 | 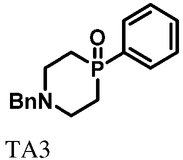 | -4 | 0 | 0.96 |
| 14 | 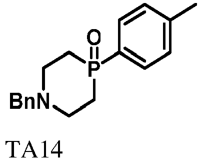 | 3 | 13 | 0.9 |
| 15 | 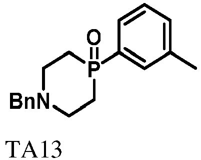 | 3 | -8 | 1.11 |
| 16 | 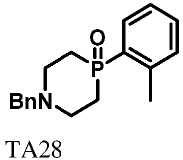 | -20 | 88 | -0.08 |
| 17 | 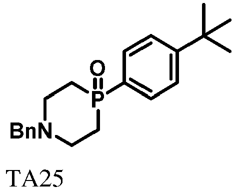 | -52 | 148 | -1 |
| 18 | 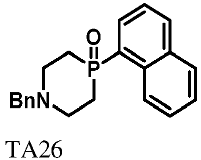 | -4 | -7 | 1.03 |
| 19 | 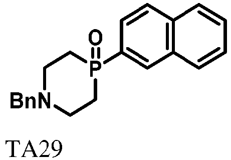 | -16 | -93 | 1.77 |
| 20 | 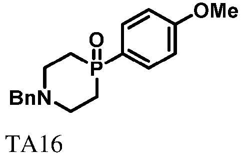 | 13 | -108 | 2.21 |
| 21 | 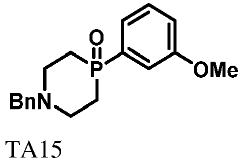 | -20 | -56 | 1.36 |
| 22 | 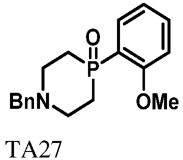 | -25 | 93 | -0.18 |
| 23 | 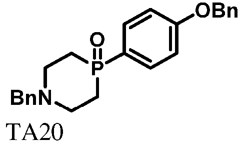 | -5 | -42 | 1.37 |
| 24 | 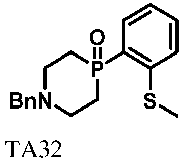 | -24 | 150 | -0.74 |
| 25 | 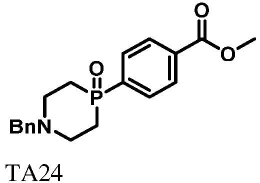 | 14 | -100 | 2.14 |
| 26 | 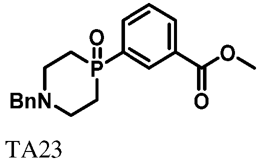 | 33 | -200 | 3.33 |
| 27 | 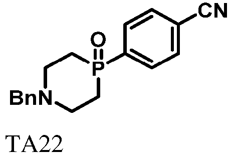 | -10 | 100 | -0.1 |
| 28 | 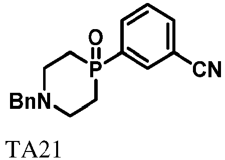 | -7 | -63 | 1.56 |
| 29 | 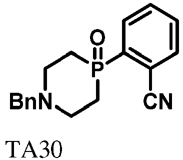 | 7 | 100 | 0.07 |
| 30 | 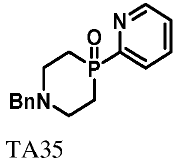 | 11 | 58.6 | 0.52 |
| 31 | 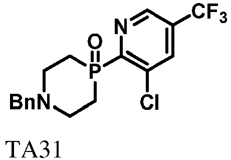 | 0 | 133 | -0.33 |
| 32 | 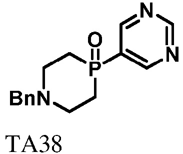 | 14.4 | 26.6 | 0.88 |
| 33 | 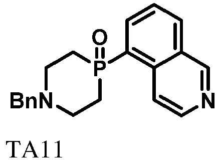 | 42 | 166 | -0.24 |
| 34 | 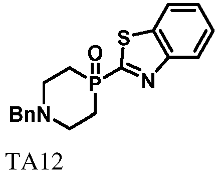 | -19 | 51 | 0.3 |
| 35 | 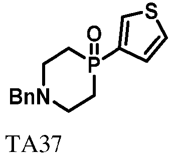 | -13 | 53 | 0.34 |
| 36 | 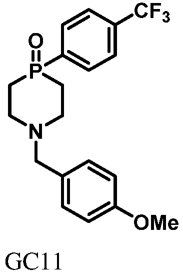 | 3 | 19.6 | 0.83 |
| 37 | 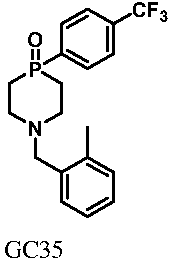 | -6 | -5.1 | 0.99 |
| 38 | 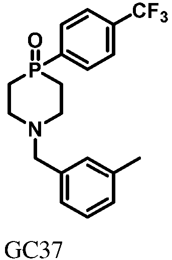 | 2 | 26.1 | 0.76 |
| 39 | 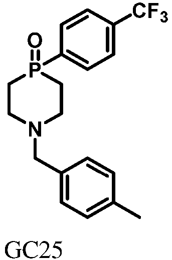 | 1 | 18.2 | 0.83 |
| 40 | 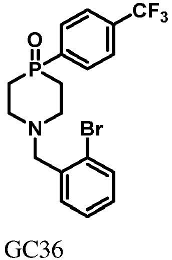 | 12 | 110.3 | 0.02 |
| 41 | 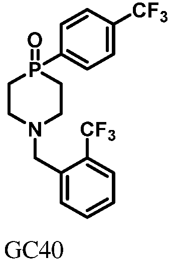 | -6.0 | 78.4 | 0.16 |
| 42 | 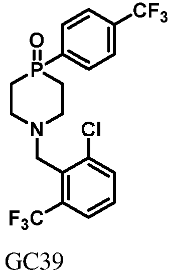 | -22.0 | 105.4 | -0.27 |
| 43 | 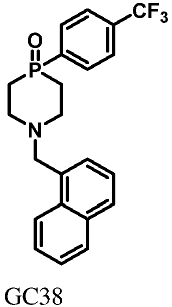 | -8.0 | 80.4 | 0.12 |
| 44 | 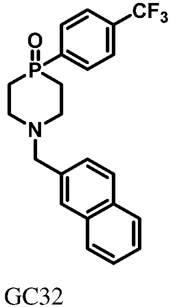 | -5.0 | 52.3 | 0.43 |
| 45 | 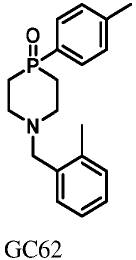 | 3.0 | 30.8 | 0.72 |
| 46 | 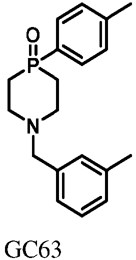 | 4.0 | 15.4 | 0.89 |
| 47 | 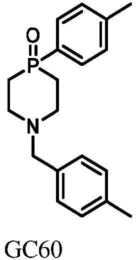 | -1.0 | -7.7 | 1.07 |
| 48 | 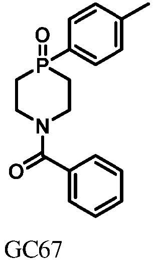 | -35.0 | 76.5 | -0.11 |
| 49 | 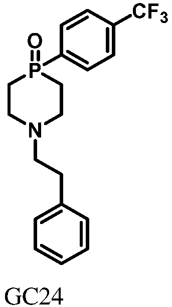 | -3.0 | 6.8 | 0.90 |
| 50 | 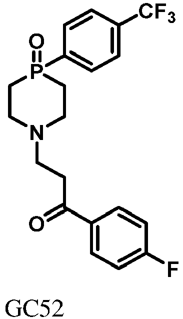 | 1.0 | 20.55 | 0.81 |
| 51 | 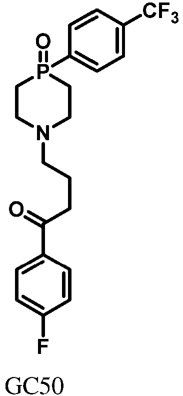 | -6.0 | 27.3 | 0.67 |
| 52 | 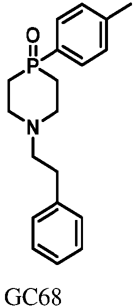 | 2.0 | 17.6 | 0.84 |
| 53 | 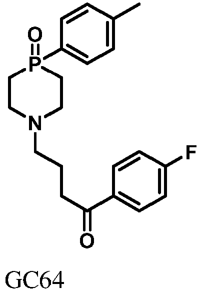 | -22.0 | 84.3 | -0.06 |
| 54 | 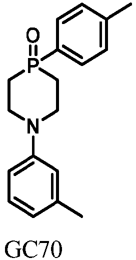 | 3.0 | 10.5 | 0.92 |
| 55 | 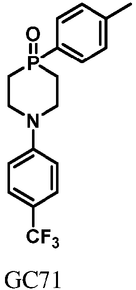 | -6.0 | 55.3 | 0.39 |
| 56 | 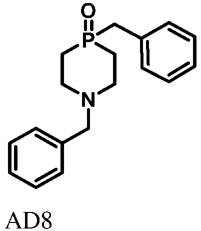 | 3 | 19.5 | 0.83 |
| 57 | 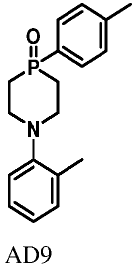 | 12 | 17.1 | 0.95 |
| 58 | 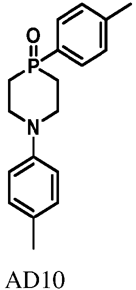 | -4 | 26.8 | 0.69 |
| 59 | 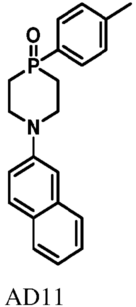 | 12 | 52.3 | 0.60 |
| 60 | 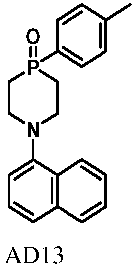 | -11 | 70.5 | 0.19 |
| 61 | 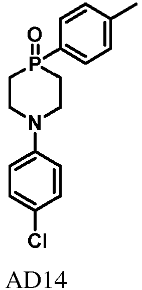 | -13 | 6.8 | 0.80 |
| 62 | 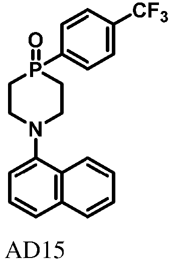 | 19 | 65.8 | 0.53 |
| 63 | 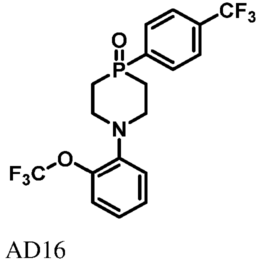 | 15 | 60.5 | 0.54 |
| 64 | 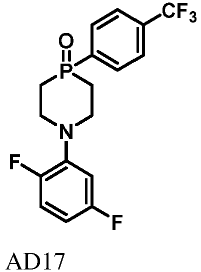 | -15 | 50.0 | 0.35 |
| 65 | 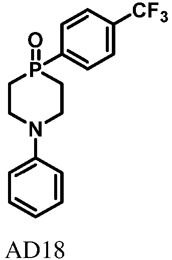 | -9 | 6.0 | 0.85 |
| 66 | 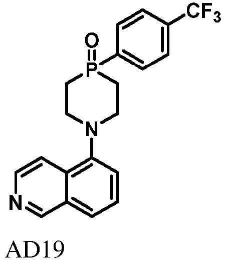 | 19 | 26.0 | 0.93 |
| 67 | 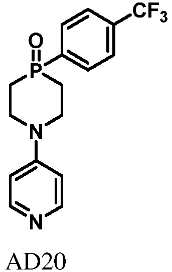 | 4 | 14.0 | 0.90 |
| 68 | 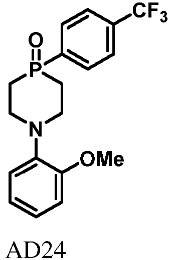 | -3 | 27.8 | 0.69 |
| 69 | 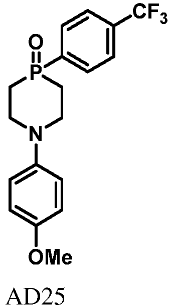 | 3 | 38.9 | 0.64 |
| 70 | 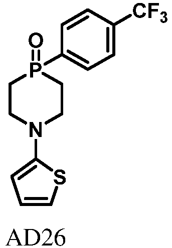 | -5 | 19.4 | 0.76 |
| 71 | 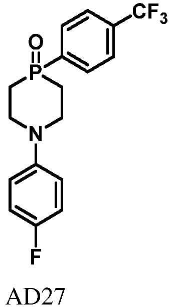 | 4 | 36.8 | 0.67 |
| 72 | 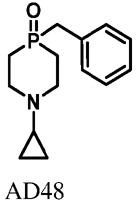 | 3 | 16.2 | 0.87 |
| 73 | 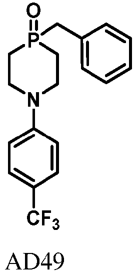 | -13 | 16.2 | 0.71 |
| 74 | 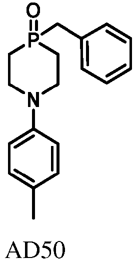 | -6 | 31.3 | 0.63 |
| 75 | 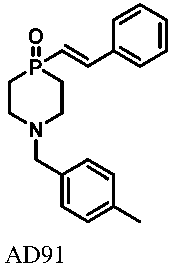 | -15 | 45.3 | 0.40 |
| 76 | 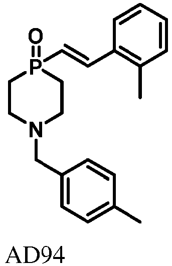 | 4 | 47.4 | 0.57 |
| 77 | 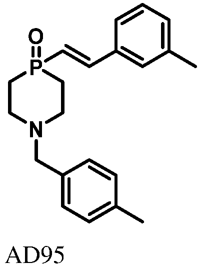 | 3 | 38.5 | 0.65 |
| 78 | 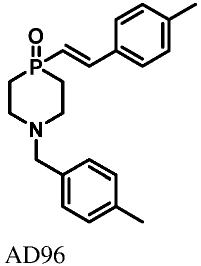 | -22 | 61.5 | 0.16 |
| 79 | 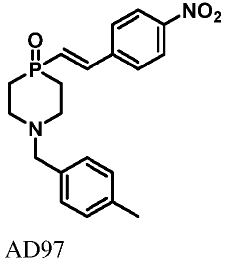 | 3 | 38.5 | 0.65 |
| 80 | 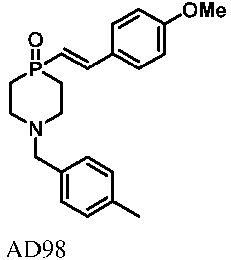 | 5 | 39.5 | 0.66 |
| 81 | 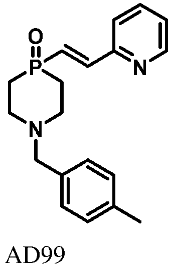 | 5 | 47.6 | 0.57 |
| 82 | 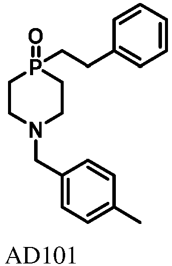 | -13 | 89.5 | 0.66 |
| 83 | 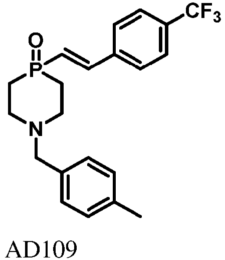 | 19 | 104.4 | 0.15 |
| 84 | 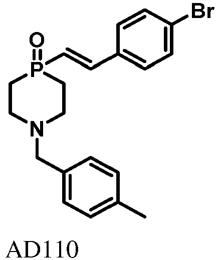 | 18 | 64.4 | 0.54 |
| 85 | 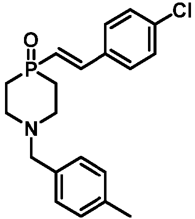 | -28 | 47.1 | 0.25 |
| | 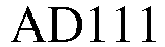 | | | |
| 86 | 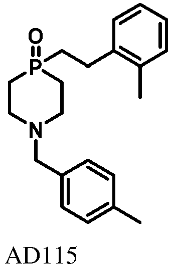 | -11 | 126.5 | -0.37 |
| 87 | 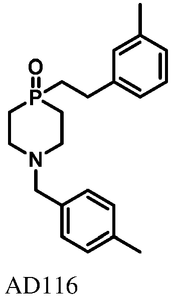 | 1 | 155.9 | -0.55 |
| 88 | 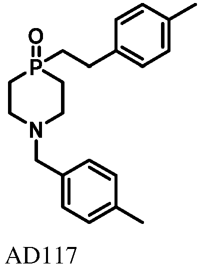 | 4 | 82.4 | 0.22 |
| 89 | 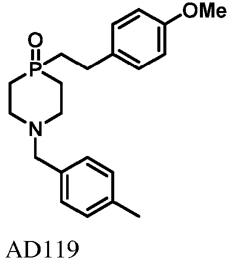 | 17 | 72.5 | 0.44 |
| 90 | 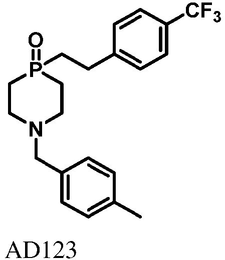 | -35 | 73.0 | -0.08 |
| 91 | 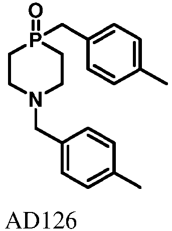 | 13 | 13.5 | 0.99 |
| 92 | 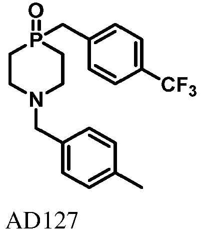 | 16 | 35.1 | 0.81 |
| 93 | 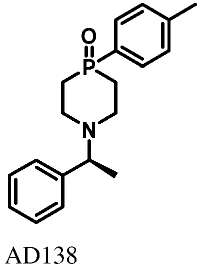 | 5.0 | 20.0 | 0.86 |
| 94 | 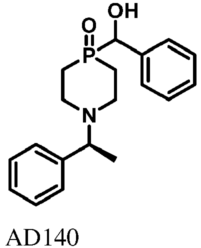 | 9.0 | 17.1 | 0.92 |
| 95 | 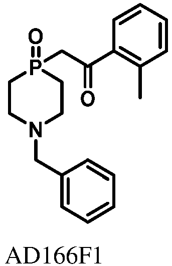 | -22 | 84.3 | -0.06 |
| 96 | 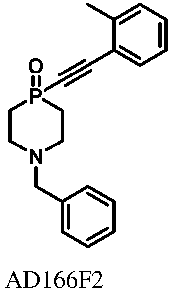 | 12 | 109.1 | 0.03 |
| 97 | 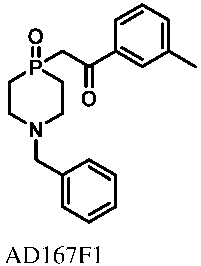 | 1 | 56.9 | 0.44 |
| 98 | 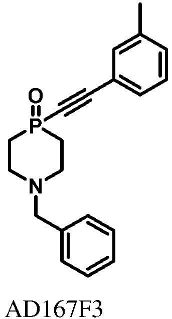 | -23 | 86.5 | -0.09 |
| 99 | 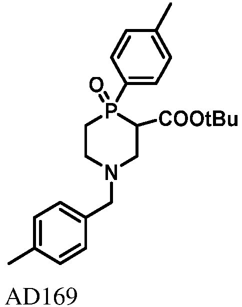 | -37 | 78.4 | -0.15 |
| 100 | 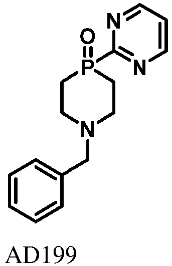 | 3 | 35.8 | 0.67 |
| 101 | 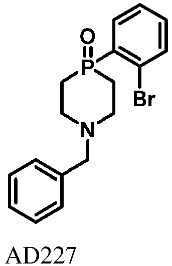 | 4.0 | 38.2 | 0.66 |
| 102 | 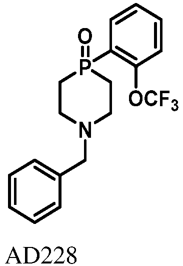 | -21.0 | 29.4 | 0.50 |
| 103 | 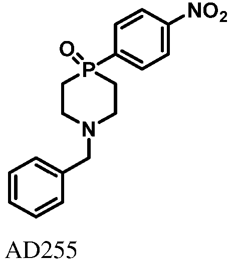 | 2.0 | 35.3 | 0.67 |
| 104 | 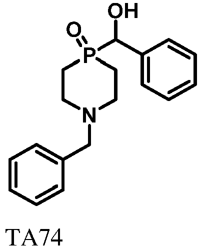 | 3 | 15.9 | 0.87 |
| 105 | 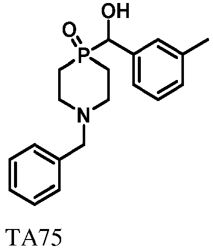 | 12 | 6.8 | 1.05 |
| 106 | 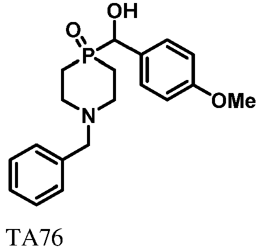 | 8 | 22.7 | 0.85 |
| 107 | 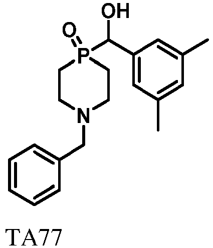 | -26 | -47.1 | 1.21 |
| 108 | 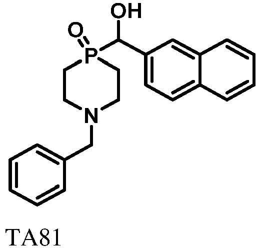 | 32 | 109.8 | 0.22 |
| 109 | 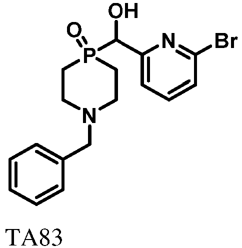 | -22 | 45.1 | 0.33 |
| 110 | 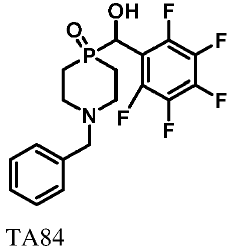 | -44 | -84.6 | 1.41 |
| 111 | 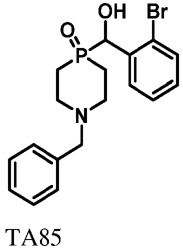 | -18 | 82.1 | 0.00 |
| 112 | 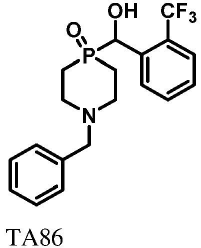 | -26 | -41.0 | 1.15 |
| 113 | 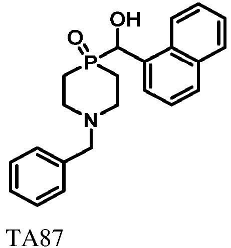 | -12 | 50.0 | 0.38 |
| 114 | 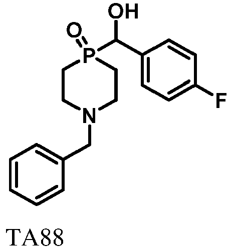 | 3 | 31.0 | 0.72 |
| 115 | 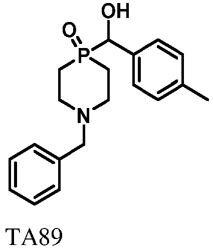 | -3 | 28.6 | 0.68 |
| 116 | 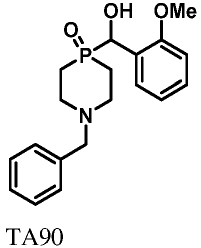 | -1 | 20.0 | 0.79 |
| 117 | 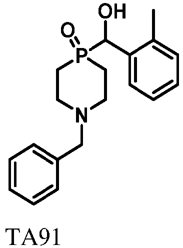 | -8 | 60.0 | 0.32 |
| 118 | 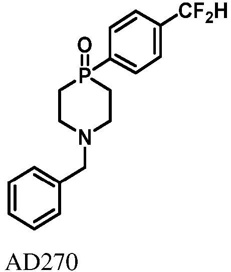 | 7 | 25.4 | 0.82 |
| 119 | 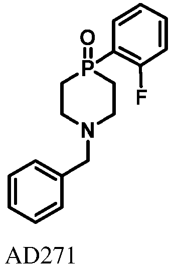 | -9 | 35.8 | 0.55 |
| 120 | 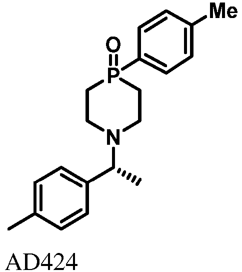 | 7.0 | 22.2 | 0.85 |
| 121 | 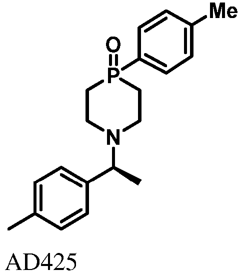 | 9.0 | 17.8 | 0.91 |

On the zebrafish screening, a lack of activity is characterized by a value of zero for *wfs1^{WT}* and a value of one hundred for *wfs1^{KO}.* All the compounds illustrated in the table show an activity regarding the locomotion of the *wfs1^{WT}* and/or the *wfs1^{KO}* zebrafish. Preferred compounds are compounds that marginally affected locomotion in *wfs1^{WT}* larvae and decreased the hyperlocomotor response in *wfs1^{KO}* larvae close to zero (with PI values close to 1).

### 2.2. Affinity and activity at S1R

The compound affinity for S1R was estimated by analyzing their inhibition of the specific [³H](+)-pentazocine binding at a single concentration of 100 nM. The dose response curve was then analyzed for the series leads. The compound activities as putative S1R agonists was analyzed using the S1R/BiP dissociation assay in S1R-overexpressing CHO cells, with expectedly agonists being able to dissociate BiP from S1R and antagonists failing to do so but preventing the PRE-084-induced dissociation.

**Table 5. S1R affinity and activity of the pharmacological or clinical S1R reference drugs in the [3H](+)-pentazocine binding assay and S1R/BiP dissociation assay in S1R-overexpressing CHO cells. a: Kᵢ < 50 nM or IC₅₀ < 400 nM; b: Kᵢ < 1 µM or IC₅₀ < 800 nM.**

| Compound | Ki (nM) | S1R/BiP IC₅₀ (nM) |
|---|---|---|
| PRE-084 | 44^{a} | 434^{b} |
| Igmesine | 39^{a} | |
| Cutamesine | 17^{a} | 363^{a} |
| Blarcamesine | 860^{b} | 520^{b} |
| Pridopidine | 81.7-240^{a} | 433^{b} |

Values that will be used a reference data to estimate the compound efficacy are summarized in Table 5 for the pharmacological or clinical reference drugs. It is concluded that reference S1R agonist show an affinity in the 17-860 nM concentration range and an activity (IC50) in the cellular assay in the 360-520 nM range.

The compounds of the invention were tested for their ability to inhibit [³H](+)-pentazocine binding. 10 compounds showed > 50% inhibition. In particular, Compound 47 (GC60) appears to be a potent S1R ligand with an estimated IC₅₀ value of 54 nM (Fig. 1).

The S1R/BiP dissociation assay showed that the compounds of the invention efficiently dissociated S1R from BiP, and thus behaved as potent agonists, with IC₅₀ values < 500 nM (Fig. 2) and a dozen of compounds with IC₅₀ values below the values determined for the reference S1R ligands. In particular Compound 47 (GC60) showed an IC₅₀ value of 199 nM, the complete concentration-response curve is shown in Fig. 3.

In particular, these data identified Compound 47 (GC60) as a potent S1R ligand and S1R agonist, superior to the pharmacological or clinical reference drugs.

### 2.3. In vivo activity as anti-amnesic drugs against Dizocilpine-induced learning impairments

Compound 47 (GC60) was administered IP in mice 10 min before Dizocilpine (0.15 mg/kg IP), administered 20 min before the Y-maze test session or the passive avoidance training session. Spatial working memory using the measure of spontaneous alternation in the Y-maze (Fig. 4A) and for long-term contextual memory using the passive avoidance test (Fig. 4B). The GC60 treatment attenuated the Dizocilpine-induced alternation deficit at the doses of 0.3-1 mg/kg (Fig. 4A). The effect at the 1 and 3 mg/kg doses were blocked by the S1R antagonist NE-100 (1 mg/kg IP), devoid of effect by itself (Fig. 4A). In the passive avoidance test, the GC60 treatment attenuated the dizocilpine-induced deficits in step-through latency (Fig. 4B) at the doses of 0.1-3 mg/kg IP, with significant effects at the doses of 1 and 3 mg/kg. These effects were blocked by NE-100 (1 mg/kg IP), devoid of effect by itself (Fig. 4B)

### 2.4. In vivo activity as neuroprotective drugs against Aβ₂₅₋₃₅-induced toxicity

Compound 47 (GC60) was administered PO 20 min before Aβ₂₅₋₃₅ peptide, administered ICV in Swiss mice. after 7 days, mice were tested for spatial working memory using the measure of spontaneous alternation in the Y-maze and for long-term contextual memory using the passive avoidance test. The GC60 treatment attenuated the Aβ₂₅₋₃₅-induced spontaneous alternation deficit at the doses of 1-30 mg/kg (Fig. 5). The effect at the 3 and 10 mg/kg doses was blocked by the S1R antagonist NE-100, injected at 10 mg/kg PO and devoid of effect by itself (Fig. 5).

In the passive avoidance test, the GC60 treatment attenuated the Aβ₂₅₋₃₅-induced deficits in step-through latency and escape latency (Fig. 6B) at the doses of 1-30 mg/kg PO, with significant effects at the doses of 10 and/or 3 mg/kg PO. Theses effect were blocked by NE-100, injected at 10 mg/kg PO and devoid of effect by itself (Fig. 6A,B).

Immediately after the behavioral tests, mice were sacrificed, and their brain removed and dissected out for biochemical analyses. The neuroinflammatory cytokines IL-1ß, IL-6 and TNFa were analyzed by Elisa. Aβ₂₅₋₃₅ induced a significant +41% increase in IL-1ß that was dose-dependently prevented by GC60, in a NE-100 sensitive manner (Fig. 7A). Aβ₂₅₋₃₅ induced a non-significant +135% increase in IL-6 that was however dose-dependently attenuated by GC60 down to control values, in a NE-100 sensitive manner (Fig. 7B). Aβ₂₅₋₃₅ induced a highly significant +70% increase in TNFa that was dose-dependently prevented by GC60, in a NE-100 sensitive manner (Fig. 13C). Active doses of GC60 were 3, 10 and 30 mg/kg PO.

The oxidative stress was analyzed using the level of lipid peroxidation in the mouse hippocampus. Aβ₂₅₋₃₅ induced a highly significant +43% increase in lipid peroxidation that was dose-dependently prevented by GC60, in a NE-100 sensitive manner (Fig. 8). Active doses of GC60 were 3, 10 and 30 mg/kg PO.

The synaptic alteration was analyzed using the level of the scaffold protein PSD-95 also known as DLG4, in the mouse hippocampus. Aβ₂₅₋₃₅ induced a significant -43% decrease in DLG4 level that was dose-dependently prevented by GC60, in a NE-100 sensitive manner (Fig. 9). Active doses of GC60 were 10 and 30 mg/kg PO.

The apoptosis in the mouse brain tissue was analyzed using the level of the pro-apoptotic marker Bax and the anti-apoptotic marker Bcl-2, in the mouse hippocampus. Aβ₂₅₋₃₅ induced a +50% increase in Bax level that was dose-dependently prevented by GC60, in a NE-100 sensitive manner (Fig. 10A). Active doses of GC60 were 10 and 30 mg/kg PO. the treatments failed to alter Bcl-2 levels, as usually observed in the Aβ₂₅₋₃₅ model (Fig. 10B).

All these analyses showed in a very coherent manner that Compound 47 (GC60), administered acutely in the 3-30 mg/kg PO dose range, prevented the memory deficits, the increases in neuroinflammation, oxidative stress, apoptosis, and synaptic alteration induced by Aβ₂₅₋₃₅. The effects were completely blocked by NE-100, confirming the selective involvement of S1R in these effects.

Other compounds of the invention were tested and showed also neuroprotective properties in memory deficits and biochemical alterations induced by Aβ₂₅₋₃₅. For example, Compound 72 (AD48), Compound 73 (AD49) and Compound 74 (AD50) showed beneficial effect in the spatial working memory test (Fig. 11A), in contextual long term memory test (Fig. 11C) and in oxidative stress measurement (Fig. 11B).

### 2.5. In vitro activity as neuroprotective drugs against cytotoxicity induced by Aβ₂₅₋₃₅ in neuroblastoma cells

SH-SY5Y cells were pre-incubated with Compound 47 (GC60) or PRE-084 at 0.3-3 µM concentration-range 1 h prior Aβ₂₅₋₃₅, 24 h incubation in cells medium. After 24 h exposure, survival of cells was assessed by MTT test. PRE-084 showed beneficial effect by reducing the 25 µM Aβ₂₅₋₃₅ toxicity (-57% at 1 µM). This effect was fully blocked by NE-100 at 1 µM.

Compound 47 (GC60) also attenuated Aβ₂₅₋₃₅ toxicity (Fig. 13), with a better beneficial effect than PRE-084 (-73% at 1 µM). This effect was fully blocked by NE-100 (1 µM).

Other compounds of the invention, Compound 50 (GC52) and Compound 54 (GC70), were also tested and showed beneficial effects against Aβ₂₅₋₃₅ toxicity (at 25 µM) at either 0.3, 1 or 3 µM tested (Fig. 14).

All these analyses showed in a coherent manner that Compound 47 (GC60) prevented the Aβ₂₅₋₃₅ induced cell toxicity. The effects were completely blocked by NE-100, confirming the selective involvement of S1R in these effects.

### 2.6. In vivo activity as beneficial effector against behavioral and biochemical alterations in Wfs1^{E864K} mice

*Wfs1^{E864K}* mice, a mouse model of Wolfram syndrome, showed vestibular deficits (red dots; Fig. 15), starting at post-natal day 24 (PND24). Using fixed-speed Rotarod analysis, the latency to fall of the *Wfs1^{E864K}* mutant mice was significantly shortened when compared with their WT littermates (Fig. 15). Compound 47 (GC60) showed no effect on *Wfs1^{WT}* mice but alleviated behavioral deficits in *Wfs1^{E864K}* mice in a significant matter (Fig. 15).

*Wfs1^{E864K}* mice showed also moderate glycemia dysregulation in females and a strong dysregulation in males, compared to *Wfs1^{WT}* mice (Fig. 16A-C). GC60 alleviated deficits in female *Wfs1^{E864K}*mice compared to *Wfs1^{WT}* mice (Fig. 16A-C), and very significantly alleviated deficits in male *Wfs1^{E864K}*mice compared to *Wfs1^{WT}* mice (Fig. 16B-D) at 0.3 mg/kg PO dose.

These results showed that Compound 47 (GC60) significantly alleviated the Wolfram-related alterations (metabolic and motor deficits) in *Wfs1^{E864K}* compared to *Wfs1^{WT}* mice.

### 2.7. In vitro activity as protective drugs against toxicity induced by glucose in pancreatic β-cell lines MIN6-Wfs1^{WT} and MIN6-Wfs1^{KD70}.

MIN6 cells were pre-incubated with Compound 47 (GC60) at 0.03-1 µM range, 1 h prior to glucose 50 mM, added during 24 h in cell medium. After 24 h of exposure, survival of cells was measured by MTT assay. In MIN6-Wfs1^{WT} cells, glucose induced a toxicity which kills 74% of cells.

Compound 47 (GC60) very significantly and dose-dependently increase the survival of cells up to 73% of survival for 0.3 µM. NE-100 at 3 µM significantly blocked the GC60 effect (Fig. 17A). In MIN6-Wfs1^{KD70} cells, glucose induced a toxicity which kills 77% of cells. GC60 very significantly and concentration -dependently fully rescued the survival of cells up to 89% of survival for 0.3 µM. NE-100 at 3 µM fully blocked the GC60 effect (Fig. 17B).

This analysis showed that Compound 47 (GC60) treatment in the 0.03-1µM dose range, increase the survival against the glucose-induced toxicity in MIN6-Wfs1^{WT} and MIN6-Wfs1^{KD70} cells.

Other compounds of the invention were also tested similarly. Compound 36 (GC11) (Fig. 18-B), Compound 39 (GC25) (Fig. 18C-D) and Compound 74 (AD50) (Fig. 18E-F) also showed a protective profile in glucose-induced toxicity in MIN6-Wfs1^{WT} and MIN6-Wfs1^{KD70} cells.

### 2.8. In vivo activity as protective drugs against behavioral deficits induced by marf mutation in drosophila CMT-2A model.

Drosophila expressing wild-type or mutant marf under the control of the pan-neuronal promoter Elav were used as a fly model of CMT-2A. They were analyzed behaviorally using a negative geotaxis test 3 times at day 1, 3, 5, and day 7 of adult age. Marf mutation induced strong deficit in locomotor activity in drosophila. Genetic supplementation with S1R gene alleviated deficits (Fig. 19).

At Day 7, Drosophila received S1R agonist PRE-084 and were submitted to negative geotaxis at Day 9. PRE-084 strongly and very significantly alleviated deficits in Drosophila which were supplemented to S1R gene, but remained without beneficial effect in non-supplemented Drosophila. These observations indicated that PRE-084 effect was directly linked to its S1R agonist activity.

In another set of experiment, Drosophila expressing marf^{WT}, marf^{R94Q} and marf^{T105M+/-} S1R gene were tested for negative geotaxis test at day 4 of adult age, after 24h of treatment with Compound 47 (GC60). Marf mutation induced strong deficit in locomotor activity in drosophila. Genetic supplementation with S1R gene alleviated deficits and GC60 strongly increase the number of drosophila which reached to top of the apparatus in negative geotaxia (Fig. 21).

## Claims

1. A compound of Formula I: 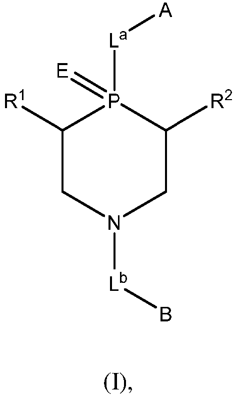 or a pharmaceutically acceptable salt thereof,
wherein
A is selected from the group consisting of phenyl, naphthyl, 5 to 10-membered mono-cyclic or bi-cyclic heteroaryl, 3 to 6-membered cycloalkyl and 3 to 6-membered cycloalkenyl, said phenyl, naphthyl, 5 to 10-membered mono-cyclic or bi-cyclic heteroaryl, 3 to 5-membered cycloalkyl and 3 to 6-membered cycloalkenyl being optionally substituted with 1 to 5 substituents **R^{a}**;
wherein each **R^{a}** is independently selected from the group consisting of -H, -F, -Cl, - Br, -CN, -NH₂, -NO₂, -CH₂F, -CHF₂, -CF₃, -OCH₃, -(CO)OH, -(CO)OCH₃, -SCH₃, -OCH₂F, -OCHF₂, -OCF₃, -O(CH₂)Ph, and C₁₋₄ alkyl;
**L^{a}** is a linker selected from the group consisting of a bond, C₁₋₅ alkanediyl, C₂₋₅ alkenediyl and C₂₋₅ alkynediyl, each optionally substituted with one, two or three substituents independently selected from the group consisting of -OH and =O;
**R¹** and **R²** are independently selected from -H and -C(O)O-C₁₋₄ alkyl;
**B** is selected from the group consisting of phenyl, naphthyl, 5 to 10-membered mono-cyclic or bi-cyclic heteroaryl, 3 to 6-membered cycloalkyl and 3 to 6-membered cycloalkenyl, said phenyl, naphthyl, 5 to 10-membered mono-cyclic or bi-cyclic heteroaryl, 3 to 6-membered cycloalkyl 3 to 6-membered cycloalkyl and 3 to 6-membered cycloalkenyl being optionally substituted with 1 to 5 substituents **R^{b}**;
wherein each **R^{b}** is independently selected from the group consisting of -H, -F, -Cl, -Br, -CN, -NH₂, -NO₂, -CH₂F, -CHF₂, -CF₃, -OCH₃, -(CO)OH, -(CO)OCH₃, -SCH₃, -OCH₂F, -OCHF₂, -OCF₃, -O(CH₂)Ph, and C₁₋₄ alkyl;
**L^{b}** is a linker selected from the group consisting of a bond, C₁₋₅ alkanediyl, C₂₋₅ alkenediyl and C₂₋₅ alkynediyl, each optionally substituted with one, two or three substituents independently selected from the group consisting of -OH and =O;
**E** is selected from the group consisting of O, S and Se;
*with the proviso* that when **E** is O and:
(a) when both of **L^{a}** and **L^{b}** is a bond and **A** is either unsubstituted phenyl or 4-fluorophenyl, then **B** is not 2,6-difluoro-4-nitrophenyl or 2,6-difluoro-4-aminophenyl;
(b) when **L^{a}** is a bond, **L^{b}** is -(CH₂)- and **B** is unsubstituted phenyl, then **A** is not unsubstituted phenyl, 4-bromophenyl, 4-fluorophenyl, 4-(trifluoromethyl)phenyl, 4-nitrophenyl or unsubstituted cyclopropanyl;
(c) when **L^{a}** is a bond, **L^{b}** is -(CH₂)- and **A** is 4-fluorophenyl, then **B** is not 4-methoxyphenyl;
(d) when **L^{a}** is -CH₂-, **L^{b}** is -(CH₂)- and **B** is unsubstituted phenyl, then **A** is not 4-fluorophenyl or unsubstituted cyclopropanyl;
(e) when **L^{a}** is a bond, **L^{b}** is -(CH₂)- and **B** is 2,4-di-methoxyphenyl, then **A** is not 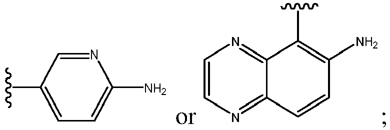
(f) when **L^{a}** is a bond and **A** is unsubstituted phenyl, then **L^{b}-B** is not -(CH₂)₃-(1-isoquinoline), -(CH₂)₂-(4-carboxyphenyl), -(CH₂)-(4-methylbenzoate) or 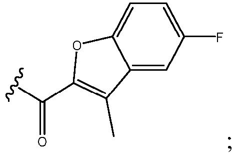 and
(g) when **L^{a}** is a bond and **A** is 3-methoxy-4-aminophenyl or 3-methoxy-4-nitrophenyl, then **L^{b}-B** is not unsubstituted phenyl, unsubstituted cyclopropanyl or -(CO)-cyclopropanyl.

2. The compound according to claim 1, wherein **E** is O.

3. The compound according to claim 1 or 2, wherein **R¹** and **R²** are -H.

4. The compound according to any one of claims 1 to 3, wherein **L^{a}** is a linker selected from the group consisting of a bond, -CH₂-, -(CH₂)₂-, -CH=CH-, -C≡C-, -CH(OH)-, -CH₂-C(O)-, -(CH₂)₂-C(O)- and -(CH₂)₃-C(O)-.

5. The compound according to any one of claims 1 to 4, wherein **L^{b}** is a linker selected from the group consisting of a bond, -CH₂-, -(CH₂)₂-, -CH=CH-, -C≡C-, -CH(OH)-, -CH(CH₃)-, -CH₂-C(O)-, -(CH₂)₂-C(O)-, -(CH₂)₃-C(O)- and -C(O)-.

6. The compound according to claim 1 selected from the group consisting of:
1-benzyl-4-(3-(trifluoromethyl)phenyl)-1,4-azaphosphinane 4-oxide;
1-benzyl-4-(2-(trifluoromethyl)phenyl)-1,4-azaphosphinane 4-oxide;
1-benzyl-4-(4-(trifluoromethoxy)phenyl)-1,4-azaphosphinane 4-oxide;
1-benzyl-4-(3-fluorophenyl)-1,4-azaphosphinane 4-oxide;
1-benzyl-4-(3,5-difluorophenyl)-1,4-azaphosphinane 4-oxide;
1-benzyl-4-(4-chlorophenyl)-1,4-azaphosphinane 4-oxide;
1-benzyl-4-(3-chlorophenyl)-1,4-azaphosphinane 4-oxide;
1-benzyl-4-(2-chlorophenyl)-1,4-azaphosphinane 4-oxide;
1-benzyl-4-(3,4-dichlorophenyl)-1,4-azaphosphinane 4-oxide;
1-benzyl-4-(2,3-dichlorophenyl)-1,4-azaphosphinane 4-oxide;
1-benzyl-4-(*p*-tolyl)-1,4-azaphosphinane 4-oxide;
1-benzyl-4-(*m*-tolyl)-1,4-azaphosphinane 4-oxide;
1-benzyl-4-(*o*-tolyl)-1,4-azaphosphinane 4-oxide;
1-benzyl-4-(4-(*tert*-butyl)phenyl)-1,4-azaphosphinane 4-oxide;
1-benzyl-4-(naphthalen-1-yl)-1,4-azaphosphinane 4-oxide;
1-benzyl-4-(naphthalen-2-yl)-1,4-azaphosphinane 4-oxide;
1-benzyl-4-(4-methoxyphenyl)-1,4-azaphosphinane 4-oxide;
1-benzyl-4-(3-methoxyphenyl)-1,4-azaphosphinane 4-oxide;
1-benzyl-4-(2-methoxyphenyl)-1,4-azaphosphinane 4-oxide;
1-benzyl-4-(4-(benzyloxy)phenyl)-1,4-azaphosphinane 4-oxide;
1-benzyl-4-(2-(methylthio)phenyl)-1,4-azaphosphinane 4-oxide;
methyl 4-(1-benzyl-4-oxido-1,4-azaphosphinan-4-yl)benzoate;
methyl 3-(1-benzyl-4-oxido-1,4-azaphosphinan-4-yl)benzoate;
4-(1-benzyl-4-oxido-1,4-azaphosphinan-4-yl)benzonitrile;
3-(1-benzyl-4-oxido-1,4-azaphosphinan-4-yl)benzonitrile;
2-(1-benzyl-4-oxido-1,4-azaphosphinan-4-yl)benzonitrile;
1-benzyl-4-(pyridin-2-yl)-1,4-azaphosphinane 4-oxide;
1-benzyl-4-(3-chloro-5-(trifluoromethyl)pyridin-2-yl)-1,4-azaphosphinane 4-oxide;
1-benzyl-4-(pyrimidin-5-yl)-1,4-azaphosphinane 4-oxide;
1-benzyl-4-(isoquinolin-5-yl)-1,4-azaphosphinane 4-oxide;
4-(benzo[d]thiazol-2-yl)-1-benzyl-1,4-azaphosphinane 4-oxide;
1-benzyl-4-(thiophen-3-yl)-1,4-azaphosphinane 4-oxide;
1-(4-methoxybenzyl)-4-(4-(trifluoromethyl)phenyl)-1,4-azaphosphinane 4-oxide;
1-(2-methylbenzyl)-4-(4-(trifluoromethyl)phenyl)-1,4-azaphosphinane 4-oxide;
1-(3-methylbenzyl)-4-(4-(trifluoromethyl)phenyl)-1,4-azaphosphinane 4-oxide;
1-(4-methylbenzyl)-4-(4-(trifluoromethyl)phenyl)-1,4-azaphosphinane 4-oxide;
1-(2-bromobenzyl)-4-(4-(trifluoromethyl)phenyl)-1,4-azaphosphinane 4-oxide;
1-(2-(trifluoromethyl)benzyl)-4-(4-(trifluoromethyl)phenyl)-1,4-azaphosphinane 4-oxide;
1-(2-chloro-6-(trifluoromethyl)benzyl)-4-(4-(trifluoromethyl)phenyl)-1,4-azaphosphinane 4-oxide;
1-(naphthalen-1-ylmethyl)-4-(4-(trifluoromethyl)phenyl)-1,4-azaphosphinane 4-oxide;
1-(naphthalen-2-ylmethyl)-4-(4-(trifluoromethyl)phenyl)-1,4-azaphosphinane 4-oxide;
1-(2-methylbenzyl)-4-(*p*-tolyl)-1,4-azaphosphinane 4-oxide;
1-(3-methylbenzyl)-4-(*p*-tolyl)-1,4-azaphosphinane 4-oxide;
1-(4-methylbenzyl)-4-(*p*-tolyl)-1,4-azaphosphinane 4-oxide;
(4-oxido-4-(*p*-tolyl)-1,4-azaphosphinan-1-yl)(phenyl)methanone;
1-phenethyl-4-(4-(trifluoromethyl)phenyl)-1,4-azaphosphinane 4-oxide;
1-(4-fluorophenyl)-3-(4-oxido-4-(4-(trifluoromethyl)phenyl)-1,4-azaphosphinan-1-yl)propan-1-one;
1-(4-fluorophenyl)-4-(4-oxido-4-(4-(trifluoromethyl)phenyl)-1,4-azaphosphinan-1-yl)butan-1-one;
1-phenethyl-4-(*p*-tolyl)-1,4-azaphosphinane 4-oxide;
1-(4-fluorophenyl)-4-(4-oxido-4-(*p*-tolyl)-1,4-azaphosphinan-1-yl)butan-1-one;
1-(m-tolyl)-4-(*p*-tolyl)-1,4-azaphosphinane 4-oxide;
4-(*p*-tolyl)-1-(4-(trifluoromethyl)phenyl)-1,4-azaphosphinane 4-oxide;
1,4-dibenzyl-1,4-azaphosphinane 4-oxide;
1-(o-tolyl)-4-(*p*-tolyl)-1,4-azaphosphinane 4-oxide;
1,4-di-p-tolyl-1,4-azaphosphinane 4-oxide;
1-(naphthalen-2-yl)-4-(*p*-tolyl)-1,4-azaphosphinane 4-oxide;
1-(naphthalen-1-yl)-4-(*p*-tolyl)-1,4-azaphosphinane 4-oxide;
1-(4-chlorophenyl)-4-(*p*-tolyl)-1,4-azaphosphinane 4-oxide;
1-(naphthalen-1-yl)-4-(4-(trifluoromethyl)phenyl)-1,4-azaphosphinane 4-oxide;
1-(2-(trifluoromethoxy)phenyl)-4-(4-(trifluoromethyl)phenyl)-1,4-azaphosphinane 4-oxide;
1-(2,5-difluorophenyl)-4-(4-(trifluoromethyl)phenyl)-1,4-azaphosphinane 4-oxide;
1-phenyl-4-(4-(trifluoromethyl)phenyl)-1,4-azaphosphinane 4-oxide;
1-(isoquinolin-5-yl)-4-(4-(trifluoromethyl)phenyl)-1,4-azaphosphinane 4-oxide;
1-(pyridin-4-yl)-4-(4-(trifluoromethyl)phenyl)-1,4-azaphosphinane 4-oxide;
1-(2-methoxyphenyl)-4-(4-(trifluoromethyl)phenyl)-1,4-azaphosphinane 4-oxide;
1-(4-methoxyphenyl)-4-(4-(trifluoromethyl)phenyl)-1,4-azaphosphinane 4-oxide;
1-(thiophen-2-yl)-4-(4-(trifluoromethyl)phenyl)-1,4-azaphosphinane 4-oxide;
1-(4-fluorophenyl)-4-(4-(trifluoromethyl)phenyl)-1,4-azaphosphinane 4-oxide;
4-benzyl-1-(4-(trifluoromethyl)phenyl)-1,4-azaphosphinane 4-oxide;
4-benzyl-1-(*p*-tolyl)-1,4-azaphosphinane 4-oxide;
(E)-1-(4-methylbenzyl)-4-styryl-1,4-azaphosphinane 4-oxide;
(E)-1-(4-methylbenzyl)-4-(2-methylstyryl)-1,4-azaphosphinane 4-oxide;
(E)-1-(4-methylbenzyl)-4-(3-methylstyryl)-1,4-azaphosphinane 4-oxide;
(E)-1-(4-methylbenzyl)-4-(4-methylstyryl)-1,4-azaphosphinane 4-oxide;
(E)-1-(4-methylbenzyl)-4-(4-nitrostyryl)-1,4-azaphosphinane 4-oxide;
(E)-4-(4-methoxystyryl)-1-(4-methylbenzyl)-1,4-azaphosphinane 4-oxide;
(E)-1-(4-methylbenzyl)-4-(2-(pyridin-2-yl)vinyl)-1,4-azaphosphinane 4-oxide;
1-(4-methylbenzyl)-4-phenethyl-1,4-azaphosphinane 4-oxide;
(E)-1-(4-methylbenzyl)-4-(4-(trifluoromethyl)styryl)-1,4-azaphosphinane 4-oxide;
(E)-4-(4-bromostyryl)-1-(4-methylbenzyl)-1,4-azaphosphinane 4-oxide;
(E)-4-(4-chlorostyryl)-1-(4-methylbenzyl)-1,4-azaphosphinane 4-oxide;
1-(4-methylbenzyl)-4-(2-methylphenethyl)-1,4-azaphosphinane 4-oxide;
1-(4-methylbenzyl)-4-(3-methylphenethyl)-1,4-azaphosphinane 4-oxide;
1-(4-methylbenzyl)-4-(4-methylphenethyl)-1,4-azaphosphinane 4-oxide;
4-(4-methoxyphenethyl)-1-(4-methylbenzyl)-1,4-azaphosphinane 4-oxide;
1-(4-methylbenzyl)-4-(4-(trifluoromethyl)phenethyl)-1,4-azaphosphinane 4-oxide;
1,4-bis(4-methylbenzyl)-1,4-azaphosphinane 4-oxide;
1-(4-methylbenzyl)-4-(4-(trifluoromethyl)benzyl)-1,4-azaphosphinane 4-oxide;
(S)-1-(1-phenylethyl)-4-(p-tolyl)-1,4-azaphosphinane 4-oxide;
4-(hydroxy(phenyl)methyl)-1-((S)-1-phenylethyl)-1,4-azaphosphinane 4-oxide;
2-(1-benzyl-4-oxido-1,4-azaphosphinan-4-yl)-1-(o-tolyl)ethan-1-one;
1-benzyl-4-(o-tolylethynyl)-1,4-azaphosphinane 4-oxide;
2-(1-benzyl-4-oxido-1,4-azaphosphinan-4-yl)-1-(m-tolyl)ethan-1-one;
1-benzyl-4-(o-tolylethynyl)-1,4-azaphosphinane 4-oxide;
tert-butyl 1-(4-methylbenzyl)-4-(p-tolyl)-1,4-azaphosphinane-3-carboxylate 4-oxide;
1-benzyl-4-(pyrimidin-2-yl)-1,4-azaphosphinane 4-oxide;
1-benzyl-4-(2-bromophenyl)-1,4-azaphosphinane 4-oxide;
1-benzyl-4-(2-(trifluoromethoxy)phenyl)-1,4-azaphosphinane 4-oxide;
1-benzyl-4-(hydroxy(phenyl)methyl)-1,4-azaphosphinane 4-oxide;
1-benzyl-4-(hydroxy(m-tolyl)methyl)-1,4-azaphosphinane 4-oxide;
1-benzyl-4-(hydroxy(4-methoxyphenyl)methyl)-1,4-azaphosphinane 4-oxide;
1-benzyl-4-((3,5-dimethylphenyl)(hydroxy)methyl)-1,4-azaphosphinane 4-oxide;
1-benzyl-4-(hydroxy(naphthalen-2-yl)methyl)-1,4-azaphosphinane 4-oxide;
1-benzyl-4-((6-bromopyridin-2-yl)(hydroxy)methyl)-1,4-azaphosphinane 4-oxide;
1-benzyl-4-(hydroxy(perfluorophenyl)methyl)-1,4-azaphosphinane 4-oxide;
1-benzyl-4-((2-bromophenyl)(hydroxy)methyl)-1,4-azaphosphinane 4-oxide;
1-benzyl-4-(hydroxy(2-(trifluoromethyl)phenyl)methyl)-1,4-azaphosphinane 4-oxide;
1-benzyl-4-(hydroxy(naphthalen-1-yl)methyl)-1,4-azaphosphinane 4-oxide;
1-benzyl-4-((4-fluorophenyl)(hydroxy)methyl)-1,4-azaphosphinane 4-oxide;
1-benzyl-4-(hydroxy(p-tolyl)methyl)-1,4-azaphosphinane 4-oxide;
1-benzyl-4-(hydroxy(2-methoxyphenyl)methyl)-1,4-azaphosphinane 4-oxide;
1-benzyl-4-(hydroxy(o-tolyl)methyl)-1,4-azaphosphinane 4-oxide;
1-benzyl-4-(4-(difluoromethyl)phenyl)-1,4-azaphosphinane 4-oxide;
1-benzyl-4-(2-fluorophenyl)-1,4-azaphosphinane 4-oxide;
(R)-4-(p-tolyl)-1-(1-(p-tolyl)ethyl)-1,4-azaphosphinane 4-oxide; and
(S)-4-(p-tolyl)-1-(1-(p-tolyl)ethyl)-1,4-azaphosphinane 4-oxide.

7. A pharmaceutical composition comprising a compound of Formula I: 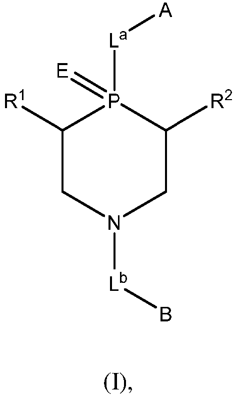 or a pharmaceutically acceptable salt thereof,
wherein
**A** is selected from the group consisting of phenyl, naphthyl, 5 to 10-membered mono-cyclic or bi-cyclic heteroaryl, 3 to 6-membered cycloalkyl and 3 to 6-membered cycloalkenyl, said phenyl, naphthyl, 5 to 10-membered mono-cyclic or bi-cyclic heteroaryl, 3 to 5-membered cycloalkyl and 3 to 6-membered cycloalkenyl being optionally substituted with 1 to 5 substituents **R^{a}**;
wherein each **R^{a}** is independently selected from the group consisting of -H, -F, -Cl, - Br, -CN, -NH₂, -NO₂, -CH₂F, -CHF₂, -CF₃, -OCH₃, -(CO)OH, -(CO)OCH₃, -SCH₃, -OCH₂F, -OCHF₂, -OCF₃, -O(CH₂)Ph, and C₁₋₄ alkyl;
**L^{a}** is a linker selected from the group consisting of a bond, C₁₋₅ alkanediyl, C₂₋₅ alkenediyl and C₂₋₅ alkynediyl, each optionally substituted with one, two or three substituents independently selected from the group consisting of -OH and =O;
**R¹** and **R²** are independently selected from -H and -C(O)O-C₁₋₄ alkyl;
**B** is selected from the group consisting of phenyl, naphthyl, 5 to 10-membered mono-cyclic or bi-cyclic heteroaryl, 3 to 6-membered cycloalkyl and 3 to 6-membered cycloalkenyl, said phenyl, naphthyl, 5 to 10-membered mono-cyclic or bi-cyclic heteroaryl, 3 to 6-membered cycloalkyl and 3 to 6-membered cycloalkenyl being optionally substituted with 1 to 5 substituents **R^{b}**;
wherein each **R^{b}** is independently selected from the group consisting of -H, -F, -Cl, -Br, -CN, -NH₂, -NO₂, -CH₂F, -CHF₂, -CF₃, -OCH₃, -(CO)OH, -(CO)OCH₃, -SCH₃, -OCH₂F, -OCHF₂, -OCF₃, -O(CH₂)Ph, and C₁₋₄ alkyl;
**L^{b}** is a linker selected from the group consisting of a bond, C₁₋₅ alkanediyl, C₂₋₅ alkenediyl and C₂₋₅ alkynediyl, each optionally substituted with one, two or three substituents independently selected from the group consisting of -OH and =O;
**E** is selected from the group consisting of O, S and Se;
and at least one pharmaceutically acceptable carrier, diluent, excipient and/or adjuvant.

8. A compound of Formula I: 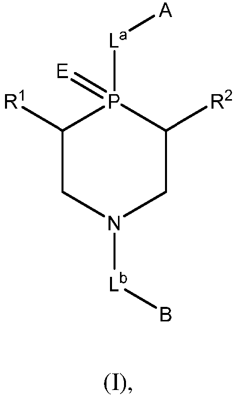 or a pharmaceutically acceptable salt thereof,
wherein
**A** is selected from the group consisting of phenyl, naphthyl, 5 to 10-membered mono-cyclic or bi-cyclic heteroaryl, 3 to 6-membered cycloalkyl and 3 to 6-membered cycloalkenyl, said phenyl, naphthyl, 5 to 10-membered mono-cyclic or bi-cyclic heteroaryl, 3 to 5-membered cycloalkyl and 3 to 6-membered cycloalkenyl being optionally substituted with 1 to 5 substituents **R^{a}**;
wherein each **R^{a}** is independently selected from the group consisting of -H, -F, -Cl, - Br, -CN, -NH₂, -NO₂, -CH₂F, -CHF₂, -CF₃, -OCH₃, -(CO)OH, -(CO)OCH₃, -SCH₃, -OCH₂F, -OCHF₂, -OCF₃, -O(CH₂)Ph, and C₁₋₄ alkyl;
**L^{a}** is a linker selected from the group consisting of a bond, C₁₋₅ alkanediyl, C₂₋₅ alkenediyl and C₂₋₅ alkynediyl, each optionally substituted with one, two or three substituents independently selected from the group consisting of -OH and =O;
**R¹** and **R²** are independently selected from -H and -C(O)O-C₁₋₄ alkyl;
**B** is selected from the group consisting of phenyl, naphthyl, 5 to 10-membered mono-cyclic or bi-cyclic heteroaryl, 3 to 6-membered cycloalkyl and 3 to 6-membered cycloalkenyl, said phenyl, naphthyl, 5 to 10-membered mono-cyclic or bi-cyclic heteroaryl, 3 to 6-membered cycloalkyl and 3 to 6-membered cycloalkenyl being optionally substituted with 1 to 5 substituents **R^{b}**;
wherein each **R^{b}** is independently selected from the group consisting of -H, -F, -Cl, -Br, -CN, -NH₂, -NO₂, -CH₂F, -CHF₂, -CF₃, -OCH₃, -(CO)OH, -(CO)OCH₃, -SCH₃, -OCH₂F, -OCHF₂, -OCF₃, -O(CH₂)Ph, and C₁₋₄ alkyl;
**L^{b}** is a linker selected from the group consisting of a bond, C₁₋₅ alkanediyl, C₂₋₅ alkenediyl and C₂₋₅ alkynediyl, each optionally substituted with one, two or three substituents independently selected from the group consisting of -OH and =O;
**E** is selected from the group consisting of O, S and Se;
for use in treating and/or preventing a disease, disorder or condition that is:
(a) associated with, caused by or a symptom of a mutation in WFS1 or WFS2 (CISD2) gene;
(b) a neurodegenerative disease, disorder or condition;
(c) selected from the group consisting of stroke, stroke-induced neuronal damage, depression, anxiety, addiction, epilepsy, multiple sclerosis, pain, neuropathic pain and cancer;
(d) associated with mitochondrial dysfunction, endoplasmic reticulum (ER) stress, or disfunction of nucleocytoplasmic transport; and/or
(e) treatable, ameliorable or preventable by activation or inhibition of sigma-1 receptor.

9. The compound for use according to claim 8, wherein the disease, disorder or condition is associated with, caused by or a symptom of a mutation in WFS1 or WFS2 (CISD2) gene.

10. The compound for use according to claim 9, wherein the disease, disorder or condition associated, caused by or a symptom of with a mutation in WFS1 or WFS2 (CISD2) gene is Wolfram syndrome or Wolfram-like syndrome.

11. The compound for use according to claim 8 or 9, wherein the disease, disorder or condition is associated with, caused by or a symptom of: (a) a mutation in WFS1 or WFS2 (CISD2) gene, and/or (b) Wolfram syndrome or Wolfram-like syndrome.

12. The compound for use according to claim 11, wherein the disease, disorder or condition associated with, caused by or a symptom of a mutation in WFS1 or WFS2 (CISD2) gene is selected from the group consisting of diabetes insipidus (DI), diabetes mellitus (DM), an optic atrophy (OA), deafness, urinary disorders, pons atrophy, ataxia, and psychiatric disorders, including bipolar disorder, schizophrenia, suicide and mania.

13. The compound for use according to claim 11 or 12, wherein the disease, disorder or condition is exhibited in an individual with Wolfram syndrome or Wolfram-like syndrome.

14. The compound for use according to claim 8, wherein the neurodegenerative disease, disorder or condition is selected from the group consisting of Alzheimer's disease (AD), frontotemporal dementia (FTD), Lewy body dementia (LBD), Parkinson's disease (PD), Huntington disease (HD), amyotrophic lateral sclerosis (ALS), multiple sclerosis (MS) and Charcot-Marie-Tooth 2A disease (CMT2A).

15. The compound for use according to claim 8, wherein the disease, disorder or condition associated with a mitochondrial dysfunction, endoplasmic reticulum (ER) stress, or disfunction of nucleocytoplasmic transport is selected from the group consisting of a mood disorder, a mitochondrial myopathy, a lysosomal storage disease, frontotemporal dementia (FTD), Charcot-Marie-Tooth 2A disease (CMT2A), stroke, hypoxia, ischemia, neonatal anoxia, spinal cord injuries, multiple sclerosis, glaucoma, diabetes mellitus, cardiomyopathy, ototoxicity and acoustic trauma.

16. The compound for use according to any one of claims 8 to 15, wherein the compound is a ligand of sigma-1 receptor.

17. The compound for use according to any one of claims 8 to 16, wherein the compound is an agonist of sigma-1 receptor.

18. The compound for use according to claim 8, wherein the disease, disorder or condition is selected from the group consisting of Wolfram syndrome, diabetes insipidus (DI), diabetes mellitus (DM), an optic atrophy (OA), deafness, urinary disorders, pons atrophy, ataxia, psychiatric disorders, bipolar disorder, schizophrenia, suicide, mania, Alzheimer's disease (AD), frontotemporal dementia (FTD), Lewy body dementia (LBD), Parkinson's disease (PD), Huntington disease (HD), amyotrophic lateral sclerosis (ALS), stroke, stroke-induced neuronal damage, depression, anxiety, addiction, epilepsy, multiple sclerosis (MS), pain, neuropathic pain, cancer, a mood disorder, a mitochondrial myopathy, a lysosomal storage disease, Charcot-Marie-Tooth 2A disease (CMT2A), hypoxia, ischemia, neonatal anoxia, spinal cord injuries, glaucoma, cardiomyopathy, ototoxicity and acoustic trauma, Rett Syndrome and Dravet Syndrome.

19. The compound for use according to any one of claims 8 to 18, wherein E is O.

20. The compound for use according to any one of claims 8 to 19, wherein **R¹** and **R²** are H.

21. The compound for use according to any one of claims 8 to 20, wherein the compound is selected from the group consisting of:
1-benzyl-4-(4-(trifluoromethyl)phenyl)-1,4-azaphosphinane 4-oxide;
1-benzyl-4-(4-fluorophenyl)-1,4-azaphosphinane 4-oxide;
1-benzyl-4-(3-(trifluoromethyl)phenyl)-1,4-azaphosphinane 4-oxide;
1-benzyl-4-(2-(trifluoromethyl)phenyl)-1,4-azaphosphinane 4-oxide;
1-benzyl-4-(4-(trifluoromethoxy)phenyl)-1,4-azaphosphinane 4-oxide;
1-benzyl-4-(3-fluorophenyl)-1,4-azaphosphinane 4-oxide;
1-benzyl-4-(3,5-difluorophenyl)-1,4-azaphosphinane 4-oxide;
1-benzyl-4-(4-chlorophenyl)-1,4-azaphosphinane 4-oxide;
1-benzyl-4-(3-chlorophenyl)-1,4-azaphosphinane 4-oxide;
1-benzyl-4-(2-chlorophenyl)-1,4-azaphosphinane 4-oxide;
1-benzyl-4-(3,4-dichlorophenyl)-1,4-azaphosphinane 4-oxide;
1-benzyl-4-(2,3-dichlorophenyl)-1,4-azaphosphinane 4-oxide;
1-benzyl-4-phenyl-1,4-azaphosphinane 4-oxide;
1-benzyl-4-(*p*-tolyl)-1,4-azaphosphinane 4-oxide;
1-benzyl-4-(*m*-tolyl)-1,4-azaphosphinane 4-oxide;
1-benzyl-4-(*o*-tolyl)-1,4-azaphosphinane 4-oxide;
1-benzyl-4-(4-(*tert*-butyl)phenyl)-1,4-azaphosphinane 4-oxide;
1-benzyl-4-(naphthalen-1-yl)-1,4-azaphosphinane 4-oxide;
1-benzyl-4-(naphthalen-2-yl)-1,4-azaphosphinane 4-oxide;
1-benzyl-4-(4-methoxyphenyl)-1,4-azaphosphinane 4-oxide;
1-benzyl-4-(3-methoxyphenyl)-1,4-azaphosphinane 4-oxide;
1-benzyl-4-(2-methoxyphenyl)-1,4-azaphosphinane 4-oxide;
1-benzyl-4-(4-(benzyloxy)phenyl)-1,4-azaphosphinane 4-oxide;
1-benzyl-4-(2-(methylthio)phenyl)-1,4-azaphosphinane 4-oxide;
methyl 4-(1-benzyl-4-oxido-1,4-azaphosphinan-4-yl)benzoate;
methyl 3-(1-benzyl-4-oxido-1,4-azaphosphinan-4-yl)benzoate;
4-(1-benzyl-4-oxido-1,4-azaphosphinan-4-yl)benzonitrile;
3-(1-benzyl-4-oxido-1,4-azaphosphinan-4-yl)benzonitrile;
2-(1-benzyl-4-oxido-1,4-azaphosphinan-4-yl)benzonitrile;
1-benzyl-4-(pyridin-2-yl)-1,4-azaphosphinane 4-oxide;
1-benzyl-4-(3-chloro-5-(trifluoromethyl)pyridin-2-yl)-1,4-azaphosphinane 4-oxide;
1-benzyl-4-(pyrimidin-5-yl)-1,4-azaphosphinane 4-oxide;
1-benzyl-4-(isoquinolin-5-yl)-1,4-azaphosphinane 4-oxide;
4-(benzo[d]thiazol-2-yl)-1-benzyl-1,4-azaphosphinane 4-oxide;
1-benzyl-4-(thiophen-3-yl)-1,4-azaphosphinane 4-oxide;
1-(4-methoxybenzyl)-4-(4-(trifluoromethyl)phenyl)-1,4-azaphosphinane 4-oxide;
1-(2-methylbenzyl)-4-(4-(trifluoromethyl)phenyl)-1,4-azaphosphinane 4-oxide;
1-(3-methylbenzyl)-4-(4-(trifluoromethyl)phenyl)-1,4-azaphosphinane 4-oxide;
1-(4-methylbenzyl)-4-(4-(trifluoromethyl)phenyl)-1,4-azaphosphinane 4-oxide;
1-(2-bromobenzyl)-4-(4-(trifluoromethyl)phenyl)-1,4-azaphosphinane 4-oxide;
1-(2-(trifluoromethyl)benzyl)-4-(4-(trifluoromethyl)phenyl)-1,4-azaphosphinane 4-oxide;
1-(2-chloro-6-(trifluoromethyl)benzyl)-4-(4-(trifluoromethyl)phenyl)-1,4-azaphosphinane 4-oxide;
1-(naphthalen-1-ylmethyl)-4-(4-(trifluoromethyl)phenyl)-1,4-azaphosphinane 4-oxide;
1-(naphthalen-2-ylmethyl)-4-(4-(trifluoromethyl)phenyl)-1,4-azaphosphinane 4-oxide;
1-(2-methylbenzyl)-4-(*p*-tolyl)-1,4-azaphosphinane 4-oxide;
1-(3-methylbenzyl)-4-(*p*-tolyl)-1,4-azaphosphinane 4-oxide;
1-(4-methylbenzyl)-4-(*p*-tolyl)-1,4-azaphosphinane 4-oxide;
(4-oxido-4-(*p*-tolyl)-1,4-azaphosphinan-1-yl)(phenyl)methanone;
1-phenethyl-4-(4-(trifluoromethyl)phenyl)-1,4-azaphosphinane 4-oxide;
1-(4-fluorophenyl)-3-(4-oxido-4-(4-(trifluoromethyl)phenyl)-1,4-azaphosphinan-1-yl)propan-1-one;
1-(4-fluorophenyl)-4-(4-oxido-4-(4-(trifluoromethyl)phenyl)-1,4-azaphosphinan-1-yl)butan-1-one;
1-phenethyl-4-(*p*-tolyl)-1,4-azaphosphinane 4-oxide;
1-(4-fluorophenyl)-4-(4-oxido-4-(*p*-tolyl)-1,4-azaphosphinan-1-yl)butan-1-one;
1-(m-tolyl)-4-(*p*-tolyl)-1,4-azaphosphinane 4-oxide;
4-(*p*-tolyl)-1-(4-(trifluoromethyl)phenyl)-1,4-azaphosphinane 4-oxide;
1,4-dibenzyl-1,4-azaphosphinane 4-oxide;
1-(*o*-tolyl)-4-(*p*-tolyl)-1,4-azaphosphinane 4-oxide;
1,4-di-*p*-tolyl-1,4-azaphosphinane 4-oxide;
1-(naphthalen-2-yl)-4-(*p*-tolyl)-1,4-azaphosphinane 4-oxide;
1-(naphthalen-1-yl)-4-(*p*-tolyl)-1,4-azaphosphinane 4-oxide;
1-(4-chlorophenyl)-4-(*p*-tolyl)-1,4-azaphosphinane 4-oxide;
1-(naphthalen-1-yl)-4-(4-(trifluoromethyl)phenyl)-1,4-azaphosphinane 4-oxide;
1-(2-(trifluoromethoxy)phenyl)-4-(4-(trifluoromethyl)phenyl)-1,4-azaphosphinane 4-oxide;
1-(2,5-difluorophenyl)-4-(4-(trifluoromethyl)phenyl)-1,4-azaphosphinane 4-oxide;
1-phenyl-4-(4-(trifluoromethyl)phenyl)-1,4-azaphosphinane 4-oxide;
1-(isoquinolin-5-yl)-4-(4-(trifluoromethyl)phenyl)-1,4-azaphosphinane 4-oxide;
1-(pyridin-4-yl)-4-(4-(trifluoromethyl)phenyl)-1,4-azaphosphinane 4-oxide;
1-(2-methoxyphenyl)-4-(4-(trifluoromethyl)phenyl)-1,4-azaphosphinane 4-oxide;
1-(4-methoxyphenyl)-4-(4-(trifluoromethyl)phenyl)-1,4-azaphosphinane 4-oxide;
1-(thiophen-2-yl)-4-(4-(trifluoromethyl)phenyl)-1,4-azaphosphinane 4-oxide;
1-(4-fluorophenyl)-4-(4-(trifluoromethyl)phenyl)-1,4-azaphosphinane 4-oxide;
4-benzyl-1-cyclopropyl-1,4-azaphosphinane 4-oxide;
4-benzyl-1-(4-(trifluoromethyl)phenyl)-1,4-azaphosphinane 4-oxide;
4-benzyl-1-(*p*-tolyl)-1,4-azaphosphinane 4-oxide;
(E)-1-(4-methylbenzyl)-4-styryl-1,4-azaphosphinane 4-oxide;
(E)-1-(4-methylbenzyl)-4-(2-methylstyryl)-1,4-azaphosphinane 4-oxide;
(E)-1-(4-methylbenzyl)-4-(3-methylstyryl)-1,4-azaphosphinane 4-oxide;
(E)-1-(4-methylbenzyl)-4-(4-methylstyryl)-1,4-azaphosphinane 4-oxide;
(E)-1-(4-methylbenzyl)-4-(4-nitrostyryl)-1,4-azaphosphinane 4-oxide;
(E)-4-(4-methoxystyryl)-1-(4-methylbenzyl)-1,4-azaphosphinane 4-oxide;
(E)-1-(4-methylbenzyl)-4-(2-(pyridin-2-yl)vinyl)-1,4-azaphosphinane 4-oxide;
1-(4-methylbenzyl)-4-phenethyl-1,4-azaphosphinane 4-oxide;
(E)-1-(4-methylbenzyl)-4-(4-(trifluoromethyl)styryl)-1,4-azaphosphinane 4-oxide;
(E)-4-(4-bromostyryl)-1-(4-methylbenzyl)-1,4-azaphosphinane 4-oxide;
(E)-4-(4-chlorostyryl)-1-(4-methylbenzyl)-1,4-azaphosphinane 4-oxide;
1-(4-methylbenzyl)-4-(2-methylphenethyl)-1,4-azaphosphinane 4-oxide;
1-(4-methylbenzyl)-4-(3-methylphenethyl)-1,4-azaphosphinane 4-oxide;
1-(4-methylbenzyl)-4-(4-methylphenethyl)-1,4-azaphosphinane 4-oxide;
4-(4-methoxyphenethyl)-1-(4-methylbenzyl)-1,4-azaphosphinane 4-oxide;
1-(4-methylbenzyl)-4-(4-(trifluoromethyl)phenethyl)-1,4-azaphosphinane 4-oxide;
1,4-bis(4-methylbenzyl)-1,4-azaphosphinane 4-oxide;
1-(4-methylbenzyl)-4-(4-(trifluoromethyl)benzyl)-1,4-azaphosphinane 4-oxide;
(S)-1-(1-phenylethyl)-4-(p-tolyl)-1,4-azaphosphinane 4-oxide;
4-(hydroxy(phenyl)methyl)-1-((S)-1-phenylethyl)-1,4-azaphosphinane 4-oxide;
2-(1-benzyl-4-oxido-1,4-azaphosphinan-4-yl)-1-(o-tolyl)ethan-1-one;
1-benzyl-4-(o-tolylethynyl)-1,4-azaphosphinane 4-oxide;
2-(1-benzyl-4-oxido-1,4-azaphosphinan-4-yl)-1-(m-tolyl)ethan-1-one;
1-benzyl-4-(o-tolylethynyl)-1,4-azaphosphinane 4-oxide;
tert-butyl 1-(4-methylbenzyl)-4-(p-tolyl)-1,4-azaphosphinane-3-carboxylate 4-oxide;
1-benzyl-4-(pyrimidin-2-yl)-1,4-azaphosphinane 4-oxide;
1-benzyl-4-(2-bromophenyl)-1,4-azaphosphinane 4-oxide;
1-benzyl-4-(2-(trifluoromethoxy)phenyl)-1,4-azaphosphinane 4-oxide;
1-benzyl-4-(4-nitrophenyl)-1,4-azaphosphinane 4-oxide;
1-benzyl-4-(hydroxy(phenyl)methyl)-1,4-azaphosphinane 4-oxide;
1-benzyl-4-(hydroxy(m-tolyl)methyl)-1,4-azaphosphinane 4-oxide;
1-benzyl-4-(hydroxy(4-methoxyphenyl)methyl)-1,4-azaphosphinane 4-oxide;
1-benzyl-4-((3,5-dimethylphenyl)(hydroxy)methyl)-1,4-azaphosphinane 4-oxide;
1-benzyl-4-(hydroxy(naphthalen-2-yl)methyl)-1,4-azaphosphinane 4-oxide;
1-benzyl-4-((6-bromopyridin-2-yl)(hydroxy)methyl)-1,4-azaphosphinane 4-oxide;
1-benzyl-4-(hydroxy(perfluorophenyl)methyl)-1,4-azaphosphinane 4-oxide;
1-benzyl-4-((2-bromophenyl)(hydroxy)methyl)-1,4-azaphosphinane 4-oxide;
1-benzyl-4-(hydroxy(2-(trifluoromethyl)phenyl)methyl)-1,4-azaphosphinane 4-oxide;
1-benzyl-4-(hydroxy(naphthalen-1-yl)methyl)-1,4-azaphosphinane 4-oxide;
1-benzyl-4-((4-fluorophenyl)(hydroxy)methyl)-1,4-azaphosphinane 4-oxide;
1-benzyl-4-(hydroxy(p-tolyl)methyl)-1,4-azaphosphinane 4-oxide;
1-benzyl-4-(hydroxy(2-methoxyphenyl)methyl)-1,4-azaphosphinane 4-oxide;
1-benzyl-4-(hydroxy(o-tolyl)methyl)-1,4-azaphosphinane 4-oxide;
1-benzyl-4-(4-(difluoromethyl)phenyl)-1,4-azaphosphinane 4-oxide;
1-benzyl-4-(2-fluorophenyl)-1,4-azaphosphinane 4-oxide;
(R)-4-(p-tolyl)-1-(1-(p-tolyl)ethyl)-1,4-azaphosphinane 4-oxide; and
(S)-4-(p-tolyl)-1-(1-(p-tolyl)ethyl)-1,4-azaphosphinane 4-oxide.
